# EUROPEAN PATENT APPLICATION

(11) **EP 4 483 714 A2**
(43) Date of publication of application: **01.01.2025**
(21) Application number: 24213074.8
(22) Date of filing: 15.12.2022
(51) Int. Cl.: A01N 43/36

(54) **COMPOUNDS USEFUL FOR THE PREPARATION OF VARIOUS AGROCHEMICALS AND MARKERS THEREOF**

(30) Priority: 15.12.2021 US 202163289671 P
(62) Divisional of application: 22851041.8
(71) Applicant: Adama Agan Ltd., 7710001 Ashdod (IL)
(72) Inventor: FOGLER, Eran, 8502500 Meitar (IL); GRABARNICK, Michael, 8502500 Meitar (IL); SILVER, David, 4201672 Netanya (IL)
(74) Representative: Modiano, Gabriella Diana

(57) **Abstract**

The present invention relates to various compounds, which are novel and useful intermediates in novel methods for preparation of certain compounds referred to as Saturated Targets and amorphous and crystal forms thereof, and certain marker compounds including Storage Marker compounds, methods for their preparation, their use, and compositions comprising them including methods to minimize their percentage content of Storage Markers in products and compositions of Saturated Target.

## Description

### TECHNICAL FIELD

The present invention relates to the field of chemistry and pesticide syntheses in particular. This invention specifically relates to synthetic intermediates, polymorphs and marker compounds and their salts, processes for their preparation and their uses.

### BACKGROUND

WO 2015/084796 and WO 2016/196593 reportedly disclose a variety of substituted cyclic amides, a method using them as a herbicide, and methods to prepare them. WO 2016/094117 discloses certain 3-oxo-3-(arylamino)propanoates, their salts and compositions, a process to prepare them and their use in preparing certain pyrrolidinones which are apparently, *inter alia*, useful as agricultural chemicals reportedly particularly as herbicides.

WO 2018/175226 reportedly discloses certain pyrrolidinones, intermediates, and methods to prepare them. We similarly note the publications WO 2020/242946, WO 2020/064260, US10,676,431, WO 2019/025156, WO 2018/222647, WO 2018/222646, WO 2018/184890,

US 2018/099935, EP 3650430, US 2018/077931, WO 2016/164201, US 2017/158638 and

US 2020010415. We further note WO01/76566 which teaches the use of amido-group containing polymeric compounds or amino-group containing polymeric compounds exemplified by polyvinylpyrrolidone, cross-linked polyvinylpyrrolidone, copolymers of vinylpyrrolidone and vinyl acetate, and polynoxylin in stabilizing compounds sensitive to an acidic environment.

The herein disclosed, inventive intermediates; markers; compositions; the processes to prepare them; and their use in processes; mixtures; compositions; crystal forms; and amorphous solid forms of the present invention are not disclosed in these previous publications. The known methods of preparation for the chiral compounds, referred to, are deficient and suffer from disadvantages including at least one of, generally expensive because they employ expensive starting materials; involve multiple synthetic steps; involve the use of, and are based on catalysis employing heavy metals such as, Palladium, Rhodium, Iridium etc.; involve a ring-closing step; and introduce chirality into the system at an early stage of synthesis, making selective preparation of a desired syn or anti isomer of compounds I challenging. There is an on-going need for alternative processes and novel synthetic intermediates that can alleviate at least some of these issues. Several further novel and non-obvious useful aspects and embodiments of the invention are direct results of the herein disclosed inventive processes and novel synthetic intermediates.

### SUMMARY OF THE INVENTION

The present invention relates to various compounds, which are novel and useful intermediates in novel methods for preparation of certain compounds referred to as Saturated Targets and amorphous and crystal forms thereof, and certain marker compounds including Storage Marker compounds, methods for their preparation, their use, and compositions comprising them including methods to minimize their percentage content of Storage Markers in products and compositions of Saturated Target.

### BRIEF DESCRIPTION OF THE FIGURES

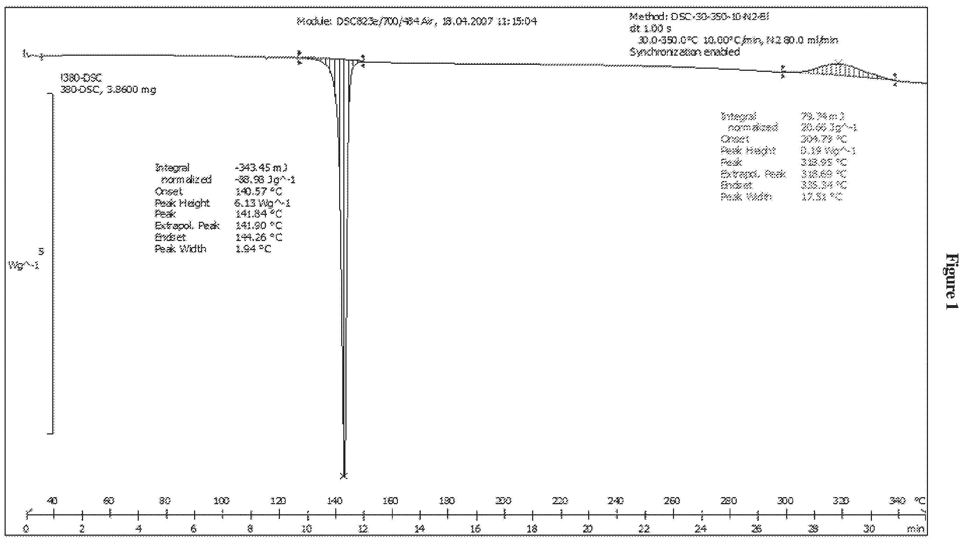

**Figure 1****.** Tetflupyrolimet **FORM I** - DSC

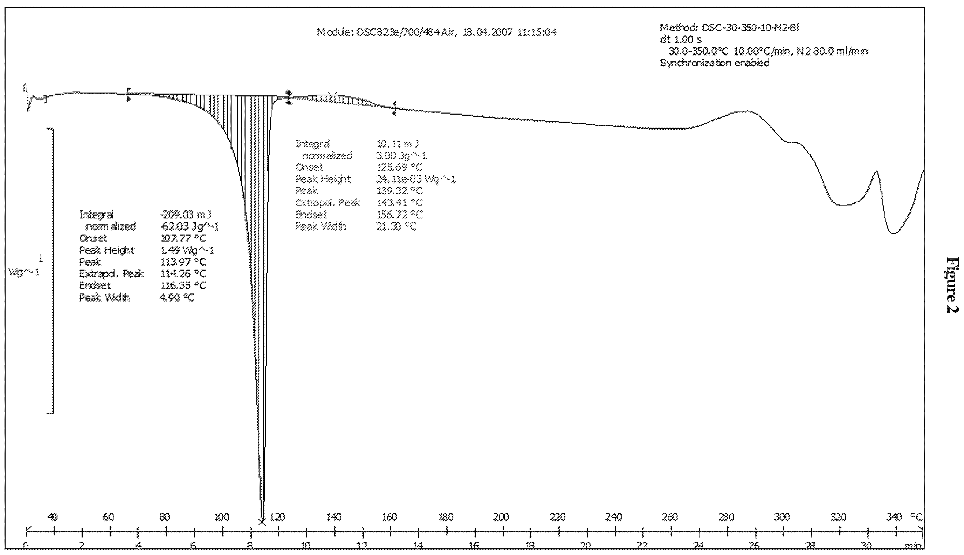

**Figure 2****.** Tetflupyrolimet **FORM II** - DSC

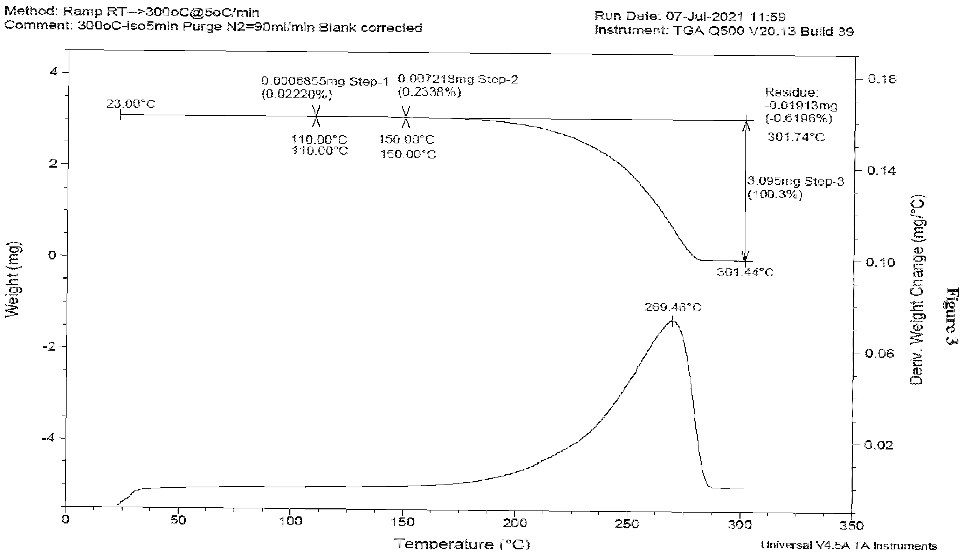

**Figure 3****.** Tetflupyrolimet **FORM I** - TGA

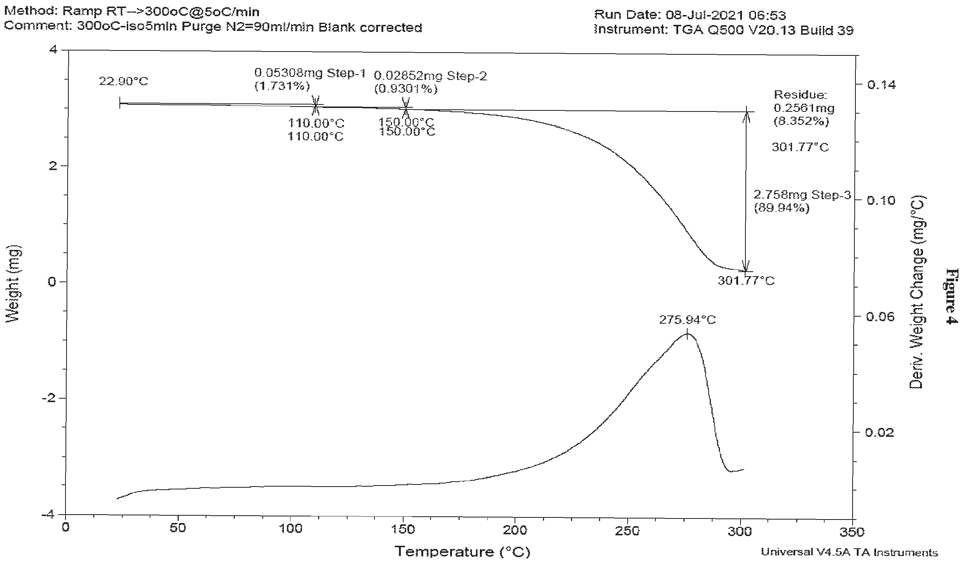

**Figure 4****.** Tetflupyrolimet **FORM II** - TGA
**Figure 5****.** Tetflupyrolimet **FORM** I - XRD
**Figure 6****.** Tetflupyrolimet **FORM II** - XRD

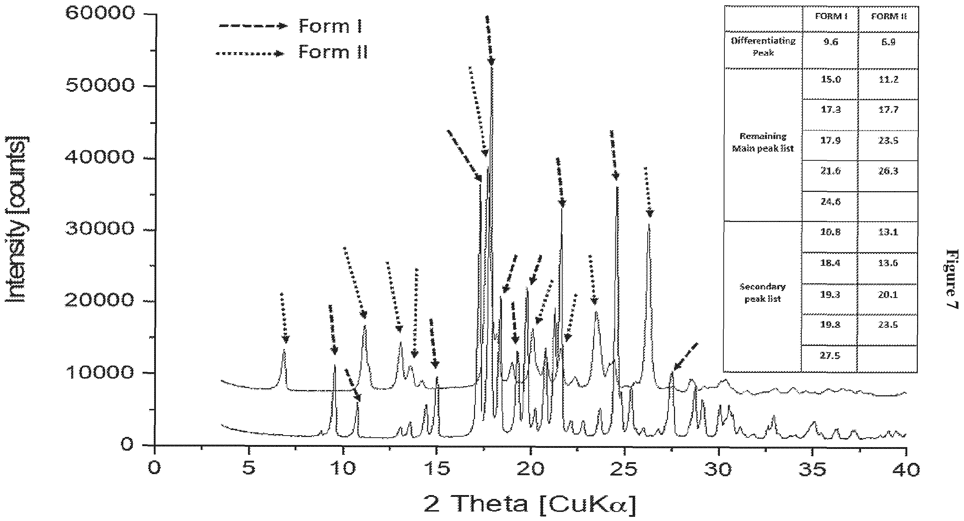

**Figure 7****.** Tetflupyrolimet **FORM I** & **FORM II** - XRD Overlay

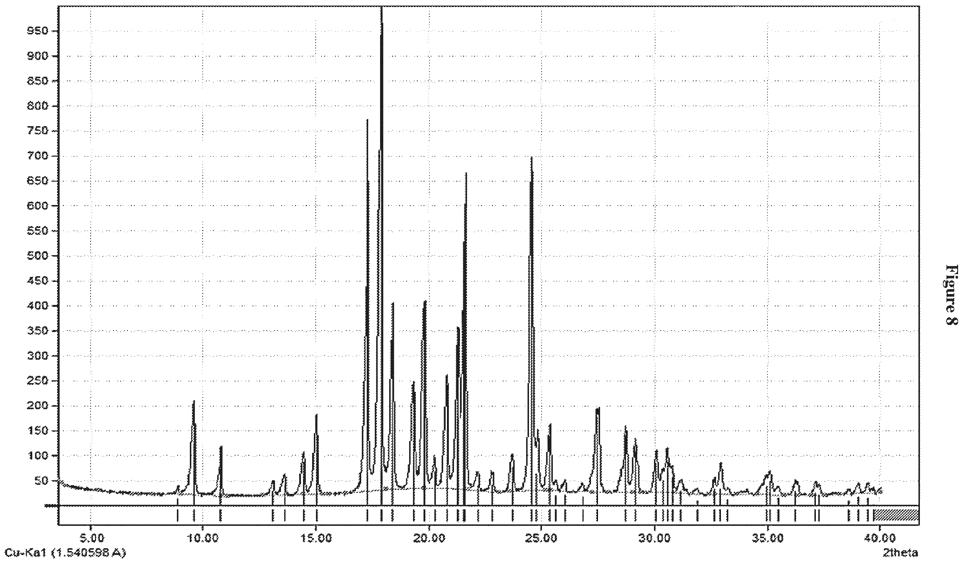

**Figure 8****.** Tetflupyrolimet **9:1 Mixture FORM I** & **FORM II** - XRD
**Figure 9****.** Tetflupyrolimet **FORM I** IR Spectrum
**Figure 10****.** Tetflupyrolimet **FORM II** IR Spectrum

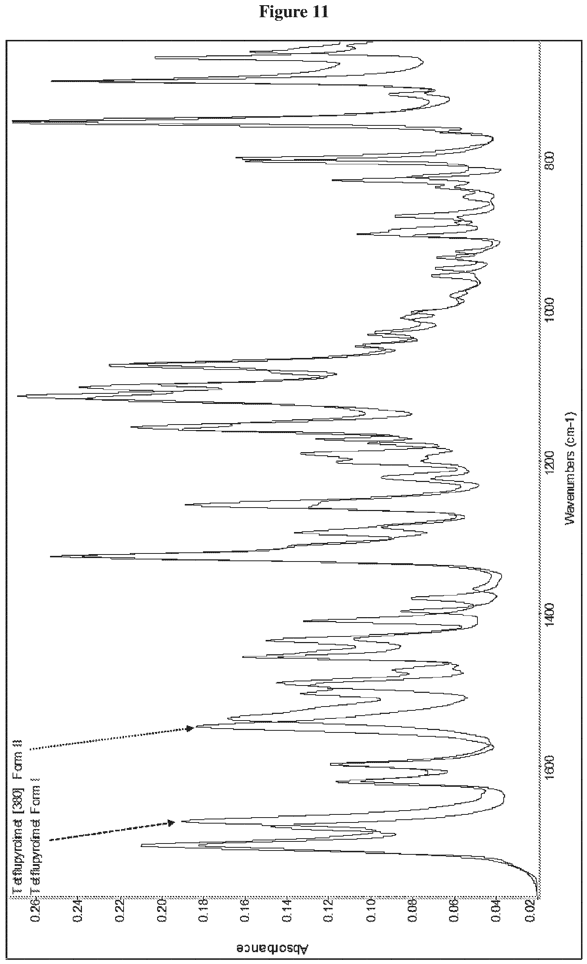

**Figure 11****.** Tetflupyrolimet **FORM I** & **FORM II** - IR Overlay Spectra

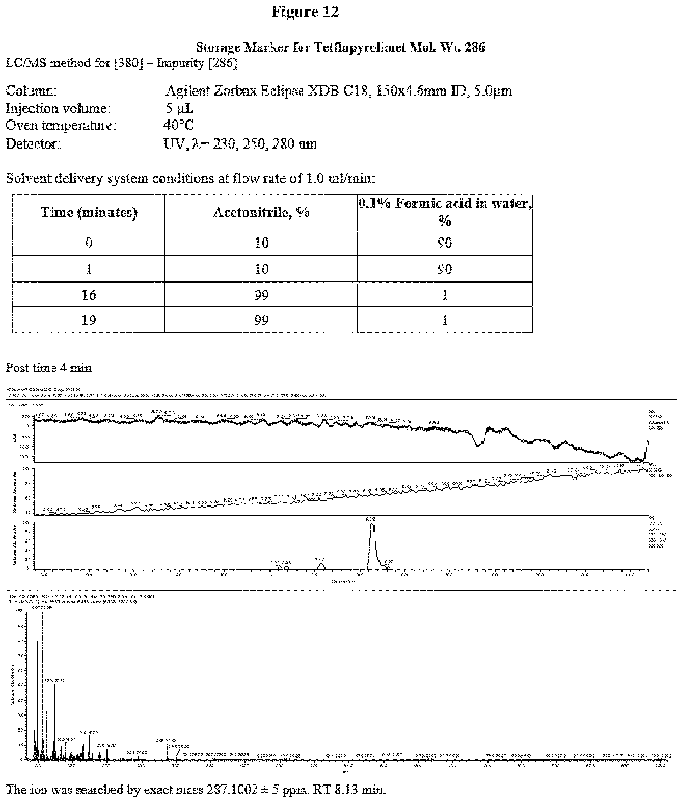

**Figure 12****. Storage Marker** - LC/MS - Storage Marker for Tetflupyrolimet, Mol. Wt. 286

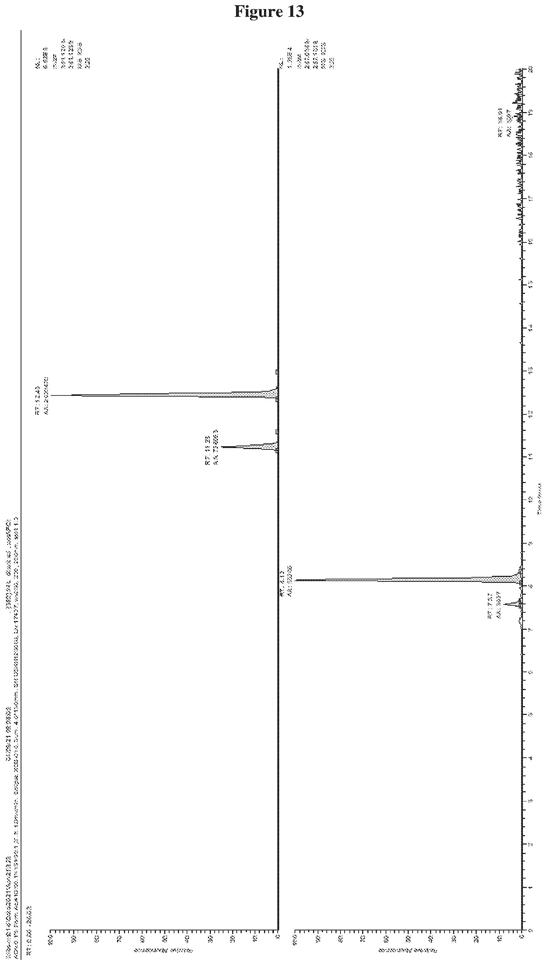

**Figure 13****. Tetflupyrolimet** + **Storage Marker** - LC/MS

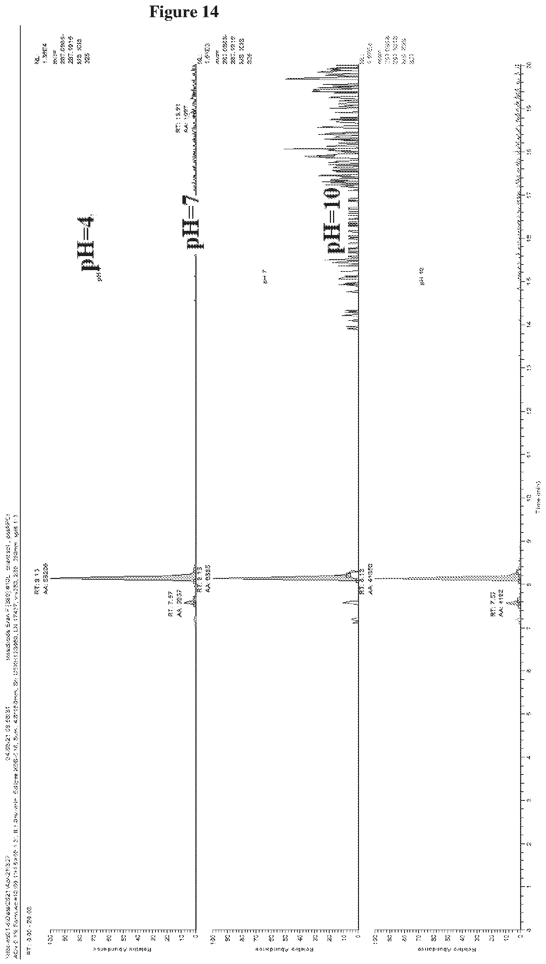

**Figure 14****. Storage Marker Mol. Wt. 286** - LC/MS

### DETAILED DESCRIPTION OF THE INVENTION

### DEFINITIONS

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The term "a" or "an" as used herein includes the singular and the plural, unless specifically stated otherwise. Therefore, the terms "a," "an," or "at least one" can be used interchangeably in this application.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Throughout the application, descriptions of various embodiments use the term "comprising": however, it will be understood by one skilled in the art, that in some specific instances, an embodiment can alternatively be described using the language "containing", "consisting essentially of" or "consisting of".

For purposes of better understanding the present teachings and in no way limiting the scope of the teachings, unless otherwise indicated, all numbers expressing quantities, percentages, or proportions, and other numerical values used in the specification and claims, are to be understood as being modified in all instances by the term "about".

Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, use of the term "about" herein specifically includes +/-10% from the indicated values in the range, unless clearly specified otherwise. In addition, the endpoints of all ranges directed to the same component or property herein are inclusive of the endpoints, are independently combinable, and include all intermediate points and ranges.

In the present invention, the following terms etc. have the following meanings.

All diagrams and references herein, to Saturated Target compounds, Unsaturated Precursor compounds, compounds III, compounds IV or Storage Marker compounds, are intended to include those analogues, isomers, salts, N-oxides, esters etc. thereof as would be understood by one ordinarily skilled in these arts to plausibly, have the function and utility intended or attributed to that referenced compound, unless specifically defined otherwise or unless the context in which they are referred to are clearly limited to the specific structures, or when their inclusion would introduce unpatentable matter or scope.

Saturated Target, Unsaturated Precursor, compound III, compound IV and Storage Marker are sometimes referred to in the plural, e.g. Saturated Targets, Unsaturated Precursors, compounds III, compounds IV and Storage Markers , to reflect the plurality of compounds encompassed by the general structures describing them, no significance should be attributed to this and no inference should be made if the singular term is used for textural convenience.

Where appropriate, disclosed structures of specific compounds are labelled with numbers to better facilitate clearer identification of compounds.

Often where appropriate, disclosed specific structures are further labelled with letters and numbers for easier comparison with general synthetic schemes disclosed herein.

Saturated Target compounds and some of the inventive intermediates from which they can be produced, Unsaturated Precursor, compounds III and compounds IV, using the inventive methods of preparation herein described, as well as the Storage Marker compounds can exist as isomers and particularly one or more stereoisomers. The various stereoisomers include enantiomers, diastereomers, atropisomers and geometric isomers. For example, Saturated Target is understood to be chiral. In cases where only one structure is drawn or labelled "1" or described as Saturated Target, the companion enantiomers, diastereomers or optical isomers etc. of 1 or Saturated Target including both optically active and racemic mixtures thereof, are explicitly intended and no inference should be taken from omission of the corresponding structures of partner compounds, unless specifically stated, or it is clear from the context in which the structure appears. Likewise, examples of intermediate and marker compounds of the invention, Unsaturated Precursor, compounds III and compounds IV and Storage Marker and corresponding Saturated Target, are isomeric and include e.g. cis-trans and or E-Z isomers etc. In cases where only one isomeric structure is drawn or labelled, all the companion isomers are explicitly intended when it would be clear to a person ordinarily skilled in these arts that they would be have similar function/characteristic and no inference should be taken from omission of the corresponding structures for textural convenience, unless specifically stated, or it is clear from the context in which the structure appears.

While it is generally understood that a crystal form of a compound cannot be fully characterized by a single peak in an X-ray powder diffraction pattern, as regards differentiating between the only two known crystalline forms of Tetflupyrolimet described herein, it is noted that each respective X-ray powder diffraction pattern displays at least one peak which is absent or at an intensity below the limit of detection, in the X-ray powder diffraction pattern for the other polymorphic form. Thus when a crystalline form is already chemically identified as Tetflupyrolimet, the two novel polymorphic forms herein disclosed can be differentiated by this single characteristic peak. Thus inventors refer to this type of peak as a differentiating peak. Clearly the differentiating peaks are each also part of each of the list of characteristic peaks.

In the definitions of the disclosed structures, the term "carbonyl" is intended to include the corresponding "carbonyl" protected as acetal of C₁-C₈ alcohol, e.g. Ethanol or C₁-C₈ diol such as ethylene glycol, 1,3-Propanediol, Propylene glycol. As an example, the term "dimethyl carbonyl" will include a corresponding acetal exemplified by MeC(OCH₂CH₂O)Me.

In this document, the term "Metal" is intended to include all forms of the metal including, though not limited to, metal salts, metal complexes etc.

In the synthetic process embodiments of the invention disclosed herein, where use of a Grignard reagent is indicated, there is a particular preference for those Grignard reagents that are not aliphatic e.g. they are not alkyl-type Grignard reagents.

Further, in the synthetic process embodiments of the invention disclosed herein, where use of a Grignard reagent is indicated, it will be clear to those skilled in these preparative arts that analogous organometallic compounds or alternative nucleophiles, can replace the Grignard reagent. Preferred examples of these alternative nucleophiles are organo-lithium; organo-copper and organo-zinc compounds for example, phenyl(3-(trifluoromethyl)phenyl)zinc, 3-(trifluoromethyl)phenyl)lithium, bis(3-(trifluoromethyl)phenyl)zinc, (3-(trifluoromethyl)phenyl)zinc(II) bromide, bis(3-(trifluoromethyl)phenyl)cuprate(I), bis(3-(trifluoromethyl)phenyl)copper etc., and clearly include combinations of these types of reagent.

In the synthetic process embodiments of the invention disclosed herein, where use of a Nucleophile e.g. Grignard reagent, is indicated, the use of a hydrogen/hydride source or hydrogenation is also intended as an option to be included within the concept of using a nucleophile, exemplified by the use of H₂/Pd, NaBH₄, CuH, or H₂/Raney Ni etc. as well as electron sources exemplified by the use of Zn/AcOH, Na/NH₃ etc.

The term Grignard reagent, includes organo-magnesium compounds in which Mg is connected to carbon including Ate complexes, Turbo Grignard etc., exemplified by iPrMgCl, MeMgCl₂Li etc. In general structures representing a group of compounds, unless stated otherwise, each instance of a substituent may independently be selected from any of the definitions of that substituent. For example, a compound within a group represented by CH(R')₃ where R' was defined as F, Cl or I could include the compound CH(F)(Cl)(I).

The present invention relates *inter alia*, to each of the compounds of formulae 2, 3 and 4 as defined below, herein referred to as Unsaturated Precursor, and intermediates III and IV, 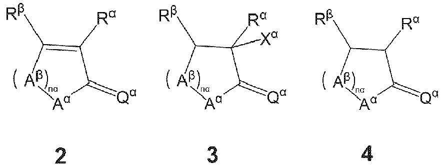 as further defined below, which are novel, useful intermediates in the herein disclosed novel methods of preparation of certain agrochemical compounds, in particular, Pyrrolidinones identified herein as Compounds of formulae **1** and generally referred to as Saturated Targets, defined below. One example of these methods is represented in the scheme below.

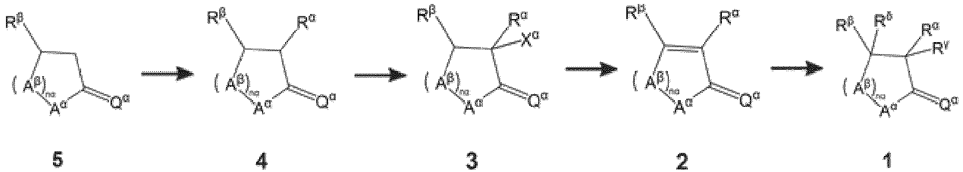

However, each of Unsaturated Precursor; Compounds **III**; and **IV**, individually is useful as an intermediate for preparation of Saturated Targets without being dependent on the others in a chain of reactions as exemplified. Advantageously Saturated Targets include those Pyrrolidinones which are Anilide Pesticides most exemplified by, 2'-fluoro-1-methyl-2-oxo-4-[3-(trifluoromethyl)phenyl]pyrrolidine-3-carboxanilide, 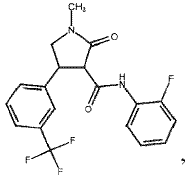 and most especially, optical isomers and/or diastereomers thereof, useful as herbicides, including the following structures,

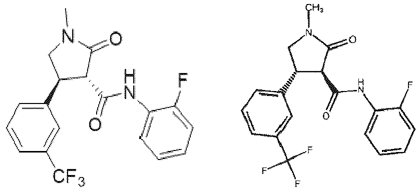

The present invention also relates to certain marker compounds including those identified herein as Storage Markers, exemplified by the compound of formula **D** below, 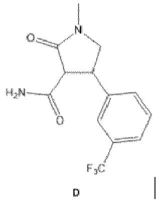 which are further produced from an exemplified Saturated Target compound having the structure below, 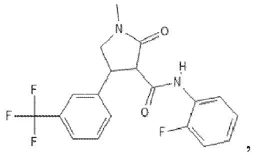 which is most especially exemplified by:
(3S,4S)-2'-fluoro-1-methyl-2-oxo-4-[3-(trifluoromethyl)phenyl]pyrrolidine-3-carboxanilide, in the following scheme, 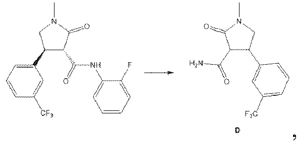 wherein the rate and degree of conversion and amount of Storage Marker of formula D produced, and its percentage content in mixtures with compositions of the Saturated Target compound from which it was produced, is mediated by the characteristics of said composition and by the history of its storage at various defined environmental conditions, particularly exposure to sunlight, and thus Storage Markers of formula **D**, are novelly, and inventively utilized as indicative markers of said composition characteristics and evidence of historical storage conditions.

The present invention thus, also relates to methods of preparation of Saturated Target compounds, and methods of preparing each of various individual intermediates herein referred to as Unsaturated Precursor and compounds III, and IV and Storage Marker compounds of formula D which are embodiment subjects of the invention as well as industrially useful methods for the efficient preparation of certain Pyrrolidinones which are Anilide Pesticides, particularly exemplified by optically active compounds of 2'-fluoro-1-methyl-2-oxo-4-[3-(trifluoromethyl)phenyl]pyrrolidine-3-carboxanilides, from the aforesaid intermediate compounds.

The invention similarly relates to novel mixtures and compositions comprising Saturated Target and Storage Marker compounds which are exemplified by mixtures of Anilide Pesticide together with above mentioned respective markers which are indicative or evidence of, for example, sub-optimal storage conditions and/or disadvantageous composition characteristics or ingredients of said mixtures.

The invention further relates to methods, ingredients and packaging useful in minimizing the percentage content of said Storage Marker compounds in compositions of Saturated Target by *inter alia*, minimizing, avoiding or mitigating the effects of, exposure of said compositions to, for example, sunlight.

The invention is clearly, inextricably linked to those novel physical/chemical characteristics and/or attributes of Saturated Target compounds which result from having been produced by a process utilizing the Unsaturated Precursors and compounds **III** and **IV** of the invention as intermediates in their preparation.

Thus chemically, a Saturated Target compound prepared utilizing any of Unsaturated Precursor; compounds, **III**; or **IV** as intermediates can have, a novel impurity profile that can include at least trace amounts of any of Unsaturated Intermediates;, **III**; or **IV** as impurities carried through from the process, and thus will novelly provide evidence of that Saturated Target compound having been prepared using the inventive compounds, Unsaturated Intermediate, **III** or **IV** as intermediates.

### Combinations of Saturated Target with any of

Unsaturated Precursor, **III** or **IV** are in themselves novel and have utility in *inter alia*, analysis of a test Saturated Target to determine its route of preparation and other uses independent of being preparative intermediates.

As mentioned above, the Saturated Target compounds having been produced by a process utilizing the compounds referred to as Unsaturated Precursor, **III** and **IV** of the invention as intermediates in their preparation, will also have novel physical characteristics and/or attributes as a result. In one non-limiting example, these characteristics include, though are not limited to, novel and useful polymorphic forms of
2'-fluoro-1-methyl-2-oxo-4-[3-(trifluoromethyl)phenyl]pyrrolidine-3-carboxanifide and its individual enantiomers/diastereomers and mixtures thereof when produced by the inventive methods and intermediates.

Thus, the invention also relates to both amorphous or non-crystalline solid forms and to each of crystalline polymorphs, Form **I** and Form **II** of (3S,4S)-N-(2-fluorophenyl)-1-methyl-2-oxo-4-[3-(trifluoromethyl)phenyl]-3-pyrrolidinecarboxamide (Tetflupyrolimet), and mixtures of both Form **I** and Form **II**, either as pure active ingredients or in combination with non-crystalline forms, carriers or additional ingredients etc. All of these mixtures are included herein within the term composition. Thus, generally the term composition in this description should be understood to include all mixtures of more than one material, including mixtures of active ingredients intermediates and markers even when there are no inactive or inert ingredients or carrier materials included.

It is well understood that often compounds having different crystalline polymorphic forms can typically display different physical characteristics in a wide range of parameters depending on the polymorph, whether as regards rates of solubilization, flowability, stability, compressibility or even brightness of reflection and many others. Given the large array of pesticidal, particularly herbicidal compositions each with its own requirements. Having alternative new and useful polymorphs of an agricultural active ingredient that allow for formulating in a wider spectrum of delivery forms is advantageous per se. and inter alia useful in allowing for a larger scope of formulating techniques. Similar advantages can often be obtained by producing non-crystalline or amorphous forms for example because of their generally faster rates of dissolution. Spray drying, freeze drying, solid solutions and adsorption on to carrier substrates are some typical methods for obtaining such solid forms although stability of sensitive compounds must be considered.

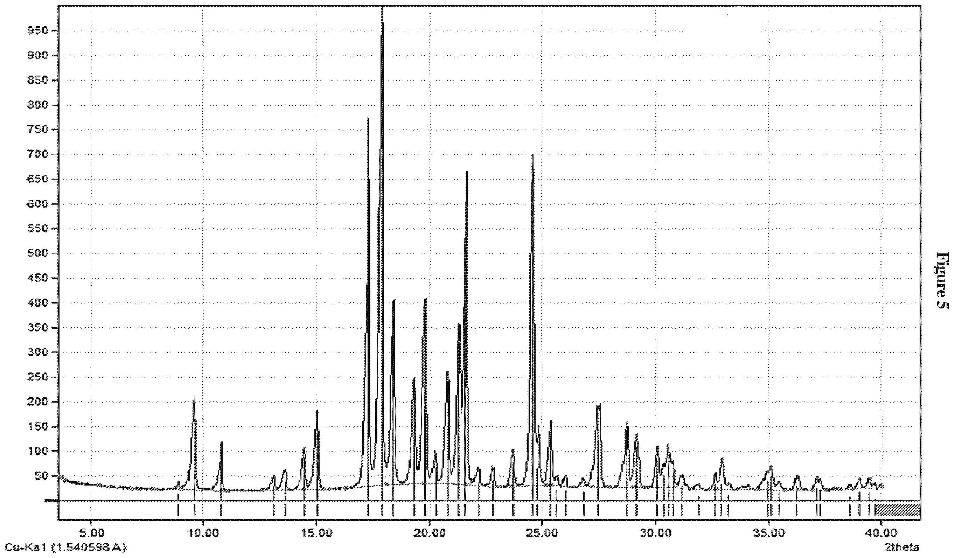

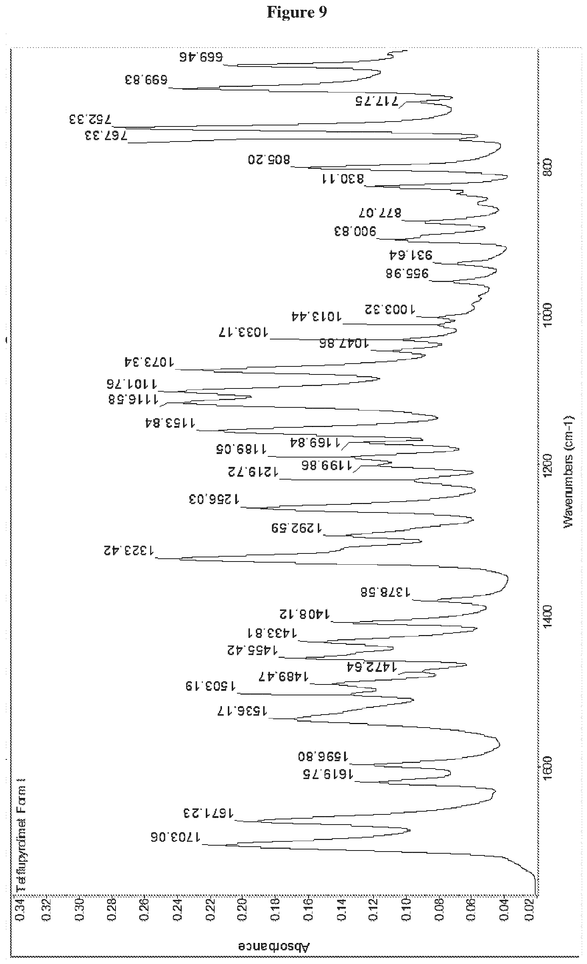

This invention specifically includes novel Tetflupyrolimet Polymorph **Form I** which exhibits at least one of the following properties:
an infrared spectrum substantially as shown in Figure 9.
an X-ray powder diffraction pattern substantially as shown in Figure 5.
an X-ray powder diffraction pattern having a differentiating peak expressed in 2θ (± 0.20) at 9.6,
an X-ray powder diffraction pattern having at least 4 of the characteristic peaks expressed in 2θ (± 0.20) at 9.6, 15.0, 17.3, 17.9, 21.6 and 24.6.

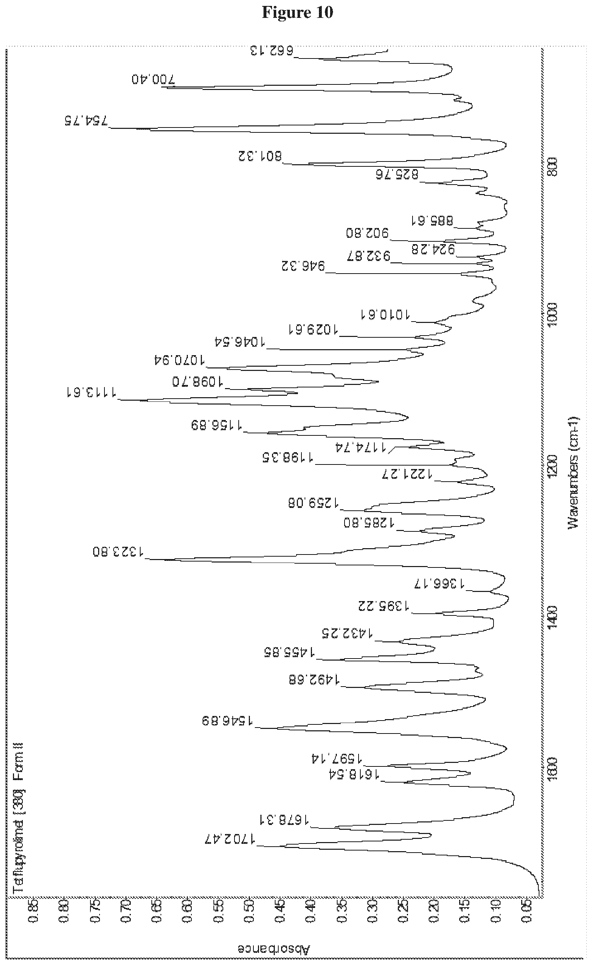

This invention specifically includes novel Tetflupyrolimet Polymorph **Form II** which exhibits at least one of the following properties:
An infrared (IR) absorption spectrum substantially as shown in Figure 10.

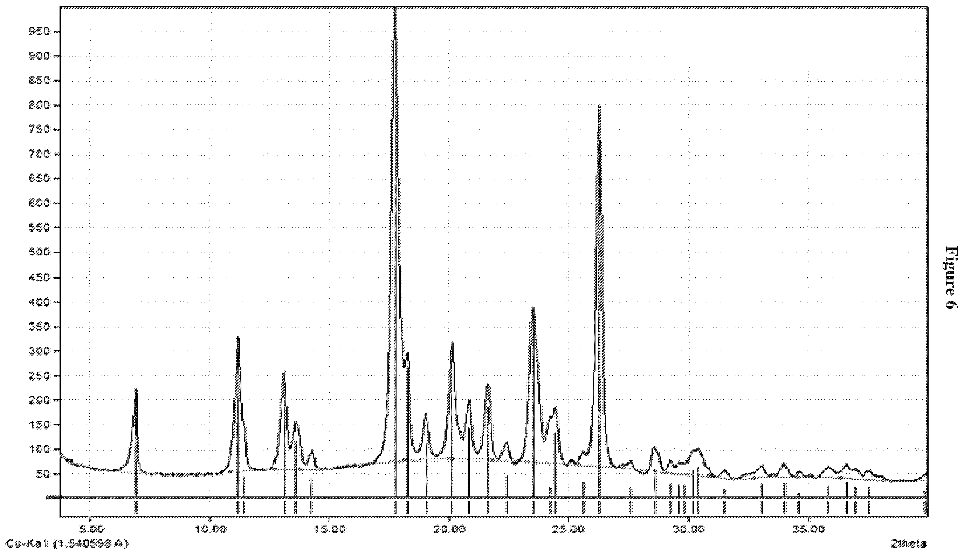

An infrared (IR) absorption spectrum having at least one characteristic peak selected from the following values expressed as cm-1 (± 1 cm-1) at 1678, 1546, 1395, 946, 924, 885, 825 and 662. An X-ray powder diffraction pattern substantially as shown in Figure 6.

An X-ray powder diffraction pattern having a differentiating peak expressed in 2θ (± 0.20) at 6.9, an X-ray powder diffraction pattern having at least 4 of the characteristic peaks expressed in 2θ (± 0.20) selected from the following values at 6.9, 11.2, 17.7, 23.5 and 26.3.

The invention additionally relates to processes for the preparation of crystalline polymorphs Form I and Form **II** of (3S,4S)-N-(2-fluorophenyl)-1-methyl-2-oxo-4-[3-(trifluoromethyl)phenyl]-3-pyrrolidinecarboxamide (Tetflupyrolimet) as described in the invention.

### EMBODIMENTS AND DESCRIPTION

The compounds of formulae **2, 3** and **4** also referred to as Unsaturated Precursor, **III** and **IV** respectively as defined infra and in the embodiments below, can each individually be prepared by general methods known in the art of synthetic organic chemistry by those skilled in these arts

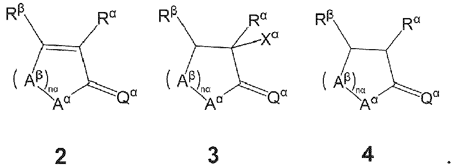

Each of the compounds, Unsaturated Precursor, **III,** and **IV** is at least one, preferably all of:
a. a novel compound heretofore undisclosed for any chemical, biological or agricultural purpose;
b. a novel synthetic intermediate heretofore undisclosed as having utility in methods of preparation of corresponding Saturated Target compounds ;
c. a novel combination partner with Saturated Target compounds remaining as a synthetic impurity after its use as a synthetic intermediate in the preparation of said Saturated Target; and
d. a marker compound probative or evidence of, the use of the novel methods of preparation of a tested sample of a Saturated Target material.

In one aspect the inventive methods for preparing Saturated Target compounds can involve a scheme which utilizes all of compounds, Unsaturated Precursor, **III** and **IV,** and clearly also includes the individual aspects of methods of preparation of each of the intermediate compounds without reference to their subsequent use as intermediates in a multistep preparation.

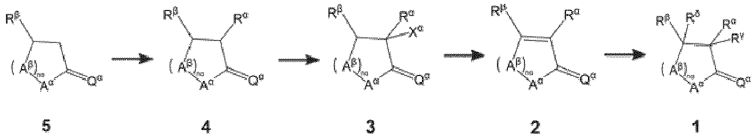

However, it is entirely within the scope of the invention and specifically contemplated by inventors that, the inventive process for preparing a Saturated Target compound **Saturated Target** will only include the use of one and/or two of the three corresponding novel intermediate compounds, without the occurrence of the other(s). Thus, it is useful to consider each of the claimed compounds as being inventively independent of each other.

Processes for preparing a Saturated Target **Saturated Target** of formula **1**, directly from a corresponding intermediate compound of formula **3** or Saturated Target formula **4**, without involvement of any of the other disclosed intermediate compounds and/or via other unclaimed intermediates is specifically contemplated by inventors. Examples will include processes of the following general schemes below, the details of which will become immediately apparent to a person skilled in these synthetic chemical arts after, and as a result of, the material having been disclosed herein. 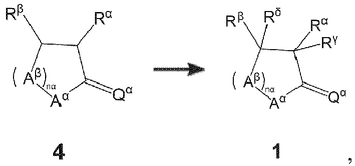 and

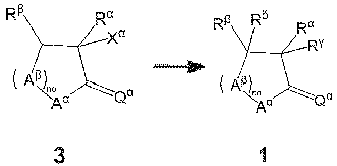

Similarly in some cases Unsaturated Precursors of formula **2**, may be prepared directly from some compounds **IV**, of formula **4**,

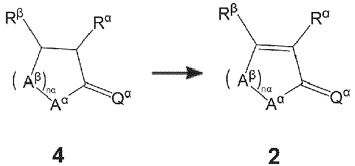

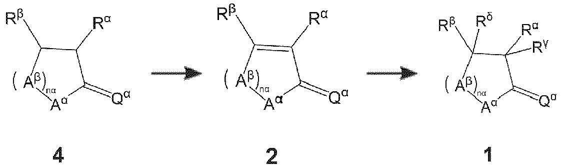

Thus, each of the Compounds, Unsaturated Precursor, **III** and **IV** is individually useful as an intermediate for preparation of Saturated Target without being dependent on the others in the chain of reactions as exemplified above.

The known methods of preparation for Saturated Targets of formula **1** are generally expensive because, they use expensive starting materials, involve multiple synthetic steps, involve the use of and/or are based on catalysis with heavy metals like Palladium, Rhodium, Iridium etc., involve closing the ring labelled **P** in the structure below, and/or introduce chirality into the system at an early stage of synthesis making selective preparation of a desired syn or anti isomer of compounds of formula **1** challenging.

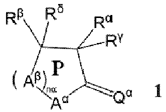

Thus in one aspect the invention provides novel intermediate compounds that may be used for a novel method of synthesis of Saturated Targets of formula **1** via reaction at the cyclic double bond in the ring system of an Unsaturated Precursor of formula **2**, this step does not require the same expensive catalysts such as Pd, Rh or Ir as were required in previous processes.

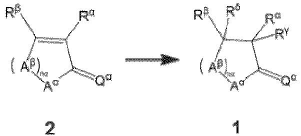

The invention also teaches and provides cheap closed-ring starting materials and intermediates that may be used for the synthesis of Unsaturated Precursor, avoiding the need for any ring closure synthetic step exemplified in the following scheme.

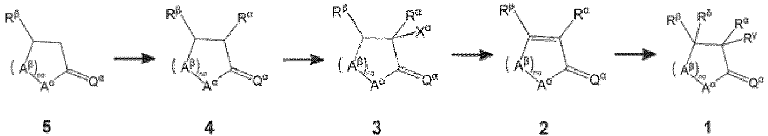

This aspect can be exemplified by the following scheme that employs the cheap and available solvent, N-Methyl pyrrolidone (NMP) as a starting material.

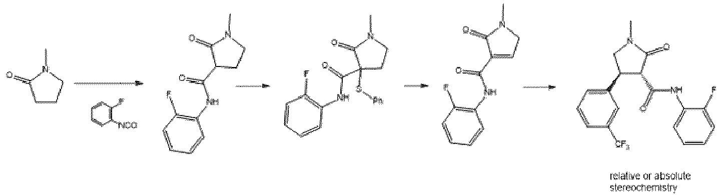

Remarkably, reaction of the lactam/ secondary amide of the structure below, 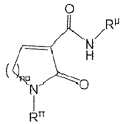 with Grignard reagent or organolithium reagent, results in a 1,4 addition, instead of simple deprotonation and de-activation of the lactam / secondary amide, or other undesired reaction, for example attack on amide.

Implementation of the inventive synthetic preparative methods enables processes without the need for expensive metals such as Pd, Rh, Pt, Ir etc., and the use of relatively cheap and readily available Grignard reagent or Organolithium reagents can also advantageously often produce some marker compounds which are useful for evidencing the use of the inventive method,. , although not all or any of the markers are always produced.

For example, in the following scheme,

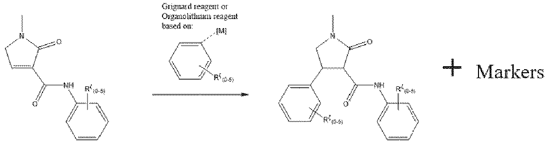

The markers that can be formed in the above scheme can include some the following structures,

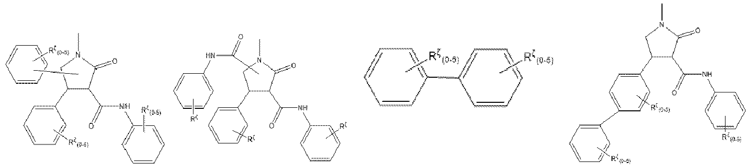

Another example can be seen in the following scheme,

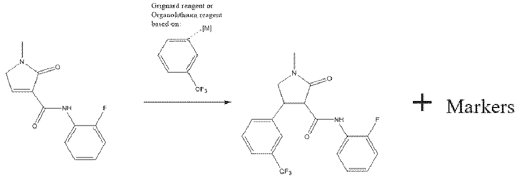

The markers that may be formed in this scheme can include some the following structures,

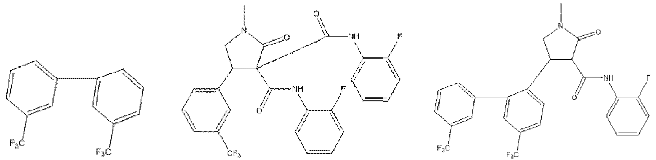

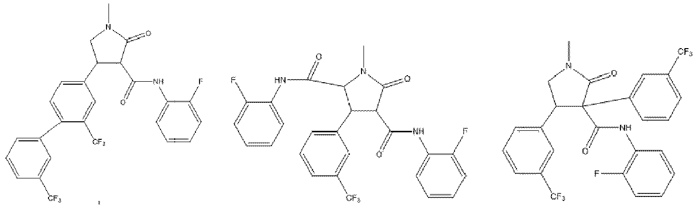

As mentioned, the use of this type of Grignard reagent and/or organolithium regent may be very desirable for production on industrial scale, reducing and/or eliminating problems including, waste issues e.g. heavy metals, B etc. use of expensive metal(s) e.g. Rh, Pd, Ir, Pt etc., reducing the number of synthetic steps, eliminating the need to purify the desired Saturated Target product from residual problematic impurities.

The use of the inventive intermediates and processes herein disclosed can also be accompanied by some or all of the following potential advantages. The improvement of Syn-anti selectivity and introduction of chirality at the late stages of synthesis reduces the risk of partial or even full racemization in the final Saturated Target product, thus improving the yield of the desired chiral compound and, accordingly, reducing the production cost.

The disclosed process also reduces the number of synthetic steps required for preparation of Saturated Target product, and allows for synthesis starting from a cyclic compound and thus avoids the need for cyclization as part of the synthesis.

The starting materials can also be significantly cheaper than previously disclosed starting materials and the process requires cheaper reagents and catalysts and obviates the need for expensive heavy metals such as Palladium and Ruthenium etc.

Some more detailed illustrative process methods disclosed below may be useful in understanding some of the aspects and embodiments of the invention. None of the process details herein below described should be considered in any way or fashion, to limit the scope of the invention as conceived by inventors. Conversely, the individual steps and process details in the following illustrations should not be considered solely as part of a specific complete synthesis scheme, but rather can be seen as individual steps that each have value without the other steps being necessarily those taught here.

As will be immediately apparent to those skilled in these arts, the disclosed methods below are not necessarily preferred methods but, are intended to provide some color, and scope, depth and breadth to more general descriptions.

Embodiments of the present invention include:

### Embodiment A1

An Unsaturated Precursor of Formula **2** and e.g. salts etc. thereof 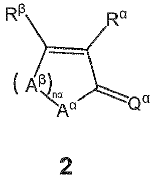 wherein,
**R^{α}** is halogen; carbon-containing group exemplified by, substituted or unsubstituted, alkyl, aryl, heterocyclic, naphthyl; ester, including aliphatic and aromatic esters exemplified by C(O)OC₆H₅, - C(O)OC₆H₄F; amide, including aliphatic and aromatic amides exemplified by -C(O)NHC₆H₅; hydrazinamide, including aliphatic and aromatic hydrazinamide exemplified by, -C(O)NHNHC₆H₅, thioesters, thionoesters and similar compounds such as -C(O)NHOC₆F₅; -C(S)SC₆H₅ etc. with the proviso that **R^{α}** is not connected to the ring via O or N, thus for example, **R^{α}** cannot be -OMe.
**R^{β}** is hydrogen or carbon-containing group exemplified by, substituted or unsubstituted, alkyl, aryl, naphthyl, heterocycles etc. connected to the ring via a C atom, or Si atom if relevant, with the proviso that
**R^{β}** is notcannot be an amine connected via N to the ring
**R^{β}** cannot be anis not ether connected via O to the ring
**R^{β}** cannot be ais not thioether connected via S to the ring
**A^{α}** is a C, N, O or S atom with or without substitution, exemplified by -N(Me)-; -O-.

Where present, a substituent of **A^{α}** may also be connected to another part of the molecule such as Q^{α}, A^{β} etc. for example in the following type of structure .

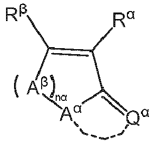

Each **A^{β}** is individually and independently a C, N, O or S atom, with or without substitution exemplified by -CH₂- or -O-.

Where present, the a substituent of each A^{β} may be connected to another part of the molecule such as **A^{α}**, **A^{β}**, **R^{β}** etc., with the proviso, that at least one of **A^{β}** is a saturated atom exemplified by CH₂. **nα** is 1, 2, or 3

Each **Q^{a}** is individually and independently O, S, or N**R^{ε}**

**R^{ε}** is a C, N, O or S atom with or without substitution exemplified by -N(Me)₂.

The substituent on **R^{ε}** may be hydrogen or a carbon- and/or nitrogen-containing group, exemplified by, substituted or unsubstituted, alkyl, aryl, naphthyl, heterocycles etc. connected to the ring via a C atom, or a Si atom if relevant.

Where present, the substituent of **R^{ε}** may also be connected to another part of the molecule such as **Q^{α}**, **A^{β}** etc in a ring via a C atom, or Si atom if relevant, for example in the structure below,

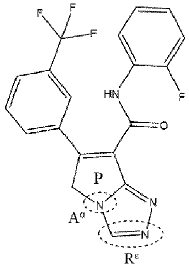

Generally, the ring moiety of the Unsaturated Precursor, labelled **P** below, is not aromatic i.e. not all atoms are capable of conjugation. Additionally, the double bond in that ring moiety indicated below, is also not part of an aromatic additional ring external to it.

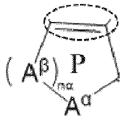

### Embodiment A2

An Unsaturated Precursor of Formula **2** and e.g. salts thereof 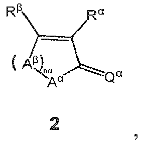 wherein, the Unsaturated Precursor does not include any covalent Nitrogen-Halogen bond, and wherein,
**R^{α}** is halogen (Hal.), C₁-C₁₀ alkyl, C₁-C₁₀ haloalkyl, **R^{µ}**, or -C(**Q^{α}**)**Q^{β}R^{µ}**. In some circumstances, **R^{α}** may also be connected to another part of the molecule such as **Q^{α}**, **A^{β}** etc. in a ring.
**Q^{β}** is O, S, N**R^{ε}**, N**R^{π}**-N**R^{π}**, N**R^{π}**-O, N**R^{π}**-S, S-N**R^{π}**, or O-N**R^{π}**
**R^{β}** is H, **R^{ε}**, or Si(**R^{ε}**)₃
**A^{α}** is a C, N, O or S atom with 0-2 substituents. The substituents, when present, may be selected from H and/or **R^{θ}** defined infra, Examples of **A^{α}** include -N(Me)-, -C(Et)H-, -O-.

In some circumstances, substituents of **A^{α}** may also be connected to another part of the molecule such as, **Q^{α}**, **A^{β}**, etc. as a ring, for example: 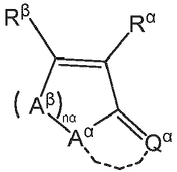
Each **A^{β}** is individually and independently a C, N, O or S atom with 0-2 substituents, for example, **A^{β}** may be, -N(Me)-, -C(Et)H-, -O- etc. Substituents on each **A^{β}** are selected from H and/or **R^{θ}** defined infra, with the proviso that at least one of the **A^{β}** atoms on the ring is a saturated atom exemplified by CH₂.
   Additionally, each **A^{β}** in some circumstances may be connected to another part of the molecule such as, **Q^{α}**, **A^{α}** or to another **A^{β}**, etc to form a ring.
**nα** is 1-3
**Q^{α}** is O, S, N**R^{ε}**, and in certain circumstances may be connected to another part of the molecule such as an **A^{β}** etc. in a ring.
Each **R^{θ}** is individually and independentlyis halogen (Hal.), hydroxy (OH), cyano (CN), nitro, amino, C₁-C₈ alkyl, C₁-C₈ cyanoalkyl, C₁-C₈ cyanoalkoxy, C₁-C₈ haloalkyl, C₁-C₈ hydroxyalkyl, C₁-C₈ nitroalkyl, C₂-C₈ alkenyl, C₂-C₈ haloalkenyl, C₂-C₈ nitroalkenyl, C₂-C₈ alkynyl, C₂-C₈ haloalkynyl, C₂-C₈ alkoxyalkyl, C₃-C₈ alkoxyalkoxyalkyl, C₂-C₈ haloalkoxyalkyl, C₂-C₈ haloalkoxyhaloalkoxy, C₃-C₆ cycloalkyl, cyclopropylmethyl, 1-methylcyclopropyl, 2-methylcyclopropyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂cycloalkylalkynyl, C₃-C₈ cycloalkyl, C₃-C₈ halocycloalkyl, C₄-C₁₀ alkylcycloalkyl, C₆-C₁₂ cycloalkylcycloalkyl, C₃-C₈ cycloalkenyl, C₃-C₈ halocycloalkenyl, C₂-C₈ haloalkoxyalkoxy, C₂-C₈ alkoxyalkoxy, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylamino, C₂-C₈ dialkylamino, C₂-C₈ halodialkylamino, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl, C₂-C₈ haloalkylcarbonyl, C₄-C₁₀ cycloalkylcarbonyl, -C(=O)OH, C₂-C₈ alkoxycarbonyl, C₂-C₈ haloalkoxycarbonyl, C₄-C₁₀ cycloalkoxycarbonyl, C₅-C₁₂ cycloalkylalkoxycarbonyl, -C(=O)NH₂, C₂-C₈ alkylaminocarbonyl, C₄-C₁₀ cycloalkylaminocarbonyl, C₃-C₁₀ dialkylaminocarbonyl, C₁-C₈ alkoxy, C₁-C₈ haloalkoxy, C₂-C₈ alkoxyalkoxy, C₂-C₈ alkenyloxy, C₂-C₈ haloalkenyloxy, C₃-C₈ alkynyloxy, C₃-C₈ haloalkynyloxy, C₃-C₈ cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈ haloalkylsulfonyloxy, C₁-C₈ alkylthio, C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₃-C₈ cycloalkylsulfonyl, formylamino, C₂-C₈ alkylcarbonylamino, C₂-C₈ haloalkylcarbonylamino, C₃-C₈ cycloalkylamino, C₂-C₈ alkoxycarbonylamino, C₁-C₆ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl, C₄-C₁₂ trialkylsilylalkoxy, **R^{σ}**, **R^{η}**S(=O)=N-, **R^{η}**S(=O)₂N, **R^{η}**-C(=O)-, **R^{η}**(**R^{η}**N=)**_{q}**S(=O)**ₚ**-cyano, formyl, C₃-C₈ alkylcarbonylalkyl, -C(C₁-C₄ alkyl)isN-O(C₁-C₄ alkyl), -C(O)NH₂, C₂-C₆ cyanoalkyl, C₃-C₆ cycloalkyl, C₄-C₈ cycloalkenyl, arylcarbonyl, arylalkenylalkyl, arylcarbonylalkyl or -CPhisN-O(C₁-C₄ alkyl), each of which are optionally substituted on ring members with up to 5 substituents independently selected from **R^{η}**.

In circumstances where **R^{θ}** is connected to another part of the molecule, the connection is through a saturated, partially unsaturated or fully unsaturated, chain containing 2 to 4 atoms selected from, up to four C atoms, up to one O atom, up to one S atom and up to two N atoms, wherein up to 2 carbon members of the chain are independently selected from C(=O) and C(=S) and a sulfur atom member of the chain is selected from, S(=O)**ᵤ**(=N**R^{ζ}**)**ᵥ**; said chain being optionally substituted with up to 5 substituents independently selected from **R^{η}** substituted on carbon and/or nitrogen atoms;
**R^{λ}** is H or **R^{µ}**,
   wherein, **R^{λ}** may be connected to another part of the molecule such as **Q^{α}**, **A^{β}** etc.
**R^{µ}** is phenyl ring or a naphthalenyl ring system, each ring or ring system being substituted or unsubstituted with up to 5 substituents independently selected from **R^{ζ};** or a 4-7 membered heterocyclic ring; or an 8-10 membered bicyclic ring system, each ring or ring system containing ring members selected from carbon atoms and 1 to 4 heteroatoms independently selected from up to two O, up to two S, and up to five N atoms, wherein up to three C ring members are independently selected from C(=O) and C(=S), and the sulfur atom ring members are independently selected from S(=O)ᵤ(=N**R^{ζ}**)**ᵥ**, each ring or ring system substituted or unsubstituted with up to 5 substituents independently selected from **R^{ζ}** on carbon atom ring members and selected from **R^{η}** on nitrogen atom ring members
**R^{π}** is H or **R^{θ}**
**R^{ε}** is C, N, O or S atom with 0-2 substituents, each selected from **R^{π}**,
   exemplified by -N(Me)₂. Substituents may be additionally connected to another part of the molecule such as **Q^{α}**, **A^{β}** etc.in a ring.
Each **R^{ζ}** is individually and independently, Hal, OH, CN, nitro, amino, C₁-C₈ alkyl, C₁-C₈ cyanoalkyl, C₁-C₈ cyanoalkoxy, C₁-C₈ haloalkyl, C₁-C₈ hydroxyalkyl, C₁-C₈ nitroalkyl, C₂-C₈ alkenyl, C₂-C₈ haloalkenyl, C₂-C₈ nitroalkenyl, C₂-C₈ alkynyl, C₂-C₈ haloalkynyl, C₂-C₈ alkoxyalkyl, C₃-C₈ alkoxyalkoxyalkyl, C₂-C₈ haloalkoxyalkyl, C₂-C₈ haloalkoxyhaloalkoxy, C₃-C₆ cycloalkyl, cyclopropylmethyl, 1-methylcyclopropyl, 2-methylcyclopropyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂ cycloalkylalkynyl, C₃-C₈ cycloalkyl, C₃-C₈ halocycloalkyl, C₄-C₁₀ alkylcycloalkyl, C₆-C₁₂ cycloalkylcycloalkyl, C₃-C₈ cycloalkenyl, C₃-C₈ halocycloalkenyl, C₂-C₈ haloalkoxyalkoxy, C₂-C₈ alkoxyalkoxy, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylamino, C₂-C₈ dialkylamino, C₂-C₈ halodialkylamino, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl, C₂-C₈ haloalkylcarbonyl, C₄-C₁₀ cycloalkylcarbonyl, -C(=O)OH, C₂-C₈ alkoxycarbonyl, C₂-C₈ haloalkoxycarbonyl, C₄-C₁₀ cycloalkoxycarbonyl, C₅-C₁₂ cycloalkylalkoxycarbonyl, -C(=O)NH₂, C₂-C₈ alkylaminocarbonyl, C₄-C₁₀ cycloalkylaminocarbonyl, C₃-C₁₀ dialkylaminocarbonyl, C₁-C₈ alkoxy, C₁-C₈ haloalkoxy, C₂-C₈ alkoxyalkoxy, C₂-C₈ alkenyloxy, C₂-C₈ haloalkenyloxy, C₃-C₈ alkynyloxy, C₃-C₈ haloalkynyloxy, C₃-C₈ cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈ haloalkylsulfonyloxy, C₁-C₈ alkylthio, C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₃-C₈ cycloalkylsulfonyl, formylamino, C₂-C₈ alkylcarbonylamino, C₂-C₈ haloalkylcarbonylamino, C₃-C₈ cycloalkylamino, C₂-C₈ alkoxycarbonylamino, C₁-C₆ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl, C₄-C₁₂ trialkylsilylalkoxy, **R^{η}**, **R^{η}**S(=O)=N-, **R^{η}**S(=O)₂N**R^{η}**-C(=O)- or **R^{η}**(**R^{η}**N=)**_{q}**S(=O)**ₚ**-, wherein the free bond projecting to the right indicates the connecting point for example to **R^{µ}**;

In some circumstances **R^{ζ}** may be connected to another part of the molecule such as **Q^{α}**, **A^{β}** etc. to form a ring.
Each **R^{η}** is individually and independently, CN, C₁-C₃ alkyl, C₁-C₈ hydroxyalkyl, C₂-C₃ alkenyl, C₂-C₃ alkynyl, C₃-C₆ cycloalkyl, C₂-C₃ alkoxyalkyl, C₁-C₃ alkoxy, C₂-C₃ alkylcarbonyl, C₂-C₃ alkoxycarbonyl, C₂-C₃ alkylaminoalkyl or C₃-C₄ dialkylaminoalkyl.
**R^{η}** may be connected to another part of the molecule such as **Q^{α}**, **A^{β}** etc. forming a ring.
Each **R^{σ}** is individually and independently, phenyl, phenylmethyl (benzyl), pyridinylmethyl, phenylcarbonyl (i.e. benzoyl), phenoxy, phenylethynyl, phenylsulfonyl or a 5- or 6-membered heterocyclic ring, each substituted or unsubstituted on ring members with up to 5 substituents independently selected from **R^{λ}**,
**q** is 0-4,
**p** is 0-4,
**u** is 0-4, and
**v** is 0-4

### Embodiment A2a

The Unsaturated Precursor of Embodiment **A2** and e.g. salts thereof wherein,
**nα** is 1, and wherein,
**R^{α}**, **R^{β}**, **Q^{α}**, **Q^{β}**, **A^{β}**, **A^{α}**, and **R^{µ}**, have the same meanings as defined in Embodiment **A2**.

### Embodiment A2b

The Unsaturated Precursor of Embodiment **A2** and e.g. salts thereof wherein,
**nα** is 2, and wherein,
**R^{α}**, **R^{β}**, **Q^{α}**, **Q^{β}**, **A^{β}**, **A^{α}**, and **R^{µ}**, have the same meanings as defined in Embodiment **A2**.

### Embodiment A2c

The Unsaturated Precursor of Embodiment **A2** and e.g. salts thereof wherein,
**R^{β}** is H and
**R^{α}**, **Q^{α}**, **Q^{β}**, **A^{β}**, **A^{α}**, **R^{µ}** and **nα**, have the same meanings as defined in Embodiment **A2**.

### Embodiment A2d

The Unsaturated Precursor of Embodiment **A2** and e.g. salts thereof wherein,
**R^{α}**, **R^{β}**, **Q^{α}**, **Q^{β}**, **A^{β}**, **A^{α}**, **R^{µ}** and **nα**, have the same meanings as defined in Embodiment **A2**, with the proviso that **R^{β}** is not H.

### Embodiment A3

The Unsaturated Precursor of any one of Embodiments **A2; A2a; A2b; A2c;** and **A2d** and e.g., salts thereof wherein,
**R^{α}** is -C(**Q^{α}**)**Q^{β}R^{µ}**, 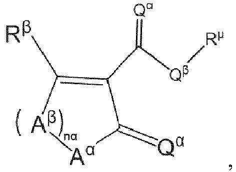
and wherein,
**R^{β}**, **Q^{α}**, **Q^{β}**, **A^{β}**, **A^{α}**, **R^{µ}** and **nα**, have the same meanings as defined in Embodiment **A2.**

### Embodiment A4

The Unsaturated Precursor of any one of Embodiments **A2; A2a; A2b; A2c; and A2d** and e.g. salts thereof, wherein,
**R^{α}** is -C(**Q^{α}**)**Q^{β}R^{µ}**, and
**A^{α}** is -N(**R^{π}**)- 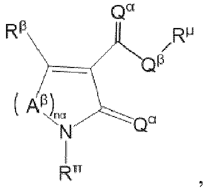
and wherein,
**R^{β}**, **Q^{α}**, **Q^{β}**, **A^{β}**, **R^{π}**, **R^{µ}** and **nα**, have the same meanings as defined in Embodiment **A2.**

### Embodiment A5

The Unsaturated Precursor of any one of Embodiments **A2; A2c;** and **A2d** and e.g. salts thereof wherein,
**R^{α}** is -C(**Q^{α}**)**Q^{β}R^{µ}**,
**A^{α}** is -N(**R^{π}**)-, and
**nα** is 1 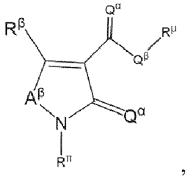
and wherein,
**R^{β}**, **Q^{α}**, **Q^{β}**, **A^{β}**, **R^{π}** and **R^{µ}**, have the same meanings as defined in Embodiment **A2**.

### Embodiment A6

The Unsaturated Precursor of any one of Embodiments **A2; A2c;** and **A2d** and e.g. salts thereof wherein,
**R^{α}** is -C(**Q^{α}**)**Q^{β}R^{µ}**
**A^{α}** is -N(**R^{π}**)-
**nα** is 1
**A^{β}** is CH₂ 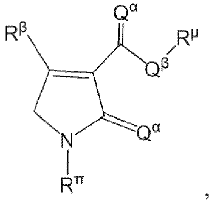
and wherein,
**R^{β}**, **Q^{α}**, **Q^{β}**, **R^{π}** and **R^{µ}**, have the same meanings as defined in Embodiment **A2**.

### Embodiment A7

The Unsaturated Precursor of any one of Embodiments **A2; A2c;** and **A2d** and e.g. salts thereof wherein,
**R^{α}** is -C(**Q^{α}**)**Q^{β}R^{µ}**,
**A^{α}** is -N(**R^{π}**)-,
**nα** is 2,
**A^{β}** is CH₂ 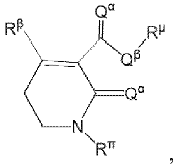
and wherein,
**R^{β}**, **Q^{α}**, **Q^{β}**, **R^{π}** and **R^{µ}**, have the same meanings as defined in Embodiment **A2**.

### Embodiment A8

The Unsaturated Precursor of any one of Embodiments **A2; A2c**; and **A2d** and e.g. salts thereof wherein,
**R^{α}** is -C(O)**Q^{β}R^{µ}**
**A^{α}** is -N(**R^{π}**)-
**nα** is 1
**A^{β}** is CH₂
**Q^{α}** is O 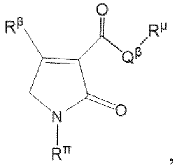
and wherein,
**R^{β}**, **Q^{β}**, **R^{π}** and **R^{µ}**, have the same meanings as defined in Embodiment **A2**.

### Embodiment A9

The Unsaturated Precursor of any one of Embodiments **A2; A2c;** and **A2d** and e.g. salts thereof wherein,
**R^{α}** is -C(O)NH**R^{µ}**
**A^{α}** is -N(**R^{π}**)-
**nα** is 1
**A^{β}** is CH₂
**Q^{α}** is 0
**Q^{β}** is NH 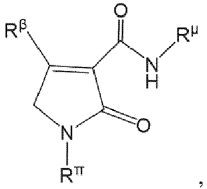
and wherein,
**R^{β}**, **R^{π}** and **R^{µ}**, have the same meanings as defined in Embodiment **A2.**

### Embodiment A10

The Unsaturated Precursor of any one of Embodiments **A2; A2c;** and **A2d** and e.g. salts thereof wherein,
**R^{α}** is -C(O)NH**R^{µ}**
**A^{α}** is -N(**R^{π}**)-
**nα** is 1
**A^{β}** is CH₂
**Q^{α}** is O,
**Q^{β}** is NH and
**R^{µ}** is phenyl ring, unsubstituted or substituted with up to 5 substituents independently selected from **R^{ζ}** 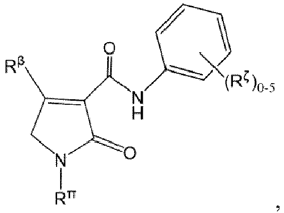
and wherein,
**R^{β}**, **R^{π}** and **R^{ζ}**, have the same meanings as defined in Embodiment **A2**.

### Embodiment A11

The Unsaturated Precursor of Embodiment **A2**, and e.g. salts thereof wherein,
**R^{α}** is -C(O)NH**R^{µ}**
**A^{α}** is -N(**R^{π}**)-
**nα** is 1
**A^{β}** is CH₂
**Q^{α}** is O
**Q^{β}** is NH
**R^{µ}** is phenyl ring, unsubstituted or substituted with up to 5 substituents independently selected from **R^{ζ}**
**R^{β}** is **R^{λ}** is phenyl ring, unsubstituted or substituted with up to 5 substituents independently selected from **R^{ζ}**

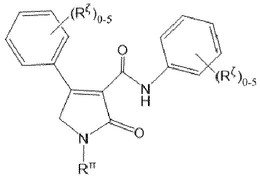

Wherein, **R^{π}** and **R^{ζ}**, have the same meanings as defined in Embodiment **A2.**

### Embodiment A12

The Unsaturated Precursor of Embodiment **A2**, and e.g. salts thereof wherein,
**R^{α}** is -C(O)NH**R^{µ}**
**A^{α}** is -N(Me)-
**nα** is 1
**A^{β}** is CH₂
**Q^{α}** is O
**Q^{β}** is NH
**R^{µ}** is phenyl ring, unsubstituted or substituted with up to 5 substituents independently selected from R^{ζ}
**R^{β}** is **R^{λ}** is phenyl ring, unsubstituted or substituted with up to 5 substituents independently selected from **R^{ζ}**, 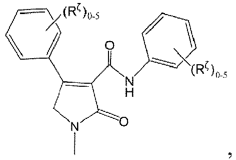
and wherein, **R^{ζ}**, has the same meaning as defined in Embodiment **A2**.

### Embodiment A13

The Unsaturated Precursor of Embodiment **A2**, and e.g. salts thereof wherein,
**Unsaturated Precursor**
**R^{α}** is -C(O)NH**R^{µ}**
**A^{α}** is -NH-
**nα** is **1**
**A^{β}** is CH₂
**Q^{α}** is O
**Q^{β}** is NH
**R^{µ}** is phenyl ring, unsubstituted or substituted with up to 5 substituents independently selected from **R^{ζ}**
**R^{β}** is **R^{λ}** is phenyl ring, unsubstituted or substituted with up to 5 substituents independently selected from **R^{ζ}** 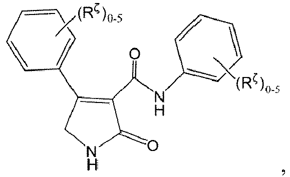
and wherein, **R^{ζ}**, has the same meaning as defined in Embodiment **A2.**

### Embodiment A14

The Unsaturated Precursor of Embodiment **A2**, and e.g. salts thereof wherein,
**R^{α}** is -C(O)NH**R^{µ}**
**A^{α}** is -NH-
**nα** is 2
**A^{β}** is CH₂
**Q^{α}** is O
**Q^{β}** is NH
**R^{µ}** is phenyl ring, unsubstituted or substituted with up to 5 substituents independently selected from **R^{ζ}**
**R^{β}** is **R^{λ}** which is a phenyl ring, unsubstituted or substituted with up to 5 substituents independently selected from **R^{ζ}** 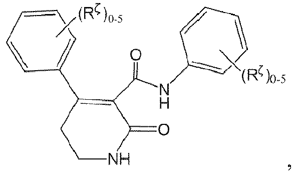
and wherein, **R^{ζ}**, has the same meaning as defined in Embodiment **A2.**

### Embodiment A15

The Unsaturated Precursor of Embodiment **A2**, and e.g. salts thereof wherein,
**R^{α}** is -C(O)NH**R^{µ}**
**A^{α}** is -N(Me)-
**nα** is 2
**A^{β}** is CH₂
**Q^{α}** is O
**Q^{β}** is NH
**R^{µ}** is phenyl ring, unsubstituted or substituted with up to 5 substituents independently selected from **R^{ζ}**
**R^{β}** is **R^{λ}** is phenyl ring, unsubstituted or substituted with up to 5 substituents independently selected from R^{ζ} 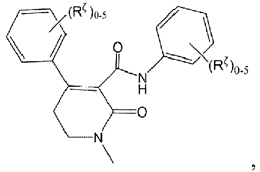
and wherein, **R^{ζ}**, has the same meaning as defined in Embodiment **A2**.

### Embodiment A16

An Unsaturated Precursor which has the following structure,

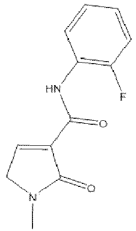

### Embodiment A16a

A pesticide synthesis intermediate compound having the following structure,

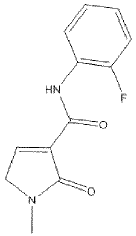

### Embodiment A17

An Unsaturated Precursor which has the following structure,

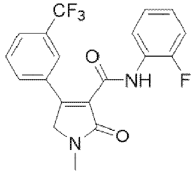

### Embodiment A17a

A pesticide synthesis intermediate compound having the following structure,

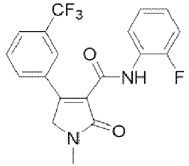

### Embodiment A18

An Unsaturated Precursor which has the following structure,

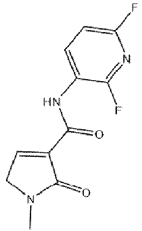

### Embodiment A19

An Unsaturated Precursor which has the following structure,

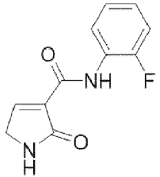

### Embodiment A20

An Unsaturated Precursor which has the following structure,

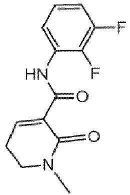

### Embodiment A21

An Unsaturated Precursor which has the following structure,

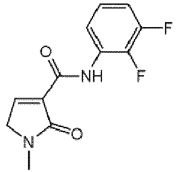

### Embodiment A22

An Unsaturated Precursor which has the following structure,

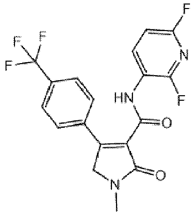

### Embodiment A23

An Unsaturated Precursor which has the following structure,

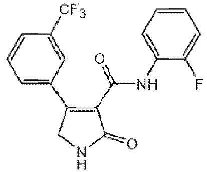

### Embodiment A24

An Unsaturated Precursor which has the following structure,

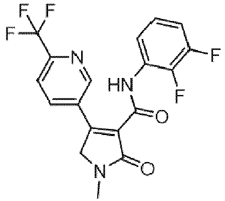

### Embodiment A25

An Unsaturated Precursor which has the following structure,

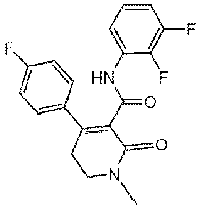

### Embodiment A26

An Unsaturated Precursor which has the following structure,

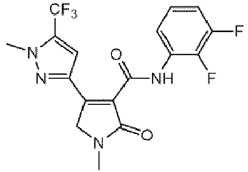

### Embodiment A27

An Unsaturated Precursor which has the following structure,

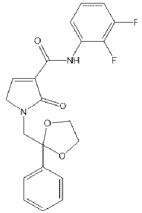

### Embodiment A28

An Unsaturated Precursor which has the following structure,

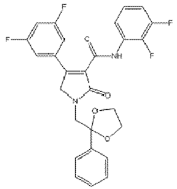

### Embodiment A29

An Unsaturated Precursor which has the following structure,

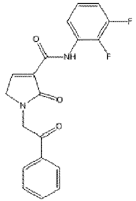

### Embodiment A30

An Unsaturated Precursor which has the following structure,

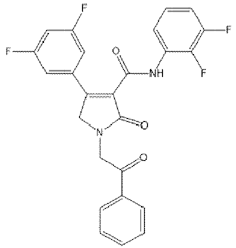

### Embodiment A31

The Unsaturated Precursor of Embodiment and e.g. salts thereof wherein,
**R^{α}** is -C(O)NH**R^{µ}**,
**A^{α}** is -N(**R^{π}**)-,
**nα** is 1,
**A^{β}** is CH₂ ,
**Q^{α}** is O,
**Q^{β}** is NH,
**R^{µ}** is a phenyl ring, unsubstituted or substituted with up to 5 substituents independently selected from **R^{ζ}** , and
**R^{β}** is H 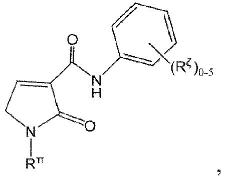
and wherein, **R^{π}** and **R^{ζ}**, have the same meanings as defined in Embodiment **A2**.

### Embodiment A32

The Unsaturated Precursor of Embodiment **A2** and e.g. salts thereof wherein,
**R^{α}** is -C(O)NH**R^{µ}** ,
**A^{α}** is -NH-,
**nα** is 1,
**A^{β}** is CH₂ ,
**Q^{α}** is O,
**Q^{β}** is NH,
**R^{µ}** is phenyl ring, unsubstituted or substituted with up to 5 substituents independently selected from **R^{ζ}**, and
**R^{β}** is H 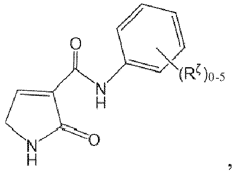
and wherein, **R^{ζ}**, has the same meaning as defined in Embodiment **A2.**

### Embodiment A33

The Unsaturated Precursor of Embodiment **A2** and e.g. salts thereof wherein,
**R^{α}** is -C(O)NH**R^{µ}**,
**A^{α}** is -NH-,
**nα** is 2,
**A^{β}** is CH₂ ,
**Q^{α}** is O,
**Q^{β}** is NH,
**R^{µ}** is a phenyl ring, unsubstituted or substituted with up to 5 substituents independently selected from **R^{ζ}** and
**R^{β}** is H 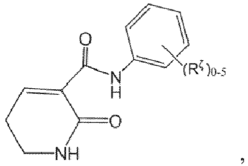
and wherein, **R^{ζ}**, has the same meaning as defined in Embodiment **A2**.

### Embodiment A34

The Unsaturated Precursor of Embodiment **A2** and e.g. salts thereof wherein,
**R^{α}** is -C(O)NH**R^{µ}**,
**A^{α}** is -N(Me)-,
**nα** is 2,
**A^{β}** is CH₂,
**Q^{α}** is O,
**Q^{β}** is NH,
**R^{µ}** is phenyl ring, unsubstituted or substituted with up to 5 substituents independently selected from **R^{ζ}**,
**R^{β}** is H, and 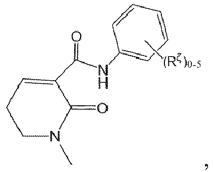
and wherein, **R^{ζ}**, has the same meaning as defined in Embodiment **A2.**

### Embodiment A35a

An Unsaturated Precursor which is defined by any of the following structures,

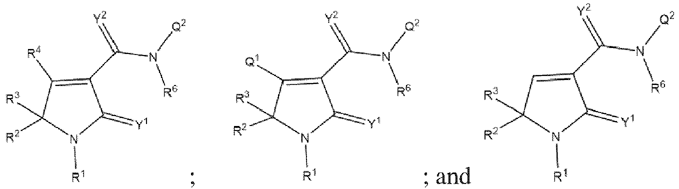

Wherein Q¹, Q², R¹, R², R³, R⁴, R⁶, Y1 and Y2 etc. have the meanings as defined in Claim 1 of PCT/US2014/068073 published as WO 2015/084796. The text extracted from item 1 on page 286, line 6 until page 289 line 23 of WO 2015/084796 being specifically incorporated by reference solely for the purpose of defining the structures of this embodiment.
Q¹ is a phenyl ring or a naphthalenyl ring system, each ring or ring system optionally substituted with up to 5 substituents independently selected from R⁷; or a 5- to 6-memberedheterocyclic ring or an 8- to 10-membered heteroaromatic bicyclic ring system, each ring or ring system containing ring members selected from carbon atoms and 1 to 4 heteroatoms independently selected from up to 2 O, up to 2 S and up to 4 N atoms, wherein up to 3 carbon ring members are independently selected from C(=O) and C(=S), and the sulfur atom ring members are independently selected from S(=O)ᵤ(=NR⁸)ᵥ, each ring or ring system optionally substituted with up to 5 substituents independently selected from R⁷ on carbon atom ring members and selected from R⁹ on nitrogen atom ring members;
Q² is a phenyl ring or a naphthalenyl ring system, each ring or ring system optionally substituted with up to 5 substituents independently selected from R¹⁰; or a 5- to 6-membered fully unsaturated heterocyclic ring or an 8- to 10-membered heteroaromatic bicyclic ring system, each ring or ring system containing ring members selected from carbon atoms and 1 to 4 heteroatoms independently selected from up to 2 U, up to 2 S and up to 4 N atoms, wherein up to 3 carbon ring members are independently selected from C(=O) and C(=S), and the sulfur atom ring members are independently selected from S(=O)ᵤ(=NR⁸)ᵥ, each ring or ring system optionally substituted with up to 5 substituents independently selected from R¹⁰ on carbon atom ring members and selected from R¹¹ on nitrogen atom ring members;
Y¹ and Y² are each independently O, S or NR¹²;
R¹ is H, hydroxy, amino, C₁-C₆ alkyl, C₁-C₆ haloalkyl, C₂-C₆ alkenyl, C₃-C₆ alkynyl, C₄-C₈ cycloalkylalkyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₂-Cg alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃C₁₀ dialkylaminocarbonyl, C₄-C₁₀ cycloalkylaminocarbonyl, C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, C₃-C₁₀ trialkylsilyl or G¹;
R² and R³ are each independently H, halogen or C₁-C₄ alkyl; or
R² and R³ are taken together with the carbon atom to which they are bonded to form a C₃-C₇ cycloalkyl ring;
R⁴ and R⁵ are each independently H; halogen or C₁-C₄ alkyl;
R⁶ is H, hydroxy, amino, C₁-C₆ alkyl, C₁-C₆ haloalkyl, C₂-C₆ alkenyl, C₃-C₆ alkynyl, C₂-C₈ alkoxy alkyl, C₂-C₈ haloalkoxyalkyl, C₂-C₈ alkylthioalkyl, C₂-C₈ alkylsulfinylalkyl, C₂-C₈ alkylsulfonylalkyl, C₂-C₈ alkylcarbonyl, C₂-C₈ haloalkylcurbonyl, C₄-C₁₀ cycloalkylcarbonyl, C₂-C₃ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, C₃-C₁₀ trialkylsilyl of G¹;
each R⁷ and R¹⁰ is independently halogen, cyano, nitro, C₁-C₈ alkyl, C₁-C₈ haloalkyl, C₁-C₈ nitroalkyl, C₂-C₈ alkenyl, C₂-C₈ haloalkenyl, C₂-C₈ nitroalkenyl, C₂-Cg alkynyl, C₂-C₈ haloalkynyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂ cycloalkylalkynyl, C₃-C₈ cycloalkyl, C₃-C₈ halocycloalkyl, C₄-C₁₀ alkylcycloalkyl, C₆-C₁₂, cycloalkylcycloalkyl, C₃-C₈ cycloalkenyl, C₃-C₈ halocycloalkenyl, C₂-C₈ alkoxyalkyl, C₂-C₈ haloalkoxyalkyl, C₃-C₈ haloalkoxyalkoxy, C₃-C₈ alkoxyalkoxy, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl, C₂-C₈ haloalkylcarbonyl, C₄-C₁₀ cycloalkylcarbonyl, -C(=O)OH, C₂-C₈ alkoxycarbonyl, C₂-C₈ haloalkoxycarbonyl, C₄-C₁₀ cycloalkoxycarbonyl, C₅-C₁₂ cycloalkylalkoxycarbonyl, -C(-O)NH₂, C₂-C₈ alkylaminocarbonyl, C₄-C₁₀ cycloalkylaminocarbonyl, C₃-C₁₀ dialkylaminocarbonyl, C₁-C₈ alkoxy, C₁-C₈ haloalkoxy, C₂-C₈ alkoxyalkoxy, C₂-C₈ alkenyloxy, C₂-C₈ haloalkenyloxy, C₃-C₈ alkynyloxy, C₃-C₈ haloalkynyloxy, C₃-C₈ cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈, haloalkylsulfonyloxy, C₁-C₈ alkylthio, C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₃-C₈ cycloalkylsulfonyl, formylamino, C₂-C₈ alkylcarbonylamino, C₂-C₈ haloalkylcarbonylamino, C₂-C₈ alkoxycarbonylamino, C₁-C₈ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl, C₄-C₁₂ trialkylsilylalkoxy or G²;
each R⁸ is independently H, cyano, C₂-C₃ alkyl carbonyl or C₂-C₃ haloalkylcarbonyl;
each R⁹ and R¹¹ is independently cyano, C₁-C₃ alkyl, C₂-C₃ alkenyl, C₂-C₃ alkynyl, C₃-C₆ cycloalkyl, C₂-C₃ alkoxyalkyl, C₁-C₃ alkoxy, C₂-C₃ alkylcarbonyl, C₂-C₃ alkoxycarbonyl, C₂-C₃ alkylaminoalkyl or C₃-C₄ dialkylaminoalkyl;
each R¹² is independently H, cyano, C₁-C₄ alkyl, C₁-C₄ haloalkyl, -(C=O)CH₃ or-(C=C)CF₃;
each G¹ is independently phenyl, phenylmethyl, pyridinylmethyl, phenylcarbonyl, phenoxy, phenylethynyl, phenylsulfonyl or a 5- or 6-membered heteroaromatic ring, each optionally substituted on ring members with up to 5 substituents independently selected from R¹³ ;
each G² is independently phenyl, phenylmethyl, pyridinylmethyl, phenylcarbonyl, phenoxy, phenylethynyl, phenylsulfonyl or a 5- or 6-membered heteroaromatic ring, each optionally substituted on ring members with up to 5 substituents independently selected from R¹⁴ ;
each R¹³ and R¹⁴ is independently halogen, cyano, hydroxy, amino, nitro, -CHO, -C(=O)OH, -C(=O)NH₂, -SO₂NH₂, C₁-C₆ alkyl, C₁-C₆ haloalkyl, C₂-C₆ alkenyl, C₂-C₆ alkynyl, C₂-C₈ alkylcarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ alkylaminosulfonyl, C₂-C₈ dialkylaminosulfonyl, C₃-C₁₀ trialkylsilyl, C₁-C₆ alkyiamino, C₂-C₈ dialkylamino, C₂-C₈ alkylcarbonylamino, C₁-C₆ alkylsulfonylamino, phenyl, pyridinyl or thienyl; and
each u and v are independently 0, 1 or 2 in each instance of S(=O)ᵤ(=NR⁸)ᵥ, provided that the sum of u and v is 0, 1 or 2;
provided that
   (a) the compound of Formula 1 is other than*N*-1*H*-benzotriazol-1-yl-2-oxo-4-phenyl-3-pyrrolidinecarboxamide;
   (b) when Q¹ comprises a 3-furanyl or 3-pyridinyl ring directly bonded to the remainder of Formula 1, then said ring is substituted with at least one substituent selected from R⁷;
   (c) when Q¹ is an unsubstituted phenyl ring, and Q² comprises a phenyl ring directly bonded to the remainder of Formula 1, then said Q² ring is substituted with R¹⁰ other than optionally substituted phenoxy or F at a 2-position, cyano or -CF₃ at the 4-positionand R³ is II or halogen;
   (d) when Q¹ is unsubstituted phenyl, and Q² comprises a pyridinyl ring directly bonded to the remainder of Formula 1, then said pyridinyl ring is substituted with at least one substituent selected from R¹⁰;
   (e) when Q¹ is a phenyl ring substituted with 4-phenyl or 4-phenoxy, said Q¹ ring is further substituted with and R⁷ susbtituent;
   (f) when Q¹ comprises a phenyl ring directly bonded to the remainder of Formula 1 and said ring is substituted with R⁷ at both ortho positions (relative to the bond to the remainder of Formula 1), then said ring is also independently substituted with R⁷ on at least one additional position;
   (g) when Q¹ is other than unsubstituted 1-naphthalenyl, then Q² is other than 2,3-difluorophenyl or 2-CF₃-phenyl;
   (h) Q² is other than optionally substituted 1*H*-pyrazol-5-yl;and
   (i) when Q² comprises a 1*H*-pyrazol-3-yl ring directly bonded to the remainder of Formula 1, said ring is substituted at the 1-position with R⁹.

### Embodiment A35b

A Saturated Target which has the following structure, 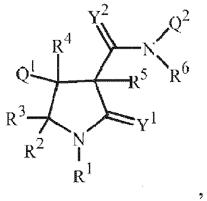 in combination, admix, mixture etc., with at least one Unsaturated Precursor which is defined by any of the following structures,

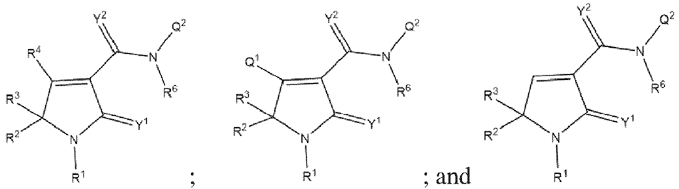

Wherein Q¹, Q², R¹, R², R³, R⁴, R6, Y1 and Y2 etc. have the meanings as defined in Claim 1 of PCT/US2014/068073 published as WO 2015/084796. The text extracted from item 1 on page 286, line 6 until page 289 line 23 of WO 2015/084796 being specifically incorporated by reference solely for the purpose of defining the structures of this embodiment. The text being reproduced in Embodiment **A35a.**

### Embodiment A35c

A Saturated Target which has the following structure, 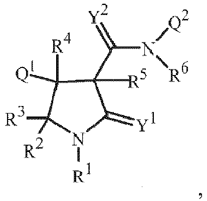 in combination, admix, mixture etc., with at least one Unsaturated Precursor which is an Unsaturated Precursor defined by any of the following structures, 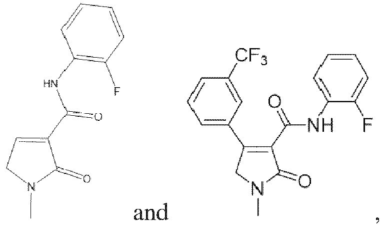 wherein Q¹, Q², R¹, R², R³, R⁴, R6, Y1 and Y2 etc. have the meanings as defined in Claim 1 of PCT/US2014/068073 published as WO 2015/084796. The text extracted from item 1 on page 286, line 6 until page 289 line 23 of WO 2015/084796 being specifically incorporated by reference solely for the purpose of defining the structures of this embodiment. The text being reproduced in Embodiment **A35a.**

### Embodiment A36a

A Saturated Target which has the following structure, 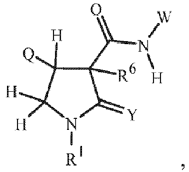 in combination, admix, mixture etc., with at least one Unsaturated Precursor which is defined by any of the following structures,

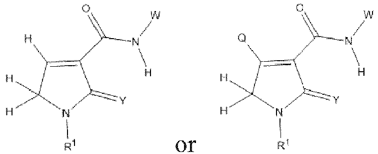

Wherein Q, R¹, R⁶, Y and W etc. have the meanings as defined in Claim 1 of PCT/US2020/034232, published as WO 2020/242946. The text from claim 1 on page 87, line 3 until page 88 line 6 of WO 2020/242946 being specifically incorporated herein by reference solely for the purpose of defining the structures of this embodiment.
wherein Q is selected from the group consisting of 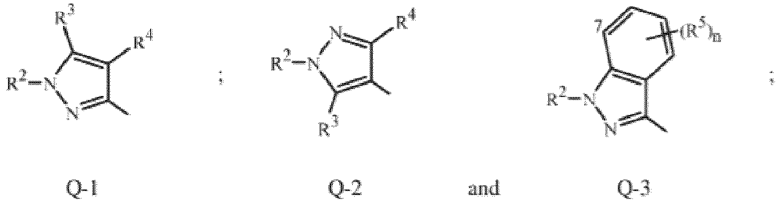
   R¹ is H, C₁-C₆ alkyl, C₁-C₆ haloalkyl, C₃-C₇ cycloalkyl orC₄-C₈ cycloalkylalkyl;
   R² is C₁-C₆ alkyl or C₁-C₆ haloalkyl;
   R³ is halogen, C₁-C₆ alkyl, C₁-C₆ haloalky, C₁-C₆, alkoxy or C₁-C haloalkoxy;
   Y is O or S,
   R⁴ is H, halogen, C₁-C₄ alkyl or C₁-C₄ haloalkyl;
   R⁵ is halogen, C₁-C₄ alkyl or C₁-C₄ haloalkyl;
   n is 1, 2, 3 or 4;
   R⁶ is H, halogen, hydroxy, C₁-C₄ alkoxy, C₁-C₄ haloalkyl or C₁-C₄ alkyl;
   W is phenyl or pyridyl, each phenyl or pyridyl optionally substituted with up to 5 R⁹; and
   each R⁹ is independently halogen, cyano, nitro, C₁-C₄ alkyl, C₁-C₄ haloalkyl, C₂-C₄ alkenyl, C₂-C₄ haloalkenyl C₂-C₄ alkynyl, C₂-C₄ haloalkynyl, C₁-C₄ nitroalkyl, C₂-C₄ nitroalkenyl, C₂-C₄ alkoxyalkyl, C₂-C₄ haloalkoxyalkyl, C₃-C₄ cycloalkyl, C₃-C₄ halocycloalkyl, cyclopropylmethyl. methylcyclopropyl, C₁-C₄ alkoxy, C₁-C₄ haloalkoxy, C₂-C₄ alkenyloxy, C₂-C₄ haloalkenyloxy, C₃-C₄ alkynyloxy, C₃-C₄ haloalkynyloxy C₃-C₄ cycloalkoxy, C₁-C₄ alkylthio, C₁-C₄ haloalkylthio, C₁-C₄ alkylsulfinyl, C₁-C₄ haloalkylsulfinyl, C₁-C₄ alkylsulfonyl, C₁-C₄ haloalkylsullonyl, hydroxy, formyl, C₁-C₄ alkylcarbonyl, C₂-C₄ alkylcarbonyloxy, C₁-C₄ alkylsulfonyloxy,
      C₁-C₄ haloaltylsulfonyloxy, amino, C₁-C₄ alkylamino, C₂-C₄ dialkylamino, formylamino, C₂-C₄ alkylcarbonylamino, -SF₅, -SCN, C₃-C₄ trialkylsilyl, trimethylsilylmethyl or trimethylsitylmethoxy;
provided the compound is other than it compound of Formula **1** wherein Q is Q-1; R¹ is H; R² is CH₃; R³ is C(CH₃)₃; R⁴ is H; R⁶ is H; Y is O, W is phenyl substituted with R⁹ at the 2-position; and R⁹ is F.

### Embodiment A37a

An Unsaturated Precursor which is defined by any of the following structures, 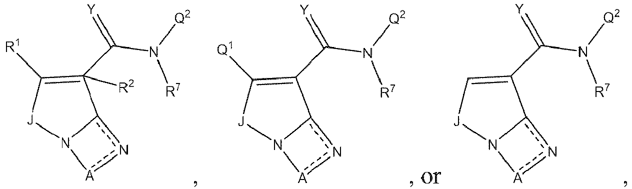 wherein Q¹, Q², R¹, R², R⁷, Y, A and J etc. have the meanings as defined in Claim 1 of PCT/US2016/030450, published as WO2016182780. The text of claim 1 on page 101, line 3 until page 106 line 28 of WO2016/182780 being specifically incorporated by reference solely for the purpose of defining the structures of this embodiment.
Q¹ is a phenyl or benzyl ring or a naphthalenyl ring system, each ring or ring system optionally substituted with up to 5 substituents independently selected from R⁹; or a 5- to 6-membered fully unsaturated heterocyclic ring or an 8- to 10-membered heteroaromatic bicyclic ring system, each ring or ring system containing ring members selected from carbon atoms and 1 to 4 heteroatoms independently selected from up to 2 O, up to 2 S and up to 4 N atoms, wherein up to 3 carbon ring members are independently selected from C(=O) and C(=S), and the sulfur atom ring members are independently selected from S(=O)ᵤ(=NR⁸)ᵥ, each ring or ring system optionally substituted with up to 5 substituents independently selected from R⁹ on carbon atom ring members and selected from R¹⁰ on nitrogen atom ring members;
Q² is a phenyl ring or a naphthalenyl ring system, each ring or ring system optionally substituted with up to 5 substituents independently selected from R¹¹; or a 5- to 6-membered fully unsaturated heterocyclic ring or an 8- to to-membered heteroaromatic bicyclic ring system, each ring or ring system containing ring members selected from carbon atoms and 1 to 4 heteroatoms independently selected from up to 2 O, up to 2 S and up to 4 N atoms, wherein up to 3 carbon ring members are independently selected from C(=O) and C(=S), and the sulfur atom ring members are independently selected from S(=O)ᵤ(=NR⁸)ᵥ, each ring or ring system optionally substituted with up to 5 substituents independently selected from R¹¹ on carbon atom ring members and selected from R¹² on nitrogen atom ring members;
R¹ and R² are each independently H, halogen, hydroxy or C₁-C₄ alkyl;
Y is O, S or NR¹⁵;
A is a saturated, partially unsaturated or fully unsaturated chain containing 2 to 4 atoms selected from up to 4 carbon, up to 1 O, up to 1 S and up to 2 N atoms, wherein up to 2 carbon members are independently selected from C(=O) and C(=S) and the sulfur atom member is selected from S(=O)ᵤ(=NR⁸)ᵥ; the said chain optionally substituted with up to 5 substituents independently selected from R³ on carbon atoms and R⁴ on nitrogen atoms;
each R³ is independently halogen, cyano, hydroxy, -CO₂H, C₁-C₄ alkyl, C₁-C₄ haloalkyl, C₁-C₄ alkoxy, C₁-C₄ alkylthio, C₁-C₄ haloalkoxy, C₂-C₄ alkoxyalkyl, C₂-C₄ alkylcarbonyl, C₂-C₄ alkoxycarbonyl, C₃-C₆ cycloalkyl of C₄-C₆ cycloalkylalkyl; or
two R³ are taken together with the carbon atom(s) to which they are bonded to form a C₃-C₇ cycloalkyl ring;
each R⁴ is independently cyano, C₁-C₄ alkyl, C₁-C₄ haloalkyl, C₁-C₄ alkoxy, C₁-C₄ alkylcarbonyl, C₁-C₄ alkoxycarbonyl or C₃-C₆ cycloalkyl;
J is -CR⁵R⁶- or -CR⁵R⁶-CR^{5a}R^{6a}- wherein the -CR⁵R⁶- moiety is directly connected to N;
R⁵ and R⁶ are each independently H, halogen, hydroxy, C₁-C₄ alkyl or C₁-C₄ alkoxy, or
R⁵ and R⁶ are taken together with the carbon atom to which they are bonded to form a C₃-C₇ cycloalkyl ring;
R^{5a} and R^{6a} are each independently H, halogen or C₁-C₄ alkyl, or
R^{5a} and R^{6a} are taken together with the carbon atom to which they are bonded to form a C₃-C₇ cycloalkyl ring,
R⁷ is H, hydroxy, amino, C₁-C₆ alkyl, C₁-C₆ haloalkyl, C₁-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₁-C₆ alkylsulfonylalkyl, C₂-C₈ alkylcarbonyl, C₂-C₈ haloalkylcarbonyl, C₄-C₁₀) cycloalkylcacbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, C₃-C₁₀ trialkylsilyl or G¹;
each R⁸ is independently H, cyano, C₂-C₃ alkylcarbonyl or C₂-C₃ haloalkylcarbonyl;
each R⁹ is independently halogen, cyano, nitro, C₁-C₈ alkyl, C₁-C₄ cyanoalkyl, C₁-C₄ cyanoalkoxy, C₁-C₈ haloalkyl, C₁-C₈ nitroalkyl, C₂-C₈ alkenyl, C₂-C₈ haloalkenyl, C₂-C₈ nitroalkenyl, C₂-C₈ alkynyl, C₂-C₈ haloalkynyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂ cycloalkylalkynyl, C₂-C₈ cycloalkyl, C₃-C₈ halocycloalkyl, C₄-C₁₀ alkylcycloalkyl, C₆-C₁₂ cycloalkylcycloalkyl, C₃-C₈ cycloalkenyl, C₁-C₆ balocycloalkenyl, C₂-C₈ alkoxyalkyl, C₂-C₈ haloalkoxyalkyl, C₃-C₈ haloalkoxyalkoxy, C₁-C₄ hydroxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl, C₂-C₈ haloalkykarbonyl, C₄-C₁₀ cycloalkylcarbonyl, -C(-O)OH, C₂-C₈ alkoxycarbonyl, C₂-C₈ haloalkoxycarbonyl, C₄-C₁₀ cycloalkoxycarbonyl, C₅-C₁₂ cycloalkylalkoxycarbonyl, -C(=O)NH₂, C₂-C₈ alkylaminocarbonyl, C₄-C₁₀ cycloalkylaminocarbonyl, C₃-C₁₀ dialkylaminocarbonyl, C₁-C₈ alkoxy, C₁-C₈ haloalkoxy, C₂-C₈ alkoxyalkoxy, C₂-C₈ alkenyloxy, C₂-C₈ haloalkenyloxy, C₂-C₈ haloalkoxyhaloalkoxy, C₃-C₈ alkynyloxy, C₃-C₈ haloalkynyloxy, C₃-C₈ cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈ haloalkylsulfonyloxy, C₁-C₈, alkylthio, C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₃-C₈ cycloalkylsulfonyl, formylamino, C₂-C₈ alkylcarbonylamino, haloalkylcarbonylamino, C₂-C₈ alkoxycarbonylamino, C₁-C₆ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl, C₄-C₁₂ trialkylsilylalkoxy or G²;
each R¹¹ is independently halogen, cyano, nitro, C₁-C₈ alkyl, C₁-C₄ cyanoalkyl, C₁-C₄ cyanoalkoxy, C₁-C₈ haloalkyl, C₁-C₈ nitroalkyl, C₂-C₈ alkenyl, C₂-C₈ haloalkenyl, C₂-C₈ nitroalkenyl, C₂-C₈ alkynyl, C₂-C₈ haloalkynyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂ cycloalkylalkynyl, C₃-C₈ cycloalkyl, C₁-C₆ halocycloalkyl, C₄-C₁₀ alkylcycloalkyl, C₆-C₁₂ cycloalkylcycloalkyl, C₃-C₈ cycloalkenyl, C₃-C₈, halocycloalkenyl, C₂-C₈ alkoxyalkyl, C₂-C₈ haloalkoxyalkyl, C₃-C₈ haloalkoxyalkoxy, C₁-C₄ hydroxyalkyl, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl, C₂-C₈ haloalkylcarbonyl, C₄-C₁₀ cycloalkylcarbonyl, -C(=O)OH, C₂-C₈ alkoxycarbonyl, C₂-C₈ haloalkoxycarbonyl, C₄-C₁₀ cycloalkoxycarbonyl, C₅-C₁₂, cycloalkylalkoxycarbonyl, -C(=O)NH₂, C₂-C₈ alkylaminocarbonyl, C₄-C₁₀ cycloalkylaminocarbonyl, C₃-C₁₀ dialkylaminocarbonyl, C₁-C₈ alkoxy, C₁-C₈ haloalkoxy, C₂-C₈ alkoxyalkoxy, C₂-C₈ alkenyloxy, C₂-C₈ haloalkenyloxy, C₂-C₈ haloalkoxyhaloalkoxy, C₃-C₈ alkynyloxy, C₃-C₈ haloalkynyloxy, C₃-C₈ cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈ haloalkylsulfonyloxy, C₁-C₈ alkylthio, C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₁-C₆ cycloalkyl sulfonyl, formylamino, C₂-C₈ alkylcarbonylamino, C₂-C₈ haloalkylcarbonylamino, C₂-C₈ alkoxycarbonylamino, C₁-C₆, alkylsulfonylamino, C₁-C₆ haloalkylsulfonylamino, -SF₅. -SCN, SO₂NH₂, C₃-C₁₂ trialkylsilyl, C₄-C₁₂ trialkylsilylalkyl, C₄-C₁₂ trialkylsilylalkoxy, or G³;
each R¹⁰ and R¹² is independently cyano, C₁-C₃ alkyl, C₂-C₃ alkenyl, C₂-C₃ alkynyl, C₃-C₆ cycloalkyl, C₂-C₃ alkoxyalkyl, C₁-C₃ alkoxy, alkylcarbonyl, C₂-C₃ alkoxycarbonyl, C₂-C₃ alkylaminoalkyl or C₃-C₄ dialkylaminoalkyl;
R¹⁵ is H, cyano, C₁-C₄ alkyl, C₁-C₄ haloalkyl. -(C=O)CH₃ or -(C=O)CF₃;
each G¹ is independently phenyl, phenylmethyl, pyridinylinethyl, pyridinyloxy, phenylcarbonyl, phenoxy, phenylethynyl, phenylsulfonyl, phenylcarbonyl(C₁-C₄ alkyl); or a 5- or 6-membered heteroaromatic ring, each optionally substituted on ring members with up to 5 substituents independently selected from R¹³;
each G² is independently phenyl, phenylmethyl, pyridinylmethyl, phenylcarbonyl, phenylcarbonylalkyl, phenoxy, phenylethynyl, phenylsulfonyl or pyridyloxy; or a 5- of 6-membered heteroaromatic ring, each optionally substituted on ring members with up to 5 substituents independently selected from R¹⁴; or R¹⁶ON=CR¹⁷-, (R¹⁸)₂C=NO-, (R¹⁹)₂NN=CR¹⁷-, (R¹⁸)₂C=NNR²⁰-, R²¹N=CR¹⁷-, (R¹⁸)₂C=N-, R²²ON=CR¹⁷C(R²³)₂- or (R¹⁸)₂C=NOC(R²³)₂-, wherein the free bond projecting to the right indicates the connecting point to Q¹;
each G³ is independently phenyl, phenylmethyl, pyridinylmethyl, phenylcarbonyl, phenylcarbonylalkyl, phenoxy, phenylethynyl, phenylsulfonyl or pyridyloxy; or a 5- or 6-membered heteroaromatic ring, each optionally substituted on ring members with up to 5 substituents independently selected from R¹⁵;
each R¹³, R¹⁴ and R¹⁵ is independently halogen, cyano, hydroxy, amino, nitro, -CHO, -C(=O)OH, -C(=O)NH₂, -SO₂NH₂, C₁-C₆ alkyl, C₁-C₆ haloalkyl, C₂-C₆ alkenyl, C₂-C₆ alkynyl, C₂-C₈ alkylcarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆, alkylsulfonyl, C₁-C₆ haloalkylsulfonyl, C₁-C₆ alkylaminosulfonyl, C₂-C₈ dialkylaminosulfonyl, C₃-C₁₀ trialkylsilyl, C₁-C₆ alkylamino, C₂-C₈ dialkylamino, C₂-C₈ alkylcarbonylamino or C₁-C₆ alkylsulfonylamino;
each R¹⁶ is independently H, C₁-C₆ alkyl, C₃-C₈ cycloalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinyialkyl, C₂-C₈ alkylsulfonylalkyl, C₂-C₈ alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, C₃-C₁₀ trialkylsilyl or G¹;
each R¹⁷ is independently H, C₁-C₆ alkyl, C₃-C₈ cycloalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₁-C₆ alkoxy, C₁-C₆ alkylthio, C₂-C₈ haloalkylthio, C₃-C₈ cycloalkylthio, C₃-C₁₀ trialkylsilyl or G¹;
each R¹⁸ is independently H, hydroxy, C₁-C₆, alkyl, C₃-C₈ cydoalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinyialkyl, C₂-C₈ alkylsulfonylalkyl, C₂-C₈ alkylcarbonyl, C₂-C₈ haloalkylcarbonyl, C₄-C₁₀ cydoalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀) cycloalkylaminocarbonyl, C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkyl sulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂₋C₈ dialkylaminosulfonyl, C₃-C₁₀ trialkylsilyl orG¹;
each R¹⁹ is independently H, C₁-C₆ alkyl, C₃-C₈ cycloalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₂-C₈ alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, C₃-C₁₀ trialkylsilyl or G¹;
each R²⁰ is independently H, C₁-C₆ alkyl, C₃-C₈ cycloalkyl, C₄-C₈ cycloalkylalkyl, C₁₋C₆ haloalkyl, C₂₋C₆ alkenyl, C₃-C₆ alkynyl, C₂-C₈ alkoxyalkyl, C₂-C₈ haloalkoxyalkyl, C₂-C₈ alkylthialkyl, C₂-C₈ alkylsulfinylalkyl, C₂-C₈ alkylsulfonylalkyl, C₁-C₆ alkoxyC₃-C₁₀ trialkylsilyl or G¹;
each R²¹ is independently H, hydroxy, amino, C₁-C₆ alkyl, C₃-C₈ cycloalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₂-C₈ alkylcarbonyl, C₂-C₈ haloalkylcarbonyl, C₄-C₁₀ cycloalkylcarbonyl, C₁-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cydoalkylsulfonyl, C₁-C₆ alkylaminosulfonyl, C₂-C₈ dialkylaminosulfonyl, C₃-C₁₀ trialkylsilyl or G¹;
each R²² is independently H, C₁-C₄ alkyl, C₃-C₈ cycloalkyl, C₄-C₈ cycloalkylalkyl, C₁-C₄ haloalkyl, C₁-C₄ alkoxy, C₁-C₄ haloalkoxy, C₃-C₄ alkoxyalkyl, C₂-C₄ alkylcarbonyl, C₂-C₄ alkoxycarbonyl or C₃-C₆ cycloalkyl;
each R²³ is independently H, halogen, cyano, hydroxy, C₁-C₄ alkyl, C₃-C₈ cycloalkyl, C₄-C₈ cycloalkylalkyl, C₁-C₄ haloalkyl, C₁-C₄ alkoxy, C₁-C₄ haloalkoxy, C₂-C₄ alkoxyalkyl, C₂-C₄ alkylcarbonyl, C₂-C₄ alkoxycarbonyl or C₃-C₆ cycloalkyl; and
each u and v are independently 0, 1 or 2 in each instance of S(=O)u(=NR8)v, provided that the sum of u and v is 0, 1. or 2; and provided the compound is other than a compound of Formula 1 wherein Q¹ is Ph(3-CF₃); Q² is Ph(2-F); R¹ is H; R² is H; Y is O; A is -CH₂CH₂-; J is -CR⁵R⁶-; R⁵ is H; R⁶ is H; and R⁷ is H.

### Embodiment A37b

A pesticide synthesis intermediate compound defined by any one of the following structures,

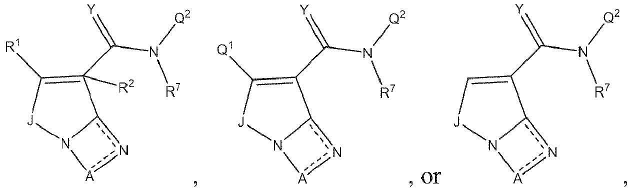

Wherein Q¹, Q², R¹, R², R⁷, Y, A and J etc. have the meanings as defined in Claim 1 of PCT/US2016/030450, published as WO2016/182780. The text of claim 1 on page 101, line 3 until page 106 line 28 of WO2016/182780 being specifically incorporated by reference solely for the purpose of defining the structures of this embodiment, the text being reproduced in Embodiment **A37a.**

### Embodiment A37c

A Saturated Target having the following structure, 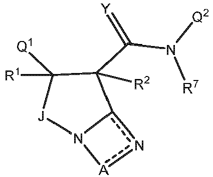 in combination, admix, mixture etc. with at least one Unsaturated Precursor which is defined by any of the following structures, 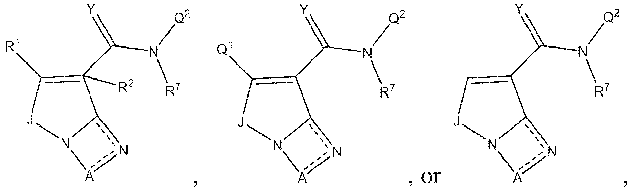 wherein Q¹, Q², R¹, R², R⁷, Y, A and J etc. have the meanings as defined in Claim 1 of PCT/US2016/030450, published as WO2016182780. The text of claim 1 on page 101, line 3 until page 106 line 28 of WO2016/182780 being specifically incorporated by reference solely for the purpose of defining the structures of this embodiment, the text being reproduced in Embodiment **A37a.**

### Embodiment A38

An Unsaturated Precursor, defined by either one of the following formulae, 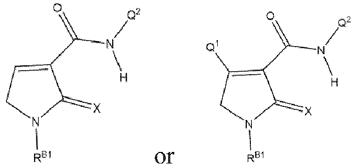 wherein Q¹, Q²,R^{B1}, and X etc. have the meanings as defined in Claim 1 of PCT/EP2020/052780 published as WO 2020/161147. The text from claim 1 on page 74, line 10 until page 75 line 10 of WO 2020/161147 being specifically incorporated by reference solely for the purpose of defining the structures of this embodiment.
R^{B1} is H, methyl, or methoxy;
X is O or S;
Q¹ is a di- or tri-substituted pyrazole, substituted on one ring nitrogen by R^{B2} and substituted
on at least one ring carbon by R^{B3}, wherein
   R⁵² is C₁-C₃ alkyl or C₁-C₃fluoroalkyl and each R^{B3} is independently halogen, C₁-
   C₃fluoroalkyl, C₁-C₃haloalkoxy, C₁-C₃alkoxy, or C₁-C₃hatoalkyl, Ct-Csfluoroalkyl. C₁-
   C₃haloalkoxy, C₁-C₃alkoxy, or C₁-C₃alkyl;
or Q¹ is a di-substituted pyrazole, substituted on one ring nitrogen by R^{B2} and on an
adjacent ring carbon by R^{B3}, wherein
   R^{B2} C₁-C₃ alkyl and R^{B3} is C₁-C₃fluoroalkyl or C₁-C₃alkyl, and R^{B2} and R^{B3} together
   with the atoms to which they are joined and Q¹ form an eight or nine-membered
   fused heterocyclic bicyclic ring system;
Q² is a phenyl, pyridinyl, or thienyl ring system, optionally substituted by 1, 2, or 3 R^{B5}
substituents; and
each R^{B5} is independently halogen, C₁-C₆alkyl, C₁-C₆haloalkyl, C₁-C₆alkoxy, C₁-C₆hatoalkoxy,
cyano, nitro, C₁₋C₆alkylthio, C₁-C₆alkylsulphinyl, or C₁-C₆alkylsulphonyl; or an N-oxide, or a
salt form thereof.

### Embodiment A38a

A Saturated Target as defined in Claim 1 of PCT/EP2020/052780 published as WO2020/161147, 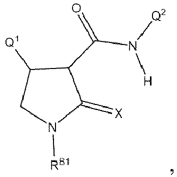 in combination, admix, mixture etc. with at least one Unsaturated Precursor each defined by any one of the following structures, 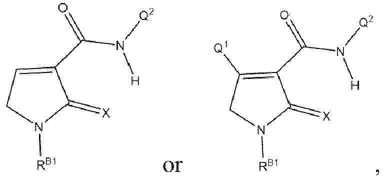 wherein Q¹, Q²,R^{B1}, and X etc. have the meanings as defined in Claim 1 of PCT/EP2020/052780 published as WO 2020/161147. The text from claim 1 on page 74, line 10 until page 75 line 10 of WO 2020/161147 being specifically incorporated by reference solely for the purpose of defining the structures of this embodiment, the text being reproduced in Embodiment **A38.**

### Embodiment A39

An Unsaturated Precursor defined by either one of the following formulae,

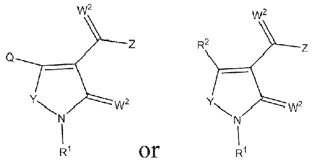

Wherein Q, W², R¹, Y and Z etc. have the meanings as defined in Claim 1 of PCT/EP2018/069001 published as WO 2019/025156. The German language text from claim 1 on page 109, line 3 until page 112 line 6 of WO 2019/025156 being specifically incorporated by reference only for the purpose of defining the structures of this embodiment.

### Embodiment A40

An Unsaturated Precursor which has the following structure,

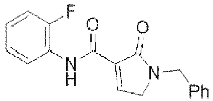

### Embodiment A41

An Unsaturated Precursor which is defined by any one of the following structures,

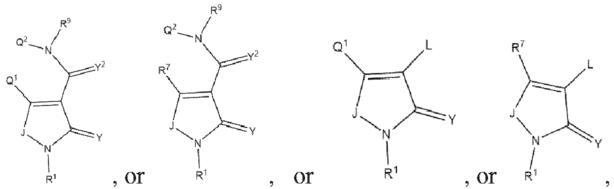

Wherein Q¹, Q², R¹, R⁷, R⁹, J, L, Y and Y² etc. have the meanings as defined in Claim 1 of PCT/US2018/035017 published as WO 2018/222647. The text from claim 1 on page 84, line 5 until page 89 line 27 of WO 2018/222647 being specifically incorporated by reference solely for the purpose of defining the structures of this embodiment.
L is selected from 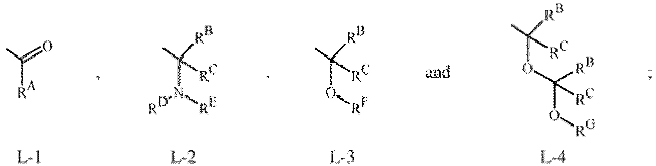
R^{A} is C₁-C₇ alkyl, C₁-C₇ haloalkyl, C₃-C₉ cycloalkyl, C₃-C₉ halocycloalkyl. C₁-C₇ alkoxy, C₁-C₇ haloalkoxy, C₃-C₉ cycloalkoxy, C₃-C₉ halocycloalkoxy, C₂-C₈ alkenyl, C₂-C₈ haloalkenyl, C₁-C₇ alkylamino, C₁-C₇ haloalkylamino, C₂-C₉ dialkylamino, C₂-C₉ halodialkylamino, C₃-C₉ cycloalkylamino or C₃-C₉ halocycloalkylamino, each substituted or unsubstituted with up to 3 substituents independently selected from R⁸ or G¹; or
R^{A} is G¹ or OG¹; or
R^{A} is taken together with R⁹ as -C(R^{I})(R^{J})C(=O)-;
R^{B} is H, C₁-C₄ alkoxy, C₁-C₄ haloalkyl or C₁-C₄ alkyl; or phenyl substituted or unsubstituted with halogen or C₁-C₄ alkyl;
R^{C} is H, C₁-C₄ alkoxy, C₁-C₄ haloalkyl orC₁-C₄ alkyl, or phenyl substituted or unsubstituted with halogen or C₁-C₄ alkyl;
R^{D} is H, C₁-C₄ alkyl or C₂-C₄ alkylcarbonyl;
R^{E} is H, hydroxy, amino, cyano, formyl, -C(O)NH₂, C₁-C₆ alkyl, C₁-C₆ haloalkyl, C₂-C₆ cyanoalkyl, C₃-C₆ cycloalkyl, C₄-C₈ cycloalkylalkyl, C₂-C₈ alkoxyalkyl, C₃-C₈ alkoxyalkoxyalkyl, C₂-C₈ haloalkoxyalkyl, C₂-C₈ alkenyl, C₂-C₈ haloalkenyl, C₂-C₈ alkenylalkyl, C₂-C₈ haloalkenylalkyl, C₂-C₈
   alkylcarbonyl, C₂-C₈ haloalkylcarbonyl, C₄-C₁₀ cycloalkylcarbonyl, C₅-C₁₀ cycloalkylcarbonylalkyl, C₂-C₈ alkoxycarbonyl, C₂-C₈ haloalkoxycarbonyl, C₄-C₁₀ cycloalkoxycaibonyl, C₂-C₈ alkylaminocarbonyl, C₃-C₁₀ dialkylaminocarbonyl, C₄-C₁₀ cycloalkylaminocarbonyl. C₁-C₆ alkoxy, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl of C₂-C₈ dialkylaminosulfonyl; or G^{E} of W^{E}G^{E};
R^{F} is H, formyl, -C(O)NH₂, C₂-C₈ alkylcarbonyl, C₂-C₈ haloalkylcarbonyl, C₄-C₁₀ cycloalkylcarbonyl. C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, -P(=O)(OH)₂, C₁-C₆ dialkylphosphoryl, C₁-C₆ haloalkylphosphoryl, C₃-C₈ cycloalkylphosphoryl, C₂-C₈ dialkoxyphosphoryl, C₆-C₁₄ dicycloalkoxyphixsphoryl, C₈-C₁₆ dicycloalkylalkoxyphosphoryl, C₂-C₁₂ bis(alkylamino)phosphoryl, C₄-C₂₄ bis(dialkylamino)phosphoryl; or G^{F} or W^{F}G^{F};
R^{G} is formyl, -C(O)NH₂, C₂-C₈ alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkylsulfinyl, C₂-C₈ haloalkylsulfinyl, C₂-C₈ cycloalkylsulfinyl, C₁-C₆ alkylsulfonyl, C₁₋C₆ haloalkylsulfonyl, C₃-C₈ cycloalkylsulfonyl, C₁-C₆ alkylaminosulfonyl, C₂-C₈ dialkylaminosulfonyl, -P(=O)(OH)₂, C₁-C₆ dialkylphosphoryl, C₁-C₆ haloalkylphosphoryl, C₃-C₈ cycloalkylphosphoryl, C₂-C₈ dialkoxyphosphoryl, C₆-C₁₄ dicycloalkoxyphosphoryl, C₈-C₁₆ dicycloalkylalkoxyphasphoryl, C₂-C₁₂ bis(alkylamino)phosphoryl, C₄-C₂₄ bis(dialkylamino)phosphoryl; or phenyl substituted or unsubstituted with R¹⁶; or W^{G}G^{G};
R¹ is H, C₁-C₄ alkoxy, C₁-C₄ haloalkyl or C₁-C₄ alkyl; or phenyl substituted or unsubstituted with halogen or C₁-C₄ alkyl;
R^{J} is H, C₁-C₄ alkoxy, C₁-C₄ haloalkyl, or C₁-C₄ alkyl; or phenyl substituted or unsubstituted with halogen or C₁-C₄ alkyl;
Q¹ is a phenyl ring or a naphthalenyl ring system, each ring or ring system substituted or unsubstituted with up to 5 substituents independently selected from R⁷, or a 4-to 7-membered heterocyclic ring; or an 8- to 10-membered bicyclic ring system, each ring or ring system containing ring members selected from carbon atoms and 1 to 5 heteroatoms independently selected from up to 2 O, up to 2 5 and up to 5 N atoms, wherein up to 3 carbon ring members are independently selected from C(=O) and C(=S), and the sulfur atom ring members are independently selected from S(=O)ᵤ(=NR¹⁴)ᵥ. each ring or ring system substituted or unsubstituted with up to 5 substituents independently selected from R¹⁰ on carbon atom ring members and selected from R¹² on nitrogen atom ring members; or
Q² is a phenyl ring or a naphthalenyl ring system, each ring or ring system substituted or unsubstituted with up to 5 substituents independently selected from R¹⁰; or a 4- to 7-membered heterocyclic ring; or an 8- to 10-membered bicyclic ring system, each ring or ring system containing ring members selected from carbon atoms and 1 to 4 heteroatoms independently selected from up to 2 O, up to 2 S and up to 5 N atoms, wherein up to 3 carbon ring members are independently selected from C(=O) and C(=S), and the sulfur atom ring members are independently selected from S(=O)ᵤ(=NR¹⁴)ᵥ, each ring or ring system substituted or unsubstituted with up to 5 substituents independently selected from R¹¹ on carbon atom ring members and selected from R¹³ on nitrogen atom ring members; or
J is -CR²R³-, -CR²R³-CR⁴R⁵-, -NR⁶- or -O-;
Y¹ and Y² are each independently O, S or NR¹⁵;
R¹ is H, hydroxy, amino, cyano, formyl, C₃-C₈ alkylcarbonylalkyl, -C(C₁-C₄ alkyl)=N-O(C₁-C₄ alkyl), -C(O)NH₂, C₁-C₆ alkyl, C₁-C₆ haloalkyl, C₂-C₆ alkenyl, C₃-C₆ alkynyl, C₂-C₆ cyanoalkyl, C₃-C₆ cycloalkyl, C₃-C₈ cycloalkenyl, C₄-C₈ cycloalkylalkyl, C₂-C₈ alkoxyalkyl, C₃-C₈ alkoxyalkoxyalkyl, C₂-C₈ haloalkoxyalkyl, C₂-C₈ haloalkenylalkyl, C₂-C₈ alkylthioalkyl, C₂-C₈ alkylsulfinylalkyl, C₂-C₈ alkylsulfonylalkyl, C₂-C₈ alkylcarbonyl, C₂-C₈ haloalkylcarbonyl, C₄-C₁₀ cycloalkylcarbonyl, C₅-C₁₀ cycloalkylcarbonylalkyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylarninocarbonyl, C₄-C₁₀ cycloalkylaminocarbonyl, C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkylsulfinyl, C₃-C₈ cycloalkylsullinyl, C₁-C₆ alkylsulfonyl, C₁-C₆ haloalkylsulfonyl, C₃-C₈ cycloalkylsulfonyl, C₁-C₆ alkylaminosuifonyl C₂-C₈ dialkylaminosulfonyl, C₃-C₁₀ trialkylsilyl; or -CPh=N-O(C₁-C₄ alkyl), each substituted or unsubstituted on ring members with up to 5 substituents independently selected from R¹³; or G¹;
R² and R³ are each independently H, halogen, hydroxy, C₁-C₄ alkyl, C₁-C₄ haloalkyl or C₁-C₄ alkoxy; or
R² and R³ are taken together with the carbon atom to which they are bonded to form a C₃-C₇ cycloalkyl ring;
R¹ and R⁵ are each independently H, halogen, hydroxy, C₁-C₄ alkyl, C₁-C₄ haloalkyl or C₁-C₄ alkoxy;
R⁶ is C₁-C₆ alkyl, C₂-C₆ alkenyl, C₃-C₆ alkynyl or C₁-C₆ alkoxy; or
R¹ and R⁶ are taken together as C₃-C₆ alkylene or -CH₂OCH₂-;
R⁷ is H, halogen, hydroxy, C₁-C₄ alkoxy, C₁-C₄ haloalkyl or C₁-C₄ alkyl;
each R⁸ is independently cyano, hydroxy, amino, nitro, -CHO, -C(=O)OH, -C(=O)NH₂, -SO₂NH₂, C₂-C₆ alkenyl, C₂₋C₆ alkynyl, C₂-C₈ alkykarbonyl, C₂-C₈ haloalkylcarbonyl, C₂-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylarninocarbonyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alklsulfonyl, C₁-C₆ haloalkylsulfonyl, C₁-C₆ alkylaminosulfonyl, C₂-C₈ dialkylaminosulfonyl C₃-C₁₀ trialkylsilyl, C₃-C₆ alkylamino, C₂-C₈ dialkyl amino, C₂-C₈ alkylcarbonylamino or C₁-C₆ alkylsulfonylamino;
R⁹ is H, hydroxy, amino, C₁-C₆ alkyl, C₁-C₆ haloalkyl, C₂-C₆, alkenyl, C₃-C₆ alkynyl, C₂-C₈ alkoxyalkyl, C₂-C₈ haloalkoxyalkyl, C₂-C₈ alkykhioalkyl, C₂-C₈ alkylsulfinylalkyl, C₂-C₈ alkylsulfonylalkyl, C₂-C₈ alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl. C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkylsulfinyl, C₃-C₈ cycloalkykulfinyl. C₁-C₆ alkylsulfonyl, C₁-C₆ haloalkylsulfooyl, C₃-C₈ cycloalkylsulfonyl, C₁-C₆ alkylaminosulfonyl, C₂-C₈ dialkylaminosulfonyl or C₃-C₁₀ trialkylsilyl or G¹;
each R¹⁰ and R¹¹ is independently halogen, hydroxy, cyano, nitro, amino, C₁-C₈ alkyl, C₁-C₈ cyanoalkyl, C₁-C₈ cyanoalkoxy, C₁-C₈ haloalkyl, C₁-C₈ hydroxyalkyl, C₁-C₈ nitroalkyl, C₂-C₈ alkenyl, C₁-C₈ haloalkenyl, C₂-C₈, nitroalkenyl, C₂-C₈ alkynyl, C₂-C₈ haloalkynyl, C₂-C₈ alkoxyalkyl, C₃-C₈ alkoxyalkoxyalky], C₂-C₈ haloalkoxyalkyl, C₂-C₈ haloalkoxyhaloalkoxy, C₃-C₆, cycloalkyl, cyclopropylmethyl, 1-methylcyclopropyl, 2-methylcyclopropyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂ cycloalkylalkynyl, C₃-C₈ cycloalkyl, C₃-C₈ halocycloalkyl, C₄-C₁₀ alkylcycloalkyl, C₆-C₁₂ cycloalkylcycloalkyl, C₃-C₈ cycloalkenyl, C₃-C₈ halocycloalkenyl, C₂-C₈ haloalkoxyalkoxy, C₂-C₈ alkoxyalkoxy. C₄-C₁₀ cycloalkoxyalkyl, C₂-C₈ alkylthioalkyl, C₂-C₈ alkylsulfinylalkyl, C₂-C₈ alkylsulfonylalkyl, C₂-C₈ alkylamino, C₂-C₈ dialkylamino, C₂-C₈ halodialkylamino, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl, C₂-C₈ haloalkylcarbonyl, C₄-C₁₀ cycloalkylcarbonyl, -C(=O)OH, C₂-C₈ alkoxycarbonyl, C₂-C₈ haloalkoxycarbonyl, C₄-C₁₀ cycloalkoxycarbonyl, C₅-C₁₂ cycloalkylalkoxycarbonyl, -C(=O)NH₂, C₂-C₈ alkylaminocarbonyl, C₄-C₁₀ cycloalkylaminocabonyl, C₃-C₁₀ dialkylaminocarbonyl, C₁-C₈ alkoxy, C₁-C₈ haloalkoxy, C₂-C₈ alkenyloxy, C₂-C₈ haloalkenyloxy, C₃-C₈ alkynyloxy, C₃-C₈ haloalkynyloxy, C₃-C₈, cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈ haloalkylsulfonyloxy, C₂-C₈ alkylthio. C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₂-C₈ cycloalkylsulfonyl, formylamino, C₂-C₈ alkylcarbonylamino, C₂-C₈ haloalkylcarbonylamino, C₃-C₈ cycloalkylamino, C₂-C₈ alkoxycarbonylamino, C₁-C₆ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl or C₄-C₁₂ trialkylsilylalkoxy; or G²; or R²⁰S(=O)=N- R²⁰S(=O)₂NR¹⁹-C(=O)- or R²⁰(R¹⁹N= )_{q}S(=O)ₚ-, wherein the free bond projecting to the right indicates the connecting point to Q¹; or
each R¹² and R¹³ is independently cyano, C₁-C₃ alkyl, C₁-C₈ hydroxyalkyl, C₂-C₃ alkenyl, C₂--C₃ alkynyl, C₃-C₆ cycloalkyl, C₂-C₃ alkoxyalkyl, C₁-C₃ alkoxy, C₂-C₃ alkylcarbonyl, C₂-C₃ alkoxycarbonyl, C₂-C₃ alkylaminoalkyl or C₃-C₄ dialkylaminoalkyl;
each R¹⁴ is independently H, cyano, C₂-C₃ alkylcarbonyl or C₂-C₃ haloalkylcarbonyl;
each R¹⁵ is independently H, cyano, hydroxy, CHO, C₁-C₄ alkyl, C₁-C₄ haloalkyl, C₁-C₄ alkoxy, C₂-C₆ alkylcarbonyl, C₂-C₆ haloalkylcarbonyl, -(C=O)CH₃ or -(C=O)CF₃;
each G¹ is independently phenyl; or a 5- or 6-membered heterocyclic ring, each substituted or unsubstituted on ring members with up to 5 substituents independently selected from R¹⁷;
each W^{E}, W^{F} and W^{G} is independently -C(=O)-, -C(=O)O-, -C(=O)NH- or - S(=O)₂-;
each G^{E}, G^{F} and G^{G} is independently phenyl substituted or unsubstituted with R¹⁶; or a 5- or 6-membered heterocyclic ring, each heterocyclic ring substituted or unsubstituted on ring members with up to 5 substituents independently selected from R¹⁶;
each G² is independently phenyl, phenylmethyl, pyridinylmethyl, phenylcarbonyl, phenoxy, phenylethynyl, phenylsulfonyl or a 5- or 6-membered heterocyclic ring, each substituted or unsubstituted on ring members with up to 5 substituents independently selected from R¹⁸;
each R¹⁶, R¹⁷ and R¹⁸ is independently halogen, cyano, hydroxy, amino, nitro, -CHO, -C(=O)OH, -C(=O)NH₂, SO₂NH₂, C₁-C₆ alkyl, C₁-C₆ haloalkyl, C₂-C₆ alkenyl, C₂-C₆ alkynyl, C₂-C₈ alkylcarbonyl, C₂-C₈ haloalkylcarbonyl, C₁-C₈ alkoxycarbonyl, C₄-C₁₀ cycloalkoxycarbonyl, C₅-C₁₂ cycloalkylalkoxycarbonyl, C₂-C₈ alkylaminocarbonyl, C₃-C₁₀ dialkylaminocarbonyl, C₁-C₆ alkoxy, C₁-C₆ haloalkoxy, C₂-C₈ alkylcarbonyloxy, C₁-C₆, alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ allylaminosulfonyl, C₁-C₈ dialkylaminosulfonyl, C₃-C₁₀ trialkylsilyl, C₁-C₆ alkylamino. C₂-C₈ dialkylamino, C₂-C₈ alkylcarbonylamino, C₁-C₆ alkylsulfonylamino, phenyl, pyridinyl or thienyl;
each R¹⁹ is independently H, cyano, C₂-C₃ alkylcarbonyl or C₂-C₃ haloalkylcarbonyl;
each R²⁰ is independently H, C₁-C₆ alkyl, C₃-C₈ cycloalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₁-C₆ alkoxy or C₃-C₁₀ trialkylsilyl; or G¹;
each u and v are independently 0, 1 or 2 in each instance of S(=O)ᵤ(=NR¹⁴)ᵥ, provided that the sum of u and v is 0, 1 or 2; and
each p and q are independently 0, 1 or 2 in each instance or R²⁰(R¹⁹N=)_{q}S(=O)ₚ-, provided that the sum or u and v is 0, 1 or 2 and when p is 0, q is other than 1 or 2.

### Embodiment A41a

A Saturated Target of the following formula, N-oxides, and salts thereof as defined in Claim 1 of PCT/US2018/035017 published as WO 2018/222647, 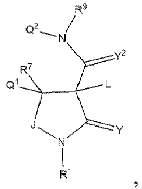 in combination with at least one Unsaturated Precursor, each defined by any one of the following structures, 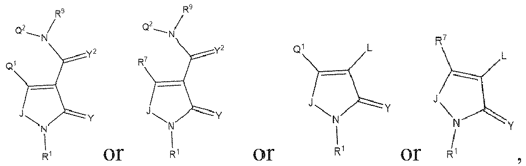 wherein Q¹, Q², R¹, R⁷, R⁹, J, L, Y and Y² etc. have the meanings as defined in Claim 1 of PCT/US2018/035017 published as WO 2018/222647. The text from claim 1 on page 84, line 5 until page 89 line 27 of WO2018222647 being specifically incorporated by reference solely for the purpose of defining the structures of this embodiment, the text being reproduced in Embodiment **A41.**

### Embodiment A42

An Unsaturated Precursor defined by any one of the formulae, 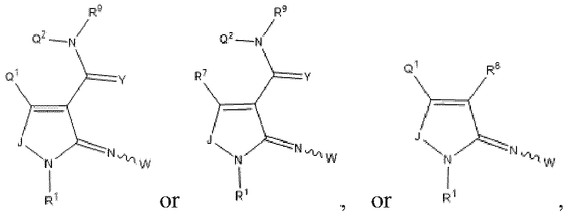 wherein Q¹, Q², R¹, R⁷, R⁸, R⁹, J, Y and W, etc. have the meanings as defined in Claim 1 of PCT/US2018/035015 published as WO 2018/222646. The text from claim 1 on page 82, line 6 until page 86 line 21 of WO 2018/222646 being specifically incorporated by reference solely for the purpose of defining the structures of this embodiment.
W is -NR^{A}R^{B} or -OR^{C};
R^{A} is H, cyano, CHO, C₂-C₄ alkylcarbonyl, C₁-C₄ alkyl; or phenyl substituted or unsubstituted with halogen or C₁-C₄ alkyl;
R^{B} is H, cyano, hydroxy, CHO, C₁-C₆ alkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₂-C₈ alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxyearbonyl, C₂-C₈ alkylaminocarbonyl, C₃-C₁₀ dialkylaminocarhonyl, C₄-C₁₀ cycloalkylaminocarbonyl, C₁-C₆ allkoxy, C₁-C₆ alkylthio, C₁-C₆ haloalkylthio, C₃-C₈ cycloalkylthio, C₁-C₆ alkylsulfinyl, C₁-C₆ haltoalkylsulfinyl, C₃-C₈ cycloalkylsidfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl or C₃-C₁₀ triallkylsilyl; or a phenyl ring or a naphthalenyl ring system, each ring or ring system substituted or unsubsliluted with up to 5 substituents independently selected from R¹⁶; or a 4- to 7-membered heterocyclic ring, substituted or unsubsliluted on ring members with up to 5 substituents independently selected from R¹⁶; or
R^{A} and R^{B} are taken together along with the nitrogen atom to which they are both bonded to form a 4-, 5- or 6-membered ring containing ring members selected from carbon, oxygen, nitrogen and -C(=O:)-; or taken together as a 6. to 10-membered bicyclic ring system; or taken together as an 8- to 13-membered tricyclic ring system, each ring or ring system containing ring members selected from carbon, nitrogen and -C(=O)- and substituted or unsubstituted with halogen, cyano or C₁-C₄ alkyl,
R^{C} is C₁-C₄ haloalkyl, C₂-C₄ haloalkylearbonyl, C₃-C₆ cycloalkyl; or phenyl substituted or unsubstituted with halogen, cyano or C₁-C₄ alkyl; or a 6-membered nitrogen containing aromatic ring substituted or unsubstituted with halogen, cyano or C₁-C₄ alkyl;
J is -CR²R³-, -CR²R³-CR⁴R⁵-, -NR⁶- or -O-;
Y is O, S or NR¹⁵;
R¹ is H, hydroxy, amino, cyano, CHO, C₃-C₈ alkylcarbonylalkyl, -C(C₁-C₄ alkyl)=NO(C₁-C₄ alkyl), -C(O)NH₂, C₁-C₆ alkyl, C₁-C₆ haloalkyl, C₂-C₆ alkenyl, C₃-C₆, alkynyl, C₂-C₆ cyanoalkyl, C₃-C₆ cycloalkyl, C₃-C₈ cycloalkenyl, C₄-C₈ cycloalkylalkyl, C₂-C₈ alkoxyalkyl, C₂-C₈ alkoxyalkoxyalkyl, C₂-C₈ haloalkoxyalkyl, C₂-C₈ haloalkenylalkyl, C₂-C₈ alkylthioalkyl, C₂-C₈ alkylsulfinylalkyl, C₂-C₈ alkylsulfonylalkyl, C₂-C₈ alkylcarbonyl, C₂-C₈ haloalkylearbonyl, C₄-C₁₀ cycloalkylcarbonyl, C₅-C₁₀ cycloalkylcarbonylalkyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ alkylthio, C₁-C₆ haloalkylthio, C₃-C₈ cycloalkylthio, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₃-C₈ cycloalkylsulfonyl, C₁-C₆ alkylaminosulfonyl, C₂-C₈ dialkylaminosulfonyl, C₃-C₁₀ trialkylsilyl; or -CPh=N-O(C₁-C₄ alkyl) where the phenyl is substituted or unsubstituted with up to 5 substituents independently selected from R¹³, or G¹;
Q¹ is a phenyl ring or a naphthalenyl ring system, each ring or ring system substituted or unsubstituted with up to 5 substituents independently selected from R¹⁰; or a 4- to 7-membered heterocyclic ring; or an 8- to 10-membered bicyclic ring system, each ring or ring system containing ring members selected from carbon atoms and 1 to 5 heteroatoms independently selected from up to 2 O, up to 2 S and up to 5 N atoms, wherein up to 3 carbon ring members are independently selected from C(=O) and C(=S), and the sulfur atom ring members are independently selected from S(=O)ᵤ(=NR¹⁴)ᵥ, each ring or ring system substituted or unsubstituted with up to 5 substituents independently selected from R¹⁰ on carbon atom ring members and selected from R¹² on nitrogen atom ring members;
Q² is a phenyl ring or a naphthalenyl ring system, each ring or ring system substituted or unsubstituted with up to 5 substituents independently selected from R¹¹; or a 4- to 7-membered heterocyclic ring, or an 8- to 10-membered bicyclic ring system, each ring or ring system containing ring members selected from carbon atoms and 1 to 4 heteroatoms independently selected from up to 2 t7, up to 2 S
   and up to 5 N atoms, wherein up to 3 carbon ring members are independently selected from C(=O) and C(=S), and the sulfur atom ring members are independently selected from S(=O)ᵤ(=NR¹⁴)ᵥ, each ring or ring system substituted or unsubstituted with up to 5 substituents independently selected from R¹¹ on carbon atom ring members and selected from R¹³ on nitrogen atom ring members;
R² and R³ arc each independently H. halogen, hydroxy, C₁-C₄ alkyl, C₁-C₄ haloalkyl or C₁-C₄. alkoxy; of
R² and R³ are taken together with the carbon atom to which they are bonded to form a C₃-C₇ cycloalkyl ring;
R⁴ and R⁵ are each independently H, halogen, hydroxy, C₁-C₄ alkyl, C₁-C₄ haloalkyl or C₁-C₄ alkoxy;
R⁶ is C₁-C₆ alkyl, C₂-C₈ alkenyl, C₁-C₆ alkynyl or C₁-C₆ alkoxy; or
R¹ and R⁶ are taken together as C₃-C₆ alkylene or -CH₂-OCH₂-;
R⁷ and R⁸ are each independently H, halogen, hydroxy, C₁-C₄ alkoxy, C₁-C₄ haloalkyl or C₁-C₄ alkyl;
R⁹ is H, hydroxy, amino, C₁-C₆ alkyl, C₁-C₆ haloalkyl, C₁-C₆ alkenyl, C₁-C₆ alkynyl, C₂-C₈ alkoxyalkyl, C₂-C₈ haloalkoxyalkyl, C₂-C₈ alkyllhioalkyl, C₂-C₈ alkylsulfinylalkyl, C₂-C₈ alkylsulfonylalkyl, C₂-C₈ alkylcarbonyl, C₂-C₈ haloalkylcarbonyl, C₄-C₁₀ cycloalkylcarbonyl, C₂-C₈ alkoxycarbonyl, C₂-C₈ haloalkoxycarbonyl, C₄-C₁₀ cycloalkoxycarbonyl, C₂-C₈ alkylaminocarbonyl, C₃-C₁₀ dialkylaminocarbonyl, C₄-C₁₀ cycloalkylaminocarbonyl, C₁-C₆ alkoxy, C₁-C₆ alkylthio, C₁-C₆ haloalkylthio, C₂-C₈ cycloalkylthio, C₁-C₆ alkylsulfinyl, C₁-C₆ haloalkylsulfinyl, C₃-C₈ cycloalkylsulfinyl, C₁-C₆ alkylsulfonyl, C₁-C₆ haloalkylsulfonyl, C₂-C₈ cycloalkylsulfonyl, C₁-C₆ alkylaminosulfonyl, C₂-C₈ dialkylaminosulfonyl or C₃-C₁₀ trialkylsilyl; or G¹;
each R¹⁰ and R¹¹ is independently halogen, hydroxy, cyano, nitro, amino, C₁-C₈ alkyl, C₁-C₈ cyanoalkyl, C₁-C₈ cyanoalkoxy, C₁-C₈ haloalkyl, C₁-C₈ hydroxyalkyl, C₁-C₈ nitroalkyl, C₂-C₈ alkenyl, C₂-C₈ haloalkenyl, C₂-C₈ nitroalkenyl, C₂-C₈ alkynyl. C₂-C₈ haloalkynyl, C₂-C₈ alkoxyalkyl, C₃-C₈ alkoxyalkoxyalkyl, C₂-C₈ haloalkoxyalkyl, C₂-C₈ haloalkoxyhaloalkoxy, C₃-C₆ cycloalkyl, cyclopropylmethyl, l-methylcyclopropyl, 2-methylcyclopropyl, C₄-C₁₀ cycloalkylalkyl, C₄-C₁₀ halocycloalkylalkyl, C₅-C₁₂ alkylcycloalkylalkyl, C₅-C₁₂ cycloalkylalkenyl, C₅-C₁₂ cycloalkylalkynyl, C₃-C₈ cycloalkyl, C₃-C₈ halocycloalkyl, C₄-C₁₀ alkylcycloalkyl, C₆-C-₁₂ cycloalkylcycloalkyl, C₃-C₈ cycloalkenyl, C₃-C₈ halocycloalkenyl, C₂-C₈ haloalkoxyalkoxy, C₂-C₈ alkoxyalkoxy, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylamino, C₂₋C₈ dialkylamino, C₂-C₈ halodialkylamino, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂C₈ alkylcarbonyl, C₂-C₈ haloalkylcarbonyl, C₄-C₁₀ cycloalkylcarbonyl, -C(=O)OH, C₂-C₈ alkoxycarbonyl, C₂-C₈ haloalkoxycarbonyl, C₄-C₁₀ cycloalkoxycarbonyl, C₅-C₁₂ cycloalkylalkoxycarbonyl, -C(=O)NH₂, C₂-C₈ alkylaminocarbonyl, C₄-C₁₀ cycloalkylaminocarbonyl, C₃-C₁₀ dialkylaminocarbonyl, C₁-C₈ alkoxy, C₁-C₈ haloalkoxy, C₂-C₈ alkoxyalkoxy, C₂-C₈ alkenyloxy, C₂-C₈ haloalkenyloxy, C₃-C₈ alkynyloxy, C₃-C₈ haloalkynyloxy, C₃-C₈ cycloalkoxy, C₃-C₈ halocycloalkoxy, C₄-C₁₀ cycloalkylalkoxy, C₃-C₁₀ alkylcarbonylalkoxy, C₂-C₈ alkylcarbonyloxy, C₂-C₈ haloalkylcarbonyloxy, C₄-C₁₀ cycloalkylcarbonyloxy, C₁-C₈ alkylsulfonyloxy, C₁-C₈ haloalkylsulfonyloxy, C₁-C₈ alkylthio, C₁-C₈ haloalkylthio, C₃-C₈ cycloalkylthio, C₁-C₈ alkylsulfinyl, C₁-C₈ haloalkylsulfinyl, C₁-C₈ alkylsulfonyl, C₁-C₈ haloalkylsulfonyl, C₃-C₈ cycloalkylsulfonyl, formylamino, C₂-C₈ alkylcarbonylamino, C₂-C₈ haloalkylcarbonylamino, C₃-C₈ cycloalkylamino, C₂-C₈ alkoxycarbonylamino, C₁-C₆ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl or C₄-C₁₂ trialkylsilylalkoxy; or G²; or R²⁰S(=O)=N-, R²⁰S(=O₂)NR¹⁹-C(=O)- or R²⁰(R¹⁹N=)_{q}S(=O)ₚ-, wherein the free bond projecting to the right indicates the connecting point to Q¹;
each R¹² and R¹³ is independently cyano, C₁-C₃ alkyl, C₁-C₈ hydroxyalkyl, C₂-C₃ alkenyl, C₂-C₃ alkynyl. C₃-C₆ cycloalkyl, C₂-C₃ alkoxyalkyl, C₁-C₃ alkoxy, C₂₋C₃ alkylcarbonyl, C₂₋C₃ alkoxycarbonyl, C₂-C₃ alkylaminoalkyl or C₃-C₄ dialkylaminoalkyl;
each R¹⁴ is independently H, cyano, C₂-C₃ alkylcarbonyl or C₂-C₃ haloalkylcarbonyl;
R¹⁵ is H, cyano, hydroxy, CHO, C₁-C₄ alkyl, C₁-C₄ haloalkyl, C₁-C₄ alkoxy, C₂-C₆ alkylcarbonyl or C₂-C₆ haloalkylcarbonyl;
each G¹ is independently phenyl; or a 5- or 6-membered heterocyclic ring, each substituted or unsubstituted on ring members with up to 5 substituents independently selected from R¹⁷;
each G² is independently phenyl, phenylmethyl, pyridinylmethyl, phenylcarhonyl, phenoxy, phenylethynyl, phenylsulfonyl or a 5- or 6-membered heterocyclic ring, each substituted or unsubstituted on ring members with up to 5 substituents independently selected from R¹⁸;
each R¹⁶, R¹⁷ and R¹⁸ is independently halogen, cyano, hydroxy, amino, nitro, -CHO, -C(=O)OH, -C(=O)NH₂, -SO₂NH₂, C₁-C₆ alkyl, C₁-C₆ haloalkyl, C₂-C₆ alkenyl, C₂-C₆ alkynyl, C₂-C₈ alkylcarbonyl, C₁-C₈ hydroxyalkyl, C₂-C₈ haloalkylcarbonyl, C₂-C₆ alkoxyalkyl, C₂-C₆ alkylaminoalkyl, C₂-C₈ alkoxycarbonyl, C₃-C₈ cycloalkyl, C₄-C₁₀ cycloalkoxycarbonyl, C₅-C₁₂ cycloalkylalkoxycarbonynyl, C₂-C₈ alkylaminocarbonyl, C₃-C₁₀ dialkylaminocarbonyl, C₃-C₈ dialkylaminoalkyl, C₁-C₆ alkoxy, C₁-C₆ haloalkoxy, C₂-C₈ alkylcarbonyloxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₁-C₆ alkylaminosulfonyl, C₂-C₈ dialkylaminosulfonyl, C₃-C₁₀ trialkylsilyl, C₁-C₆ alkylamino, C₂-C₈ dialkylamino, C₂-C₈ alkylcarbonylamino, C₁-C₆ alkylsulfonylamino, phenyl, pyridinyl or thienyl;
each R¹⁹ is independently H, cyano, C₂-C₃ alkylcarbonyl or C₂-C₃ haloalkylcarbonyl;
each R²⁰ is independently H, C₁-C₆ alkyl, C₃-C₈ cycloalkyl, C₄-C₈ cycloalkylalkyl, C₁-C₆ haloalkyl, C₂-C₆ alkenyl, C₃-C₆ alkynyl, C₂-C₈ alkoxyalkyl, C₂-C₈ haloalkoxyalkyl, C₂-C₈ alkylthioalkyl, C₂-C₈ alkylsulfinylalkyl, C₂-C₈ alkylsulfonylalkyl, C₁-C₆ alkoxy, C₃₋C₁₀ trialkylsilyl or G¹;
each u and v are independently 0, 1 or 2, provided that the sum of u and v is 0, 1 or 2; and
each p and q are independently 0, 1 or 2, provided that the sum of u and v is 0, 1 or 2 and when p is 0, q is other than 1, or 2.

### Embodiment A42a

A Saturated Target, N-oxides salts and stereoisomers thereof, wherein the Saturated Target is a compound as defined in Claim 1 of PCT/US2018/035015 published as WO2018/222646 published as WO2018222646, of the following formula, 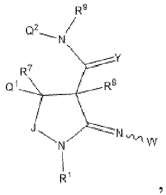 in combination with at least one Saturated Precursor each of which defined by any one of the following structures,

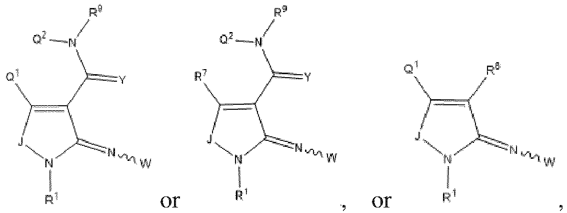

Wherein Q¹, Q², R¹, R⁷, R⁸, R⁹, J, Y and W, etc. have the meanings as defined in Claim 1 of PCT/US2018/035015 published as WO 2018/222646. The text from claim 1 on page 82, line 6 until page 86 line 21 of WO2018/222646 being specifically incorporated herein by reference solely for the purpose of defining the structures of this embodiment, the text being reproduced in Embodiment **A42.**

### Embodiment A43

An unsaturated Precursor defined by any one of the following formulae, 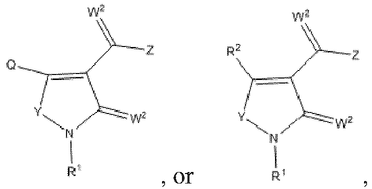 wherein Q,W², R¹, R², Y and Z etc. have the meanings as defined in Claim 1 of PCT/EP2018/057628 published as WO 2018/184890. The German language text from claim 1 on page 133, line 9 until page 136 line 4 of WO 2018/184890 being specifically incorporated herein by reference solely for the purpose of defining the structures of this embodiment.

### Embodiment A43a

A Saturated Target of the following formula and salts thereof, wherein the Saturated Target is a compound as defined in Claim 1 of PCT/EP2018/057628 published as WO2018/184890 of the following formula, 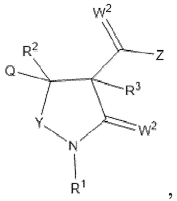 in combination with at least one Unsaturated Precursor, each defined by either one of the structures, 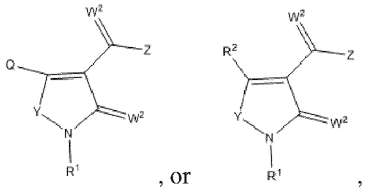 wherein Q,W², R¹, R², Y and Z etc. have the meanings as defined in Claim 1 of PCT/EP2018/057628 published as WO2018/184890. The German language text from claim 1 on page 133, line 9 until page 136 line 4 of WO2018184890 being specifically incorporated herein by reference solely for the purpose of defining the structures of this embodiment.

### Embodiment A44

The Unsaturated Precursor of any one of Embodiments **A1-A16, A31-A34** and e.g. salts thereof wherein,
**R^{α}** is -C(**Q^{α}**)-**CHA**, and
**CHA** is a chiral, carbon-containing group, exemplified by, substituted or unsubstituted, cyclic or non-cyclic, amino alcohol, 2-Oxazolidone, alcohol, amine, sulfinamide sugar, amino acid derivatives (including amino acid), alkyl, aryl, heterocyclic, naphthyl, ester including aliphatic and aromatic esters exemplified by (S)-1-(2-fluorophenyl)ethan-1-amine, (S)-1-phenylethan-1-ol, and wherein,
**R^{β}**, **Q^{α}, Q^{β}, A^{β}, A^{α}**, **R^{µ}** and **nα**, have the same meanings as defined in Embodiments **A1-A16, A31-A34.**

### Embodiment A45

The Unsaturated Precursor of Embodiment **A2** and e.g. salts thereof wherein,
**R^{α}** is -C(**Q^{α}**)-**CHA**, and 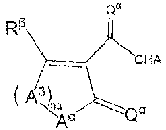
**CHA** is a chiral, carbon-containing group exemplified by, a substituted or unsubstituted, cyclic or non-cyclic, amino alcohol, 2-Oxazolidone, alcohol, amine, sulfinamide sugar, alkyl, aryl, heterocyclic, naphthyl, ester including aliphatic and aromatic esters , and wherein,
**R^{β}**, **Q^{α}, Q^{β}, A^{β}, A^{α}**, **R^{µ}** and **nα**, have the same meanings as defined in Embodiment **A2.**

### Embodiment A46

The Unsaturated Precursor of Embodiment **A2** and e.g. salts thereof wherein,
**R^{α}** is -C(**Q^{α}**)-**CHA**

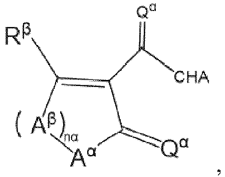

CHA are chiral auxiliaries as mentioned in: *Chirality.* 2019;1-37 and referenced within Key Chiral Auxiliary Applications (Second Edition)(ed.: Roos, G.), Academic Press, Boston, 2014 ISBN 978-0-12-417034-6*,* and those referenced within:,
Glorius, F.; Gnas, Y. (2006). "Chiral Auxiliaries - Principles and Recent Applications". Synthesis. 2006 (12): 1899-1930*,* and those referenced within Jamali, Fakhreddin (1993). "Chapter 14: Stereochemically Pure Drugs: An Overview". In Wainer, Irving W. (ed.). Drug Stereochemistry: Analytical Methods and Pharmacology. Marcel Dekker, Inc. pp. 375-382*,* and those referenced within*:*
Evans, D. A.; Helmchen, G.; Rüping, M. (2007). "Chiral Auxiliaries in Asymmetric Synthesis". In Christmann, M (ed.). Asymmetric Synthesis - The Essentials. Wiley-VCH Verlag GmbH & Co. pp. 3-9*,* and those referenced within:
J. Am. Chem. Soc. 97 (23): 6908-6909*,* and wherein,
**R^{β}**, **Q^{α}, Q^{β}, A^{β}, A^{α}**, **R^{µ}** and **nα,** have the same meanings as defined in Embodiment **A2.**

### Embodiment A46a

The Unsaturated Precursor of Embodiment A2 and e.g. salts thereof wherein,
**R^{α}** is -C(**Q^{α}**)-**CHA** 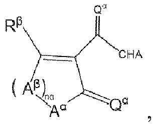 wherein,
**CHA** is any of, (R)-BINOL, (S)-BINOL, trans-2-Phenylcyclohexanol, 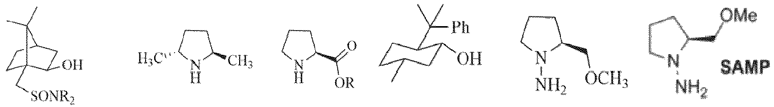 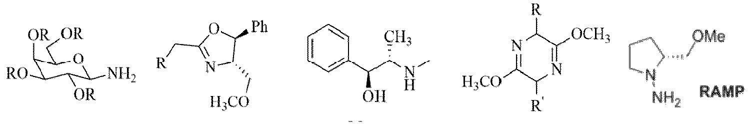 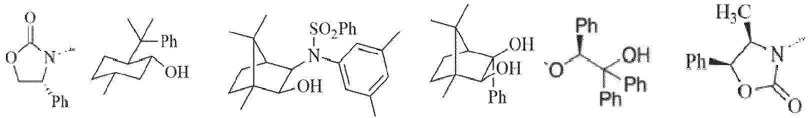 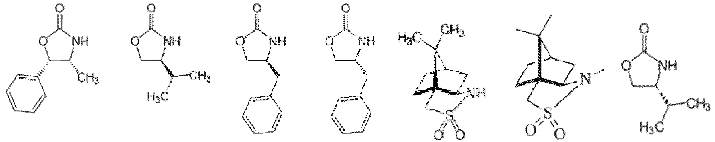 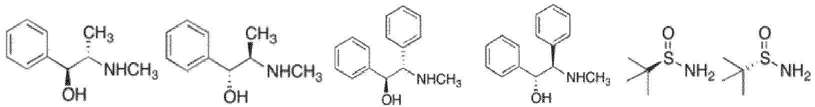 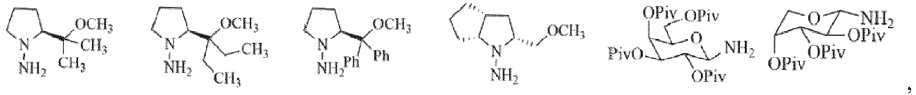
and wherein,
R = **R^{ζ}**, and wherein,
**R^{β}**, **Q^{α}, Q^{β}, A^{β}, A^{α}**, **R^{µ} , R^{ζ}** and **nα**, have the same meanings as defined in Embodiment A2.

### Embodiment A47

The Unsaturated Precursor of any one of Embodiments **A1-A16, A31-A34** and e.g. salts thereof wherein,
**R^{β}**, **Q^{α}**, **Q^{β}, A^{β}, A^{α}**, **R^{µ}** and **nα**, have the same meanings as defined in Embodiment **A2,** or the same meanings as defined in any one of Embodiments **A1-A16, A31-A34,** and wherein,
**R^{α}** is -C(**Q^{α}**)-**CHA**, wherein, CHA are chiral auxiliaries as mentioned in:
   Chirality. 2019;1-37 and referenced within Key Chiral Auxiliary Applications (Second Edition)(ed.: Roos, G.), Academic Press, Boston, 2014 ISBN 978-0-12-417034-6*,* and those referenced within:
   Glorius, F.; Gnas, Y. (2006). "Chiral Auxiliaries - Principles and Recent Applications". Synthesis. 2006 (12): 1899-1930*,* and those referenced within Jamali, Fakhreddin (1993). "Chapter 14: Stereochemically Pure Drugs: An Overview". In Wainer, Irving W. (ed.). Drug Stereochemistry: Analytical Methods and Pharmacology. Marcel Dekker, Inc. pp. 375-382*,* and
   those referenced within:
      Evans, D. A.; Helmchen, G.; Rüping, M. (2007). "Chiral Auxiliaries in Asymmetric Synthesis". In Christmann, M (ed.). Asymmetric Synthesis - The Essentials. Wiley-VCH Verlag GmbH & Co. pp. 3-9*,*
      and those referenced within:
         J. Am. Chem. Soc. 97 (23): 6908-6909*,* and

### Embodiment A47a

The Unsaturated Precursor of any one of Embodiments **A1-A16, A31-A34** and e.g. salts thereof wherein,
**R^{α}** is -C(**Q^{α}**)-**CHA**, and
**CHA** is any of, (R)-BINOL, (S)-BINOL, trans-2-Phenylcyclohexanol, 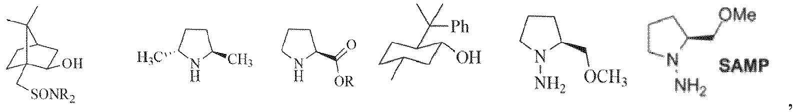 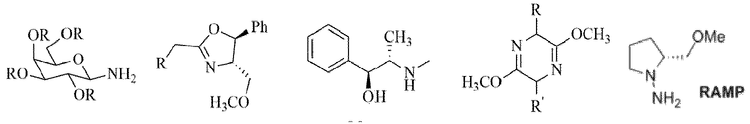 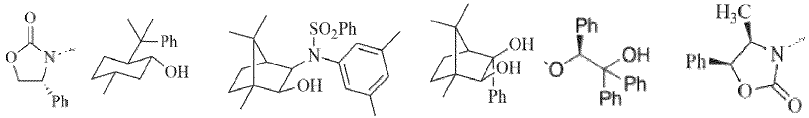 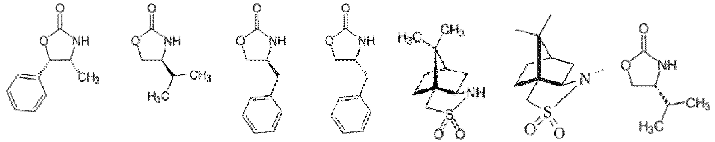 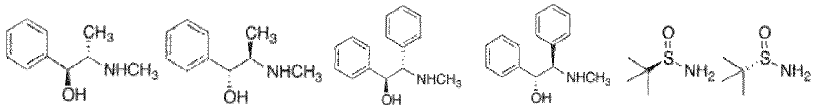 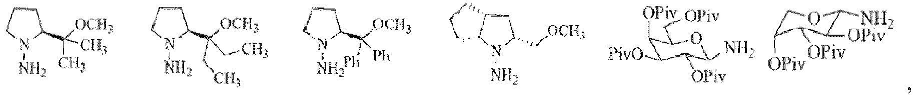
and wherein, **R** is **R^{ζ}**, and wherein,
**R^{β}**, **Q^{α}, Q^{β}, A^{β}, A^{α}**, **R^{µ}**, **R^{ζ}** and **nα**, have the same meanings as defined in any one of Embodiments
**A1-A16, A31-A34.**

### Embodiment A48

The Unsaturated Precursor of Embodiment **A2** and e.g. salts thereof wherein,
**R^{α}** is -C(**Q^{α}**)-**CHA** 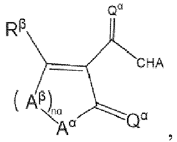 wherein
**CHA** is a chiral **Q^{β}** (**R^{θ}**)₃, **Q^{β}** H(**R^{θ}**)₂, or **Q^{β}** H₂**R^{θ}**, and wherein,
**R^{β}**, **Q^{α}, Q^{β}, A^{β}, A^{α}**, **R^{µ}** and **nα**, have the same meanings as defined in Embodiment **A2.**

### Embodiment A49

The Unsaturated Precursor of Embodiment **A1-A16, A31-A34** and e.g. salts thereof wherein,
**R^{α}** is -C(**Q^{α}**)-**CHA** 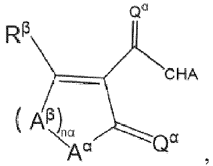 wherein
**CHA** is a chiral **Q^{β}**(**R^{θ}**)₃, **Q^{β}** H(**R^{θ}**)₂, or **Q^{β}** H₂**R^{θ}**, and wherein, **R^{β}**, **Q^{α}**, **Q^{β}, A^{β}, A^{α}**, **R^{µ}** and **nα**, have the same meanings as defined in any one of Embodiments **A1-A16, A31-A34.**

### Embodiment A50

An Unsaturated Precursor which has the following structure,

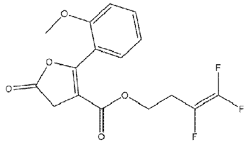

### Embodiment B1

The process as described generally above, for preparing a Saturated Target of formula **1** from an Unsaturated Precursor of formula **2** 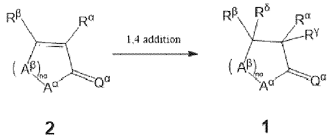 comprising substitution of the 1,4 system exemplified by 1,4-addition using Grignard reagent, organo-lithium reagent, organo-zinc reagent, hydrogenation , hydride source, electron source (reduction, exemplified by Zn/AcOH) or enzyme wherein each of
**R^{β}**, **A^{α}**, **A^{β}, nα, Q^{α}** and **R^{ε},** have the same meanings as defined in embodiment **A2,** and
**R^{α}** is H, halogen or carbon-containing group exemplified by, substituted or unsubstituted, alkyl, aryl, heterocyclic, naphthyl, ester including aliphatic and aromatic esters exemplified by, C(O)OC₆H₅, - C(O)OC₆H₄F, amide including aliphatic and aromatic exemplified by -C(O)NHC₆H₅), hydrazinamide including aliphatic and aromatic exemplified by,
-C(O)NHNHC₆H₅), thioesters, thionoesters and similar compounds exemplified by, -C(O)NHOC₆F₅); and -C(S)SC₆H₅) etc., wherein
**R^{α}** is not connected to the ring via O or N atoms, such that for example, **R^{α}** is not -OMe, and wherein, whenever H is added at the 4 position of the 1,4 system, **R^{α}** is notis not H, and wherein **R^{γ}** is H or carbon-containing group such as substituted or unsubstituted, alkyl, aryl, naphthyl etc. **R^{δ}** is H or carbon-containing group such as substituted or unsubstituted, alkyl, aryl, naphthyl etc.

### Embodiment B1a

A Saturated Target of formula **1** as defined in embodiment **B1** in combination with an amount of the intermediate Unsaturated Precursor as defined in any one of embodiments **A1** to **A52** and **B1**, from which it was produced, as an impurity.

### Embodiment B1b

The process as described generally above, for preparing a Saturated Target of formula 1 from Unsaturated Precursor of formula **2** 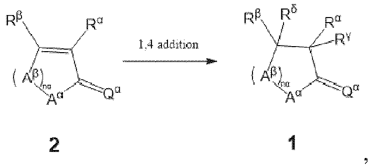 comprising substitution of the 1,4 system exemplified by,, 1,4-addition using Grignard reagent, organo-lithium reagent, organo-zinc reagent, hydrogenation , hydride source, electron source, reduction, exemplified by Zn/AcOH or enzyme, wherein each of
**R^{β}**, **A^{α}**, **A^{β}, nu, Q^{α}** and **R^{ε},** have the same meanings as defined in embodiment **A2,** and wherein
**R^{α}** is H, halogen or carbon-containing group exemplified by, substituted or unsubstituted, alkyl, aryl, heterocyclic, naphthyl, ester including aliphatic and aromatic esters e.g. C(O)OC₆H₅, or - C(O)OC₆H₄F), amide including aliphatic and aromatic e.g. -C(O)NHC₆H₅), hydrazinamide including aliphatic and aromatic e.g. -C(O)NHNHC₆H₅), thioesters, thionoesters and similar compounds such as -C(O)NHOC₆F₅); -C(S)SC₆H₅) etc.,
**R^{γ}** is H or carbon-containing group such as substituted or unsubstituted, alkyl, aryl, naphthyl etc.
**R^{δ}** is H or carbon containing group such as substituted or unsubstituted, alkyl, aryl, naphthyl etc. With the proviso that
**R^{α}** is not connected to the ring via O or N atoms, such that for example, **R^{α}** is not -OMe, and that, whenever H is added at the 4 position of the 1,4 system, **R^{α}** is not H with the exception that when **A^{α}** is N(Me) and **R^{β}** is m-(trifluoromethyl)benzene,
m-(trifluoromethyl)benzene, or 6-(trifluoromethyl)pyridin-3-yl, then **R^{α}** may be H for example in the following scheme:

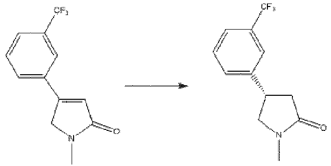

### Embodiment B1c

A process for preparing a Saturated Target from a corresponding Unsaturated Precursor according to any one of processes exemplified by Embodiments **B1** and **B1b** etc., comprising, combining the Unsaturated Precursor, a nucleophile exemplified by Grignard reagent, organo-lithium reagent, organo-zinc reagent, hydrogen source, e.g. H₂/Pd/C or hydride source or electron source exemplified by Zn/AcOH, and at least one solvent to affect a 1,4 system substitution on the Unsaturated Precursor e.g. a 1,4-addition reaction, to obtain the Saturated Target generally defined by the following formula,

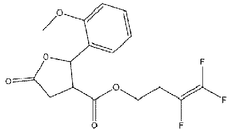

Preferably, depending of the reaction condition one or more of the isomers is in excess, e.g. trans isomers or cis isomers:

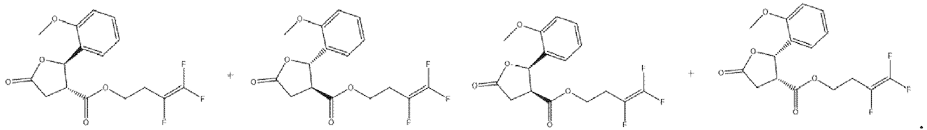

### Embodiment B1d

The process as described generally previously, for preparing a Saturated Target from a corresponding Unsaturated Precursor comprising substitution of the Unsaturated Precursor 1,4 system e.g. 1,4-addition using Grignard reagent, organo-lithium reagent, organo-zinc reagent, hydrogenation or hydride source. In this process, the Saturated Target product obtained has excess in one or more of the possible isomers, said process represented by a general scheme each selected from the following group: 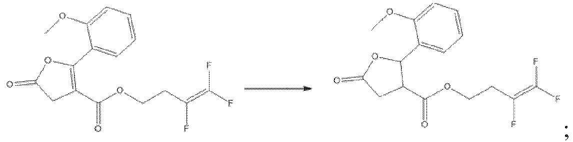 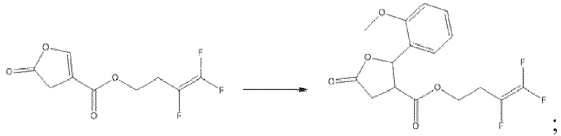 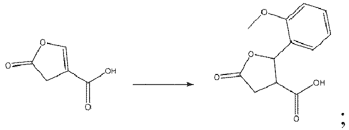 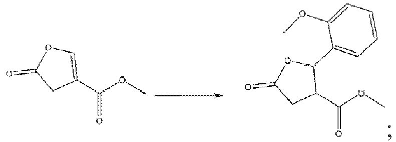 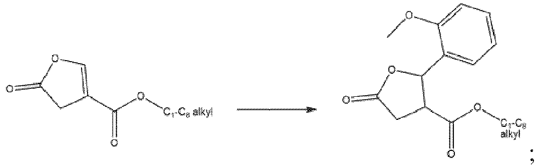 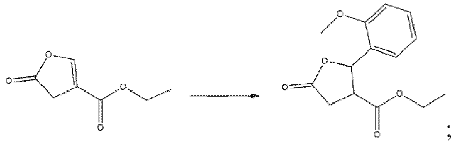 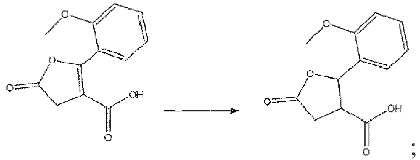 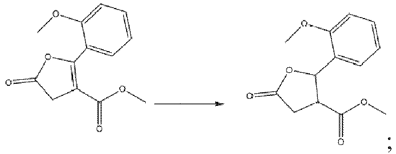 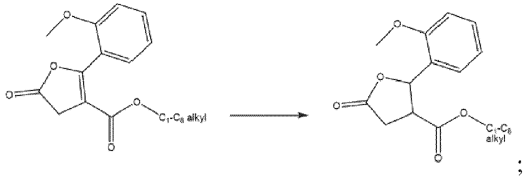 and

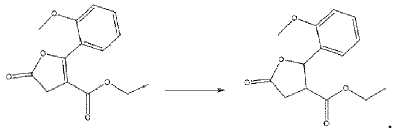

### Embodiment B2

The process according to any one of processes exemplified by Embodiments **B1** and **B1b** etc., comprising, combining an Unsaturated Precursor, a nucleophile exemplified by Grignard reagent, organo-lithium reagent, organo-zinc reagent, hydrogen source, e.g. H₂/Pd/C or hydride source or electron source exemplified by Zn/AcOH, and solvent to affect a 1,4 system substitution of the Unsaturated Precursor e.g. a 1,4-addition reaction to obtain the Saturated Target.

### Embodiment B2a

The process as described generally above, for preparing a Saturated Target of formula **1** from an Unsaturated Precursor of formula **2** 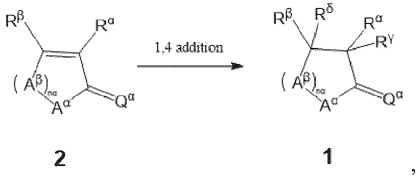 comprising substitution of the 1,4 system exemplified by, 1,4-addition using Grignard reagent, organo-lithium reagent, organo-zinc reagent, hydrogenation or hydride source,
In this process, the Saturated Target product, has excess in one or more of the possible isomers, wherein each of **R^{α}, R^{β}**, **A^{α}**, **A^{β}, nα**, **Q^{α}** and **R^{ε},** have the same meanings as defined in embodiment **A2,** and
**R^{γ}** is H or **R^{α}**
**R^{δ}** is H or **R^{α}**

### Embodiment B3

The process of according to any one of Embodiments **B2** and **B2a** comprising, combining an Unsaturated Precursor, a nucleophile exemplified by a Grignard reagent, organo-lithium reagent, organo-zinc reagent, or a hydride source, a solvent and one or more catalytic components each chosen from, catalyst; pre catalyst and co-catalyst, to affect a 1,4 system substitution of the Unsaturated Precursor e.g. a 1,4-addition reaction to obtain the Saturated Target.

### Embodiment B3a

The process according to any one of Embodiments **B2** and **B2a** comprising, combining an Unsaturated Precursor, a nucleophile exemplified by a Grignard reagent, organo-lithium reagent, organo-zinc reagent, or a hydride source, a solvent and one or more catalytic components each chosen from, catalyst; pre catalyst and co-catalyst, to affect a 1,4 system substitution of the Unsaturated Precursor exemplified by a 1,4-addition reaction to obtain the Saturated Target. In this process, the, Saturated Target product has one or more of the possible isomers in excess.

### Embodiment B4

The process according to any one of Embodiments **B2; B2a; B3;** and **B3a** comprising, further including within the combination, an additive which is a source for a reversibly bound adduct, to the Unsaturated Precursor, such as Lewis acid, to provide an improvement in the 1,4-addition reaction on the Unsaturated Precursor to obtain the Saturated Target, in any of the following schemes or any combination thereof, wherein RGA is the reversibly bound adduct. 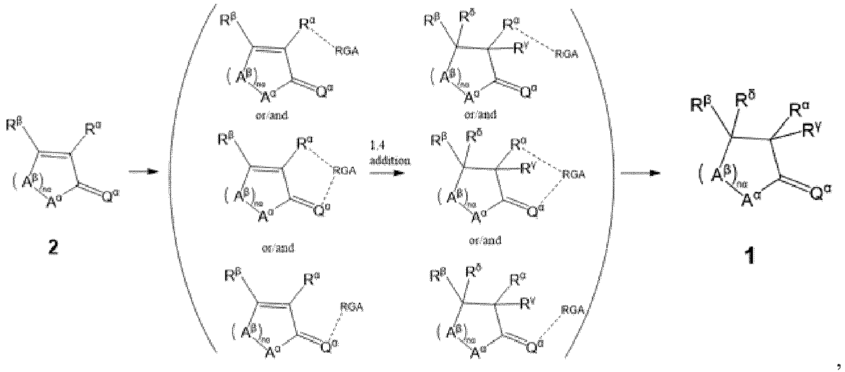
wherein said improvement provided is any of,
   protecting part of the Unsaturated Precursor from un-desired reaction,
   increasing pathway selectivity;
   increasing the selectivity for specific desired isomer;
   increasing the reactivity of the Unsaturated Precursor; and
   enabling reduction in the amount of the **R^{δ}** source e.g. Grignard reagent, required
wherein, **R^{α}, R^{β}**, **R^{γ}, A^{α}**, **A^{β}, nu, Q^{α}** and **R^{ε}** and **R^{δ}** have the same meaning as defined in any one of Embodiments **B1; B1a, B1b; B2** and **B2a.**

### Embodiment B5

The process of any one of Embodiments **B2; B2a; B3; B3a;** and **B4** further comprising a step of purifying the obtained Saturated Target product.

### Embodiment B6

The process of any one of Embodiments **B2; B2a; B3; B3a; B4;** and **B5,** further comprising a step of crystalizing the obtained Saturated Target product.

### Embodiment B6a

The process of any one of Embodiments **B2; B2a; B3; B3a; B4;** and **B5,** further comprising a step of obtaining a non-crystalline solid Saturated Target product by subjecting the Saturated Target obtained by the process of any one of Embodiments **B2; B2a; B3; B3a; B4** to spray drying, freeze drying, etc. or by adsorbing a solution thereof onto solid carrier material.

### Embodiment B7

The process of any one of Embodiments **B2** to **B6** comprising combining an Unsaturated Precursor, a nucleophile exemplified by a Grignard reagent, organo-lithium reagent, organo-zinc reagent, or a hydride source; solvent; one or more catalytic components each selected from, catalyst; pre catalyst; co-catalyst and an additive which is a source for a reversibly bound adduct, to an Unsaturated Precursor, to affect a 1,4-addition reaction to obtain the Saturated Target, further comprising steps of purifying and crystallizing the reaction product to obtain crystalline, purified, Saturated Target product.

### Embodiment B8

The process of Embodiment **B7** wherein the combination step comprises a Grignard reagent; organo-lithium reagent; organo-zinc reagent, or a hydride source, with solvent such as diethyl ether, and two or more catalytic components each selected from catalyst; pre catalyst; co-catalyst and mixtures thereof and Lewis acid.

### Embodiment B9

The process of any one of Embodiments **B1** to **B8** comprising, dissolving the Unsaturated Precursor in a solvent exemplified by, diethyl ether, and preferably adding a catalytic component comprising one or more of, catalyst; pre catalyst; co-catalyst such as a combination of both CuBr.SMe₂ (Copper(I) bromide dimethyl sulfide complex) and *R*-BINAP or (S)-N-((S)-1-(butylamino)-1-oxo-3-phenylpropan-2-yl)-2-(((E)-2-(diphenylphosphaneyl) benzylidene) amino)-3-methylbutanamide 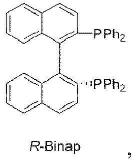 agitating the reaction mass by e.g. stirring, or by mixing in a continuous reactor and the like, and optionally adding a source for a reversibly bound adduct, to the Unsaturated Precursor, such as Lewis acid exemplified by Trimethylsilyl chloride, and continuing agitation for a suitable time exemplified by about 20 minutes. Adjusting the temperature to optimal levels preferably a cold temperature, for example of between -20°C to -25°C. Adding nucleophile, for example a Grignard reagent or a hydride source, and agitating for a period of time to achieve an optimal product by balancing parameters typified by considerations of, desired quality, and acceptable yield within a reasonable reaction time. Quenching the reaction mass, for example into aqueous NH₄Cl solution, and optionally extracting with suitable solvent. Optionally, washing the combined organic layer with for example, saturated brine solution, and optionally drying e.g. over Na₂SO₄ and optionally concentrating to obtain the Saturated Target product.

### Embodiment B10

The process of Embodiment **B9** further comprising a step of purifying the obtained Saturated Target product.

### Embodiment B11

The process of any one of Embodiments **B9** or **B10** further comprising a step of crystalizing the obtained Saturated Target product.

### Embodiment B12

The process of Embodiment **B1** comprising:
In a first vessel **A,** dissolving an Unsaturated Precursor in a solvent exemplified by diethyl ether, and agitating at a suitable temperature. Optionally cooling the reaction mass is to a suitable temperature such as between -20°C to -25°C, and adding Lewis acid, for example, Trimethylsilyl chloride, and agitating for an appropriate period of time.

In a second vessel **B,** introducing a catalytic component comprising one or more of, catalyst; pre-catalyst; co-catalyst exemplified by a combination of both CuBr.SMe₂ (Copper(I) bromide dimethyl sulfide complex) and *R*-BINAP

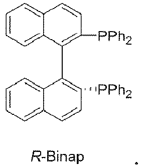

Adding a nucleophilic component, for example a Grignard reagent, organo-lithium reagent, organo-zinc reagent, or a hydride source, to vessel **B,** at a suitable temperature, (preferably cold), and agitating for some time, adding the contents of vessel **A** to vessel **B** at a suitable temperature (preferably cold), at an appropriate rate of addition, and agitating the mass for a suitable time, typically 20 minutes. Quenching the reaction mass e.g. into aq. NH₄Cl solution, and optionally extracting with suitable solvent. Optionally, washing the combined organic layer with e.g. saturated brine solution, and if desired, drying e.g. over Na₂SO₄, and optionally concentrating to obtain the Saturated Target product.

### Embodiment B13

The process of Embodiment **B12,** further comprising a step of purifying the obtained Saturated Target.

### Embodiment B14

The process of any one of Embodiments **B12** and **B13** further comprising a step of crystalizing the obtained Saturated Target product.

### Embodiment B15

The process of any one of Embodiments **B1** to **B14** wherein the Unsaturated Precursor is any of the embodiments **A1** - **A35** as defined above comprising, substitution of the 1,4 system of the Unsaturated Precursor e.g. 1,4-addition using Grignard reagent, organo-lithium reagent, organo-zinc reagent, or hydride source.

### Embodiment B15a

The use of the Unsaturated Precursor of any one of the Embodiments **A1** - **A35** defined above, as an intermediate in the method of preparation of a Saturated Target as defined in any one of Embodiments **B1** to **B14.**

### Embodiment B15b

A Saturated Target as defined in **B1** in combination with the intermediate Unsaturated Precursor via which it was produced using any one of the methods of Embodiments **B1** to **B14,** as an impurity.

### Embodiment B15c

The use of an Unsaturated Precursor of any one of the embodiments **B1** defined above, as an intermediate in the method of preparation of a Saturated Target as defined in any one of Embodiments **B1** to **B14.**

### Embodiment B15d

A Saturated Target as defined in **B1** in combination with the intermediate Unsaturated Precursor via which it was produced by the methods of any one of Embodiments **B1** to **B14,** as an impurity.

### Embodiment B16

The process of any one of Embodiments **B1** - **B15** wherein the Unsaturated Precursor is the compound of Embodiment **A10,** exemplified by the scheme, 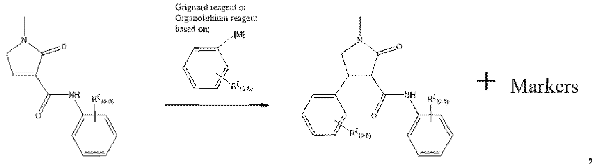 wherein, the markers that can be formed in the above scheme can include one; some, or all of the following structures, 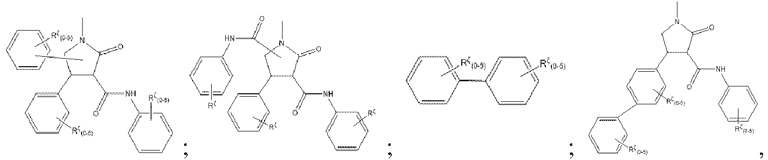 comprising, substitution of the Unsaturated Precursor 1,4 system such as 1,4-addition using Grignard reagent or/and organo-lithium reagent, exemplified by
(3-(trifluoromethyl)phenyl)lithium).

### Embodiment B16a

The process of any one of Embodiments **B1** - **B15** wherein the Unsaturated Precursor is the compound of Embodiment **A10** exemplified by the following scheme, 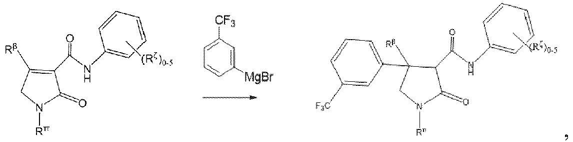 comprising, substitution of the Unsaturated Precursor 1,4 system such as 1,4-addition using Grignard reagent, exemplified by 3-(trifluoromethyl)phenyl magnesium bromide, and 3-(trifluoromethyl)phenyl]magnesium chloride etc.

### Embodiment B16b

The process of any one of Embodiments **B1** - **B15** wherein the Unsaturated Precursor is the compound of Embodiment **A10,** exemplified by the following scheme, 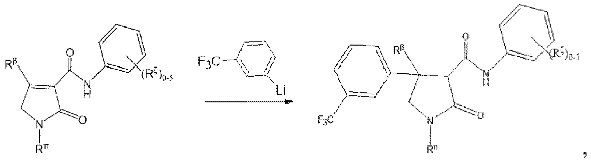 comprising, substitution of the Unsaturated Precursor 1,4 system such as 1,4-addition using organo-lithium reagent, exemplified by 3-(trifluoromethyl)phenyl lithium.

### Embodiment B16c

The process of any one of Embodiments **B1** - **B15** wherein the Unsaturated Precursor is the compound of Embodiment **A10,** exemplified by the scheme, 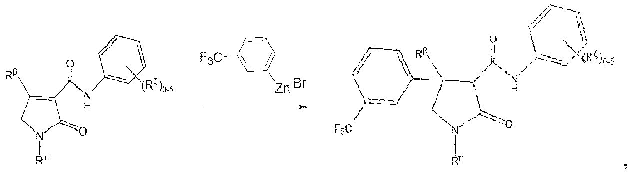 comprising, substitution of the Unsaturated Precursor 1,4 system such as 1,4-addition using organo-zinc reagent, exemplified by 3-(trifluoromethyl)phenyl zinc(II) bromide).

### Embodiment B16d

The process of any one of Embodiments **B1** - **B15** wherein the Unsaturated Precursor is the compound of Embodiment **A10,** exemplified by the following scheme, 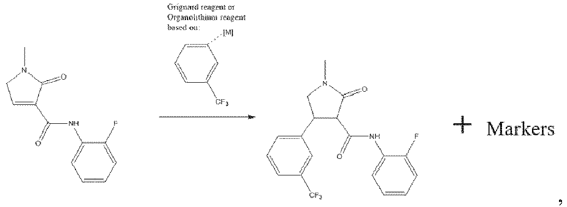 wherein, the markers that can be formed in the above scheme can include one; some or all of the following structures, 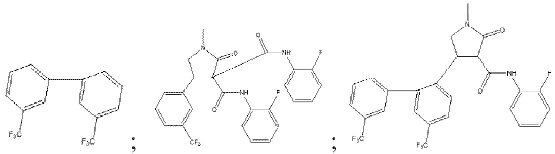 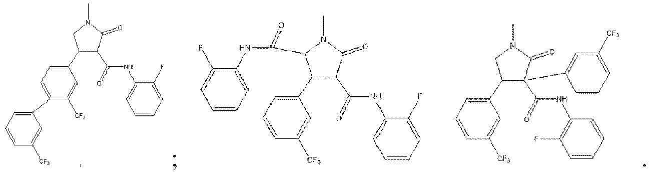 comprising, substitution of the Unsaturated Precursor 1,4 system such as 1,4-addition using Grignard reagent or/and organo-lithium reagent, exemplified by
3-(trifluoromethyl)phenyl)lithium).

### Embodiment B17

The process exemplified by the following scheme, 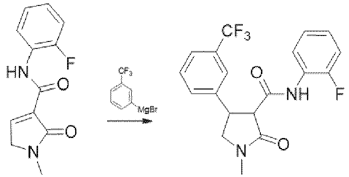 comprising, substitution of the Unsaturated Precursor 1,4 system such as 1,4-addition using Grignard reagent, exemplified by 3-(trifluoromethyl)phenyl magnesium bromide.

### Embodiment B18

A process exemplified by the scheme, 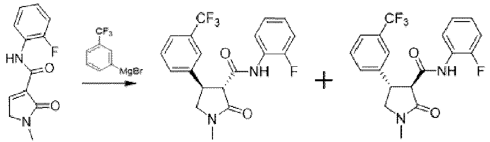 comprising substitution of the 1,4 system such as a 1,4-addition using Grignard reagent, exemplified by 3-(trifluoromethyl)phenyl magnesium bromide.

### Embodiment B18a

The process of embodiment **B18** exemplified by the scheme, 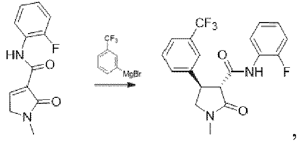 comprising substitution of the 1,4 system such as a 1,4-addition using Grignard reagent, exemplified by 3-(trifluoromethyl)phenyl magnesium bromide.

### Embodiment B18b

The process of Embodiment **B18** exemplified by the scheme, 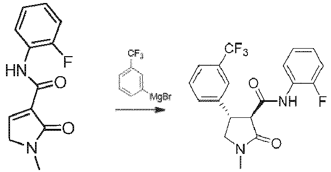 comprising substitution of the 1,4 system such as a 1,4-addition using Grignard reagent, exemplified by [3-(trifluoromethyl)phenyl] magnesium bromide)

### Embodiment B18c

A process exemplified by the following scheme, 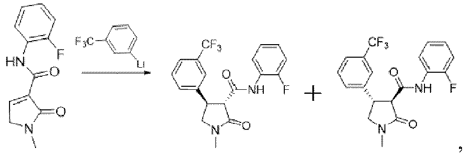 comprising substitution of the 1,4 system such as a 1,4-addition using organo-lithium reagent, exemplified by 3-(trifluoromethyl)phenyl lithium.

### Embodiment B18d

The process exemplified by the following scheme, 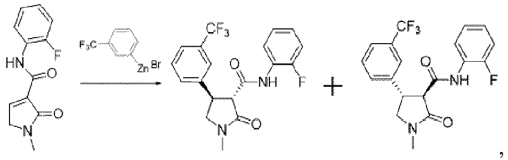 comprising substitution of the 1,4 system such as a 1,4-addition using organo-zinc reagent, exemplified by 3-(trifluoromethyl phenyl zinc(II) bromide.

### Embodiment B19

A process exemplified by the following scheme, 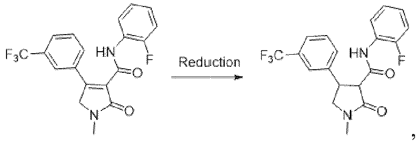 comprising, substitution of the 1,4 system by reduction of the 1- Methyl-2H-pyrrol-5-one, double bond, exemplified by the use of a hydride source.

### Embodiment B19a

The process of embodiment **B19** exemplified by the scheme, 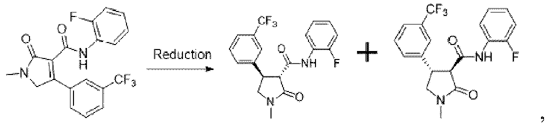 comprising, substitution of the 1,4 system by reduction of the double bond in the 1-Methyl-2H-pyrrol-5-one group, exemplified by the use of a hydride source.

### Embodiment B19b

The process exemplified by the following scheme, 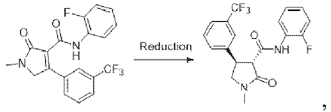 comprising, substitution of the 1,4 system by reduction of the double bond in the 1-Methyl-2H-pyrrol-5-one group, exemplified by the use of a hydride source.

### Embodiment B19c

The process exemplified by the following scheme, 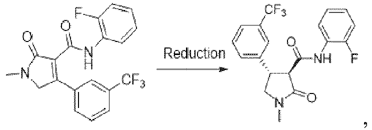 comprising, substitution of the 1,4 system by reduction of the double bond in the 1-Methyl-2H-pyrrol-5-one group, exemplified by the use of a hydride source.

### Embodiment B20

A process represented by the scheme, 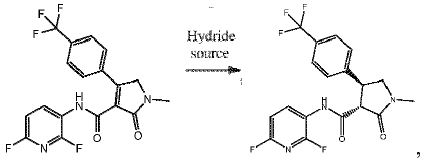 comprising, substitution of the 1,4 system by reduction of the double bond in the 1-Methyl-2H-pyrrol-5-one group, exemplified by the use of a hydride source.

### Embodiment B21a

The process for obtaining a Saturated Target from an Unsaturated Precursor represented by the following scheme, 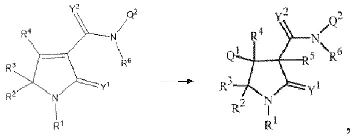 said process comprising substitution of the 1,4 system such as 1,4-addition using Grignard reagent or hydride source, wherein the Saturated Target is a compound as defined in Claim 1 of PCT/US2014/068073 published as WO 2015/084796, Wherein Q¹, Q², R¹, R², R³, R⁴, R⁶, Y¹ and Y² etc. have the meanings as defined in Claim 1 of PCT/US2014/068073 published as WO 2015/084796. The text from claim 1 on page 286, line 6 until page 289, line 23 of WO 2015/084796 being specifically incorporated herein by reference solely for the purpose of defining the structures of this embodiment. The text being reproduced in Embodiment **A35a**

### Embodiment B21b

A process for obtaining a Saturated Target from an Unsaturated Precursor represented by the following scheme, 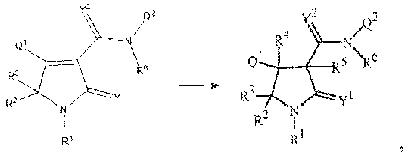 said process comprising substitution of the 1,4 system such as 1,4-addition using Grignard reagent or hydride source, wherein the Saturated Target is a compound as defined in Claim 1 of PCT/US2014/068073 published as WO 2015/084796, Wherein Q¹, Q², R¹, R², R³, R⁴, R⁶, Y¹ and Y² etc. have the meanings as defined in Claim 1 of PCT/US2014/068073 published as WO 2015/084796. The text from claim 1 on page 286, line 6 until page 289, line 23 of WO 2015/084796 being specifically incorporated herein by reference solely for the purpose of defining the structures of this embodiment. The text being reproduced in Embodiment **A35a.**

### Embodiment B21c

The process for obtaining a Saturated Target from Unsaturated Precursor, 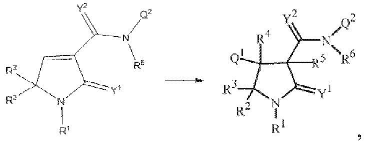 said process comprising substitution of the 1,4 system such as 1,4-addition using Grignard reagent or hydride source, wherein the Saturated Target is a compound as defined in Claim 1 of PCT/US2014/068073 published as WO 2015/084796, Wherein Q¹, Q², R¹, R², R³, R⁴, R⁶, Y¹ and Y² etc. have the meanings as defined in Claim 1 of PCT/US2014/068073 published as WO 2015/084796. The text from claim 1 on page 286, line 6 until page 289, line 23 of WO 2015/084796 being specifically incorporated herein by reference solely for the purpose of defining the structures of this embodiment. The text being reproduced in Embodiment **A35a**

### Embodiment B22

A process exemplified by the following scheme, 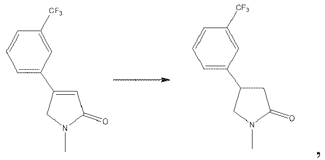 comprising, substitution of the Unsaturated Precursor 1,4 system.

### Embodiment B23

A process exemplified by the following scheme, 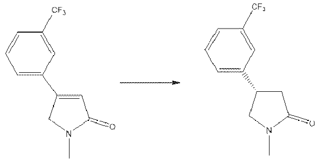 comprising, substitution of the Unsaturated Precursor 1,4 system.

### Embodiment B24

A process exemplified by the following scheme, 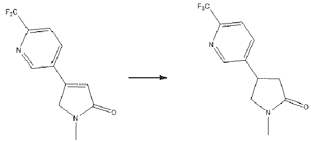 comprising, substitution of the Unsaturated Precursor 1,4 system.

### Embodiment B25

A process exemplified by the following scheme, 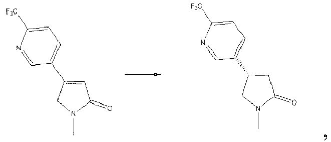 comprising, substitution of the Unsaturated Precursor 1,4 system.

### Embodiment B26

A process exemplified by the following scheme, 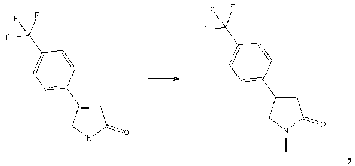 comprising, substitution of the Unsaturated Precursor 1,4 system.

### Embodiment B27a

A process exemplified by the following scheme, 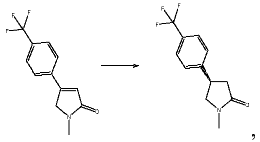 comprising, substitution of the Unsaturated Precursor 1,4 system.

### Embodiment B27b

A process exemplified by the following scheme, 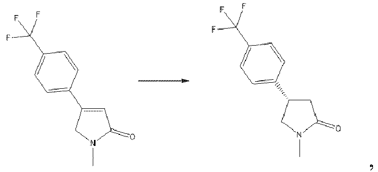 comprising, substitution of the Unsaturated Precursor 1,4 system.

### Embodiment B28a

A process exemplified by the scheme, 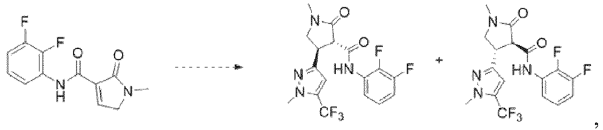 comprising substitution of the 1,4 system such as a 1,4-addition

### Embodiment B28b

The process exemplified by the following scheme, 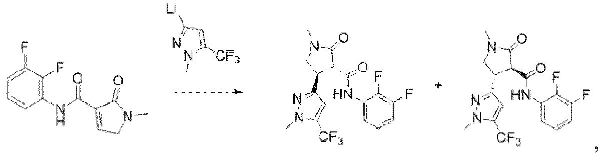 comprising substitution of the 1,4 system such as a 1,4-addition using organo-lithium reagent, exemplified by 1-methyl-5-(trifluoromethyl)-1H-pyrazol-3-yl lithium.

### Embodiment B29a

The process exemplified by the following scheme, 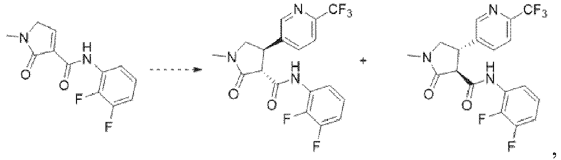 comprising substitution of the 1,4 system such as a 1,4-addition

### Embodiment B29b

The process exemplified by the following scheme, 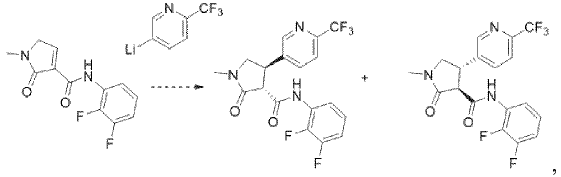 comprising substitution of the 1,4 system such as a 1,4-addition organo-lithium reagent, exemplified by (6-(trifluoromethyl)pyridin-3-yl)lithium

### Embodiment B30a

The process exemplified by the following scheme, 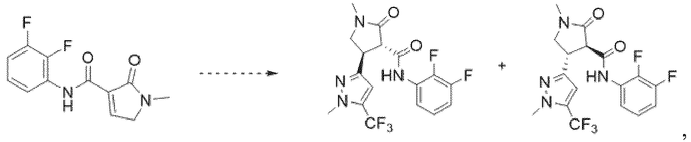 comprising substitution of the 1,4 system such as a 1,4-addition.

### Embodiment B30b

The process exemplified by the following scheme, 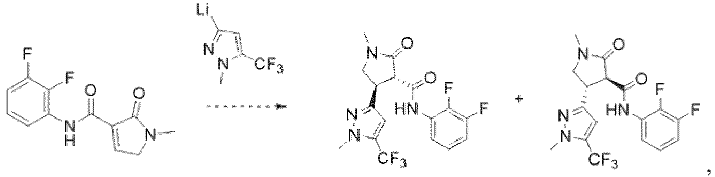 comprising substitution of the 1,4 system such as a 1,4-addition using organo-lithium reagent exemplified by 1-methyl-5-(trifluoromethyl)-1H-pyrazol-3-yl lithium

### Embodiment B31

The process of any one of Embodiments B1-B30b wherein an excess in one or more of the possible isomers is obtained.

### Embodiment B32

The process of any one of Embodiments B1-B30b wherein an excess of the anti-isomer/s is obtained.

### Embodiment B33

The process of any one of Embodiments B1-B30b wherein an excess of the syn-isomer/s is obtained.

### Embodiment B34

The process of any one of Embodiments B1-B33 wherein an excess of of one or more of the possible chiral isomers is obtained relative to the others.

### Embodiment B35

The process of any one of Embodiments B1-B34 wherein an excess of chiral anti-isomer is obtained.

### Embodiment B36

The process of any one of Embodiments B1-B34 wherein an excess of chiral, anti-isomer with S,S configuration, is obtained

### Embodiment B37

The process according to any one of Embodiments **B1-B36** wherein magnesium, lithium, zinc, iPrMgCl, iPrMgBr, iPrMgl, hexyllithium, butyllithium, tert-butyllithium, 4-methyl-pentyl-lithium, isobutyl lithium or combinations thereof are used to prepare the reagent for the substitution of the 1,4 system, in-situ and/or before it's use in the substitutionreaction.

### Embodiment B38

The process according to any one of of Embodiments **B1-B36** wherein magnesium halogen exchange or lithium halogen exchange is used for the preparation of the reagent for the substitution of the 1,4 system in-situ and/or before it's use.

### Embodiment B39

The process according to any one of Embodiments **B1-B38** conducted in the presence of metal.

### Embodiment B40

The process according to Embodiment **B39** wherein the metal is selected from Cu; Ni; Ti; Co; and Fe.

### Embodiment B41

The process according to Embodiment **B39** wherein the metal is selected from Cu; Ni; and Ti.

### Embodiment B42

The process according to Embodiment **B39** wherein the metal is Cu.

### Embodiment B43

The process according to Embodiment **B39** wherein the metal is Ni.

### Embodiment B44

The process according to any one of Embodiments **B39-B43** wherein the metal is present in catalytic amount.

### Embodiment B45

The process according to any one of Embodiments **B1-B44** conducted in the presence of a chiral compound optionally connected to, or part of, a solid material or substrate.

### Embodiment B46

The process according to Embodiment **B45** wherein the chiral compound is selected from a group containing, naturally occuring, synthetic, modified, (e.g., substituted etc.), amino acids exemplified by N,N-dimethyl-L-prolinium and L-proline; peptides exemplified by, (S)-N-((S)-1-butylamino-1-oxo-3-phenylpropan-2-yl-2- (E)-2-diphenylphosphaneyl benzylidene amino-3-methylbutanamide; phosphorus ligand exemplified by, R-BINAP; proteins; enzymes; and sugars,

### Embodiment B47

The process according to any one of Embodiments **B45-B46** wherein the chiral compound is selected from (S)-N-(S)-1-(butylamino-1-oxo-3-phenylpropan-2-yl-2-(E)-2-diphenylphosphaneyl benzylidene amino-3-methyl butanamide.

### Embodiment B48

The process according to any one of Embodiments **B45-B47** wherein the chiral compound is present in catalytic amount.

### Embodiment B49

The process according to any one of Embodiments **B45-B48** wherein Lewis bases are present in the workup exemplified by, NH₄⁺Cl⁻ (ammonium chloride); NH₄⁺OH⁻ (ammonium hydroxide); NH₃; CN⁻ ion (for ex. from KCN or NaCN); pyridine; DMA; DMSO; DMF; TEMADA; OH⁻ ion; PH₃; and H₂PO₃⁻ ion; etc.

### Embodiment B50

The process according to Embodiment **B49** wherein the Lewis bases in the workup are selected from, NH₄⁺Cl⁻ (ammonium chloride); NH₄⁺OH⁻ (ammonium hydroxide); NH₃; and CN⁻ ion for example, from KCN or NaCN.

In some cases the presence of Lewis bases can improve the workup and/or increase the yield.

### Embodiment B51

The process according to any one of Embodiments **B1-B50** conducted in the presence of strong bases exemplified by, MeMgCl; MeMgBr; iPrMgCl; iPrMgBr; BuLi; tert-BuLi; hexyllithium; NaH; and KH, etc.

In some cases, the presence of strong bases enables reducing the amount of other ingredients required

### Embodiment C1a

A compound of Formula **3** and e.g. salts etc. thereof 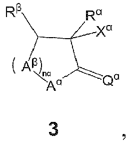 wherein,
**X^{α}** is SPh, SePh, S**G^{α},** Se**G^{α}**
**G^{α}** is alkyl, haloalkyl, phenyl, phenyl substituted with 0-5 groups each selected from halogen; alkyl; and haloalkyl groups, and
**R^{α}, R^{β}**, **A^{α}**, **A^{β},** not and **Q^{α}** have the same meanings as defined in Embodiment **A2**

### Embodiment C1b

A compound of Formulae **3** and e.g. salts etc. thereof 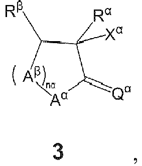 wherein,
**X^{α}** is halogen, and
**R^{α}, R^{β}**, **A^{α}**, **A^{β}, not** and **Q^{α}** have the same meanings as defined in Embodiment **A2**

### Embodiment C1c

A compound of Formula **3** and e.g. salts etc. thereof 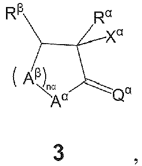 wherein,
**X^{α}** is N(**G^{α}**)₂, N⁺(O⁻)(**G^{α}**)₂ [i.e. the N-Oxide of N(**G^{α}**)₂];
**G^{α}** is alkyl, haloalkyl, phenyl, phenyl substituted with 0-5 groups each selected from halogen; alkyl; and haloalkyl groups; and
**R^{α}, R^{β}**, **A^{α}**, **A^{β}, not** and **Q^{α}** have the same meanings as defined in Embodiment **A2**

### Embodiment C2

A compound of Formula **3** and e.g. salts etc. thereof 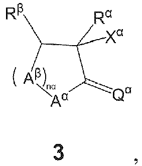 wherein, the compound of Formula 3 does not include any covalent Nitrogen-Halogen bond, and wherein,
**X^{α}** is halogen, SPh, SePh, S**G^{α}**, Se**G^{α}**;
**G^{α}** is alkyl, haloalkyl, phenyl, phenyl substituted with 0-5 groups each selected from halogen; alkyl; and haloalkyl groups; and **R^{α}, R^{β}**, **A^{α}**, **A^{β},** not and **Q^{α}** have the same meanings as defined in

### Embodiment A2

### Embodiment C2b

A Saturated Target compound of formula 1, in combination with the intermediate compound of formula **3,** via which it was produced, as an impurity wherein the intermediate is a compound as defined in any one of Embodiments **C1a**; **C1b**; **C1c**; and **C2,** and **R^{γ}** has the same meaning as defined in any one of Embodiments **B1; B1a, B1b; B2** and **B2a.**

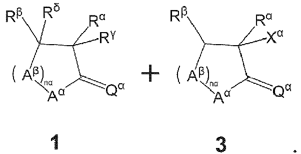

### Embodiment C3a

The compound of Embodiment **C2** and e.g. salts thereof as a process intermediate and/or marker wherein
**R^{α}** is -C(**Q^{α}**)**Q^{β}R^{µ},**
**X"** is SPh, SePh, S**G^{α}**, Se**G^{α}**
**G^{α}** is alkyl, haloalkyl, phenyl, phenyl substituted with 0-5 groups each selected from halogen; alkyl; and haloalkyl, 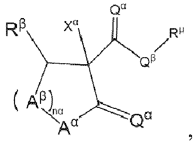 wherein,
**R^{β}**, **Q^{α}, Q^{β}, A^{β}, A^{α}**, **R^{µ}** and **nα,** have the same meanings as defined in Embodiment **A2.**

### Embodiment C3b

The compound of Embodiment **C2** and e.g. salts thereof as a process intermediate and/or marker wherein,
**R^{α}** is -C(**Q^{α}**)**Q^{β}R^{µ}**,
**X^{α}** is halogen, N(**G^{α}**)₂, N⁺(O⁻)(**G^{α}**)₂; 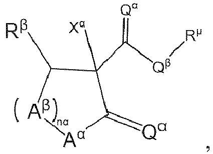 and wherein,
**R^{β}**, **Q^{α}, Q^{β}, A^{β}, A^{α}**, **R^{µ}** and **nα,** have the same meanings as defined in Embodiment **A2.**

### Embodiment C4a

The compound of Embodiment **C2** and e.g. salts thereof as a process intermediate and/or marker wherein,
**R^{α}** is -C(**Q^{α}**)**Q^{β}R^{µ}**;
**A^{α}** is -N(**R^{π}**)-;
**X^{α}** is SPh, SePh, S**G^{α}**, Se**G^{α}**;
**G^{α}** is alkyl, haloalkyl, phenyl, phenyl substituted with 0-5 groups each selected from halogen; alkyl; and haloalkyl, 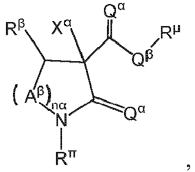 and wherein,
**R^{β}**, **Q^{α}**, **Q^{β}, A^{β}, R^{π}**, **R^{µ}** and **nα,** have the same meanings as defined in Embodiment **A2.**

### Embodiment C4b

The compound of Embodiment **C2** and e.g. salts thereof, as a process intermediate and/or marker wherein,
**R^{α}** is -C(**Q^{α}**)**Q^{β}R^{µ}**, and
**A^{α}** is -N(**R^{π}**)-
**X^{α}** is halogen, N(**G^{α}**)₂, N⁺(O⁻)(**G^{α}**)₂ 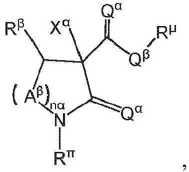 and wherein,
**R^{β}**, **Q^{α}**, **Q^{β}, A^{β}, R^{π}**, **R^{µ}** and **nα,** have the same meanings as defined in Embodiment **A2.**

### Embodiment C5a

The compound of Embodiment **C2** and e.g. salts thereof wherein,
**R^{α}** is -C(**Q^{α}**)**Q^{β}R^{µ}**,
**A^{α}** is -N(**R^{π}**)-, and
**nα** is 1
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups, 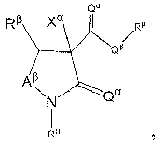 and wherein,
**R^{β}**, **Q^{α}**, **Q^{β}, A^{β}, R^{π}** and **R^{µ}**, have the same meanings as defined in Embodiment **A2.**

### Embodiment C5b

The compound of Embodiment **C2** and e.g. salts thereof, as a process intermediate and/or marker wherein,
**R^{α}** is -C(**Q^{α}**)**Q^{β}R^{µ}**;
**A^{α}** is -N(**R^{π}**)-;
**nα** is 1;
**X^{α}** is halogen 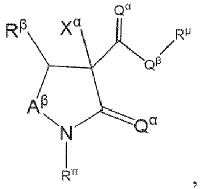 and wherein,
**R^{β}**, **Q^{α}**, **Q^{β}, A^{β}, R^{π}** and **R^{µ}**, have the same meanings as defined in Embodiment **A2.**

### Embodiment C5c

The compound of Embodiment **C2** and e.g. salts thereof wherein,
**R^{α}** is -C(**Q^{α}**)**Q^{β}R^{µ}**,
**A^{α}** is -N(**R^{π}**)-, and
**nα** is 1
**X^{α}** is SPh, SePh,
**G^{α}** is alkyl, haloalkyl, phenyl, phenyl substituted with 0-5 groups each selected from halogen; alkyl; and haloalkyl,groups, 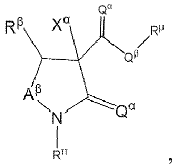 and wherein,
**R^{β}**, **Q^{α}, Q^{β}, A^{β}, R^{π}** and **R^{µ}**, have the same meanings as defined in Embodiment **A2.**

### Embodiment C6a

The compound of Embodiment **C2** and e.g. salts thereof, as a process intermediate and/or markerwherein,
**R^{α}** is -C(**Q^{α}**)**Q^{β}R^{µ}**
**A^{α}** is -N(**R^{π}**)-
**nα** is 1
**A^{β}** is CH₂
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups,, 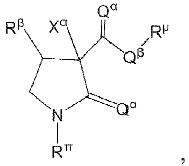 and wherein,
**R^{β}**, **Q^{α}**, **Q^{β}, R^{π}** and **R^{µ}**, have the same meanings as defined in Embodiment **A2.**

### Embodiment C6b

The compound of Embodiment **C2** and e.g. salts thereof, as a process intermediate and/or marker wherein,
**R^{α}** is -C(**Q^{α}**)**Q^{β}R^{µ}**
**A^{α}** is -N(**R^{π}**)-
**nα** is 1
**A^{β}** is CH₂
**X"** is halogen 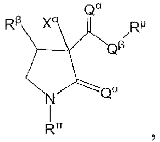 and wherein,
**R^{β}**, **Q^{α}**, **Q^{β}, R^{π}** and **R^{µ}**, have the same meanings as defined in Embodiment **A2.**

### Embodiment C7a

The compound of Embodiment **C2** and e.g. salts thereof, as a process intermediate and/or marker wherein,
**R^{α}** is -C(**Q^{α}**)**Q^{β}R^{µ}**,
**A^{α}** is -N(**R^{π}**)-,
**nα** is 2,
**A^{β}** is CH₂
**X^{α}** is SPh, SePh, S**G^{α}**, Se**G^{α}**
G^{α} is alkyl, haloalkyl, phenyl, phenyl substituted with 0-5 groups each selected from halogen; alkyl; and haloalkyl groups, 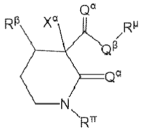
And wherein,
R^{β}, Q^{α}, Q^{β}, R^{π} and R^{µ}, have the same meanings as defined in Embodiment A2.

### Embodiment C7b

The compound of Embodiment **C2** and e.g. salts thereof, as a process intermediate and/or marker wherein,
**R^{α}** is -C(**Q^{α}**)**Q^{β}R^{µ}**,
**A^{α}** is -N(**R^{π}**)-,
**nα** is 2,
**A^{β}** is CH₂
**X^{α}** is halogen 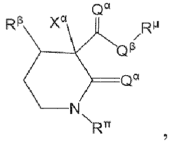 and wherein,
**R^{β}**, **Q^{α}, Q^{β}, R^{π}** and **R^{µ}**, have the same meanings as defined in Embodiment **A2.**

### Embodiment C8a

The compound of Embodiment **C2** and e.g. salts thereof wherein,
**R^{α}** is -C(O)**Q^{β}R^{µ}**
**A^{α}** is -N(**R^{π}**)-
**nα** is 1
**A^{β}** is CH₂
**Q^{α}** is O
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups,, 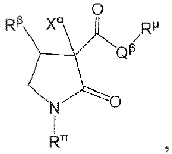 and wherein,
**R^{β}**, **Q^{β}, R^{π}** and **R^{µ}**, have the same meanings as defined in Embodiment **A2.**

### Embodiment C8b

The compound of Embodiment **C2** and e.g. salts thereof wherein,
**R^{α}** is -C(O)**Q^{β}R^{µ}**
**A^{α}** is -N(**R^{π}**)-
**nα** is 1
**A^{β}** is CH₂
**Q^{α}** is O
**X^{α}** is N⁺(O⁻)(**G^{α}**)₂ 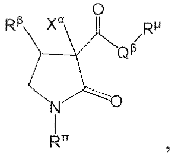 and wherein,
**R^{β}**, **Q^{β}, R^{π}** and **R^{µ}**, have the same meanings as defined in Embodiment **A2.**

### Embodiment C9a

The compound of Embodiment **C2** and e.g. salts thereof, as a process intermediate and/or marker wherein,
**R^{α}** is -C(O)NHR^{µ}
**A^{α}** is -N(**R^{π}**)-
**nα** is 1
**A^{β}** is CH₂
**Q^{α}** is O
**Q^{β}** is NH
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups, 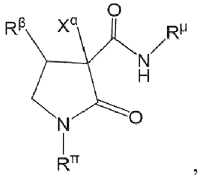 and wherein,
**R^{β}**, **R^{π}** and **R^{µ}**, have the same meanings as defined in Embodiment **A2.**

### Embodiment C9b

The compound of Embodiment **C2** and e.g. salts thereof wherein,
**R^{α}** is -C(O)NH**R^{µ}**
**A^{α}** is -N(**R^{π}**)-
**nα** is 1
**A^{β}** is CH₂
**Q^{α}** is O
**Q^{β}** is NH
**X^{α}** is SPh, SePh, 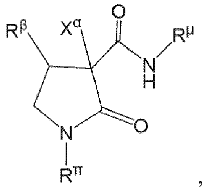 and wherein,
**R^{β}**, **R^{π}** and **R^{µ}**, have the same meanings as defined in Embodiment **A2.**

### Embodiment C9c

The compound of Embodiment **C2** and e.g. salts thereof, as a process intermediate and/or marker wherein,
**R^{α}** is -C(O)NH**R^{µ}**
**A^{α}** is -N(**R^{π}**)-
**nα** is 1
**A^{β}** is CH₂
**Q^{α}** is O
**Q^{β}** is NH
**X^{α}** is halogen 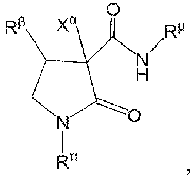 and wherein,
**R^{β}, R^{π}** and **R^{µ},** have the same meanings as defined in Embodiment **A2.**

### Embodiment C10

The compound of Embodiment **C2** and e.g. salts thereof wherein,
**R^{α}** is -C(O)NH**R^{µ}**
**A^{α}** is -N**(R*^{π}*)-**
**nα** is 1
**A^{β}** is CH₂
**Q^{α}** is O,
**Q^{β}** is NH and
**R^{µ}** is phenyl ring, unsubstituted or substituted with up to 5 substituents each independently selected from **R^{ζ}**
**X^{α}** is SPh, SePh, S**G^{α},** Se**G^{α},**
**G^{α}** is alkyl, haloalkyl, phenyl, phenyl substituted with 0-5 groups each selected from halogen; alkyl; and haloalkyl groups, 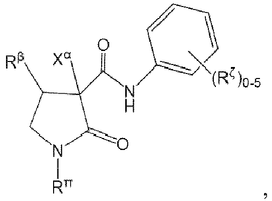 and wherein,
**R^{β}, R^{π}** and **R^{ζ},** have the same meanings as defined in Embodiment A2.

### Embodiment C11

The compound of Embodiment **C2** and e.g. salts thereof wherein,
**R^{α}** is -C(O)NH**R^{µ}**
**A^{α}** is -N(**R^{π}**)-
**nα** is 1
**A^{β}** is CH₂
**Q^{α} is O**
**Q^{β}** is NH
**R^{µ}** is phenyl ring, unsubstituted or substituted with up to 5 substituents independently selected from **R^{ζ}**
**R^{β}** is **R^{λ}** is phenyl ring, unsubstituted or substituted with up to 5 substituents each independently selected from **R^{ζ}**
**X^{α}** is SPh, SePh, 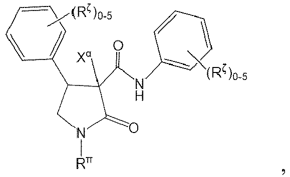 and wherein,
**R^{π}** and **R^{ζ},** have the same meanings as defined in Embodiment **A2.**

### Embodiment C12

The compound of Embodiment **C2** and e.g. salts thereof wherein,
**R^{α}** is -C(O)NH**R^{µ}**
**A^{α}** is -N(Me)-
**nα is 1**
**A^{β}** is CH₂
**Q^{α}** is O
**Q^{β}** is NH
**R^{µ}** is phenyl ring, unsubstituted or substituted with up to 5 substituents each independently selected from **R^{ζ}**
**R^{β} = R^{λ}** is phenyl ring, unsubstituted or substituted with up to 5 substituents each independently selected from **R^{ζ},**
**X^{α}** is SPh, SePh, 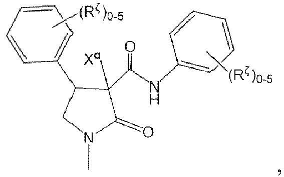 and wherein,
**R^{ζ},** has the same meaning as defined in Embodiment **A2.**

### Embodiment C12a

The compound of Embodiment **C2** and e.g. salts thereof wherein,
**R^{α}** is -C(O)NH**R^{µ}** ,
**A^{α}** is -N(Me)-,
**nα** is 1,
**A^{β}** is CH₂ ,
**Q^{α}** is O,
**Q^{β}** is NH,
**R^{µ}** is phenyl ring, unsubstituted or substituted with up to 5 substituents each independently selected from **R^{ζ},**
**R^{β}** = R^{λ} is phenyl ring, unsubstituted or substituted with up to 5 substituents each independently selected from **R^{ζ},**
**X^{α}** is Halogen, 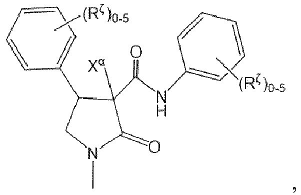 and wherein,
**R^{ζ},** has the same meaning as defined in Embodiment **A2.**

### Embodiment C12b

The compound of Embodiment **C2** and e.g. salts thereof wherein,
**R^{α}** is -C(O)NH**R^{µ}**,
**A^{α} is** -N(Me)-,
**nα** is 1,
**A^{β}** is CH₂ ,
**Q^{α}** is O,
**Q^{β}** is NH,
**R^{µ}** is phenyl ring, unsubstituted or substituted with up to 5 substituents each independently selected from **R^{ζ},**
**R^{β} = R^{λ}** is phenyl ring, unsubstituted or substituted with up to 5 substituents each independently selected from **R^{ζ},**
**X^{α}** is N⁺(O⁻)**(G^{α})**₂,
**G^{α}** is alkyl, haloalkyl, phenyl, phenyl substituted with 0-5 groups each selected from halogen; alkyl; and haloalkyl groups, 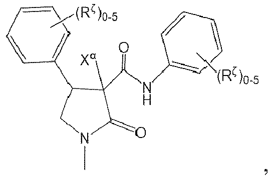 and wherein,
**R^{ζ},** has the same meaning as defined in Embodiment **A2.**

### Embodiment C13

The compound of Embodiment **C2** and e.g. salts thereof wherein,
**R^{α}** is -C(O)NH**R^{µ}** ,
**A^{α}** is -NH-,
**nα** is 1,
**A^{β}** is CH₂ ,
**Q^{α}** is O,
**Q^{β}** is NH,
**R^{µ}** is phenyl ring, unsubstituted or substituted with up to 5 substituents each independently selected from **R^{ζ},**
**R^{β}** =R^{λ} is a phenyl ring, unsubstituted or substituted with up to 5 substituents each independently selected from **R^{ζ},**
**X^{α}** is SPh, SePh, S**G^{α}**, Se**G^{α}**, , N⁺(O⁻)(**G^{α}**)₂
**G^{α}** is alkyl, haloalkyl, phenyl, phenyl substituted with 0-5 groups each selected from halogen; alkyl; and haloalkyl groups, 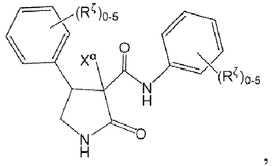 and wherein,
**R^{ζ},** has the same meaning as defined in Embodiment **A2.**

### Embodiment C14

The compound of Embodiment **C2** and e.g. salts thereof wherein,
**R^{α}** is -C(O)NH**R^{µ}** ,
**A^{α}** is -NH-,
**nα** is 2,
**A^{β}** is CH₂ ,
**Q^{α}** is O,
**Q^{β}** is NH,
**R^{µ}** is phenyl ring, unsubstituted or substituted with up to 5 substituents each independently selected from **R^{ζ},**
**R^{β}** = R^{λ} is phenyl ring, unsubstituted or substituted with up to 5 substituents independently selected from R^{ζ}
X^{α} is SPh, SePh, S**G^{α}**, Se**G^{α}**, N⁺(O⁻)(**G^{α}**)₂
**G^{α}** is alkyl, haloalkyl, phenyl, phenyl substituted with 0-5 groups each selected from halogen; alkyl; and haloalkyl groups, 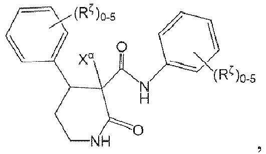 and wherein,
**R^{ζ},** has the same meaning as defined in Embodiment **A2.**

### Embodiment C15

The compound of Embodiment **C2** and e.g. salts thereof wherein,
**R^{α}** is -C(O)NH**R^{µ}**,
**A^{α}** is -N(Me)-,
**nα** is 2,
**A^{β}** is CH₂,
**Q^{α}** is O,
**Q^{β}** is NH,
**R^{µ}** is phenyl ring, unsubstituted or substituted with up to 5 substituents each independently selected from **R^{ζ},**
**R^{β}** = R^{λ} is phenyl ring, unsubstituted or substituted with up to 5 substituents each independently selected from **R^{ζ},**
**X^{α}** is SPh, SePh, S**G^{α}**, Se**G^{α}**, N⁺(O⁻)(**G^{α}**)₂,
**G^{α}** is alkyl, haloalkyl, phenyl, phenyl substituted with 0-5 groups each selected from halogen; alkyl; and haloalkyl groups, 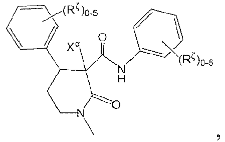
wherein, **R^{ζ},** has the same meaning as defined in Embodiment **A2.**

### Embodiment C16

The compound of any one of Embodiments **C1-C15** and e.g. salts etc. thereof wherein, **X^{α}** is SPh, and wherein,
**R^{α}, R^{β}, A^{α}, A^{β}, nα,** and **Q^{α}** have the same meanings as in Embodiment **A2**

### Embodiment C16a

The compound of any one of Embodiments **C1-C15** and e.g. salts etc. thereof, wherein, **X^{α}** is N(Me)₂, and wherein,
**R^{α}, R^{β}, A^{α}, A^{β}, nα,** and **Q^{α}** have the same meanings as in Embodiment **A2**

### Embodiment C16b

A compound which has the following formula,

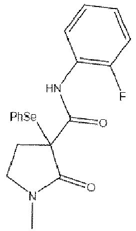

### Embodiment C16c

A compound which has the following formula,

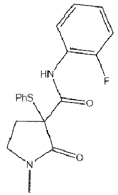

### Embodiment C16d

A compound which has the following formula,

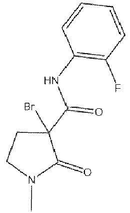

### Embodiment C16e

A compound for use as a pesticide, preferably, a herbicide synthetic process intermediate and/or marker compound of the following structure,

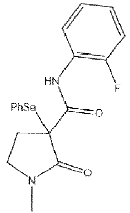

### Embodiment C16f

A compound for use as a pesticide, preferably, a herbicide synthetic process intermediate and/or marker compound of the following structure,

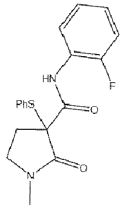

### Embodiment C16g

A compound for use as a pesticide, preferably, a herbicide synthetic process intermediate and/or marker compound of the structure,

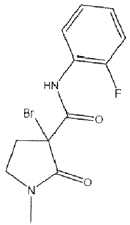

### Embodiment C16h

A compound for use as a pesticide, preferably, a herbicide synthetic process intermediate and/or marker compound of the following structure,

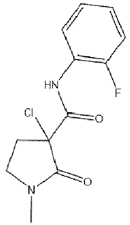

### Embodiment C17a

A compound whichhas the following formula,

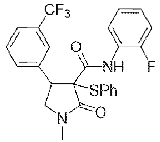

### Embodiment C17b

A compound whichhas the following formula,

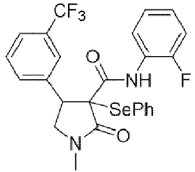

### Embodiment C17c

A compound which has the following formula,

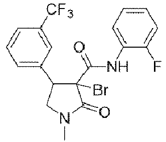

### Embodiment C17d

A compound which has the following formula,

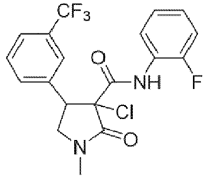

### Embodiment C17f

A compound for use as a pesticide, preferably, a herbicide synthetic process intermediate and/or marker compound of the following structure,

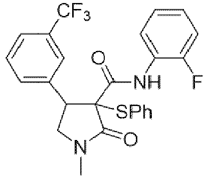

### Embodiment C17g

A compound for use as a pesticide, preferably, a herbicide synthetic process intermediate and/or marker compound of the following structure,

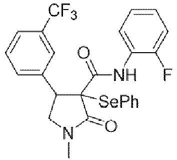

### Embodiment C17h

A compound for use as a pesticide, preferably, a herbicide synthetic process intermediate and/or marker compound of the following structure,

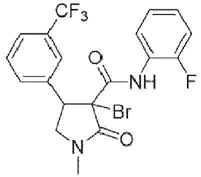

### Embodiment C17i

A compound for use as a pesticide, preferably, a herbicide synthetic process intermediate and/or marker compound of the following structure,

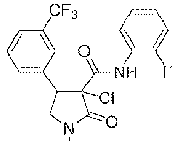

### Embodiment C18a

A compound which has the following formula,

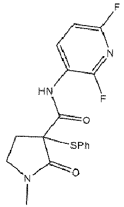

### Embodiment C18b

A compound which has the following formula,

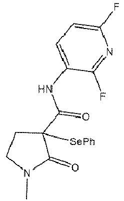

### Embodiment C19a

A compound which has the following formula,

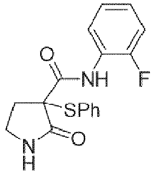

### Embodiment C19b

A compound which has the following formula,

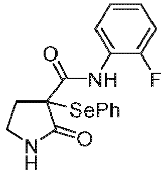

### Embodiment C20a

A compound which has the following formula,

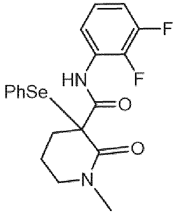

### Embodiment C20b

A compound which has the following formula,

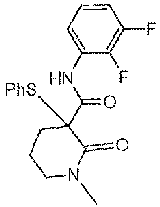

### Embodiment C21a

A compound which has the following formula,

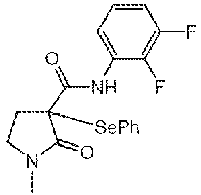

### Embodiment C21b

A compound which has the following formula,

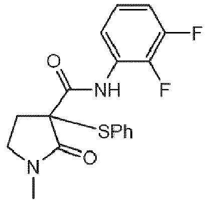

### Embodiment C22a

A compound which has the following formula,

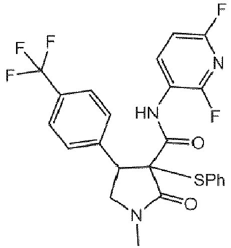

### Embodiment C22b

A compound which has the following formula,

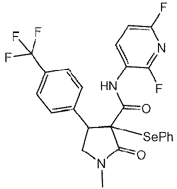

### Embodiment C23a

A compound which has the following formula,

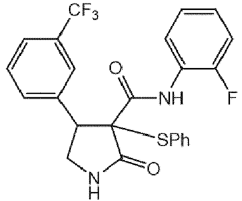

### Embodiment C23b

A compound which has the following formula,

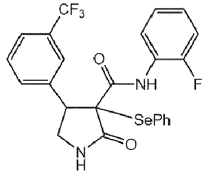

### Embodiment C24a

A compound which has the following formula,

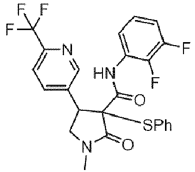

### Embodiment C24b

A compound which has the following formula,

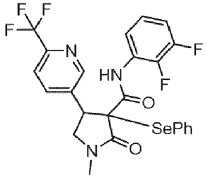

### Embodiment C25a

A compound which has the following formula,

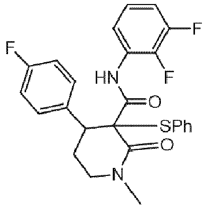

### Embodiment C25b

A compound which has the following formula,

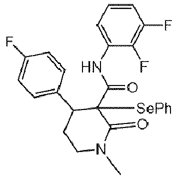

### Embodiment C26a

A compound which has the following formula,

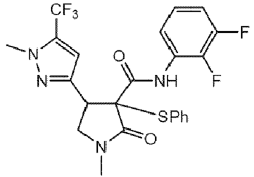

### Embodiment C26b

A compound which has the following formula,

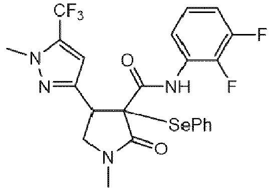

### Embodiment C27a

A compound which has the following formula,

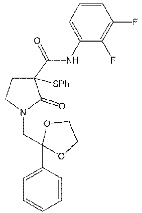

### Embodiment C27b

A compound which has the following formula,

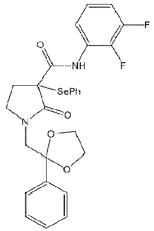

### Embodiment C28a

A compound which has the following formula,

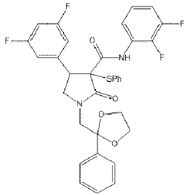

### Embodiment C28b

A compound which has the following formula,

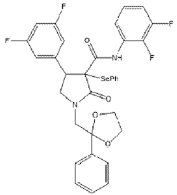

### Embodiment C29a

A compound which has the following formula,

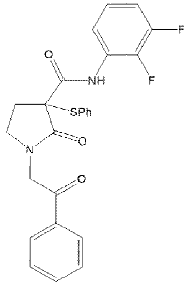

### Embodiment C29b

A compound which has the following formula,

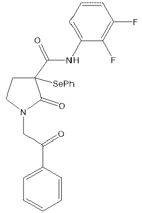

### Embodiment C30a

A compound which has the following formula,

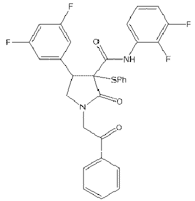

### Embodiment C30b

A compound which has the following formula,

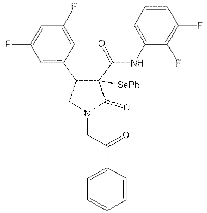

### Embodiment C31a

The compound of Embodiment **C2** and e.g. salts thereof wherein,
**R^{α}** is -C(O)NH**R**^{µ}
**A^{α}** is -N(**R*^{π}***)-
**nα is 1**
**A^{β}** is CH₂
**Q^{α}** is O
**Q^{β}** is NH
**R^{µ}** is phenyl ring, unsubstituted or substituted with up to 5 substituents each independently selected from **R^{ζ}**
**R^{β} is H**
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups, 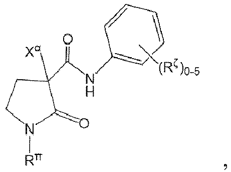 and wherein,
**R*^{π}*** and **R^{ζ},** have the same meanings as defined in Embodiment **A2.**

### Embodiment C31b

The compound of Embodiment **C2** and e.g. salts thereof wherein,
**R^{α}** is -C(O)NH**R^{µ}**
**A^{α}** is -N(**R*^{π}***)-
**nα** is 1
**A^{β}** is CH₂
**Q^{α} is O**
**Q^{β}** is NH
**R^{µ}** is phenyl ring, unsubstituted or substituted with up to 5 substituents each independently selected from **R^{ζ}**
**R^{β}** is H
**X^{α}** is Halogen 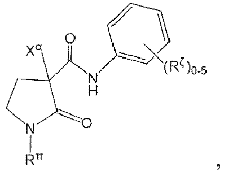 and
wherein,
**R*^{π}*** and **R^{ζ},** have the same meanings as defined in Embodiment **A2.**

### Embodiment C32a

The compound of Embodiment **C2** and e.g. salts thereof wherein,
**R^{α}** is -C(O)NH**R^{µ}**
**A^{α}** is -NH-
**nα** is 1
**A^{β}** is CH₂
**Q^{α}** is O
**Q^{β}** is NH
**R^{µ}** is phenyl ring, unsubstituted or substituted with up to 5 substituents each independently selected from **R^{ζ}**
**R^{β} is H**
**X^{α}** is SPh, SePh, S**G^{α}**, Se**G^{α}**
G^{α} is alkyl, haloalkyl, phenyl, phenyl substituted with 0-5 groups each selected from halogen; alkyl; and haloalkyl groups, 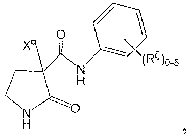 and
wherein,
**R^{ζ},** has the same meaning as defined in Embodiment **A2.**

### Embodiment C32b

The compound of Embodiment **C2** and e.g. salts thereof wherein,
**R^{α}** is -C(O)NH**R^{µ}** ,
**A^{α}** is -NH-,
**nα** is 1,
**A^{β}** is CH₂ ,
**Q^{α}** is O,
**Q^{β}** is NH,
**R^{µ}** is phenyl ring, unsubstituted or substituted with up to 5 substituents each independently selected from **R^{ζ},**
**R^{β}** is H,
**X^{α}** is Halogen, 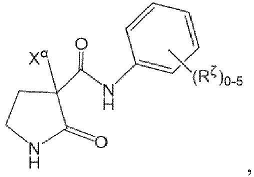 and wherein,
**R^{ζ},** has the same meaning as defined in Embodiment **A2.**

### Embodiment C33

The compound of Embodiment **C2** and e.g. salts thereof wherein,
**R^{α}** is -C(O)NH**R^{µ}** ,
**A^{α}** is -NH-,
**nα** is 2,
**A^{β}** is CH₂ ,
**Q^{α}** is O,
**Q^{β}** is NH,
**R^{µ}** is phenyl ring, unsubstituted or substituted with up to 5 substituents each independently selected from **Rζ**
**R^{β}** is H
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups, 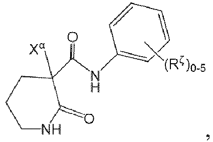 and
wherein,
**R^{ζ},** has the same meaning as defined in Embodiment **A2.**

### Embodiment C34

The compound of Embodiment **C2** and e.g. salts thereof wherein,
**R^{α}** is -C(O)NH**R^{µ}**
**A^{α}** is -N(Me)-
**nα** is 2
**A^{β}** is CH₂
**Q^{α}** is O
**Q^{β}** is NH
**R^{µ}** is phenyl ring, unsubstituted or substituted with up to 5 substituents each independently selected from **R^{ζ}**
**R^{β}** is H
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups, 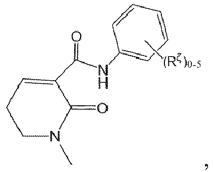 and wherein,
**R^{ζ},** has the same meaning as defined in Embodiment **A2.**

### Embodiment C35a

A compound defined by any one of the following structures, 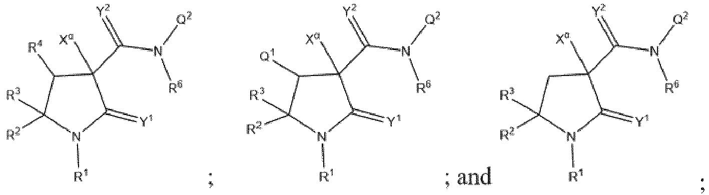 wherein Q¹, Q², R¹, R², R³, R⁴, R6, Y1 and Y2 etc. have the meanings as defined in Claim 1 of PCT/US2014/068073 published as WO 2015/084796. The text extracted from claim 1 on page 286, line 6 until page 289 line 23 of WO 2015/084796 being specifically incorporated by reference solely for the purpose of defining the structures of this embodiment, the text being reproduced in

### Embodiment A35a.

Wherein
X^{α} = SPh, SePh, SG^{α}, SeG^{α}
G^{α} = alkyl, haloalkyl, phenyl, phenyl substituted with 0-5 groups each selected from halogen; alkyl; and haloalkyl groups.

### Embodiment C35b

A Saturated Target compound having the following structure, 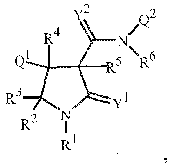 in combination with at least one compound defined by any one of the following formulae: 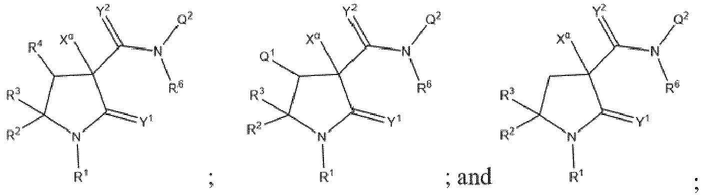 wherein Q¹, Q², R¹, R², R³, R⁴, R6, Y1 and Y2 etc. have the meanings as defined in Claim 1 of PCT/US2014/068073 published as WO 2015/084796. The text extracted from claim 1 on page 286, line 6 until page 289 line 23 of WO 2015/084796 being specifically incorporated by reference solely for the purpose of defining the structures of this embodiment, the text being reproduced in

### Embodiment A35a

And Wherein
**X^{α} =** SPh, SePh, SG^{α}, SeG^{α}
**G^{α}** = alkyl, haloalkyl, phenyl, phenyl substituted with 0-5 groups each selected from halogen; alkyl; and haloalkyl groups.

### Embodiment C36a

A compound defined by any one of the following structures, 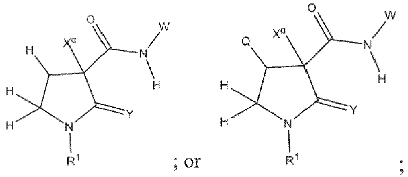
wherein Q, R¹, Y and W have the meanings as defined in Claim 1 of PCT/US2020/034232, published as WO2020/242946. The text from claim 1 on page 87, line 3 until page 88 line 6 of WO2020242946 being specifically incorporated herein by reference only for the purpose of defining the structures of this embodiment, the text being reproduced in Embodiment **A36a,** and wherein, **X^{α}** = SPh, SePh, SG^{α}, SeG^{α}, and
**G^{α}** = alkyl, haloalkyl, phenyl, phenyl substituted with 0-5 groups each selected from halogen; alkyl; and haloalkyl groups.

### Embodiment C36b

A Saturated Target compound having the structure, 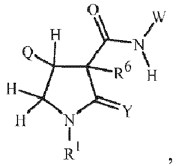 in combination with at least one compound defined by either one of the following formulae, 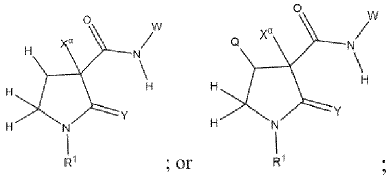
wherein Q, R¹, R⁶, Y and W etc. have the meanings as defined in Claim 1 of PCT/US2020/034232, published as WO 2020/242946. The text from claim 1 on page 87, line 3 until page 88 line 6 of WO2020/242946 being specifically incorporated herein by reference solely for the purpose of defining the structures of this embodiment the text being reproduced in Embodiment **A36a,** and wherein,
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups.

### Embodiment C37a

A compound defined by any one of the following formulae, 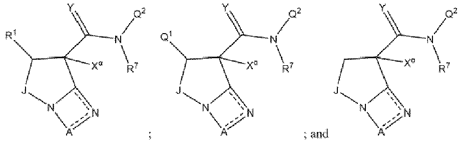
Wherein Q¹, Q², R¹, R², R⁷, Y, A and J etc. have the meanings as defined in Claim 1 of PCT/US2016/030450, published as WO 2016/182780. The text of claim 1 on page 101, line 3 until page 106 line 28 of WO 2016/182780 being specifically incorporated by reference solely for the purpose of defining the structures of this embodiment, the text being reproduced in Embodiment **A37a,** and wherein,
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups.

### Embodiment C37b

A compound for use as a pesticide, preferably, a herbicide synthetic process intermediate and/or marker having any one of the following structures, 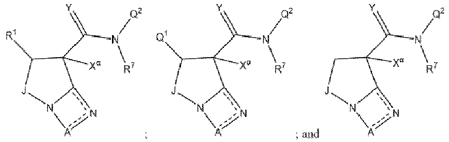
Wherein Q¹, Q², R¹, R², R⁷, Y, A and J etc. have the meanings as defined in Claim 1 of PCT/US2016/030450, published as WO 2016/182780. The text of claim 1 on page 101, line 3 until page 106 line 28 of WO 2016/182780 being specifically incorporated by reference solely for the purpose of defining the structures of this embodiment, the text being reproduced in Embodiment **A37a**,and wherein
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups.

### Embodiment C37c

A Saturated Target compound having the following structure, 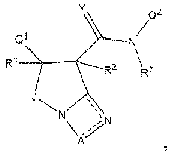 in combination with at least one compound defined by any one of the following structures, 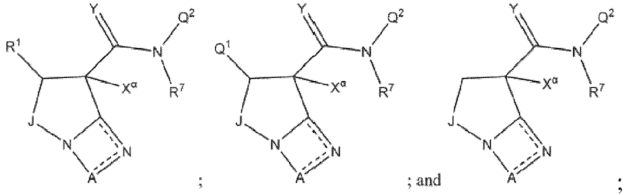 and wherein
Q¹, Q², R¹, R², R⁷, Y, A and J have the meanings as defined in Claim 1 of PCT/US2016/030450, published as WO 2016/182780. The text of claim 1 on page 101, line 3 until page 106 line 28 of WO 2016/182780 being specifically incorporated by reference solely for the purpose of defining the structures of this embodiment, the text being reproduced in Embodiment **A37a**,and wherein,
**X^{α}** is SPh, SePh, SG**^{α},** Se**G^{α},**
**G^{α}** is alkyl, haloalkyl, phenyl, phenyl substituted with 0-5 groups each selected from halogen; alkyl; and haloalkyl groups.

### Embodiment C38

A compound defined by either one of the following formulae, 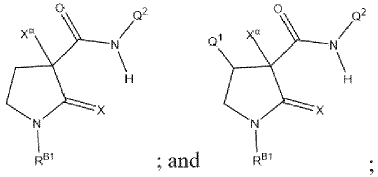
wherein Q¹, Q², R^{B1}, and X have the meanings as defined in Claim 1 of PCT/EP2020/052780 published as WO 2020/161147. The text from claim 1 on page 74, line 10 until page 75 line 10 of WO 2020/161147 being specifically incorporated by reference solely for the purpose of defining thee structures of this embodiment, the text being reproduced in Embodiment A38,
and wherein,
**X^{α}** is SPh, SePh, S**G^{α}**, Se**G^{α}**
G^{α} is alkyl, haloalkyl, phenyl, phenyl substituted with 0-5 groups each selected from halogen; alkyl; and haloalkyl groups.

### Embodiment C38a

A Saturated Target compound of the following Formula as defined in Claim 1 (B) of PCT/EP2020/052780 published as WO 2020/161147, 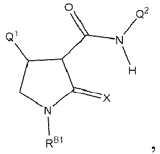 in combination with at least one compound defined by any one of the following structures, 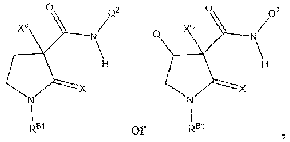
wherein Q¹, Q²,R^{B1}, and X etc. have the meanings as defined in Claim 1 of PCT/EP2020/052780 published as WO 2020/161147. The text from claim 1 on page 74, line 10 until page 75 line 10 of WO 2020/161147 being specifically incorporated by reference solely for the purpose of defining the structures of this embodiment, the text being reproduced in Embodiment A38, and wherein
**X^{α}** is SPh, SePh, S**G^{α}**, Se**G^{α}**,
**G^{α}** is alkyl, haloalkyl, phenyl, phenyl substituted with 0-5 groups each selected from halogen; alkyl; and haloalkyl groups.

### Embodiment C39

a compound defined by either one of the following formulae, 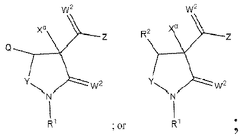 wherein Q,W², R¹, Y and Z etc. have the meanings as defined in Claim 1 of PCT/EP2018/069001 published as WO2019/025156. The German language text from claim 1 on page 109, line 3 until page 112 line 6 of WO2019025156 being specifically incorporated by reference only for the purpose of defining the structures of this embodiment.

And Wherein
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups.

### Embodiment C40

A compound defined by any one of the following formulae, 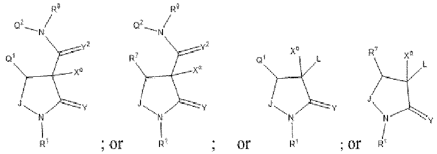
wherein Q¹, Q², R¹, R⁷, R⁹, J, L, Y and Y² etc. have the meanings as defined in Claim 1 of PCT/US2018/035017 published as WO 2018/222647. The text from claim 1 on page 84, line 5 until page 89 line 27 of WO 2018/222647 being specifically incorporated by reference solely for the purpose of defining the structures of this embodiment, the text being reproduced in Embodiment **A41,** and wherein,
**X^{α}** is SPh, SePh, S**G^{α}**, Se**G^{α}**;
**G^{α}** is alkyl, haloalkyl, phenyl, phenyl substituted with 0-5 groups each selected from halogen; alkyl; and haloalkyl groups.

### Embodiment C41

A **Saturated Target** of the following formula, N-oxides, and salts thereof as defined in Claim 1 of PCT/US2018/035017 published as WO 2018/222647, 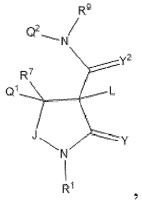 in combination with at least one compound each of which defined by any one of the following structures, 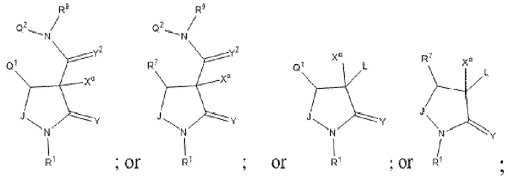 wherein,
Q¹, Q², R¹, R⁷, R⁹, J, L, Y and Y² etc. have the meanings as defined in Claim 1 of PCT/US2018/035017 published as WO 2018/222647, the text from claim 1 on page 84, line 5 until page 89 line 27 of WO 2018/222647 being specifically incorporated by reference solely for the purpose of defining the structures of this embodiment, the text being reproduced in Embodiment **A41,** and wherein,
**X^{α}** is SPh, SePh, S**G^{α}**, Se**G^{α}**;
**G^{α}** is alkyl, haloalkyl, phenyl, phenyl substituted with 0-5 groups each selected from halogen; alkyl; and haloalkyl groups.

### Embodiment C42

A compound defined by any one of the following formulae, 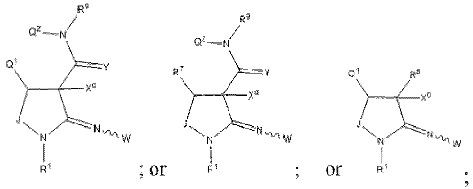
wherein Q¹, Q², R¹, R⁷, R⁸, R⁹, J, Y and W, etc. have the meanings as defined in Claim 1 of PCT/US2018/035015 published as WO2018/222646. The text from claim 1 on page 82, line 6 until page 86 line 21 of WO2018/222646 being specifically incorporated by reference solely for the purpose of defining the structures of this embodiment
And Wherein
X^{α} is SPh, SePh, SG^{α}, SeG^{α}
G^{α} is alkyl, haloalkyl, phenyl, phenyl substituted with 0-5 groups each selected from halogen; alkyl; and haloalkyl groups

### Embodiment C42a

A Saturated Target, N-oxides salts and stereoisomers thereof, wherein the Saturated Target is a compound as defined in Claim 1 of PCT/US2018/035015 published as WO 2018/222646, having the following formula, 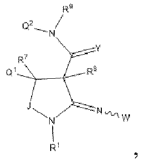 in combination with at least one compound each defined by any one of the following structures, 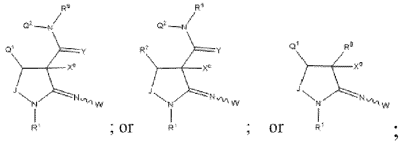 wherein,
Q¹, Q², R¹, R⁷, R⁸, R⁹, J, Y and W, etc. have the meanings as defined in Claim 1 of PCT/US2018/035015 published as WO 2018/222646. The text from claim 1 on page 82, line 6 until page 86 line 21 of WO2018/222646 being specifically incorporated herein by reference solely for the purpose of defining the structures of this embodiment, the text being reproduced in Embodiment **A42,** and wherein,
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups.

### Embodiment C43

A compound defined by either one of the formulae, 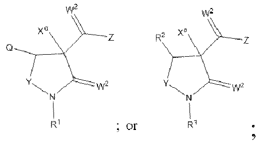 wherein,
Q,W², R¹, R², Y and Z etc. have the meanings as defined in Claim 1 of PCT/EP2018/057628 published as WO2018/184890. The text from claim 1 on page 133, line 9 until page 136 line 4 of WO2018184890 being specifically incorporated herein by reference solely for the purpose of defining the structures of this embodiment.
And Wherein
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups

### Embodiment C43a

A Saturated Target and salts thereof wherein the Saturated Target and salts thereof wherein the Saturated Target is a compound as defined in Claim 1 of PCT/EP2018/057628 published as WO2018/184890 formula, 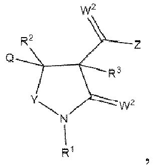 in combination with at least one compound defined by either one of the following structures, 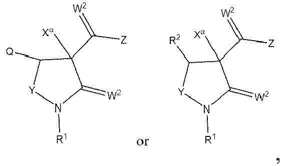 wherein,
Q,W², R¹, R², Y and Z etc. have the meanings as defined in Claim 1 of PCT/EP2018/057628 published as WO 2018/184890. The German language text from claim 1 on page 133, line 9 until page 136 line 4 of WO 2018/184890 being specifically incorporated herein by reference solely for the purpose of defining the structures of this embodiment, and wherein
**X^{α}** is SPh, SePh, S**G^{α}**, Se**G^{α}**
**G^{α}** is alkyl, haloalkyl, phenyl, phenyl substituted with 0-5 groups each selected from halogen; alkyl; and haloalkyl groups

### Embodiment D1a

The process as described generally above for preparing an Unsaturated Precursor of formula **2** from a compound of formula 3 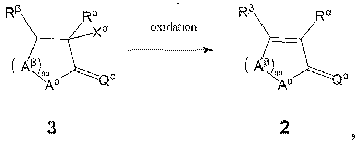 comprising oxidation of the corresponding compound of formula **3** using an Oxidant such as *meta-*Chloroperoxybenzoic acid (mCPBA) or Hydrogen peroxide (H₂O₂), wherein each of **X^{α}, R^{α}, R^{β}, A^{α}, A^{β}, nα,** and **Q^{α},** have the same meanings as in embodiment **C1a.**

This process is preferred when **X^{α}** is thioether or selenoether.

### Embodiment D1b

The process as described generally above for preparing a Unsaturated Precursor of formula **2** from a compound of formula **3** 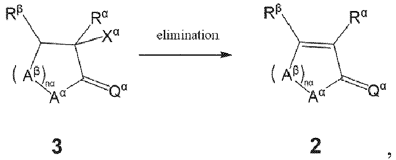 comprising an elimination reaction utilizing an activator such as a Base or a Lewis acid, wherein each of **X^{α}, R^{α}, R^{β}, A^{α}, A^{β}, nα,** and **Q^{α},** have the same meanings as in embodiment **C1a.**

### Embodiment D2a

The process of Embodiment **D1a** comprising, dissolving a compound of formula **3**in suitable solvent e.g. Acetic acid, agitating (e.g. stirring etc.) for a period of time exemplified by about 20mins, adding portion wise, an Oxidant exemplified by *meta*-Chloroperoxy benzoic acid (mCPBA), or Hydrogen peroxide (H₂O₂), at suitable temperature. Then, agitating the reaction mass at substantially the same temperature for some time e.g. about 2 hrs. The reaction mass can be quenched e.g. into aq.NaHCOs solution, and extracted with suitable solvent typically two times. The combined organic layer can be concentrated to obtain the corresponding Unsaturated Precursor of formula **2.**

### Embodiment D2b

The process of Embodiment **D1a** comprising, dissolving a compound of formula **3**in suitable solvent e.g. chlorobenzene, agitating (e.g. by stirring etc.) for a time exemplified by about 20mins, adding portion wise, an Activator exemplified by Base or Lewis acid at suitable temperature. Then, agitating the reaction mass at substantially the same temperature for some time e.g. about 2 hrs. The reaction mass can be quenched e.g. into NH₄Cl solution, and extracted with suitable solvent typically two times. The combined organic layer can be concentrated to obtain the corresponding Unsaturated Precursor of formula **2.**

### Embodiment D3a

A process exemplified by the following scheme, 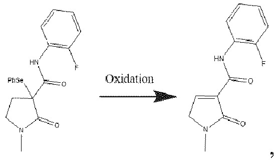 comprising, oxidation of the compound of the following formula, 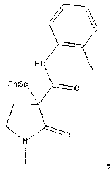 using oxidant, exemplified by *meta*-Chloroperoxy benzoic acid (mCPBA), or Hydrogen peroxide (H₂O₂). to obtain the corresponding Unsaturated Precursor compound.

### Embodiment D3b

A process exemplified by the following scheme, 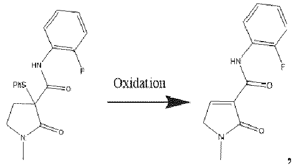 comprising, oxidation of the compound of the following formula, 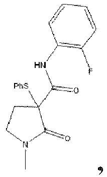 using oxidant, exemplified by *meta*-Chloroperoxy benzoic acid (mCPBA), or Hydrogen peroxide (H₂O₂). to obtain the corresponding Unsaturated Precursor.

### Embodiment D3c

A process exemplified by the following scheme, 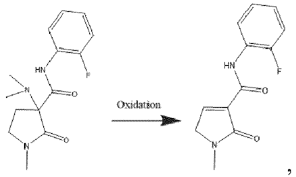 comprising, oxidation of the compound of the following formula, 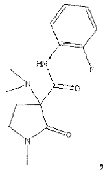 using oxidant exemplified by *meta*-Chloroperoxy benzoic acid (mCPBA), or Hydrogen peroxide (H₂O₂). to obtain the corresponding Unsaturated Precursor compound.

### Embodiment D4a

A process exemplified by the following scheme, 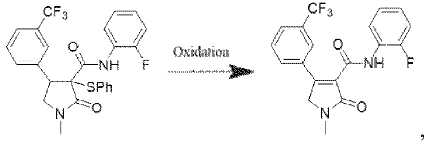 comprising, oxidation of the compound of the following formula, 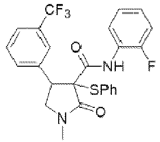 using oxidant exemplified by *meta*-Chloroperoxy benzoic acid (mCPBA), or Hydrogen peroxide (H₂O₂). to obtain the corresponding Unsaturated Precursor compound.

### Embodiment D4b

The process exemplified by the following scheme, 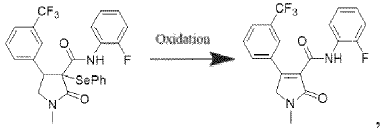 comprising, oxidation of the compound of the following formula, 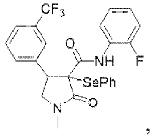 using oxidant exemplified by *meta*-Chloroperoxy benzoic acid (mCPBA), or Hydrogen peroxide (H₂O₂). to obtain the corresponding Unsaturated Precursor compound.

### Embodiment D5a

The process exemplified by the following scheme, 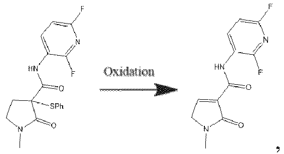 comprising, oxidation of the compound of the following formula, 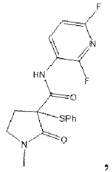 using oxidant exemplified by *meta*-Chloroperoxy benzoic acid (mCPBA), or Hydrogen peroxide (H₂O₂). to obtain the corresponding Unsaturated Precursor compound.

### Embodiment D5b

The process exemplified by the following scheme, 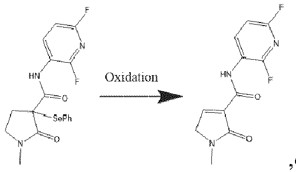 comprising, oxidation of the compound of the following formula, 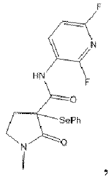 using oxidant exemplified by *meta*-Chloroperoxy benzoic acid (mCPBA), or Hydrogen peroxide (H₂O₂). to obtain the corresponding Unsaturated Precursor compound.

### Embodiment D6a

The process exemplified by the scheme, 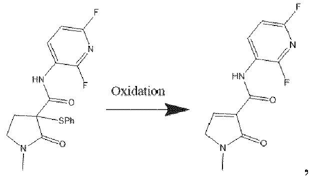 comprising, oxidation of the compound of the following formula, 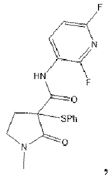 using oxidant exemplified by *meta*-Chloroperoxy benzoic acid (mCPBA), or Hydrogen peroxide (H₂O₂) to obtain the corresponding Unsaturated Precursor compound.

### Embodiment D6b

The process exemplified by the following scheme, 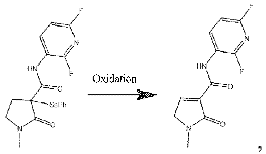 comprising, Oxidation of the compound of the following formula, 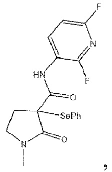 using oxidant exemplified by *meta*-Chloroperoxy benzoic acid (mCPBA), or Hydrogen peroxide (H₂O₂) to obtain the corresponding Unsaturated Precursor compound.

### Embodiment D7a

The process exemplified by the scheme, 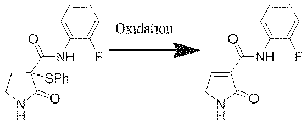 comprising, Oxidation of the compound of the following formula, 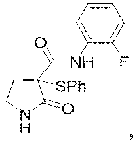 using oxidant exemplified by *meta*-Chloroperoxy benzoic acid (mCPBA), or Hydrogen peroxide (H₂O₂) to obtain the corresponding Unsaturated Precursor compound.

### Embodiment D7b

The process exemplified by the following scheme, 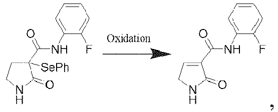 comprising, oxidation of the compound of the following formula, 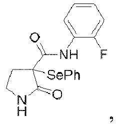 using oxidant exemplified by *meta*-Chloroperoxy benzoic acid (mCPBA), or Hydrogen peroxide (H₂O₂) to obtain the corresponding Unsaturated Precursor compound.

### Embodiment D8a

The process exemplified by the following scheme, 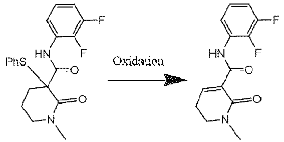 comprising, Oxidation of the compound of the following formula, 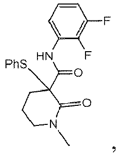 using oxidant exemplified by *meta*-Chloroperoxy benzoic acid (mCPBA), or Hydrogen peroxide (H₂O₂) to obtain the corresponding Unsaturated Precursor compound.

### Embodiment D8b

The process exemplified by the following scheme, 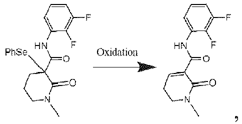 comprising, Oxidation of the compound of the following formula, 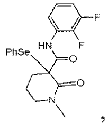 using Oxidant exemplified by *meta*-Chloroperoxy benzoic acid (mCPBA), or Hydrogen peroxide (H₂O₂) to obtain the corresponding Unsaturated Precursor compound.

### Embodiment D9a

The process exemplified by the following scheme, 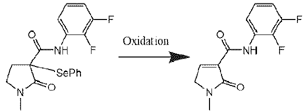 comprising, Oxidation of the compound of the following formula, 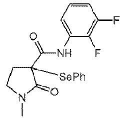 using Oxidant exemplified by *meta*-Chloroperoxy benzoic acid (mCPBA), or Hydrogen peroxide (H₂O₂) to obtain the corresponding Unsaturated Precursor compound.

### Embodiment D9b

The process exemplified by the scheme, 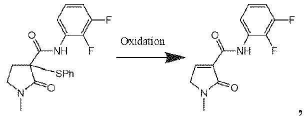 comprising, oxidation of the compound of the following formula, 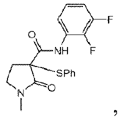 using Oxidant exemplified by *meta*-Chloroperoxy benzoic acid (mCPBA), or Hydrogen peroxide (H₂O₂) to obtain the corresponding Unsaturated Precursor compound.

### Embodiment E1

A compound of Formula **4** and e.g. salts etc. thereof, 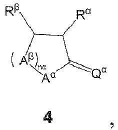 for use as an intermediate in the method of preparation of a corresponding Saturated Target of formula 1, 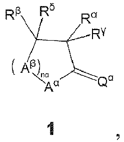 wherein, **R^{α}, R^{β}, A^{α}, A^{β}, nα** and **Q^{α}** have the same meanings as defined in Embodiment **A2,** and **R^{δ}** and **R^{γ}** have the same meanings as defined in Embodiment **B1.**

### Embodiment E2

A Saturated Target compound in combination with the intermediate compound of Formula **4** as defined in Embodiment **E1** via which it was produced, as an impurity.

### Embodiment F1a

The process as generally described above, for preparing a compound of formula **3** from a compound of formula **4,** 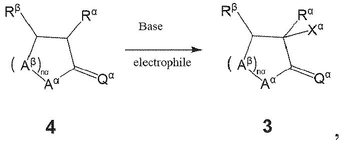 comprising addition of a **X^{α}** "leaving" group by utilizing Base and Electrophile,
wherein each of **X^{α}, R^{α}, R^{β}, A^{α}, A^{β}, nα,** and **Q^{α},** have the same meanings as in embodiment **C1a.** This process is preferred when **R^{β}** is H.

### Embodiment F1b

The process as described generally above, for preparing a compound of formula 3 from a compound of formula 4, 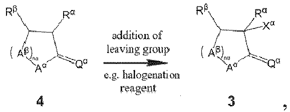 comprising addition of a **X^{α}** leaving group by utilizing a leaving group source such as halogenation reagent, wherein each of **X^{α}, R^{α}, R^{β}, A^{α}, A^{β}, nα,** and **Q^{α},** have the same meanings as in embodiment **C1a.**

### Embodiment F1c

The process as described generally above, for preparing a compound of formula **3** from a compound of formula **4** 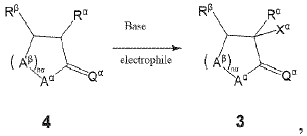 comprising addition of a **X^{α}** group, by utilizing Base and Electrophile, wherein each of **X^{α}, R^{α}, R^{β}, A^{α}, A^{β}, nα,** and **Q^{α},** have the same meanings as in embodiment **C1b.** This process is preferred when R^{β} is H.

### Embodiment F2a

The process of Embodiment **F1a** comprising, dissolving a compound of formula 4 in suitable solvent with Base such as Sodium Hydride at a specified temperature, typically by cooling, agitating (e.g. stirring etc.), for a time exemplified by about 20mins, adding Electrophile exemplified by any of diphenyl disulfide; phenyl hypochlorothioite; phenyl hypobromoselenoite, dissolved in suitable solvent at substantially the same temperature, agitating the reaction mass at substantially the same temperature for some time. The reaction mass can be quenched e.g. into NH₄Cl solution and extracted with suitable solvent typically two or more times, the combined organic layer can be washed and dried over Na₂SO₄ and concentrated to obtain the corresponding compound of formula 3. This process is preferred when R^{β} is H.

### Embodiment F2b

The process of Embodiment **F1b** comprising, dissolving a compound of formula 4 in suitable solvent together with a leaving group source exemplified by a halogenation reagent (e.g. Bromine source), at a specific temperature, optionally adding a Base and/or catalyst, (catalytic component comprising one or more of, catalyst; pre-catalyst; co-catalyst). Agitating the reaction mass (e.g. stirring etc.), at substantially the same temperature for some time exemplified by about 2 hours, quenching the reaction mass and extracting with a suitable solvent. Preferably the mass is washed again and/or the combined organic layers are washed, optionally dried e.g. over Na₂SO₄ and concentrated to obtain the corresponding compound of formula 3.

### Embodiment F3

The process of any one of Embodiments **F2a** or **F2b,** further comprising a step of purifying the compound of formula 3 obtained from the process.

### Embodiment F4

The process of any one of Embodiments **F1-F3** employing any of diphenyl disulfide, and phenyl hypochlorothioite. as Electrophile.

### Embodiment G1

A Storage Marker having the following structure, and e.g. salts thereof, 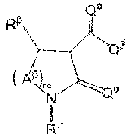 wherein,
**R^{β}, Q^{α}, Q^{β}** and **R*^{π}*,** have the same meanings as defined in Embodiment **A2**

### Embodiment G2

A Storage Marker compound of formula D, obtainable by exposure of Tetflupyrolimet to sunlight

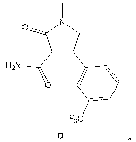

### Embodiment DD1

A Storage Marker compound of the following formula, 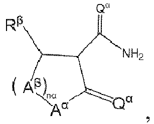 wherein,
**R^{β}, Q^{α}, A^{β}, A^{α}** and **nα,** have the same meanings as defined in Embodiment **A2.**

### Embodiment DD2

A Storage Marker compound of the following formula, 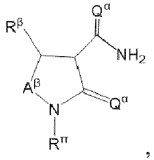 wherein,
**R^{β}, Q^{α}, A^{β}** and **R*^{π}*,** have the same meanings as defined in Embodiment **A2.**

### Embodiment DD3

A Storage Marker compound of the following formula, 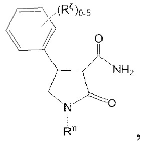 wherein,
**R*^{π}*** and **R^{ζ},** have the same meanings as defined in Embodiment **A2.**

### Embodiment DD4

A Storage Marker compound of the following formula, 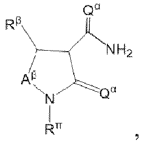 wherein,
**R^{β}, Q^{α}, A^{β}** and **R*^{π}*,** have the same meanings as defined in Embodiment **A2.**

### Embodiment DD5

A Storage Marker compound of the following formula, 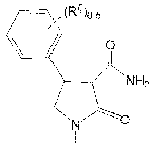 wherein, each **R^{ζ},** has the same meanings as defined in Embodiment **A2.**

### Embodiment DD6

A Storage Marker compound of the following formula,

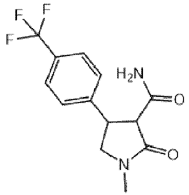

### Embodiment DD7

A Storage Marker compound of the following formula,

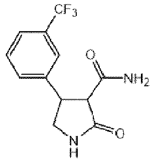

### Embodiment DD8

A Storage Marker compound of the following formula,

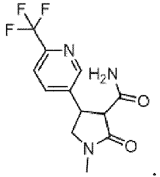

### Embodiment DD9

A Storage Marker compound of the following formula,

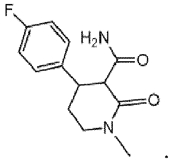

### Embodiment DD10

A Storage Marker compound of the following formula,

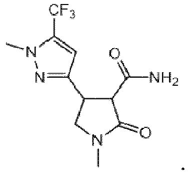

### Embodiment DD11

A Storage Marker compound of the following formula,

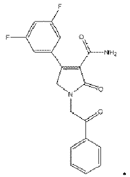

### Embodiment H1

A process for preparing a Storage Marker compound from a corresponding Saturated Target compound, in the following general scheme, wherein the Saturated Target is exposed to sunlight for sufficient time for detectable quantities of the corresponding Storage Marker to be formed when measured by an analytical method exemplified by HPLC and particularly by HPLC-MS. 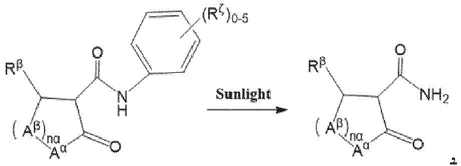 wherein,
**R^{β}, A^{β}, A^{α}, R^{ζ}** and **nα,** have the same meanings as defined in Embodiment **A2.**

### Embodiment H2

A process for preparing a Storage Marker compound from a corresponding Saturated Target compound, in the following general scheme, 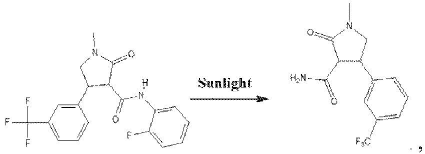 comprising exposing a composition, such as a solution, of the Saturated Target to sunlight for sufficient time for detectable quantities of the corresponding Storage Marker to be formed in the composition, and particularly, quantities above the limit of quantitation, using analytical techniques exemplified by HPLC-MS.

### Embodiment H2a

A process for preparing the corresponding Storage Marker compound from a Saturated Target compound which is, (3S,4S)-2'-fluoro-1-methyl-2-oxo-4-[3-(trifluoromethyl)phenyl]pyrrolidine-3-carboxanilide, in the following scheme, 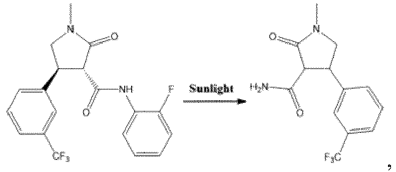 comprising exposing a composition, such as a solution, of the (3S,4S)-2'-fluoro-1-methyl-2-oxo-4-[3-(trifluoromethyl)phenyl]pyrrolidine-3-carboxanilide to sunlight for sufficient time for detectable quantities of the corresponding Storage Marker to be formed in the composition, and particularly, quantities above the limit of quantitation, using analytical techniques exemplified by HPLC-MS.

### Embodiment H3

The process of either one of Embodiments H2, or **H2a,** wherein the composition comprises a solution, especially an aqueous solution, that is maintained at a predefined level of acidity/basicity, preferably, of pH, by a method exemplified by inclusion of a suitable buffer in the preferably, aqueous, solution.

### Embodiment H4

The process of Embodiment **H3,** wherein the solution of the Saturated Target is maintained at a level of Acidity/Basicity exemplified by an aqueous solution of Saturated Target having a pH selected from about 4+1; 7±1; and 10±1, by inclusion of effective concentrations of suitable buffers in the preferably aqueous solution.

### Embodiment H5

The process of any one of Embodiments **H2; H2a; H3;** and **H4,** wherein the rate at which the corresponding Storage Marker is formed from a Saturated Target compound by exposure to sunlight, is further mediated by the selection of the acidity/basicity of the composition of comprising the Saturated Target.

### Embodiment H6

The process of any one of Embodiments **H3, H4** and **H5,** wherein the composition is an aqueous solution, and wherein the rate at which the corresponding Storage Marker is formed from the Saturated Target by exposure to sunlight, is mediated by the selection of the pH of the aqueous solution of the Saturated Target.

### Embodiment H7

A process for minimizing the rate of conversion of 2'-fluoro-1-methyl-2-oxo-4-[3-(trifluoromethyl)phenyl]pyrrolidine-3-carboxanifide in a composition thereof, to the corresponding Storage Marker, comprising minimizing and/or avoiding the exposure to sunlight of said composition and/or mitigating the converting effect of said exposure to sunlight.

### Embodiment H8

A process for minimizing the rate of conversion of Tetflupyrolimet in a composition thereof to the corresponding Storage Marker, comprising minimizing the time and extent to which said composition is exposed to sunlight and/or mitigating the converting effect of said exposure to sunlight.

### Embodiment H9

A process for minimizing the rate of conversion of Tetflupyrolimet in a composition thereof, to the corresponding Storage Marker, , comprising including in said composition, at least one of, ingredient; excipient; adjuvant; carrier; or any additive that can mitigate the converting effect of said exposure to sunlight, or reduce the rate at which said conversion would occur on exposure to sunlight absent said ingredient, excipient, adjuvant, carrier or additive.

### Embodiment H10

A process for minimizing the rate of conversion of Tetflupyrolimet in a product comprising a Tetflupyrolimet composition, including solutions thereof, to the corresponding Storage Marker, during manufacturing, packaging, labeling, transportation, and storage of said product, comprising mitigating or overcoming the effects of exposure to sunlight of said product, by including said composition within light-resistant, preferably substantially opaque, packaging and/or containers (e.g. a container-closure system), that protects the contents from the converting effects of sunlight by virtue of the specific properties of the material of which it is composed, including any coating applied to it, including clear, colorless, or translucent containers made light-resistant by means of a light-resistant or opaque covering or by use of secondary packaging, or within amber colored containers, wherein light-resistance and opacity relates to the wavelengths of light mediating conversion of Tetflupyrolimet to the corresponding Storage Marker.

### Embodiment H11

The process and/or composition of any one of Embodiments **H1** to **H10** wherein the Saturated Target compound, preferably, Tetflupyrolimet, is in a liquid, solution, emulsion or suspension composition.

### Embodiment H12

A process for minimizing the rate at which an aqueous solution of Tetflupyrolimet is converted to the corresponding Storage Marker compound when said solution is exposed to sunlight, comprising maintaining the pH of said aqueous solution to a range of between about 6 to about 8 and preferably about 7.

### Embodiment H13

A process to determine the time period that a stored composition comprising a Saturated Target compound, especially, a Tetflupyrolimet composition, was exposed to sunlight while in storage, said process comprising measuring and quantifying the relative content of the corresponding Storage Marker compound as compared to the content of the Saturated Target in the composition, and evaluating the period of exposure to sunlight by reference to a standard calibrating rate of conversion in one or more reference compositions comprising the Saturated Target compound.

### Embodiment H14

The process of Embodiment **H13** wherein said measurement and quantifying of said relative content is by a method exemplified by calculating the ratio of the respective areas under the resolved characterizing peaks of each compound in a test sample by appropriate HPLC analysis.

### Embodiment H15

The process of any one of embodiments **H13** and **H14,** wherein said standard calibrating rate is determined for a reference composition with the same acidity/basicity as the stored test composition.

### Embodiment H16

The process of any one of Embodiments **H13, H14** and **H15,** wherein said standard calibrating rate is determined for a reference composition packaged in the same container system as the stored test composition.

### Embodiment I1

A stored packaged composition comprising a combination of detectable quantities of a Storage Marker compound and a corresponding Saturated Target compound from which it was converted, by a process exemplified by the process of Embodiment **H2.**

### Embodiment I2

The stored packaged composition of Embodiment **I1** comprising detectable, preferably measurable, quantities of a Storage Marker compound of the following formula, 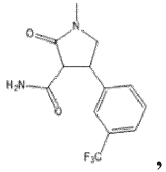 including any isomer or enantiomer thereof, in combination with a Saturated Target compound of the following formula, 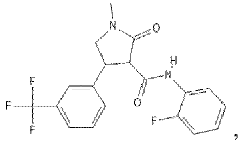 including any isomer or enantiomer thereof, especially exemplified by (3S,4S)-2'-fluoro-1-methyl-2-oxo-4-[3-(trifluoromethyl)phenyl]pyrrolidine-3-carboxanilide, of the following formula,

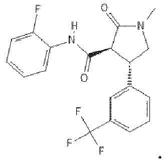

### Embodiment I3

The stored packaged composition of any one of Embodiment **I1** and **I2,** wherein the composition comprises a solution, especially an aqueous solution, of the Saturated Target that is maintained at a predefined level of acidity/basicity, preferably pH, by a method exemplified by inclusion of a suitable buffer system in the preferably aqueous, solution.

### Embodiment I4

The stored packaged composition of Embodiment **I3,** wherein the solution of the Saturated Target is maintained at a level of Acidity/Basicity exemplified by an aqueous solution of the Saturated Target having a pH selected from about 4±1; about 7±1; and about 10±1 maintained, by inclusion of suitable buffers in the preferably aqueous solution at concentrations effective to maintain said level of Acidity/Basicity.

### Embodiment I5

The stored packaged composition of any one of Embodiments **I1 - I4,** wherein the rate at which the Storage Marker compound, especially the compound of the following formula, 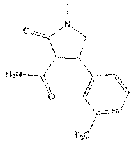 was formed from the corresponding Saturated Target, especially from the compound of the following formula, 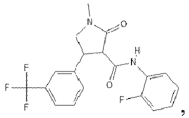 by exposure to sunlight on storage, was further mediated by the selection of the acidity/basicity of the composition of said Saturated Target over the storage period.

### Embodiment 16

A packaged aqueous-based composition of Tetflupyrolimet characterized in that the rate at which the Tetflupyrolimet is converted to a corresponding Storage Marker of the following formula, 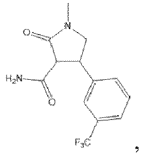 including any isomer, enantiomer or mixture thereof, when the composition is exposed to sunlight during storage is minimized, wherein said composition is adapted to maintain pH during storage, at a range of between about 6 to about 8 and preferably about 7, by inclusion of an ingredient, excipient or other component, exemplified by a buffer or buffering system in the composition.

### Embodiment I7

An agricultural product comprising a composition of an 2'-fluoro-1-methyl-2-oxo-4-[3-(trifluoromethyl)phenyl]pyrrolidine-3-carboxanilide especially, Tetflupyrolimet, characterized in that the rate at which conversion to the corresponding Storage Marker of the following formula, 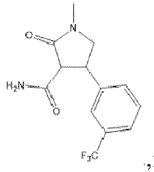 including any isomer, enantiomer or mixture thereof, occurs during storage, is minimized, wherein said product is adapted to limit, reduce or minimize the time and extent or to substantially avoid exposure to sunlight of said composition within said product during storage, wherein said minimization is as compared to an agricultural Tetflupyrolimet product without said adaptation.

### Embodiment 18

An agricultural product comprising a Tetflupyrolimet composition protected to a detectable degree against sunlight-mediated conversion to the corresponding Storage Marker compound of the following formula, 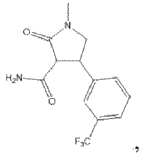 during manufacturing, packaging, labeling, transportation, or storage, of said composition, characterized in that the product comprises said composition contained within and/or shielded by substantially light-resistant, preferably, opaque packaging and/or a container-closure system that protects the contents from the effects of sunlight by virtue of the specific properties of the material of which it is composed, including any coating applied to it, including clear and colorless or translucent container made light-resistant by means of an opaque covering or by use of secondary packaging, or within amber colored containers.

### Embodiment 19

A Tetflupyrolimet composition characterized by a minimized rate or extent of sunlight-mediated conversion of Tetflupyrolimet to the corresponding Storage Marker of the following formula, 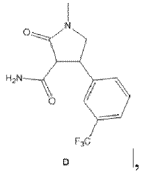 comprising at least one of, ingredient, excipient, adjuvant, carrier or additive that can mitigate the converting effect of exposure to sunlight or reduce the rate at which said conversion would occur on exposure to sunlight absent said ingredient, excipient, adjuvant, carrier or additive.

### Embodiment 110

A Tetflupyrolimet composition stabilized to at least a measurable degree against sunlight-mediated conversion to the corresponding Storage Marker, during manufacturing, packaging, labeling, transportation, and storage, characterized in that said composition comprises at least one of, ingredient, excipient, adjuvant, carrier or additive that can mitigate the converting effect of exposure to sunlight and/or reduce the rate at which said conversion occurs absent said ingredient, excipient, adjuvant, carrier or additive.

### Embodiment I11

A product comprising a Tetflupyrolimet composition shielded against sunlight-mediated conversion to the corresponding Storage Marker during any of, manufacturing; packaging; labeling; transportation; or storage; of said composition, characterized in that the product comprises said Tetflupyrolimet composition contained within substantially light-resistant, preferably, opaque packaging substantially as detailed in Embodiment **H10.**

### Embodiment I12

A Tetflupyrolimet composition stabilized to at least a measurable degree against conversion to the corresponding Storage Marker or displaying a measurably reduced rate of conversion to the corresponding Storage Marker during manufacturing, packaging, labeling, transportation, and storage, as compared to the standard calibrating rate of conversion in one or more reference compositions comprising the Tetflupyrolimet, characterized in that said composition comprises at least one component which is any, some or all, of,
a) a buffer which maintains the pH of the composition to 7± 1;
b) a UV absorber;
c) an ingredient etc. that exhibits the absorption spectra substantially similar to that of the Tetflupyrolimet exemplified by a suitable dye, colorant or pigment;
d) an ingredient etc. capable of reducing the amount of sunlight impinging on the material;
e) an opacifier;
f) a reflecting pigment such as titanium dioxide;
g) a light protecting coating over a least a portion of said Tetflupyrolimet in said composition;
h) a complex of Tetflupyrolimet with a complexing agent exemplified by an inclusion complex with e.g. a cyclodextrin;
wherein said reference compositions comprise Tetflupyrolimet absent said component.

### f

### Embodiment I14

A buffered Tetflupyrolimet composition comprising less than a measurable, preferably less than a detectable, amount of, the corresponding Storage Marker compound after storage for a period equal to, or greater than 2 months in a sunlit environment.

### Embodiment I15

The product and or composition of any one of embodiments **11** to I**12** wherein the Saturated Target preferably, Tetflupyrolimet is in a liquid, solution, emulsion, or suspension composition.

### Embodiment J1

A crystalline polymorph **Form I** of
N-(2-fluorophenyl)-1-methyl-2-oxo-4-[3-(trifluoromethyl)phenyl]-3-pyrrolidinecarboxamide which exhibits at least one of the following properties:
an infrared spectrum substantially as shown in **Figure 9****.**
an X-ray powder diffraction pattern substantially as shown in **Figure 5****.**
an X-ray powder diffraction pattern having a differentiating peak expressed in 20 (± 0.20) at 9.6,
an X-ray powder diffraction pattern having at least 4 of the characteristic peaks expressed in 20 (± 0.20) at 9.6, 15.0, 17.3, 17.9, 21.6 and 24.6.

### Embodiment J2

A crystalline polymorph **Form I** of Tetflupyrolimet which exhibits at least one of the following properties:
an infrared spectrum substantially as shown in **Figure 9****.**
an X-ray powder diffraction pattern substantially as shown in **Figure 5****.**
an X-ray powder diffraction pattern having a differentiating peak expressed in 20 (± 0.20) at 9.6,
an X-ray powder diffraction pattern having at least 4 of the characteristic peaks expressed in 20 (± 0.20) at 9.6, 15.0, 17.3, 17.9, 21.6 and 24.6.

### Embodiment J3

A composition comprising the crystalline polymorph **Form I** Tetflupyrolimet as defined in any one of Embodiments **J1** or **J2.**

### Embodiment J4

A pesticidal, preferably, herbicidal composition comprising the crystalline polymorph **Form I** of any one of embodiments **J1** and **J2;** and an herbicidally acceptable diluent or carrier.

### Embodiment J5

A pesticidal, preferably, herbicidal composition prepared from a crystalline polymorph **Form I** of any one of embodiments **J1** and **J2.**

### Embodiment K1

A crystalline polymorph Form II of
N-(2-fluorophenyl)-1-methyl-2-oxo-4-[3-(trifluoromethyl)phenyl]-3-pyrrolidinecarboxamide which exhibits at least one of the following properties:
An infrared (IR) absorption spectrum substantially as shown in **Figure 10****.**

An infrared (IR) absorption spectrum having at least one characteristic peak selected from the following values expressed as cm-1 (± 1 cm-1) at 1678, 1546, 1395, 946, 924, 885, 825 and 662. An X-ray powder diffraction pattern substantially as shown in **Figure 6****.**

An X-ray powder diffraction pattern having a differentiating peak expressed in 20 (± 0.20) at 6.9, an X-ray powder diffraction pattern having at least 4 of the characteristic peaks expressed in 20 (± 0.20) selected from the following values at 6.9, 11.2, 17.7, 23.5 and 26.3.

### Embodiment K2

A crystalline polymorph Form **II** of Tetflupyrolimet which exhibits at least one of the following properties:
An infrared (IR) absorption spectrum substantially as shown in **Figure 10****.**

An infrared (IR) absorption spectrum having at least one characteristic peak selected from the following values expressed as cm-1 (± 1 cm-1) at 1678, 1546, 1395, 946, 924, 885, 825 and 662. An X-ray powder diffraction pattern substantially as shown in **Figure 6****.**

An X-ray powder diffraction pattern having a differentiating peak expressed in 20 (± 0.20) at 6.9, an X-ray powder diffraction pattern having at least 4 of the characteristic peaks expressed in 20 (± 0.20) selected from the following values at 6.9, 11.2, 17.7, 23.5 and 26.3.

### Embodiment K3

A composition comprising the crystalline polymorph Form **II** according to any one of Embodiments **K1** or **K2.**

### Embodiment K4

A pesticidal, preferably, herbicidal composition comprising the crystalline polymorph Form **II** according to any one of Embodiments **K1** and **K2;** and a pesticidally, preferably, herbicidally acceptable diluent or carrier.

### Embodiment K5

A pesticidal, preferably, herbicidal composition prepared from the crystalline polymorph Form **II** according to any one of Embodiments **K1** and **K2.**

### Embodiment K6

A crystallization method for preparing the crystalline polymorph **Form I** according any one of embodiments **J1** and **J2** comprising recrystallization from *tert*-butyl methyl ether.

### Embodiment K6a

A method for preparing the crystalline polymorph **Form I** according any one of embodiments **J1** and **J2** comprising:
1. dissolving (3R,4S)-1-methyl-2-oxo-4-(3-(trifluoromethyl)phenyl)pyrrolidine-3-carboxylic acid in acetonitrile (200 mL),
2. adding 2-fluoroaniline and N-methylimidazole,
3. mixing the resulting contents for a period exemplified by 10 min at ambient temperature,
4. adding Chloro-N,N,N',N'-tetramethylformamidinium hexafluorophosphate,
5. stirring the resulting mixture at ambient temperature for a period exemplified by for 10h,
6. removing the solvent e.g. by evaporation,
7. dissolving the residue in e.g. dichloromethane ,
8. washing with e.g. sodium hydrosulfate aqueous solution and brine,
9. drying and concentrating under reduced pressure to obtain crude product,
10. recrystallizing crude product from tert-butyl methyl ether.

### Embodiment K7

A method of preparing a crystalline polymorph **Form II** according to any one of embodiments **K1** and **K2** comprising: trituration with heptane.

### Embodiment K7a

A method of preparing a crystalline polymorph **Form II** according to any one of embodiments **K1** and **K2** comprising,
1. dissolving N-(2-fluorophenyl)-1-methyl-2-oxo-2,5-dihydro-1H-pyrrole-3-carboxamide in Diethyl ether,
2. adding e.g. CuBr.SMe₂,
3. adding e.g. 2,2'-bis(diphenylphosphaneyl)-1,1'-binaphthalene,
4. stirring the reaction mass for a period exemplified by 20 minutes.
5. cooling the reaction mass,
6. adding e.g. Trimethylsilyl trifluoromethanesulfonate
7. stirring for a period exemplified by 20 minutes,
8. adding 3-(trifluoromethyl) phenyl) magnesium bromide
9. stirring for a period exemplified by 2 hours,
10. quench the reaction mass into e.g. aq. NH₄Cl solution
11. extract with ethyl acetate
12. wash combined organic layers with brine solution,
13. dry over Na₂SO₄ and concentrate to obtain crude
14. purifyby e.g. column chromatography using Ethyl acetate /Hexane (20:80) as eluent, and
15. triturate the solid obtained with heptane (5 mL) .

### Embodiment M1

A Saturated Target of formula **1** as defined in embodiment **B1** in combination with an amount of any one of the marker, impurity, degradant compounds having the following formulae: 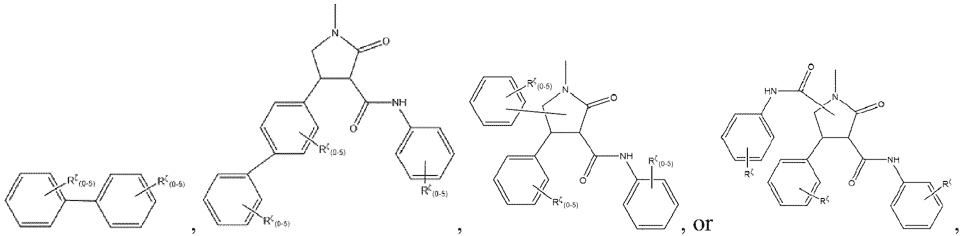 wherein, each **R^{ζ}** is as defined in any one of embodiments **A1** to **A52** and **B1**

### Embodiment M2

A Saturated Target of formula **1** as defined in embodiment **B1** in combination with an amount of any one of the marker, impurity, degradant compounds having the following formulae:

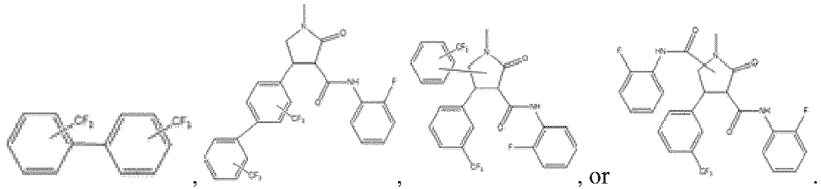

### Embodiment M3

A Saturated Target of formula **1** as defined in embodiment **B1** in combination with an amount of any one of the marker, impurity, degradant compounds having the following formulae:

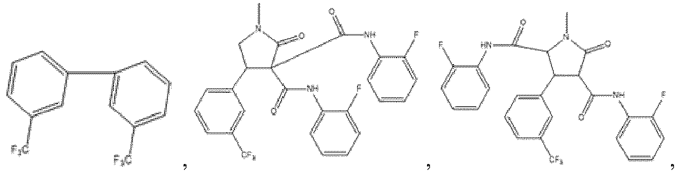

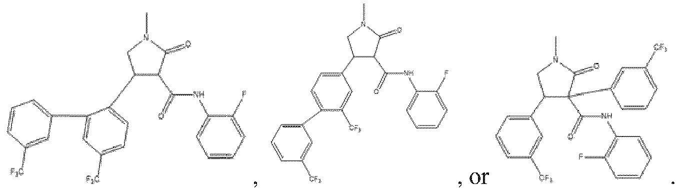

### Embodiment M4

A Saturated Target of formula **1** as defined in embodiment **B1** in combination with an amount of any one of the marker, impurity, degradant compounds having the following formulae: 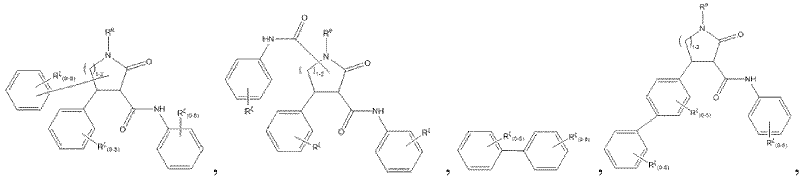 wherein each R^{θ} and each **R^{ζ}** is as defined in Embodiment **A2.**

### Embodiment N1

General procedure for the Preparation of a Storage Marker from a Saturated Target: 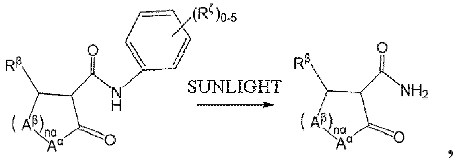 wherein,
**R^{β}, A^{β}, A^{α}** and **R^{ζ},** have the same meanings as defined in Embodiment **A2.**

### Embodiment N2

General Procedure for Preparation of a Storage Marker useful for calibration of pH dependency, based on three aqueous compositions of a Saturated Target each buffered at a different pH.

Three lots of about 50 mg of Saturated Target are each suspended in about 2ml of a 2% solution of acetonitrile in water, and sonicated for about 20 minutes.

About 0.1 ml of one of three different buffers is added to each, of pH=4, pH=7 and pH=10 respectively.

The three buffered solutions are each filtered and introduced to quartz tubes and exposed to natural sunlight for approximately 2 months.

Analysis by HPLC-MS shows the formation of the corresponding Storage Marker in each solution in pH related concentrations.

### Embodiment P1

A compound of the following formula, 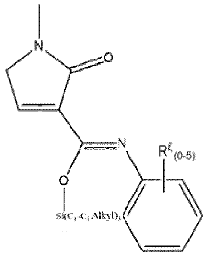 or salts or N-oxides thereof wherein,
Each **R^{ζ},** has the same meaning as defined in Embodiment **A2.**

### Embodiment P1a

A synthetic intermediate compound for the preparation of a Saturated Target of the following formula, 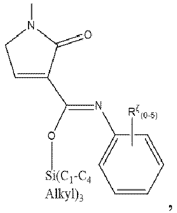 or salts or N-oxides thereof wherein,
**R^{ζ},** has the same meaning as defined in Embodiment **A2.**

### Embodiment P2

A compound of the following formula, 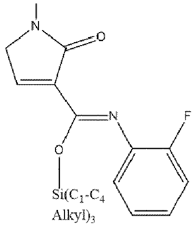 or salts or N-oxides thereof.

### Embodiment P2a

A synthetic intermediate compound for the preparation of a Saturated Target of the following formula, 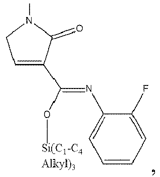 or salts or N-oxides thereof.

### Embodiment P3

A compound of the following formula, 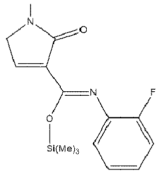 or salts or N-oxides thereof.

### Embodiment P3a

A synthetic intermediate compound for the preparation of a Saturated Target of the following formula, 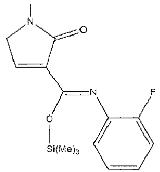 or salts or N-oxides thereof.

### Embodiment Q1

A compound of the following formula, 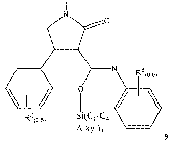 or salts or N-oxides thereof wherein,
each **R^{ζ},** has the same meaning as defined in Embodiment A2.

### Embodiment Q2

A compound of the following formula, 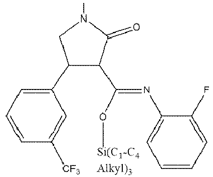 or salts or N-oxides thereof.

### Embodiment Q3

A compound of the following formula, 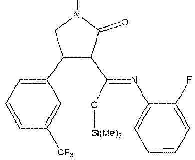 or salts or N-oxides thereof.

### Preparation A

### Preparation of N-(2-fluorophenyl)-1-methyl-2-oxopyrrolidine-3-carboxamide as an exemplified example of a synthetic intermediate compound of Embodiment E1, from N-Methylpyrrolidone (1-methylpyrrolidin-2-one).

1-methylpyrrolidin-2-one in e.g. THF is cooled to and maintained between -75°C -78°C.

e.g. Lithium diisopropylamide in THF is added.

The reaction mass is stirred for e.g. ~30 minutes.

e.g. 1-fluoro-2-isocyanatobenzene dissolved in THF is added dropwise over a period e.g. ~ 5min.

The reaction mass is allowed to warm to ambient and stirred for e.g. ~ 2 hr.

The reaction mixture is quenched e.g. into saturated NH₄Cl and extracted with e.g. ethyl acetate.

The combined organic layer is washed with saturated NaCl solution, dried over e.g. Na₂SO₄ and concentrated to obtain the crude compound.

The crude compound is purified by e.g. column chromatography using e.g. ethyl acetate/hexane as eluent to obtain N-(2-fluorophenyl)-1-methyl-2-oxopyrrolidine-3-carboxamide.

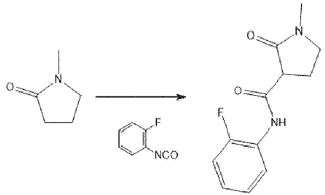

A specific example:
10.08 mmol of 1-methylpyrrolidin-2-one (1 gm,) in 10 ml THF (10 vol) was cooled to between 75°C -78°C. 20.16 mmol of 2M Lithium diisopropylamide in 10.1 ml THF (2.16 gm,) was added at the same temperature of between about -75°C -78°C -75 to -78°C.

The reaction mass was stirred at between about -75°C to -78°C for about 30 minutes.

10.08 mmol of 1-fluoro-2-isocyanatobenzene (1.4 gm) dissolved in THF (5 ml) was added dropwise over a period of about 5 minutes.

The reaction mass was allowed to warm to between about 20°C - 25°C and stirred for approximately 2 hr.

The reaction mixture was quenched into saturated NH₄Cl and extracted with ethyl acetate.

The combined organic layer was washed with saturated NaCl solution, dried over Na₂SO₄ and concentrated to obtain the crude compound.

The crude compound was purified by column chromatography over silica gel (60-120 mesh) using ethyl acetate/hexane as eluent to get 400 mg of N-(2-fluorophenyl)-1-methyl-2-oxopyrrolidine-3-carboxamide as a light brown solid which approximates to17% yield, with a purity of about 88% as analyzed by HPLC .

¹H-NMR (DMSO-d6): δ 10.08(s,1H)8.04-7.98 (m, 1H), 7.29-7.24 (m,1H), 7.17-7.12(m, 2H), 3.73-3.68 (t, 8.8 Hz, 1H), 3.39-3.36(m, 2H), 2.77(s, 3H), 2.25-2.20(m, 2H).

LCMS (EI): *m*/*z* 237.3 [M+1].

### Preparation B

### Preparation of the synthesis intermediate N-(2-fluorophenyl)-1-methyl-2-oxo-3-(phenylthio)pyrrolidine-3-carboxamide as an example of a compound of formula 3 as previously defined, from N-(2-fluorophenyl)-1-methyl-2-oxopyrrolidine-3-carboxamide.

N-(2-fluorophenyl)-1-methyl-2-oxopyrrolidine-3-carboxamide is dissolved in e.g. THF. Then e.g. Sodium Hydride is added at a temperature e.g. of between about 0°C - 5°C.

The reaction mass is stirred at a temperature of e.g. between ~0°C - 5°C for e.g. about 20 minutes. Then, e.g. diphenyl disulfide dissolved in e.g. THF is added at a temperature of e.g. between about 0-5 °C.

The reaction mass is stirred at e.g. about 20°C-25°C for e.g. ∼12 hours.

The reaction mass is quenched into e.g. aqueous NH₄Cl solution and extracted with two lots of e.g. ethyl acetate (twice).

The combined organic layers are washed with e.g. saturated NaCl solution, dried over e.g. Na2SO₄ and concentrated to obtain a crude compound.

The crude compound is purified by e.g. column chromatography over e.g. silica gel using e.g. ethyl acetate/hexane as eluent to obtain N-(2-fluorophenyl)-1-methyl-2-oxo-3-(phenylthio) pyrrolidine-3-carboxamide, as a solid.

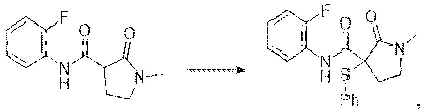

A specific example:
21.18 mmol of N-(2-fluorophenyl)-1-methyl-2-oxopyrrolidine-3-carboxamide (5 gm) was dissolved in 50 ml of THF (10 Vol). 42.37 mmol of Sodium Hydride (1g) was added at a temperature of between about 0°C - 5°C.

The reaction mass was stirred at a temperature of between about 0°C - 5°C for about 20 minutes. 25.42mmol of Diphenyl disulfide (5.5 g) dissolved in THF (5 ml) was added at a temperature of between about 0-5°C.

The reaction mass was stirred at about 20°C-25°C for approximately 12 hours.

The reaction mass was quenched into Aqueous NH₄Cl solution and extracted with two lots of about 50 ml ethyl acetate (twice).

The combined organic layers were washed with saturated NaCl solution, dried over Na2SO4 and concentrated to obtain a crude compound.

The crude compound was purified by column chromatography over Silica gel using Ethyl acetate /Hexane as eluent to obtain 3.8 gm of N-(2-fluorophenyl)-1-methyl-2-oxo-3-(phenylthio) pyrrolidine-3-carboxamide, as an off-white solid which approximates to about 52% yield, having a purity of about 98% as per LC-MS analysis.

¹H-NMR (DMSO-d6): δ 10.16(s,1H) 8.15-8.13 (t, 8 Hz,1H), 7.50-7.43 (m,3H), 7.38-7.34 (m,2H), 7.24-7.29(m, 1H), 7.19-7.11(m, 2H), 3.30-3.27 (dd, 8 Hz, 1H), 3.22-3.25(m, 1H), 2.81(s, 3H) 2.70-2.62(m, 1H), 2.22-2.16(m, 1H).

LCMS (ES): m/z 345.4 [M+1].

### Preparation C

### Synthesis of N-(2-fluorophenyl)-1-methyl-2-oxo-2,5-dihydro-1H-pyrrole-3-carboxamide as an exemplified Unsaturated Precursor, from N-(2-fluorophenyl)-1-methyl-2-oxo-3-(phenylthio) pyrrolidine-3-carboxamide.

N-(2-fluorophenyl)-1-methyl-2-oxo-3-(phenylthio)pyrrolidine-3-carboxamide is dissolved in e.g. acetic acid and stirred for e.g. about 20 min. Then e.g. meta-Chloroperoxy- benzoic acid is added portion-wise at e.g. ambient temperatures. The reaction mass is stirred for e.g. about 2 hrs.

The reaction mass is quenched into e.g. aq.NaHCO₃ solution and extracted twice with e.g. ethyl acetate. The combined organic layers are concentrated to obtain the crude compound.

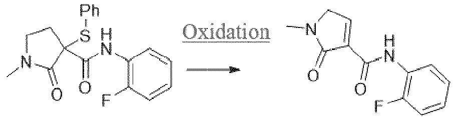

### A specific example:

0.29 mol of N-(2-fluorophenyl)-1-methyl-2-oxo-3-(phenylthio)pyrrolidine-3-carboxamide (100mg) was dissolved in 2 ml of Acetic acid (20 vol), and stirred for about 20 min. to obtain a substantially clear solution. 0.29 mmol *of meta*-Chloroperoxybenzoic acid (50 mg mCPBA) was added portion-wise at approximately 20°C-25°C. The reaction mass was stirred at about 20°C-25°C for about 2 hrs. The reaction mass was then quenched into aq.NaHCO₃ solution and extracted twice with ethyl acetate. The combined organic layers were concentrated to obtain the crude compound. Analysis of the crude material by LC-MS revealed a yield of about 48% of
N-(2-fluorophenyl)-1-methyl-2-oxo-2, 5-dihydro-1H-pyrrole-3-carboxamide.
¹H-NMR (DMSO-d6): δ 11.16(s,1H), 8.41-8.36 (dt,8 Hz, 1H), 8.09(s, 1H) 7.35-7.30 (m,1H), 7.23-7.12(m, 2H),4.26 (s,2H), 3.03(s,3H). LCMS (EI): m/z 393.4 [M+1].

### Preparation D1

### Synthesis of (3S,4S)-N-(2-fluorophenyl)-1-methyl-2-oxo-4-(3-(trifluoromethyl)phenyl) pyrrolidine-3-carboxamide as an exemplified Saturated Target from the corresponding Unsaturated Precursor, N-(2-fluorophenyl)-1-methyl-2-oxo-2,5-dihydro-1H-pyrrole-3-carboxamide, using Trimethyl silyl chloride and BINAP.

N-(2-fluorophenyl)-1-methyl-2-oxo-2,5-dihydro-1H-pyrrole-3-carboxamide is dissolved in e.g. Diethyl ether then Copper(I) bromide dimethyl sulfide complex is added, followed by R-BINAP.

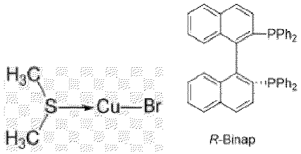

The reaction mass is stirred for e.g. about 20 minutes. The reaction mass is maintained at a cold temperature exemplified by a temperature of between about -20°C and -25°C. Trimethylsilyl chloride is added and stirred for e.g. approximately 10 minutes. Then e.g. 3-(trifluoromethyl) phenyl) magnesium bromide solution is added and stirred for e.g. approximately 2 hours. The reaction mass is quenched into e.g. aqueous NH₄Cl solution, and extracted with e.g. ethyl acetate. The combined organic layers are washed with e.g. saturated brine solution, dried over e.g. Na₂SO₄ and concentrated to obtain crude product.

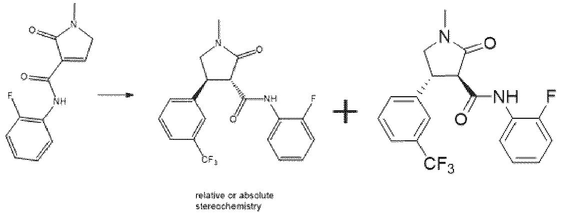

A specific example:
2.13 mmol of N-(2-fluorophenyl)-1-methyl-2-oxo-2,5-dihydro-1H-pyrrole-3-carboxamide (500mg,) was dissolved in 25 ml of Diethyl ether. 0.1 mmol of Copper(I)bromide dimethyl sulfide complex (22 mg CuBr.SMe₂) was added, followed by 0.12 mmol of R-BINAP (∼75 mg,) at a temperature of about -20°C -25°C.

The reaction mass was stirred at approximately -20°C to -25°C for about 20 minutes.

The reaction mass was kept to between about -20°C to -25°C. About 4.7 mmol of Trimethylsilyl chloride (∼506 mg,) was added and stirred for approximately 10 minutes. 6.39 mmol of 1.62M, 3-(trifluoromethyl) phenyl) magnesium bromide solution (1.6 gm, 3.9 ml) was added at a temperature between about -20 -to -25°C and stirred for approximately 2 hours.

The reaction mass quenched into aqueous NH₄Cl solution, and extracted with ethyl acetate. The combined organic layers were washed with saturated brine solution, dried over Na₂SO₄ and concentrated to obtain roughly 1g of crude product having a purity of about 54% purity as measured by quantitative HPLC analysis representing a crude assay corrected yield of approximately 77%. ¹H-NMR (DMSO-d6): δ 10.11(s,1H), 7.99-7.95(m, 1H) 7.74 (s,1H), 7.69-7.68 (m,1H), 7.63-7.58 (m,2H), 7.28-7.24(m, 1H), 7.15-7.12(m, 2H), 4.08-4.02 (m, 2H), 3.82-3.77(t, 8 Hz, 1H), 3.48-3.47(m, 1H), 2.84(s, 3H).

LCMS (ES): m/z 381.5 [M+1].

### Preparation D2

### Synthesis of (3S,4S)-N-(2-fluorophenyl)-1-methyl-2-oxo-4-(3-(trifluoromethyl) phenyl) pyrrolidine-3-carboxamide as an exemplified Saturated Target, from N-(2-fluorophenyl)-1-methyl-2-oxo-2,5-dihydro-1H-pyrrole-3-carboxamide as the corresponding Unsaturated Precursor, using Trimethylsilyl trifluoro methane sulfonate and (S)-N-((S)-1-(butylamino)-1-oxo-3-phenylpropan-2-yl)-2-(((E)-2-(diphenylphosphaneyl) benzylidene) amino)-3-methylbutanamide as ligand.

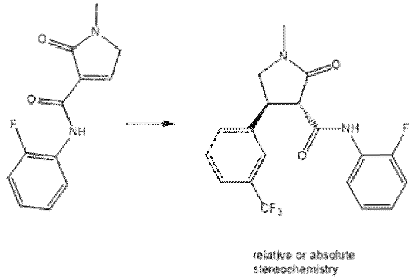

2.13 mmol of N-(2-fluorophenyl)-1-methyl-2-oxo-2,5-dihydro-1H-pyrrole-3-carboxamide (500mg,) was dissolved in 25 ml of Diethyl ether. 0.1 mmol of Copper(I) bromide dimethyl sulfide complex (22 mg CuBr.SMe₂) was added, followed by 0.12 mmol of (S)-N-((S)-1-(butylamino)-1-oxo-3-phenylpropan-2-yl)-2-(((E)-2-(diphenylphosphaneyl) benzylidene) amino)-3-methylbutanamide (76 mg,) at 20-25°C.

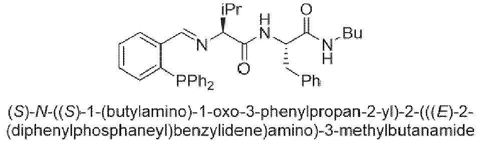

The reaction mass was stirred at about 20°C-25°C o C for approximately 20 minutes. The reaction mass was cooled to, and maintained between -20°C to -25°C, and Trimethylsilyl trifluoromethanesulfonate (1g) 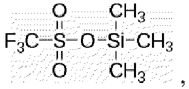 was added and stirred for about10 minutes.

6.39 mmol of 1.62M, 3-(trifluoromethyl) phenyl) magnesium bromide (1.6 gm, 3.9 ml) 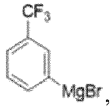 was added at a temperature between about -20 -to -25°C and stirred for approximately 2 hours. The reaction mass was quenched into aqueous NH₄Cl solution and extracted with ethyl acetate. The combined organic layer was washed with saturated brine solution, dried over Na2SO4 and concentrated to obtain a product which was purified by column chromatography over Silica gel (60-120 mesh) using Ethyl acetate/Hexane as eluent and 210 mg of (anti)-N-(2-fluorophenyl)-1-methyl-2-oxo-4-(3-(trifluoromethyl) phenyl) pyrrolidine-3-carboxamide was obtained as an off-white solid representing approximately a 26% yield, with a purity of about 67% as analyzed by HPLC. The product comprised a 1:1.32 ratio of enantiomers such as R,R/S,S by Chiral HPLC analysis.

### Preparation D3

### Synthesis of (3S,4S)-N-(2-fluorophenyl)-1-methyl-2-oxo-4-(3-(trifluoromethyl) phenyl) pyrrolidine-3-carboxamide as an exemplified Saturated Target, from N-(2-fluorophenyl)-1-methyl-2-oxo-2,5-dihydro-1H-pyrrole-3-carboxamide as corresponding Unsaturated Precursor, avoiding the use of trimethylsilyl type Lewis acids e.g. TMSI, TMSCl, TMSOTf etc.

2.13 mmol of N-(2-fluorophenyl)-1-methyl-2-oxo-2,5-dihydro-1H-pyrrole-3-carboxamide (500mg) was dissolved in 25 ml of Diethyl ether. 0.12 mmol of R-BINAP (~75 mg) was added at a temperature of about 20°C -25°C and the reaction mass was stirred for approximately 20 minutes. 6.39 mmol of 1.62M, 3-(trifluoromethyl) phenyl) magnesium bromide (1.6 gm, 3.9 ml) was added at a temperature between about -20 -to -25°C and stirred for approximately 2 hours.

The reaction mass was quenched into aqueous NH₄Cl solution, and twice extracted with ethyl acetate (2x25 mL). The combined organic layer was washed with saturated brine solution, dried over Na₂SO₄ and concentrated to obtain 260 mg of a crude product, which was,
(anti)-N-(2-fluorophenyl)-1-methyl-2-oxo-4-(3-(trifluoromethyl)phenyl)pyrrolidine-3-carboxamide, as off-white solid representing a 32% yield, with a purity of approximately 94% as measured by HPLC.

### Preparation D4

### Synthesis of (3S,4S)-N-(2-fluorophenyl)-1-methyl-2-oxo-4-(3-(trifluoromethyl) phenyl) pyrrolidine-3-carboxamide as an exemplified Saturated Target, from N-(2-fluorophenyl)-1-methyl-2-oxo-2,5-dihydro-1H-pyrrole-3-carboxamide as corresponding Unsaturated Precursor, using Boron trifluoride-diethyl ether complex, BF₃.Et₂O.

0.42 mmol N-(2-fluorophenyl)-1-methyl-2-oxo-2,5-dihydro-1H-pyrrole-3-carboxamide (100mg) was dissolved in 5 ml Diethyl ether.

0.02 mmol Copper(I) bromide dimethyl sulfide complex (4 mg CuBr.SMe₂) was added, followed by 0.02 mmol BINAP (15 mg) at 20-25°C.

The reaction mass was stirred at about 20°C -25°C for about 20 minutes.

The reaction mass was cooled to and maintained at between about -20°C and about -25°C and 0.92 mmol Boron trifluoride diethyl etherate (0.12 mL) was added and stirred for approximately 20 minutes.

1.26mmol of 3-(trifluoromethyl) phenyl) magnesium bromide (0.78 mL of 1.62M) at about -20°C -to -25°C was added and stirred for approximately 2 hours.

The reaction mass was quenched into aqueous NH₄Cl solution, twice extracted with ethyl acetate (2x5mL). The combined organic layer was washed with saturated brine solution, dried over Na₂SO₄ and concentrated to obtain 300 mg of crude (anti)-N-(2-fluorophenyl)-1-methyl-2-oxo-4-(3-(trifluoromethyl) phenyl) pyrrolidine-3-carboxamide representing more than 6% yield, with a purity of about 12% as measured by HPLC.

### Preparation E

### Preparation of a Storage Marker from three compositions of Tetflupyrolimet as an exemplified Saturated Target each buffered at a different pH.

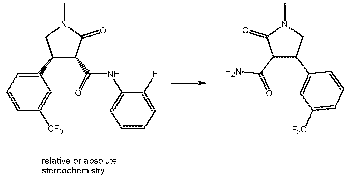

Three lots of about 50 mg of Tetflupyrolimet were each suspended in about 2ml of a 2% solution of acetonitrile in water, and sonicated for about 20 minutes.

Into each, about 0.1 ml of a different buffer was added, pH=4, pH=7 and pH=10 respectively.

The three solutions were each filtered and introduced to quartz tubes and exposed to natural sunlight for approximately 2 months.

Analysis by HPLC-MS showed the formation of Storage Marker in each solution.

The solutions buffered at pH 4 and pH 10 contained approximately an order of magnitude higher concentration of the Storage Marker as compared to the solution buffered at pH=7.

### LC/MS method for Saturated Target [380] and Storage Marker- Impurity [286]

| | |
|---|---|
| Column: | Agilent Zorbax Eclipse XDB C18, 150x4.6mm ID, 5.0µm |
| Injection volume: | 5 µL |
| Oven temperature: | 40°C |
| Detector: | UV, □ = 230, 250, 280 nm |

### Solvent delivery system conditions at flow rate of 1.0 ml/min:

| **Time (minutes)** | **Acetonitrile, %** | **0.1% Formic acid in water, %** |
|---|---|---|
| 0 | 10 | 90 |
| 1 | 10 | 90 |
| 16 | 99 | 1 |
| 19 | 99 | 1 |

| | | |
|---|---|---|
| Post time 4 min | | |

### Preparation F

### Synthesis of (3S,4S)-N-(2-fluorophenyl)-1-methyl-2-oxo-4-(3-(trifluoromethyl) phenyl) pyrrolidine-3-carboxamide from N-(2-fluorophenyl)-1-methyl-2-oxo-4-(3-(trifluoromethyl)phenyl)-2,5-dihydro-1H-pyrrole-3-carboxamide.

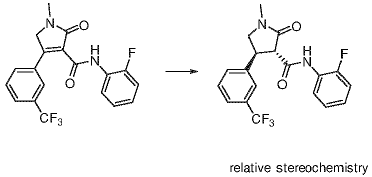

A 5 mL round bottomed flask is flame dried and purged with argon.

0.009 mmol (2 mg) of Stryker's reagent [(PPh₃)CuH]₆ as Pre-Catalyst/Catalyst and 0.0017mmol (2mg) of either (*R*)-DTBM-SEGPHOS or (*S*)-(+)-DTBM-SEGPHOS as

Pre-Catalyst/Catalyst is added.

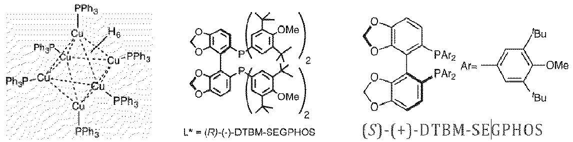

1 ml THF is added and the solution cooled to approximately 0°C. 3.36 mmol (224 µl) of Polymethylhydrosiloxane (PMHS) is added as a Hydride source, which is introduced e.g. *via* syringe followed by 1.81 mmol *t*-BuOH (173µl) and 0.83 mmol N-(2-fluorophenyl)-1-methyl-2-oxo-4-(3-(trifluoromethyl)phenyl)-2,5-dihydro-1H-pyrrole-3-carboxamide (313 mg). The mixture is stirred at about 0°C until reaction is complete as evidenced by quantitative TLC typically between 3 and 12 hours.

The reaction is quenched by pouring into saturated NaHCO₃ and diluted with Diethyl ether in water (Et₂O/H2O) and then stirred for approximately 2 hours at ambient temperatures.

The aqueous layer is extracted twice with Diethyl ether and the combined organic layers are washed with brine, dried over anhydrous MgSO₄, filtered, and concentrated by rotary evaporation. The obtained crude material is purified by column chromatography over Silica gel (60-120 mesh) using Ethyl acetate/Hexane as eluent to obtain (anti)-N-(2-fluorophenyl)-1-methyl-2-oxo-4-(3-(trifluoromethyl) phenyl) pyrrolidine-3-carboxamide as an off-white solid. About 70% yield, with a purity of approximately 90 % as measured by HPLC. A ratio of enantiomers of around 1:9 by Chiral HPLC analysis is obtainable in principle. Depending on the choice of the chiral catalytic component enantiomeric excess can be directed to the desired product. This and the other specific preparations can also be conducted with alternative reagents and conditions as would be understood by practitioners of these arts to have equivalent function and/or effect

### Preparation G

### Synthesis of 1-methyl-2-oxopyrrolidine-3-carboxylic acid:

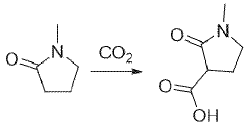

50.5 mmol of 1-methylpyrrolidin-2-one (5 g) was dissolved in 50 ml THF (10 vol) and cooled to and maintained at, between about -75°C to -78°C.

101 mmol of 2M Lithium diisopropylamide (10.2 gm, 50 ml) was added at between about -75°C to -78°C. The reaction mass was stirred at -75 to -78°C for approximately 30 minutes. The reaction mass was added drop wise onto solid CO₂ and stirred for about 1 hr. The reaction mass was allowed to warm to about 20°C-25°C gradually and stirred for about 1 hr. The reaction mixture was quenched into dilute HCl and extracted with 10% isopropyl alcohol in dichloromethane (4 × 25ml), dried over Na₂SO₄ and concentrated to obtain 2.2 gm of crude 1-methyl-2-oxopyrrolidine-3-carboxylic acid as brown oil. 30.5% yield, purity ^{∼}58% as per HPLC.

### Preparation H

### Synthesis of N-(2-fluorophenyl)-1-methyl-2-oxopyrrolidine-3-carboxamide using Propyl phosphonic anhydride (T3P)

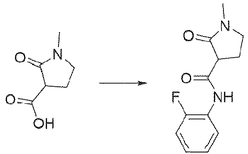

6.99 mmol of 1-methyl-2-oxopyrrolidine-3-carboxylic acid (1g) was dissolved in 20 ml Dimethylformamide (20 vol), and cooled to about 0-5°C. 20.97mmol of Triethylamine (2.1g), and 7.68 mmol of 2-fluoroaniline (850 mg) was added followed by 13.98 mmol of T3P (9ml of 50% solution in EtOAc) at between 0°C-5°C. The reaction mass was stirred for approximately 2hrs at between about 0°C - 5°C. The reaction mass was added into water and extracted with 2 × 150ml ethyl acetate. The combined organic layer was washed with water followed by brine solution, dried over sodium sulphate and concentrated to obtain the crude product. The crude product was dissolved in 5 ml Dichloromethane and precipitated with 20ml n-Heptane, The obtained solid was stirred and filtered to obtain 700 mg of

N-(2-fluorophenyl)-1-methyl-2-oxopyrrolidine-3-carboxamide as off-white solid. 42.4 % yield having a purity of ∼99% as per HPLC analysis.

### Preparation I

### Synthesis of N-(2-fluorophenyl)-1-methyl-2-oxopyrrolidine-3-carboxamide via acyl chloride

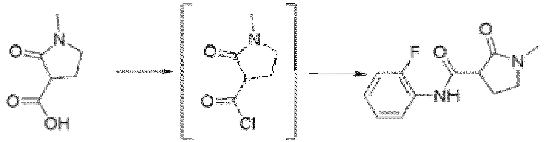

13.98 mmol 1-methyl-2-oxopyrrolidine-3-carboxylic acid (2g) was dissolved in 20 ml Dichloromethane (10 vol). 2.73 mmol Dimethylformamide (0.2 ml), was added and cooled to about 0°C-5°C. 20.97 mol Oxalyl chloride (2.6g) was added at about 0°C-5°C. Th reaction mass was stirred for approximately 2 hr. at about 0-5°C. Acyl chloride formation was monitored by TLC by quenching in ethyl alcohol.

The reaction mass was concentrated to dryness, diluted with fresh dichloromethane and added to a stirred solution of 13.98 mmol 2-fluoroaniline (1.5 gm) and 27.96mmol triethylamine (2.8g) in 10ml Dichloromethane(5 vol) at about 0°C -5°C.

The reaction mass was warmed to about 20°C-25°C and stirred for approximately 2 hrs. The reaction mass was quenched into water and extracted with Dichloromethane (2x150 ml). The combined organic layer was washed with water followed by brine solution, dried over sodium sulphate and concentrated to obtain crude product. 500 mg of N-(2-fluorophenyl)-1-methyl-2-oxopyrrolidine-3-carboxamide was obtained. 15.1% yield, 88.8% purity as per LCMS.

### Preparation J

### Synthesis of N-(2-fluorophenyl)-1-methyl-2-oxo-3-(phenylselenyl)pyrrolidine-3-carboxamide

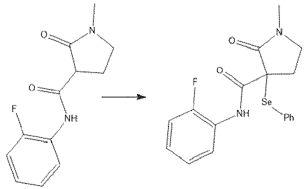

63.5 mmol of N-(2-fluorophenyl)-1-methyl-2-oxopyrrolidine-3-carboxamide (15g) was dissolved in 60 ml THF. 127.1mmol of NaH (3.0 g,) was added at about 0°C-5°C. The reaction mass was stirred at about 0-5°C for approximately 20 minutes. 69.9 mmol of Phenylselenyl bromide (16.5g) dissolved in THF was added at about 0-5°C and stirred for approximately 2hrs. at 0-5°C. The reaction mass was quenched into ice cold water, extracted with ethyl acetate. The combined organic layer was concentrated to obtain crude compound which was purified by column chromatography over Silica gel using Ethyl acetate/Hexane as eluent to obtain 24g of N-(2-fluorophenyl)-1-methyl-2-oxo-3-(phenylselenyl) pyrrolidine-3-carboxamide as an off-white solid. Yield = 96%, purity = 97.1% as per LCMS.

¹H-NMR (DMSO-d6): δ 10.26(s,1H), 8.20-8.16 (m, 1H), 7.59-7.56 (m,2H), 7.47-7.43 (m,1H), 7.35-7.31 (m,2H), 7.27-7.22(m, 1H), 7.19-7.15(m, 1H), 7.13-7.11(m, 1H), 3.30-3.25(m,1H), 3.18-3.12(m,1H), 2.77(s, 3H), 2.55-2.64(m, 1H), 2.18-2.13(m, 1sH).

LCMS (EI): m/z 393.4(M+1).

### Preparation K

### A process scheme starting with a compound of Embodiment E1 via a compound of Embodiment C1a and then via an Unsaturated Precursor compound of Embodiment A1 or Embodiment A2, and therefrom a Saturated Target exemplified by, though not limited to, Tetflupyrolimet.

### Synthesis of N-(2-fluorophenyl)-1-methyl-2-oxo-2,5-dihydro-1H-pyrrole-3-carboxamide

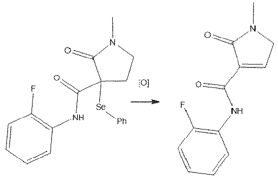

38.2 mmol of N-(2-fluorophenyl)-1-methyl-2-oxo-3-(phenylselanyl)pyrrolidine-3-carboxamide (15g) was dissolved in 150 ml dichloromethane (MDC 10 vol). 76.5mmol H₂O₂ (2.6 ml) was added at about 0-5°C. The reaction mass was stirred at 0-5°C for approximately 2 hrs. The reaction mass was quenched into ice cold saturated NaHCO₃ solution. The obtained solid was stirred, filtered and vacuum-dried. The obtained solid was suspended in 150ml n-Heptane, stirred, filtered and dried to obtain 7.1 gm of N-(2-fluorophenyl)-1-methyl-2-oxo-2,5-dihydro-1H-pyrrole-3-carboxamide (Unsaturated Precursor) as off-white solid.

Yield = 79.3%,

purity = 98.1% as per LCMS.

¹H-NMR (DMSO-d6): δ 11.16(s,1H), 8.41-8.36 (dt,8 Hz, 1H), 8.09(s, 1H) 7.35-7.30 (m,1H), 7.23-7.12(m, 2H),4.26 (s,2H), 3.03(s,3H). LCMS (EI): m/z 393.4 [M+1].

### Preparation L Tetflupyrolimet polymorph Form I

### Preparation of polymorphs:

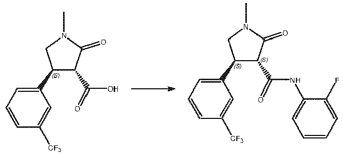

(3R,4S)-1-methyl-2-oxo-4-(3-(trifluoromethyl)phenyl)pyrrolidine-3-carboxylic acid (21.2 g, 73.8 mmol) (WO 2018/175226) was dissolved in acetonitrile (200 mL), 2-fluoroaniline (9.02 g, 81.2 mmol) and N-methylimidazole (30.3 g, 369 mmol) was added to the solution. The resulting mixture was stirred for 10 min at ambient temperature and Chloro-N,N,N',N'-tetramethyl formamidinium hexafluorophosphate (22.8 g, 81.2 mmol) was added to the mixture in a single portion. The resulting mixture was stirred at ambient temperature for 10 h, then the solvent was evaporated, the residue was dissolved in dichloromethane (500 mL) and washed with a saturated sodium hydrosulfate aqueous solution (2 × 100 mL) and brine (1 × 100 mL), dried and concentrated under reduced pressure to give the crude product, which was recrystallized from tert-butyl methyl ether (20 mL) to give (3S,4S)-N-(2-fluorophenyl)-1-methyl-2-oxo-4-(3-(trifluoromethyl) phenyl) pyrrolidine-3-carboxamide (10.1 g, 36%) with chiral enantiomers ratio of >99:1 according to chiral HPLC.

### Preparation M (Tetflupyrolimet polymorph Form II)

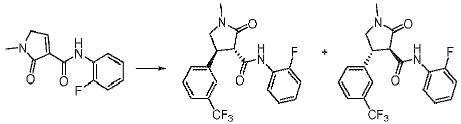

In a 50 mL two neck round-bottomed flask, N-(2-fluorophenyl)-1-methyl-2-oxo-2, 5-dihydro-1H-pyrrole-3-carboxamide (500 mg, 2.13 mmol) was dissolved in dry Diethyl ether (25ml), CuBr.SMe₂ (22 mg, 0.1 mmol), was added, followed by 2,2'-bis(diphenylphosphanyl)-1,1'-binaphthalene (79 mg, 0.12 mmol) at ambient temperatures. Reaction mass was stirred at ambient temperature for 20 minutes. Reaction mass was cooled to -20 to -25°C using dry-ice/acetonitrile, Trimethylsilyl trifluoromethanesulfonate (1 gm, 4.68 mmol) was added and stirred for 20 minutes. 1.62 M 3-(trifluoromethyl) phenyl) magnesium bromide was added via SmL syringe (1.6 gm, 6.39 mmol 3.9 ml) at -20 to -25°C and stirred for 2 hours, at which time TLC analysis indicated completion of reaction (30:70 ethyl acetate: hexanes, Rε of starting material 0.3, Rε product 0.5). Reaction mass quenched into aq. NH₄Cl solution (20 mL), extracted with ethyl acetate (3x50 mL). Combined organic layers were washed with saturated brine solution (100 mL), dried over Na₂SO₄ and concentrated in a 250 mL round-bottomed flask with 600mm Hg vacuum rotated at 60 rpm at 40°C for 30 min. to obtain crude (1g) which was purified by column chromatography over Silica gel (60-120 mesh) using Ethyl acetate /Hexane (20:80) as eluent and the obtained solid was triturated with heptane (5 mL) to obtain 360 mg of anti-(3,4)-N-(2-fluorophenyl)-1-methyl-2-oxo-4-(3-(trifluoromethyl) phenyl) pyrrolidine-3-carboxamide as off-white solid (44.3% yield, purity 94.6 % as per HPLC) with chiral enantiomers ratio of 50.5:49.4 according to chiral HPLC.

### Preparation N

### Synthesis of 4-ethyl-N-(2-fluorophenyl)-1-methyl-2-oxopyrrolidine-3-carboxamide using organo-zinc.

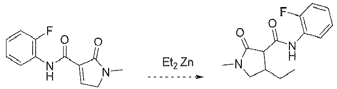

(S)-N-((S)-1-(butylamino)-1-oxo-3-phenylpropan-2-yl)-2-(((E)-2-(diphenylphosphaneyl)benzylidene)- amino)-3-methylbutanamide (75mg, 0.12mmol) and Copper(I) trifluoromethanesulfonate benzene complex (CuOTf)₂·C₆H₆ ( 25mg, 0.05 mmol) was dissolved in Toluene (6 ml, 20 vol), N-(2-fluorophenyl)-1-methyl-2-oxo-2,5-dihydro-1H-pyrrole-3-carboxamide (300mg, 1.28 mmol), was added and stirred for 10 min at 20-25°C. Reaction mass was cooled to -35 to -30°C, Diethyl zinc (3.84 mL, 1 M, 3 eq) was added to the reaction mixture. The reaction mass was left to stir at -35-30°C for 4h. Reaction mass quenched into aq. NH₄Cl solution, extracted with ethyl acetate (2x25 mL). Combined organic layer was washed with saturated brine solution, dried over Na₂SO₄ and concentrated to obtain crude which was purified by column chromatography over Silica gel (60-120 mesh) using Ethyl acetate/Hexane as eluent and obtained 120 mg of 4-ethyl-N-(2-fluorophenyl)-1-methyl-2-oxopyrrolidine-3-carboxamide as off-white solid (35.5% yield, purity 88.9 % HPLC and 58.3% LCMS purity) with chiral enantiomers ratio of 49.4:49.4 according to Chiral HPLC.

LCMS (ES): m/z 265.2(M+1).

### Preparation O

### Synthesis of N-(2,3-difluorophenyl)-1-methyl-2-oxopyrrolidine-3-carboxamide using Propylphosphonic anhydride (T3P).

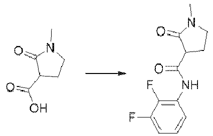

1-methyl-2-oxopyrrolidine-3-carboxylic acid (5g, 34.9 mmol) was dissolved in Dimethylformamide (20 ml, 4 vol), cooled to 0°-5°C, added Triethylamine (10.5 g, 103.9 mmol), 2,3-difluoroaniline (4.9 g, 37.9mmol) followed by T3P Propyl phosphonic anhydride (44 g ,138.3 mmol; 50% solution in EtOAc) at 0°-5°C. Reaction mass was stirred for 2 hrs at 0-5°C. Reaction mass was added into water and extracted with ethyl acetate (2x50 ml). Combined organic layer was washed with water followed by brine solution, dried over sodium sulphate and concentrated to obtain solid.

To this solid heptane (10 vol) was added and stirred for 1hr, filtered and dried under vacuum to obtain 8g of N-(2,3-difluorophenyl)-1-methyl-2-oxopyrrolidine-3-carboxamide as off-white solid 90.9 % yield, purity 98.9% as per LCMS.

¹H NMR (DMSO-d6): δ 10.27(s,1H), 7.95-7.77 (m, 1H), 7.21-7.16 (m,2H), 3.74-3.69 (t, 18 Hz, 1H), 3.43-3.32 (m, 2H), 2.89(s, 3H), 2.32-2.20(m, 2H). LCMS (EI): m/z 255.2(M+1).

### Preparation P

### Synthesis of N-(2,3-difluorophenyl)-1-methyl-2-oxo-3-(phenylselanyl)pyrrolidine-3-carboxamide:

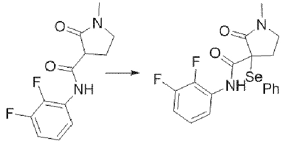

N-(2,3-difluorophenyl)-1-methyl-2-oxopyrrolidine-3-carboxamide (2g, 7.8 mmol) was dissolved in THF (20 ml), added NaH (0.629 g, 26.2mmol) at 0-5°C. Reaction mass was stirred at 0-5°C for 20 minutes. Added Phenyl selenyl bromide (2 g, 8.5 mmol) dissolved in THF (5 mL) at 0-5°C and stirred for 2 hrs. Reaction mass was quenched into cold water, extracted with ethyl acetate (2x50 mL). Combined organic layer was concentrated to obtain crude compound which was purified by column chromatography over Silica gel using Ethyl acetate /Hexane as eluent to get 2.2 g of N-(2,3-difluorophenyl)-1-methyl-2-oxopyrrolidine-3-carboxamide as light yellow solid. 68.7% yield, purity 98.2% as per LCMS.

1H NMR (DMSO-d6): δ 10.4(s,1H), 7.99-7.94 (m, 1H), 7.58-7.56 (m,2H), 7.47-7.43 (m,1H), 7.35-7.31 (m,2H), 7.20-7.12(m, 2H), 3.31-3.27 (m, 1H), 3.18 (m, 1H), 2.79(s, 3H), 2.64-2.55 (m, 1H), 2.18-2.13(m, 1H). LCMS (EI): m/z 411.2(M+1).

### Preparation Q

### Synthesis of N-(2,3-difluorophenyl)-1-methyl-2-oxo-2, 5-dihydro-1H-pyrrole-3-carboxamide

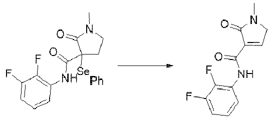

N-(2,3-difluorophenyl)-1-methyl-2-oxo-3-(phenylselenyl) pyrrolidine-3-carboxamide (2g, 4.8 mmol) was dissolved in Dichloromethane (20 ml, 10 vol) added H₂O₂ (1.1 g, 32.3 mmol; 30% solution) at 0-5°C. Reaction mass was stirred at 0-5°C for 2 hrs. Reaction mass was quenched into ice cold saturated NaHCO₃ solution and extracted with Dichloromethane (2x50 mL). The Organic layers were washed with NaHCO₃ solution (3x20 mL), dried under Na₂SO₄ and concentrated to get crude. The crude compound was suspended in IPA (SmL), stirred for 2 hrs. filtered the solid and dried to obtain 1 g of
N-(2,3-difluorophenyl)-1-methyl-2-oxo-2,5-dihydro-1H-pyrrole-3-carboxamide as light yellow solid 83.3% yield, purity 96.1% as per LCMS.
1H NMR (CDCl3): δ 11.1(s,1H), 8.24-8.20 (m, 1H), 8.0-7.99(bt, 1.6 Hz, 1H) 7.10-7.03 (m,1H), 6.99-6.90(m, 1H), 4.12-4.11 (bd,2H), 3.16(s,3H). LCMS (EI): m/z 253.2(M+1).

### Preparation R

### Synthesis of N-(2,3-difluorophenyl)-1-methyl-2-oxo-4-(6-(trifluoromethyl) pyridin-3-yl) pyrrolidine-3-carboxamide.

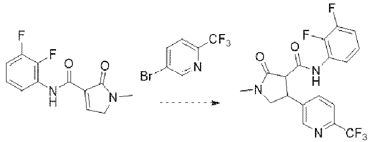

In a 25 mL two neck round-bottomed flask, 5-bromo-2-(trifluoromethyl) pyridine (0.80 g, 3.57 mmol) was dissolved in dry diethyl ether (8 mL, 10 vol). Reaction mass was cooled to -78 to -70°C, added *n*- Butyl lithium (4.26 mL, 2.5 M, 3 eq) to the reaction mixture. The reaction mass was allowed to stir at -78-70°C for 1h. In another flask N-(2,3-difluorophenyl)-1-methyl-2-oxo-2,5-dihydro-1H-pyrrole-3-carboxamide (0.3 g, 1.19 mmol), Copper(I) bromide dimethyl sulfide complex (12.2 mg, 0.059 mmol) and 2,2'-bis(diphenylphosphaneyl)-1,1'-binaphthalene (44.4 mg, 0.071 mmol) in Et₂O (9 mL, 30 vol) were taken (added), and the reaction mixture was cooled to - 78-70°C. To this reaction mass, lithiated 5-bromo-2-(trifluoromethyl) pyridine was added dropwise at -78-70°C and stirred for 1hr. Reaction mass quenched into aq. NH₄Cl solution, extracted with ethyl acetate (2x50 mL). Combined organic layer was washed with saturated brine solution, dried over Na₂SO₄ and concentrated to get crude, which was purified by column chromatography over Silica gel (60-120 mesh) using Ethyl acetate /Hexane as eluent and obtained 60 mg of N-(2,3-difluorophenyl)-1-methyl-2-oxo-4-(6-(trifluoromethyl) pyridin-3-yl) pyrrolidine-3-carboxamide as a light-yellow solid. 12.7% yield, purity 67.1% by LCMS.

1H NMR (CDCl3): δ 10.06 (s, 1H), 7.96-7.89 (m, 2H), 7.03-6.99 (m, 2H), 6.93- 6.89 (m, 2H), 4.21-4.18 (m, 1H), 3.87-3.82(m, 1H), 3.65-3.63 (m, 1H), 3.54-3.49 (m, 1H), 3.05 (s, 3H). LCMS (ES): m/z 400.3 (M+1).

### Preparation S

### Synthesis of N-(2-fluorophenyl)-1-methyl-2-oxo-4-(6-(trifluoromethyl) pyridin-3-yl) pyrrolidine-3-carboxamide.

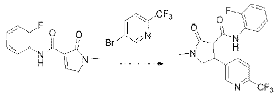

In a 25 mL two neck round-bottomed flask, 5-bromo-2-(trifluoromethyl) pyridine (0.84 g, 3.73 mmol) was dissolved in dry diethyl ether (8 mL, 10 vol). Reaction mass was cooled to -78 to -70°C, added *n*- Butyl lithium (4.4 mL, 2.5 M, 3 eq) to the reaction mixture. The reaction mass was allowed to stir at -78-70°C for 1h. In another flask N-(2-fluorophenyl)-1-methyl-2-oxo-2,5-dihydro-1H-pyrrole-3-carboxamide (0.3 g, 1.28 mmol), Copper(I) bromide dimethyl sulfide complex (13 mg, 0.063 mmol) and 2,2'-bis(diphenylphosphaneyl)-1,1'-binaphthalene (47 mg, 0.075 mmol) in Et₂O (9 mL, 3 vol) were taken and the reaction mixture was cooled to -78-70°C. To this reaction mass (RM), lithiated 5-bromo-2-(trifluoromethyl) pyridine was added dropwise at -78-70°C and stirred for 1hr. Reaction mass quenched into aqueous NH₄Cl solution, extracted with ethyl acetate (2x50 mL). Combined organic layer was washed with saturated brine solution, dried over Na₂SO₄ and concentrated to get crude, which was purified by column chromatography over Silica gel (60-120 mesh) using Ethyl acetate /Hexane as eluent and obtained 50 mg of N-(2-fluorophenyl)-1-methyl-2-oxo-4-(6-(trifluoromethyl) pyridin-3-yl) pyrrolidine-3-carboxamide as oily liquid 10.2% yield, purity 49.1% by LCMS.

LCMS (ES): m/z 382.3 (M+1).

### Preparation T

### Synthesis of N-(2-fluorophenyl)-1-methyl-2-oxo-4-(3-(trifluoromethyl)phenyl)pyrrolidine-3-carboxamide from N-(2-fluorophenyl)-1-methyl-2-oxo-4-(3-(trifluoromethyl)phenyl)-2,5-dihydro-1H-pyrrole-3-carboxamide (Procedure-1)

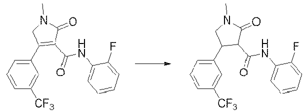

In a 25 mL round-bottomed flask (RBF), N-(2-fluorophenyl)-1-methyl-2-oxo-4-(3-(trifluoromethyl) phenyl)-2,5-dihydro-1H-pyrrole-3-carboxamide (100 mg, 0.26 mmol) was dissolved in Acetic acid (Sml, 50 vol). Reaction mass was cooled to 0 to 5°C, added Zn dust (0.17 g, 2.64 mmol). The RM was stirred at 20-25°C for 12h. RM was filtered and concentrated to get crude. Crude LCMS indicates 30.6% of anti-N-(2-fluorophenyl)-1-methyl-2-oxo-4-(3-(trifluoromethyl) phenyl) pyrrolidine-3-carboxamide.

LCMS (ES): m/z 381.3 (M+1).

### Preparation U

### Synthesis of N-(2-fluorophenyl)-1-methyl-2-oxo-4-(3-(trifluoromethyl)phenyl)pyrrolidine-3-carboxamide from N-(2-fluorophenyl)-1-methyl-2-oxo-4-(3-(trifluoromethyl)phenyl)-2,5-dihydro-1H-pyrrole-3-carboxamide (Preparation Procedure #2)

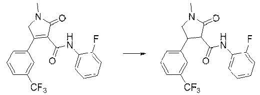

In a 25 mL RBF, N-(2-fluorophenyl)-1-methyl-2-oxo-4-(3-(trifluoromethyl)phenyl)-2,5-dihydro-1H-pyrrole-3-carboxamide (50 mg, 0.13 mmol) was dissolved in methanol (5 ml, 50 vol). Reaction mass was cooled to 0 to 5°C, added Nickel (II) chloride hexahydrate (15 mg, 0.06 mmol) and followed by Sodium borohydride (5 mg, 0.13 mmol). The RM was stirred at 20-25°C for 12h. The reaction was monitored by LCMS. LCMS indicated the formation of desired mass up to ∼15%. LCMS (ES): m/z 381.3 (M+1).

### Preparation V

### Synthesis of N-(2-fluorophenyl)-1-methyl-2-oxo-4-(3-(trifluoromethyl)phenyl)pyrrolidine-3-carboxamide from N-(2-fluorophenyl)-1-methyl-2-oxo-4-(3-(trifluoromethyl)phenyl)-2,5-dihydro-1H-pyrrole-3-carboxamide

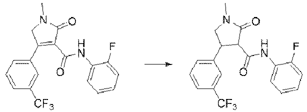

In a 25 mL RBF, N-(2-fluorophenyl)-1-methyl-2-oxo-4-(3-(trifluoromethyl) phenyl)-2,5-dihydro-1H-pyrrole-3-carboxamide (50 mg, 0.13mmol) was dissolved in methanol (Sml, 50vol), added Tris(triphenylphosphine)rhodium(I) chloride (12 mg, 0.013 mmol). The RM was stirred under H₂ pressure. The pressure was applied using hydrogen bladder at 20-25 °C for 12h. The reaction was monitored using LCMS. RM was quenched with 2N NaOH solution (0.2 mL) and extracted with ethyl acetate (2x5 mL) and concentrated to obtain crude. The crude LCMS indicated the formation of desired mass up to ∼10%.

LCMS (ES): m/z 381.2(M+1).

### Preparation W

### Synthesis of (3S,4S)-N-(2-fluorophenyl)-1-methyl-2-oxo-4-(3-(trifluoromethyl)phenyl)pyrrolidine-3-carboxamide from N-(2-fluorophenyl)-1-methyl-2-oxo-2,5-dihydro-1H-pyrrole-3-carboxamide (Procedure-#3).

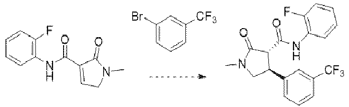

In a 25 mL two neck RBF, 1-bromo-3-(trifluoromethyl) benzene (0.28 g, 1.28 mmol) was dissolved in diethyl ether (3 mL, 10 vol). The reaction mass was cooled to -78 to -70°C, added n-Butyl lithium (1.02mL, 2.5 M, 2 eq) to the reaction mixture. The RM was allowed to stir at -78-70°C for 1h. This lithiated solution was used for the reaction with N-(2-fluorophenyl)-1-methyl-2-oxo-2,5-dihydro-1H-pyrrole-3-carboxamide (Int **[234]).** In another flask, N-(2-fluorophenyl)-1-methyl-2-oxo-2,5-dihydro-1H-pyrrole-3-carboxamide (0.1 g, 0.427 mmol), Copper(I) bromide dimethyl sulfide complex (4 mg, 0.021 mmol) and (S)-N-((S)-1-(butylamino)-1-oxo-3-phenylpropan-2-yl)-2-(((E)-2-(diphenylphosphaneyl) benzylidene) amino)-3-methylbutanamide (15 mg, 0.025 mmol) in Et₂O (5 mL) were taken and cooled to -78-70°C. To this reaction mass added lithiated 1-bromo-3-(trifluoromethyl) benzene at -78-70°C and stirred for 4 hr. The reaction was monitored by LCMS and TLC. The TLC indicated the formation of new spot and LCMS indicated the presence of desired mass. The reaction mass was quenched using aq. NH₄Cl solution and extracted with ethyl acetate (2x25 mL). The combined organic layer was washed with saturated brine solution and dried over Na₂SO₄. The organic layer was concentrated under reduced pressure below 45°C to obtain crude. The crude obtained was purified by column chromatography over Silica gel (60-120 mesh) using Ethyl acetate /Hexanes as eluent. Obtained 30 mg of anti-N-(2-fluorophenyl)-1-methyl-2-oxo-4-(3-(trifluoromethyl) phenyl) pyrrolidine-3-carboxamide as a light-yellow solid (18.5% yield, purity 30.9 % by LCMS).

LCMS (ES): m/z 381.2(M+1).

### Preparation X

### Synthesis of (3S,4S)-N-(2,3-difluorophenyl)-1-methyl-2-oxo-4-(3-(trifluoromethyl)phenyl)pyrrolidine-3-carboxamide.

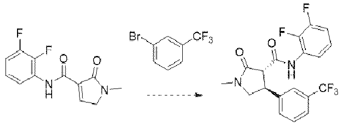

In a 25 mL two neck RBF, 1-bromo-3-(trifluoromethyl) benzene (0.8 g, 3.57mmol) was dissolved in dry diethyl ether (10 mL, 12.5 vol). The reaction mass was cooled to -78 to -70°C followed by addition of n- Butyl lithium (2.8 mL, 2.5 M, 2 eq) to the reaction mixture. The RM was allowed to stir at -78 to -70°C for 1h. This lithiated solution was used for the reaction with N-(2,3-difluorophenyl)-1-methyl-2-oxo-2,5-dihydro-1H-pyrrole-3-carboxamide (Int **[252]**).

In another flask N-(2,3-difluorophenyl)-1-methyl-2-oxo-2,5-dihydro-1H-pyrrole-3-carboxamide (0.3 g, 1.19 mmol), Copper(I) bromide dimethyl sulfide complex (12 mg, 0.059 mmol) and (S)-N-((S)-1-(butylamino)-1-oxo-3-phenylpropan-2-yl)-2-(((E)-2-(diphenylphosphaneyl) benzylidene) amino)-3,3-dimethylbutanamide (43 mg, 0.071 mmol) in Et₂O (6 mL, 20 vol) were taken . The resultant reaction mass was cooled to -78-70°C followed by addition of lithiated 1-bromo-3-(trifluoromethyl) benzene at -78-70°C. Then stir the reaction mass at -78-70 for 4 h after 4 h reaction mass allowed to warm to 20-25°C and maintained at 20-25°C for 12 h. The reaction mass was monitored by TLC and LCMS. The TLC shows the formation of new spot, similarly, LCMS indicated the formation of desired mass. Then reaction mass was quenched with saturated aq. NH₄Cl solution followed by the extraction with ethyl acetate (3x25 mL). The combined ethyl acetate layers were washed with saturated brine solution and dried over Na₂SO₄. Then ethyl acetate was evaporated under reduced pressure below 45°C to afford crude compound. The obtained crude was then purified by column chromatography over Silica gel (60-120 mesh) using Ethyl acetate/Hexanes as eluent. Obtained 55 mg of N-(2,3-difluorophenyl)-1-methyl-2-oxo-4-(3-(trifluoromethyl) phenyl) pyrrolidine-3-carboxamide as a light-yellow solid (11.2% yield, purity 72.3 % by LCMS).

LCMS (ES): m/z 399 (M+1).

### Preparation Y

### Synthesis of N-(2,6-difluoropyridin-3-yl)-1-methyl-2-oxopyrrolidine-3-carboxamide using Propyl phosphonic anhydride (T3P)

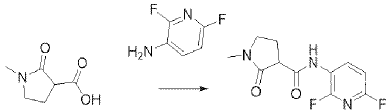

1-methyl-2-oxopyrrolidine-3-carboxylic acid (5g, 34.9 mmol) was dissolved in Dimethylformamide (20 mL, 4vol), cooled to 0°-5°C, added Triethylamine (10.5 g, 103.9 mmol), 2,6-difluoropyridin-3-amine (5g, 38.4mmol) followed by Propyl phosphonic anhydride (T3P) (44 g, 138.3 mmol; 50% solution in EtOAc) at 0-5°C. Reaction mass was stirred for 2 hrs at 0-5°C. Reaction mass was added into water and extracted with ethyl acetate (3x 100 ml). Combined organic layer was washed with water followed by brine solution. The organic was dried over sodium sulphate and concentrated to obtain solid.

To this solid, added 10V of n-Heptane (10 vol) and stirred for 1 hr. Then filtered the solid and dried under vacuum to get 5.5 g of N-(2,6-difluoropyridin-3-yl)-1-methyl-2-oxopyrrolidine-3-carboxamide as off-white solid (61.7% yield, purity 91.6% as per LCMS).

¹H NMR (DMSO-d6): δ 10.30 (s,1H), 8.60-8.54 (m, 1H), 7.19-7.16 (m,1H), 3.71-3.67 (t, 18 Hz, 1H), 3.43-3.32 (m, 2H), 2.89(s, 3H), 2.31-2.20(m, 2H). LCMS (EI): m/z 256.2(M+1).

### Preparation Z

### Synthesis of N-(2,6-difluoropyridin-3-yl)-1-methyl-2-oxo-3-(phenylselanyl)pyrrolidine-3-carboxamide

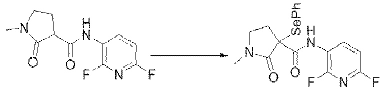

To the stirred solution of NaH (1.25 g, 52.08 mmol) in THF (10 mL) at 0-5°C, was added N-(2,6-difluoropyridin-3-yl)-1-methyl-2-oxopyrrolidine-3-carboxamide (4 g, 15.68 mmol) was dissolved in THF (20 mL). Reaction mass was stirred at 0-5°C for 20 minutes. Added Phenyl selenyl bromide (4.07 g, 17.25 mmol) dissolved in THF (10 mL) at 0-5°C and stirred for 2 hrs. Reaction mass was quenched into cold water, extracted with ethyl acetate (3x 50mL). Combined organic layer was concentrated to get crude compound which was purified by column chromatography over Silica gel using Ethyl acetate/Hexane as eluent to get 4.2g of N-(2,6-difluoropyridin-3-yl)-1-methyl-2-oxo-3-(phenylselanyl) pyrrolidine-3-carboxamide as light yellow solid. 65.6% yield, purity 99.2 % as per LCMS.

1H NMR (DMSO-d6): δ 10.23 (brs,1H), 8.71-8.64 (m, 1H), 7.59-7.56 (m,2H), 7.47-7.43 (m,1H), 7.35-7.31 (m,2H), 7.19-7.16(m, 1H), 3.39-3.28 (m, 1H), 3.24-3.18 (m, 1H), 2.79 (s, 3H), 2.63-2.55 (m, 1H), 2.19-2.14(m, 1H). LCMS (EI): m/z 412.2(M+1).

### Preparation AA

### Synthesis of N-(2,6-difluoropyridin-3-vl)-1-methyl-2-oxo-2,5-dihydro-1H-p,yrrole-3-carboxamide:

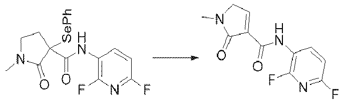

N-(2,6-difluoropyridin-3-yl)-1-methyl-2-oxo-3-(phenylselenyl) pyrrolidine-3-carboxamide (4g, 9.73mmol) was dissolved in dichloromethane (40 mL, 10 vol), added H₂O₂ (2.2g, 64.8 mmol; 30% aq solution) at 0-5°C. Reaction mass was stirred at 0-5 °C for 2 hrs. Reaction mass was quenched into saturated solution of NaHCO₃ at 0-5°C followed by Dichloromethane (2x100 mL) extraction. The Organic layers were washed with NaHCO3 solution (4x50 mL), dried over anhydrous Na₂SO₄ and concentrated to get crude. The crude compound was suspended in IPA (5 mL), stirred for 2 h filtered the solid and dried to get 1.5 g of N-(2,6-difluoropyridin-3-yl)-1-methyl-2-oxo-2,5-dihydro-1H-pyrrole-3-carboxamide as light yellow solid 62.5% yield, purity 91.6% as per LCMS.

1H NMR (CDCl3): δ 11.1 (s,1H), 8.98-8.92 (m, 1H), 8.0 (s, 1H),6.83 (m, 1H) 4.13 (s,2H), 3.15(s, 3H). LCMS (EI): m/z 254.2(M+1).

### Preparation AB

### Synthesis of (3R,4R)-N-(2,6-difluoropyridin-3-yl)-1-methyl-2-oxo-4-(4-(trifluoromethyl) phenyl)-pyrrolidine-3-carboxamide:

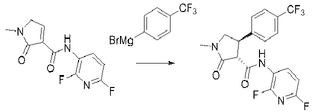

In a 25 mL two neck RBF, N-(2,6-difluoropyridin-3-yl)-1-methyl-2-oxo-2,5-dihydro-1H-pyrrole-3-carboxamide (300 mg, 1.18 mmol) was dissolved in dry Diethyl ether (10 ml), added CuBr.SMe₂ (12 mg, 0.05 mmol), followed by (R)-2,2'-bis(diphenylphosphanyl)-1,1'-binaphthalene (44 mg, 0.071mmol) at 20-25°C. Reaction mass was stirred at 20-25°C for 20 minutes. Reaction mass was cooled to -20 to -25°C using dry-ice/acetonitrile, added Trimethylsilyl trifluoromethanesulfonate (0.57 g, 2.6mmol) and stirred for 20 minutes. Added 4-(trifluoromethyl) phenyl) magnesium bromide (2.2 mL, 1.6M) at -20 to -25°C and stirred for 2 hours, at which time TLC analysis indicated completion of reaction (50:50 ethyl acetate: hexanes, Rε starting material = 0.3, Rε product = 0.5. Reaction mass quenched into aqueous NH₄Cl solution (20 mL), extracted with ethyl acetate (2x50 mL). Combined organic layers were washed with saturated brine solution (100 mL), dried over Na₂SO₄ and concentrated to get crude which was purified by column chromatography over Silica gel (60-120 mesh) using Ethyl acetate /Hexane (20:80) as eluent and obtained to get 120 mg of (3R,4R)-N-(2,6-difluoropyridin-3-yl)-1-methyl-2-oxo-4-(4-(trifluoromethyl)phenyl)pyrrolidine-3-carboxamide as a light yellow solid (24.6% yield, purity 89.8% as per LCMS) with chiral enantiomers ratio of 47.5:47.8 according to chiral HPLC.

1H NMR (CDCl3): δ 10.07 (s, 1H), 8.76-8.69 (m, 1H) 7.66-7.64 (m, 2H), 7.51-7.48 (m, 2H), 6.79-6.76 (m,1H), 4.16-4.09 (m, 1H), 3.83-3.79 (m, 1H), 3.65-3.62(d, 9.6 Hz, 1H), 3.50-3.56 (m, 1H), 3.07 (s, 3H). LCMS (ES): m/z 400.3(M+1).

### Preparation AC

### Synthesis of 1-Benzyl-N-(2-fluorophenyl)-2-oxopyrrolidine-3-carboxamide using Propylphosphonic anhydride (T3P).

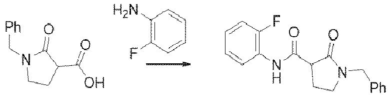

1-benzyl-2-oxopyrrolidine-3-carboxylic acid (3g, 13.69 mmol), (see WO2010/142801), was dissolved in Dimethylformamide (15 mL, 5 vol), cooled to 0°-5°C, added Triethylamine (4.15g, 41.0 mmol), 2-fluoroaniline (1.67g, 15.06 mmol) followed by Propyl phosphonic anhydride (T3P)

(8.7g, 27.36 mmol; 50% solution in EtOAc) at 0-5°C. Reaction mass was stirred for 2 hrs. at 0-5°C. Reaction mass was added into water and extracted with ethyl acetate (2x 100ml). Combined organic layer was washed with water followed by brine solution, dried over sodium sulphate and concentrated to get solid. To this solid heptane (10 vol) was added and stirred for 1 h, filtered and dried under vacuum to get 4.0 g of 1-Benzyl-N-(2-fluorophenyl)-2-oxopyrrolidine-3-carboxamide as off-white solid. 95.2% yield, purity 94.6% as per LCMS.

1H NMR (DMSO-d6): δ 10.12 (s,1H), 8.06-8.01 (m, 1H), 7.37-7.30 (m, 2H), 7.28-7.24 (m, 4H), 7.20-7.14 (m, 2H), 4.44 (s, 2H), 3.85-3.81 (t, J = 8 Hz, 1H), 3.36-3.22 (m, 2H), 2.31-2.22 (m, 2H). LCMS (EI): m/z 313.3 (M+1).

### Preparation AD

### Synthesis of 1-Benzyl-N-(2-fluorophenyl)-2-oxo-3-(phenylselanyl) pyrrolidine-3-carboxamide:

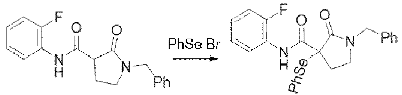

To the stirred solution of NaH (974 mg, 40.59 mmol) in THF (10 mL) at 0-5°C, was added 1-Benzyl-N-(2-fluorophenyl)-2-oxopyrrolidine-3-carboxamide (3.8 g, 12.17 mmol) dissolved in THF (20 mL, 5.2 vol). Reaction mass was stirred at 0-5°C for 20 minutes. Added Phenyl selenyl bromide (3.16 g, 13.39 mmol) dissolved in THF (10 mL) at 0-5°C and stirred for 2 hrs. Reaction mass was quenched into cold water, extracted with ethyl acetate (3x 50mL). Combined organic layer was concentrated to get crude compound which was purified by column chromatography over Silica gel using Ethyl acetate/Hexane as eluent to get 5.1 g of 1-Benzyl-N-(2-fluorophenyl)-2-oxo-3-(phenylselenyl) pyrrolidine-3-carboxamide as light yellow solid (89.4% yield, purity 98.6% as per LCMS).

1H NMR (DMSO-d6): δ 10.12 (brs,1H), 8.33-8.29 (t, J = 8Hz, 1H), 7.61-7.59 (m, 2H), 7.40-7.30 (m, 8H), 7.12-7.10 (m, 3H), 4.56- 4.41 (dd, 2H), 3.26-3.12 (m, 2H), 2.68-2.60 (m, 1H), 2.28-2.22 (m, 1H).

LCMS (EI): m/z 469.3(M+1).

### Preparation AE

### Synthesis of 1-Benzyl-N-(2-fluorophenyl)-2-oxo-2,5-dihydro-1H-pyrrole-3-carboxamide:

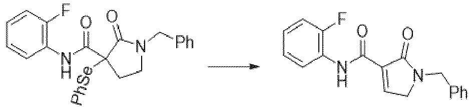

1-Benzyl-N-(2-fluorophenyl)-2-oxo-3-(phenylselanyl) pyrrolidine-3-carboxamide (5 g, 10.68 mmol) was dissolved in dichloromethane (50 mL, 10 vol), added H₂O₂ (2.4g, 71.22 mmol; 30% solution) at 0-5°C. Reaction mass was stirred at 0-5 °C for 2 hrs. Reaction mass was quenched into ice cold saturated NaHCO₃ solution, and extracted with Dichloromethane (3x100 mL). The Organic layers were washed with NaHCO3 solution (2x100 mL), dried under Na2SO4 and concentrated to get crude. The crude compound was suspended in IPA (10 mL, 2 vol), stirred for 2 h filtered the solid and dried to get 2.2 g of 1-Benzyl-N-(2-fluorophenyl)-2-oxo-2,5-dihydro-1H-pyrrole-3-carboxamide as off-white solid. 66.6 % yield, purity 94.4% as per LCMS.

LCMS (EI): m/z 311.2 (M+1).

### Preparation AF

### Synthesis of (3S,4S)-1-benzyl-N-(2-fluorophenyl)-2-oxo-4-(3-(trifluoromethyl)phenyl) pyrrolidine-3-carboxamide

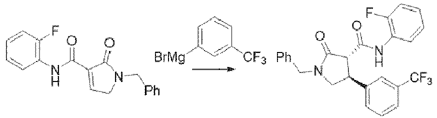

In a 25 mL two neck RBF, 1-Benzyl-N-(2-fluorophenyl)-2-oxo-2,5-dihydro-1H-pyrrole-3-carboxamide (300 mg, 0.96 mmol) was dissolved in dry Diethyl ether (9 ml), added CuBr.SMe₂ (mg, mmol), followed by (R)-2,2'-bis(diphenylphosphanyl)-1,1'-binaphthalene (10mg, 0.04mmol), at 20-25°C. Reaction mass was stirred at 20-25°C for 20 minutes. Reaction mass was cooled to -20 to -25°C using dry-ice/acetonitrile, added Trimethylsilyl trifluoromethanesulfonate (0.46g, 2.1 mmol) and stirred for 20 minutes. Added (3-(trifluoromethyl) phenyl) magnesium bromide (1.8 mL, 1.6M) at -20 to -25°C and stirred for 2 hours. Reaction mass quenched into aq. NH₄Cl solution (20 mL), extracted with ethyl acetate (2x50 mL). Combined organic layers were washed with saturated brine solution (100 mL), dried over Na₂SO₄ and concentrated to get crude which was purified by column chromatography over Silica gel (60-120 mesh) using Ethyl acetate /Hexane as eluent and obtained 50 mg of 1-benzyl-N-(2-fluorophenyl)-2-oxo-4-(3-(trifluoromethyl)phenyl)pyrrolidine-3-carboxamide as an oily liquid.

16.6% yield, purity 42.5% as per LCMS.

1H NMR (DMSO-d6): δ 10.15 (s, 1H), 8.0-7.97 (m, 1H) 7.69 (s, 1H), 7.65-7.56 (m, 3H), 7.39-7.35 (m, 2H), 7.32-7.30 (m, 4H), 7.29-7.24 (m, 2H), 4.54-4.42 (m, 2H), 4.19-4.17 (m, 1H), 4.10-4.08 (q, J = 8Hz, 1H), 3.80--3.75 (t, J = 12 Hz, 1H), 3.48-3.46 (m, 1H).

LCMS (ES): m/z 457.3 (M+1).

### Preparation AG

### Synthesis of (3S,4R)-N-(2,3-difluorophenyl)-1-methyl-4-(1-methyl-5-(trifluoromethyl)-1H-pyrazol-3-yl)-2-oxopyrrolidine-3-carboxamide

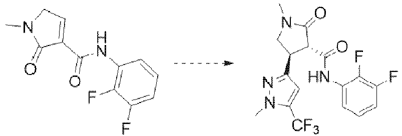

In a 25 mL two neck RBF, 3-bromo-1-methyl-5-(trifluoromethyl)-1H-pyrazole (810 mg, 3.57 mmol) was dissolved in dry diethyl ether (10 mL, 12.5 vol). Reaction mass was cooled to -78 to - 70°C, added n- Butyl lithium (4.28 mL, 2.5M, 3.0 eq) to the reaction mixture. The RM was allowed to stir at -78-70°C for 1hr. In another flask N-(2,3-difluorophenyl)-1-methyl-2-oxo-2,5-dihydro-1H-pyrrole-3-carboxamide (300 mg, 1.19 mmol), Copper(I) bromide dimethyl sulfide complex (12 mg, 0.059 mmol) and 2,2'-bis(diphenylphosphanyl)-1,1'-binaphthalene (44 mg, 0.071 mmol) in dry diethyl ether (9 mL, 30 vol) were taken and the reaction mixture was cooled to -78-70°C. To this RM, lithiated N-(2,3-difluorophenyl)-1-methyl-2-oxo-2,5-dihydro-1H-pyrrole-3-carboxamide was added dropwise at -78-70°C and stirred for 1 hr. Reaction mass quenched into aq. NH₄Cl solution, extracted with ethyl acetate (2x25 mL). Combined organic layer was washed with saturated brine solution, dried over Na₂SO₄ and concentrated to get crude, which was purified by column chromatography over Silica gel (60-120 mesh) using Ethyl acetate /Hexane as eluent and obtained 30 mg of (3S,4R)-N-(2,3-difluorophenyl)-1-methyl-4-(1-methyl-5-(trifluoromethyl)-1H-pyrazol-3-yl)-2-oxopyrrolidine-3-carboxamide with its enantiomer as a colourless liquid. 6% yield, purity 43.2% by LCMS.

LCMS (ES): m/z 403.3 (M+1).

### Preparation AH

### Synthesis of (3S,4S)-N-(2-fluorophenyl)-1-methyl-2-oxo-4-(3-(trifluoromethyl) phenyl) pyrrolidine-3-carboxamide using (CuOTf)₂.C6H6

N-(2-fluorophenyl)-1-methyl-2-oxo-2,5-dihydro-1H-pyrrole-3-carboxamide (500mg, 2.13 mmol) was dissolved in dry Diethyl ether (20 mL, 4 vol), added Copper(I) bromide dimethyl sulphide complex (22 mg, 0.1 mmol) and (S)-N-((S)-1-(butylamino)-1-oxo-3-phenylpropan-2-yl)-2-(((E)-2-(diphenylphosphaneyl) benzylidene) amino)-3-methylbutanamide (76 mg, 0.12 mmol) at 20-25°C and stirred at 20-25°C for 20 minutes. Reaction mass was cooled to -17 to -23°C, added Trimethylsilyl trifluoromethanesulfonate (1 g, 4.68 mmol) and stirred for 10 min. Added 3-(trifluoromethyl) phenyl) magnesium bromide (7.9 mL, 0.8M, 3.0 eq) at -17 -to -23°C and stirred for 2 hours. Reaction mass quenched into aq. NH₄Cl solution, extracted with ethyl acetate (2x25 mL). Combined organic layers were washed with saturated brine solution, dried over Na₂SO₄ and concentrated to get 960 mg of crude (3S,4S)-N-(2-fluorophenyl)-1-methyl-2-oxo-4-(3-(trifluoromethyl) phenyl) pyrrolidine-3-carboxamide as colourless liquid with chiral enantiomers ratio of 1:1.43 (excess of the S,S enantiomer) according to chiral HPLC.

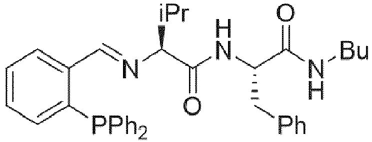

### Preparation AI

### Synthesis of 1-methylpiperidin-2-one

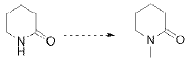

Taken Sodium hydride (9.0 g, 378.7 mmol) in THF (50 mL, 5.5 vol) at 0-5°C, added piperidin-2-one (25 g, 252.5 mmol) was dissolved in THF (50 mL, 10 vol). After stirred for 30 min, Methyl iodide (89.5 g, 631.3 mmol) was added to the reaction mixture dropwise. RM was slowly warmed to 20-25°C and stirred for 6h. Reaction mass was quenched into dilute HCL, extracted with 10% MeOH: DCM (2x250 mL). Combined organic layer were dried in sodium sulfate and concentrated to get crude compound which was purified by column chromatography over Silica gel using Ethyl acetate /Hexane as eluent to get 27.0 g of 1-methylpiperidin-2-one as colourless liquid (94.7% yield, purity 94.5% as per LCMS).

1H NMR (DMSO-d6): δ 3.23-3.20 (m, 1H), 2.78 (s, 3H), 2.18-2.15 (t, *J*is 12Hz, 2H), 1.73-1.65 (m, 4H).

LCMS (EI): m/z 114.0 (M+1).

### Preparation AJ

### Synthesis of 1-methyl-2-oxopiperidine-3-carboxylic acid:

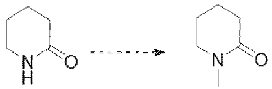

Taken Sodium hydride (5.3 g, 221.23 mmol) in THF (50 mL, 9.4 vol) at 0-5°C, added 1-methylpiperidin -2-one (5g, 44.24 mmol) was dissolved in THF (25 mL, 5 vol). The RM was warmed to 60-65°C and stirred at this temperature for 1 hr. After stirred for 1hr, Diethyl carbonate (31.3 g, 265.26 mmol) was added to the reaction mixture dropwise and stirred at 60-65°C for 12h. Reaction mass was quenched into cold water and extracted with 2-MeTHF. The aqueous layer was acidified with HCL, extracted with 10% IPA: DCM (5x50 mL). The combined organic layers were dried under Na2SO4 and concentrated to get crude. The crude compound was suspended in IPA (5 vol), stirred for 2 h filtered the solid and dried to get 4.7 g of 1-methyl-2-oxopiperidine-3-carboxylic acid as off-white solid. 68.1 % yield, purity 94.7% as per LCMS. 1H NMR (DMSO-d6): δ 12.56 (s, 1H), 3.33-3.20 (m, 3H), 2.82 (s, 3H), 1.99-1.90 (m, 1H), 1.88-1.69 (m, 3H).

LCMS (EI): m/z 158.1 (M+1).

### Preparation AK

### Synthesis of N-(2,3-difluorophenyl)-1-methyl-2-oxopiperidine-3-carboxamide

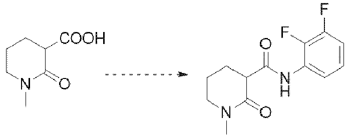

1-methyl-2-oxopiperidine-3-carboxylic acid (4 g, 25.4 mmol) was dissolved in Dimethylformamide (20ml, 5 vol), cooled to 0°-5°C, added Triethylamine (7.7 g, 76.4 mmol), 2,3-difluoroaniline (3.6 g, 28.0 mmol) followed by T3P (32.4 g, 101.9 mmol; 50% solution in EtOAc) at 0°-5°C. Reaction mass was stirred for 2 hrs. at 0-5°C. Reaction mass was added into water and extracted with ethyl acetate (2x50 ml). Combined organic layer was washed with water followed by brine solution, dried over sodium sulphate and concentrated to get solid. To this solid heptane (10 vol) was added and stirred for 1 hr, filtered and dried under vacuum to get 2.1 g of N-(2,3-difluorophenyl)-1-methyl-2-oxopiperidine-3-carboxamide as off-white solid. 29.4 % yield, purity 92.2 % as per LCMS.

1H NMR (DMSO-d6): δ 10.29 (s,1H), 7.83-7.7.78 (m, 1H), 7.20-7.13 (m,2H), 3.62-3.58 (t, 16 Hz, 1H), 3.37-3.25 (m, 2H), 2.89(s, 3H), 2.05-2.0(m, 2H), 1.99-1.85 (m, 1H), 1.76-1.70 (m, 1H). LCMS (EI): m/z 269.2(M+1).

### Preparation AL

### Synthesis of N-(2,3-difluorophenyl)-1-methyl-2-oxo-3-(phenylselenyl) piperidine-3-carboxamide

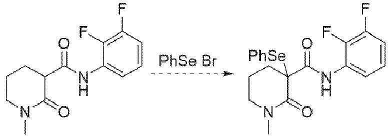

N-(2,3-difluorophenyl)-1-methyl-2-oxopiperidine-3-carboxamide (2 g, 7.46 mmol) was dissolved in THF (20 ml, 10 vol), added NaH (0.89 g, 37.3 mmol) at 0-5°C. Reaction mass was stirred at 0-5°C for 30 minutes. Added Phenyl selenyl bromide (1.76 g, 7.46 mmol) dissolved in THF (5 mL) at 0-5°C and stirred for 2 hrs. Reaction mass was quenched into cold water, extracted with ethyl acetate (2x50 mL). Combined organic layer was concentrated to get crude compound which was purified by column chromatography over Silica gel using Ethyl acetate/Hexane as eluent to get 2.6 g of N-(2,3-difluorophenyl)-1-methyl-2-oxo-3-(phenylselenyl) piperidine-3-carboxamide as light yellow solid. 83.8% yield, purity 93.1% as per LCMS.

1H NMR (DMSO-d6): δ 10.94 (s,1H), 7.98-7.94 (m, 1H), 7.53-7.51 (m,2H), 7.45-7.41 (m,1H), 7.34-7.31 (m, 2H), 7.19-7.11 (m, 2H), 3.40-3.31 (m, 2H), 2.93 (s, 3H), 2.23-2.17 (m, 1H), 2.19-1.88 (m, 2H), 1.67-1.64 (m, 1H).

LCMS (EI): m/z 425.2(M+1).

### Preparation AM

### Synthesis of N-(2,3-difluorophenyl)-1-methyl-2-oxo-1,2,5,6-tetrahydropyridine-3-carboxamide

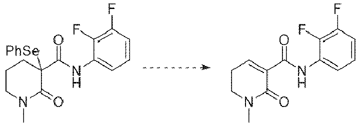

N-(2,3-difluorophenyl)-1-methyl-2-oxo-3-(phenylselenyl) piperidine-3-carboxamide (2.5 g, 5.89 mmol) was dissolved in MDC (25 ml, 10 vol), added H₂O₂ (1.3 g, 39.30 mmol; 30% solution) at 0-5°C. Reaction mass was stirred at 0-5 ° C for 2 hrs. Reaction mass was quenched into ice cold saturated NaHCO₃ solution, and extracted with Dichloromethane (2x50 mL). The Organic layers were washed with NaHCO3 solution (3x20 mL), dried under Na2SO4 and concentrated to get crude. The crude compound was suspended in IPA (5 mL), stirred for 2 h filtered the solid and dried to get 1.2 g of N-(2,3-difluorophenyl)-1-methyl-2-oxo-1,2,5,6-tetrahydropyridine-3-carboxamide as light yellow solid 80% yield, purity 98.3% as per LCMS.

1H NMR (DMSO-d6): δ 12.32 (s, 1H), 8.18-8.13 (m, 1H), 7.86-7.84 (t, Jis8 Hz, 1H) 7.23-7.11 (m, 2H), 3.53-3.49 (t, Jis16 Hz, 2H), 2.99 (s, 3H), 2.66-2.61 (m, 2H).

LCMS (EI): m/z 267.2 (M+1).

### Preparation AN

### Synthesis of anti-N-(2,3-difluorophenyl)-4-(4-fluorophenyl)-1-methyl-2-oxopiperidine-3-carboxamide

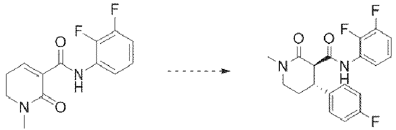

In a 25 mL two neck RBF, 1-bromo-4-fluorobenzene (530 mg, 3.0 mmol) was dissolved in dry THF (6 mL, 11 vol). Reaction mass was cooled to -78 to -70°C, added n- Butyl lithium (1.8 mL, 2.5M, 1.5 eq) to the reaction mixture. The RM was allowed to stir at -78-70°C for 1h. In another flask N-(2,3-difluorophenyl)-1-methyl-2-oxo-1,2,5,6-tetrahydropyridine-3-carboxamide (250 mg, 0.93mmol), Copper(I) bromide dimethyl sulfide complex (9 mg, 0.046 mmol) and 2,2'-bis(diphenylphosphanyl)-1,1'-binaphthalene (35 mg, 0.056 mmol) in THF (5 mL, 20 vol) were taken and the reaction mixture was cooled to -78-70°C. To this RM, lithiated 1-bromo-4-fluorobenzene was added dropwise at -78-70°C and stirred for 1 h. Reaction mass quenched into aq. NH₄Cl solution, extracted with ethyl acetate (2x50 mL). Combined organic layer was washed with saturated brine solution, dried over Na₂SO₄ and concentrated to get crude, which was purified by column chromatography over Silica gel (60-120 mesh) using Ethyl acetate /Hexane as eluent and obtained 40 mg of anti-N-(2,3-difluorophenyl)-4-(4-fluorophenyl)-1-methyl-2-oxopiperidine-3-carboxamide as off-white solid. 11.7% yield, purity ~90% by LCMS.

1H NMR (DMSO-d6): δ 9.91 (s, 1H), 7.54-7.50 (m, 1H), 7.32-7.29 (m, 2H), 7.14-7.06 (m, 4H), 3.84- 3.81 (d, 1H), 3.55-3.42 (m, 3H), 2.96 (s, 3H), 2.07-1.96 (m, 2H).

LCMS (ES): m/z 363.3 (M+1).

### Preparation ANa

### Synthesis of (3S,4S)-N-(2-fluorophenyl)-1-methyl-2-oxo-4-(3-(trifluoromethyl) phenyl) pyrrolidine-3-carboxamide using (R)-(+)-1,1 '-Bi(2-naphthol) in Methyl tert-butyl ether as solvent.

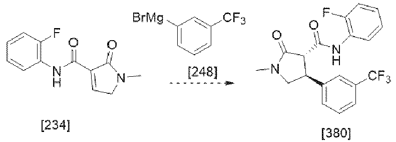

In a 25 mL flask A, (R)-(+)-1,1'-Bi(2-naphthol) (91 mg 0.320 mmol) was taken in 2 mL of dry Methyl tert-butyl ether at 20-25°C and stirred for 50 min. In flask B, (3-(trifluoromethyl)phenyl) magnesium bromide (5.3 mL, 4.26 mmol, 0.8 M in Et₂O) was slowly added into Bis-[2-(N,N-dimethylaminoethyl ether (683 mg 4.27 mmol) in 3 mL dry Methyl tert-butyl ether at 0-5°C under nitrogen and then was stirred for 30 min. The mixture A was then introduced into the mixture B at 0-5°C. Then the RM was warmed to 20-25°C, and stirred for 1 hr. Then RM was cooled to 0-5°C, N-(2-fluorophenyl)-1-methyl-2-oxo-2,5-dihydro-1H-pyrrole-3-carboxamide (500 mg 2.13 mmol) in Methyl tert-butyl ether (5 mL) was added dropwise. The mixture was warmed to 20-25°C and stirred for 12 h. The reaction was quenched with 10% aqueous HCl solution and extracted with ethyl acetate (25 mLx2). The combined organic layers were dried with anhydrous Na₂SO₄ and evaporated in vacuo to get 800 mg of crude. The crude product was purified by column chromatography over Silica gel (60-120 mesh) using Ethyl acetate/Hexane (20:80) as eluent and obtained 50 mg of (3S,4S)-N-(2-fluorophenyl)-1-methyl-2-oxo-4-(3-(trifluoromethyl) phenyl) pyrrolidine-3-carboxamide with chiral enantiomers ratio of (R,R enantiomer :S,S enantiomer) 1:2 according to chiral HPLC.

LCMS (ES): m/z 381.2 (M+1).

### Preparation AO

### Synthesis of N-(2-fluorophenyl)-1-methyl-2-oxo-3-(phenylthio)-4-(3-(trifluoromethyl) phenyl) pyrrolidine-3-carboxamide

To the stirred solution of NaH (0.94 g, 39.47 mmol) in THF (10 mL) at 0-5°C, was added N-(2-fluorophenyl)-1-methyl-2-oxo-4-(3-(trifluoromethyl) phenyl) pyrrolidine-3-carboxamide (1.5g, 3.94 mmol) in THF (10 mL). Reaction mass was stirred at 0-5°C for 20 minutes. Added Diphenyldisulphide (1.29 g, 5.92 mmol) in THF (10 mL) at 0-5°C.The RM was stirred at 20-25°C for 48 hrs. Reaction mass was quenched into cold water, extracted with ethyl acetate (3x 50mL). Combined organic layers was concentrated to get crude compound which was purified by column chromatography over Silica gel using Ethyl acetate/Hexane as eluent to get 1.5 g of N-(2-fluorophenyl)-1-methyl-2-oxo-3-(phenylthio)-4-(3-(trifluoromethyl) phenyl) pyrrolidine-3-carboxamide as off-white solid. 78.9% yield, purity 96.6 % as per LCMS.

1H NMR (DMSO-d6): δ 10.28 (s,1H), 7.68-7.64 (m, 1H), 7.57-7.55 (m, 1H), 7.52-7.42 (m, 5H), 7.38-7.32 (m, 1H), 7.19-7.02 (m, 2H), 4.05-3.97 (m, 2H), 3.37-3.33 (m, 1H), 2.91 (s, 3H). LCMS (EI): m/z 489.3 (M+1).

### Preparation AP

### Synthesis of N-(2-fluorophenyl)-1-methyl-2-oxo-4-(3-(trifluoromethyl) phenyl)-2,5-dihydro-1H-pyrrole-3-carboxamide

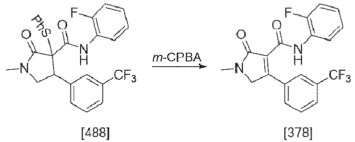

N-(2-fluorophenyl)-1-methyl-2-oxo-3-(phenylthio)-4-(3-(trifluoromethyl) phenyl) pyrrolidine-3-carboxamide (1.5g, 3.07mmol) was dissolved in dichloromethane (15mL, 10 vol), added *m*-CPBA (1.05g, 6.14 mmol) at 20-25°C. Reaction mass was stirred at 20-25°C for 4hrs. Reaction mass was quenched into ice cold saturated NaHCO₃ solution, and extracted with Dichloromethane (2x50 mL). The Organic layers were washed with NaHCO3 solution (2x50 mL), dried under Na₂SO₄ and concentrated to get solid. The solid was suspended in heptane (10 mL), stirred for 2 h filtered the solid and dried to get 600 mg of N-(2-fluorophenyl)-1-methyl-2-oxo-4-(3-(trifluoromethyl) phenyl)-2,5-dihydro-1H-pyrrole-3-carboxamide as off-white solid (54.5 % yield, purity 82.8% as per LCMS).

1H NMR (CDCl3): δ 11.52 (s,1H), 8.40-8.36 (m, 1H), 7.92-7.90 (m, 1H), 7.79 (s, 1H), 7.73-7.71 (m, 1H), 7.61-7.57 (m, 1H), 7.14-7.04 (m, 3H), 4.33 (s, 2H), 3.21 (s, 3H). LCMS (EI): m/z 379.2 (M+1).

It is recognized by one skilled in the art that various functional groups can be converted into others to provide different Saturated Target compounds encompassed by the general Formula 1 as defined above. For a valuable resource that illustrates the interconversion of functional groups in a simple and straightforward fashion, see Larock, R. C., Comprehensive Organic Transformations: A Guide to Functional Group Preparations, 2nd Ed., Wiley-VCH, New York, 1999.

It is recognized that some reagents and reaction conditions described above for preparing Saturated Target compounds and the corresponding novel and inventive intermediates, may not be compatible with certain functionalities present in the inventive intermediates from which they are prepared. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. Protective Groups in Organic Synthesis, 2nd ed.; Wiley: New York, 1991).

One skilled in the art will recognize that, in some cases, after the introduction of a given reagent as it is depicted in any individual scheme, it may be necessary to perform additional routine synthetic steps not described in detail to complete the synthesis of *inter alia* Saturated Target compounds encompassed by the general Formula 1 as defined above and the novel and inventive intermediates detailed herein. One skilled in the art will also recognize that it may be necessary to perform a combination of the steps illustrated in the above schemes in an order other than that implied by the particular scheme depicted to prepare the compounds including the Saturated Target compounds.

One skilled in the art will also recognize that The Saturated Target compounds and the inventive intermediate, impurity, marker, and degradant compounds including *inter alia,* the compounds as encompassed by the general formulae **II, III, IV** and **D,** described and defined herein can be subjected to various electrophilic, nucleophilic, radical, organometallic, oxidation, and reduction reactions to add substituents or modify existing substituents.

Without further elaboration, it is believed that one skilled in the art using this disclosed description can utilize the present invention to its fullest extent. The above examples, described embodiments and descriptions of processes are, therefore, to be construed as merely illustrative, and not limiting of the disclosure in any way whatsoever.

Inventors claim protection for all working embodiments of their invention, if inadvertently an unworkable embodiment has been unintentionally included within the scope of a broad description or definition, inventors clarify that all unworkable embodiments are definitely and unreservedly excluded from claims for protection. In other words, an embodiment that is unworkable is, by definition, outside the breadth of scope of protection requested.

Inventors claim protection for all novel and non-obvious embodiments of their invention, if inadvertently an embodiment that is not novel has been unintentionally included within the scope of a broad description or definition, inventors clarify that all such anticipated embodiments are and have been excluded from claims for protection. In other words, if in a multi-component embodiment that includes several compounds is determined on examination to include an anticipated member of the group of components encompassed, then by definition, inventors had no intention to include it and that compound is and always was outside the breadth of scope of protection requested. The error in inadvertently including an anticipated compound within a broad list of compounds through textural inexactitude should be remedied through deletion of that erroneous group member, without effecting the remaining compounds of that list or group.

The present invention is further defined by the following items:
1. A process **B1** for preparing a Saturated Target of formula 1 from an Unsaturated Precursor of formula **2** according to the following Scheme, 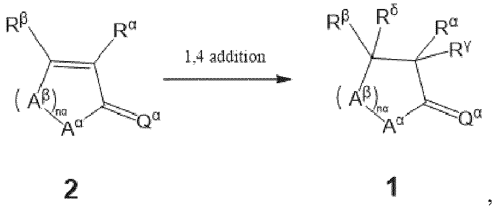
   comprising substitution of the 1,4 system exemplified by 1,4-addition using Grignard reagent, organo-lithium reagent, organo-zinc reagent, hydrogenation , hydride source, electron source (reduction, exemplified by Zn/AcOH) or enzyme wherein each of **R^{β}, A^{α}, A^{β}, nα, Q^{α}** and **R^{ε}**, have the same meanings as defined in compound **A2**, and
   **R^{α}** is H, halogen or carbon-containing group exemplified by, substituted or unsubstituted, alkyl, aryl, heterocyclic, naphthyl, ester including aliphatic and aromatic esters exemplified by, C(O)OC₆H₅, -C(O)OC₆H₄F, amide including aliphatic and aromatic exemplified by - C(O)NHC₆H₅), hydrazinamide including aliphatic and aromatic exemplified by, -C(O)NHNHC₆H₅), thioesters, thionoesters and similar compounds exemplified by, -C(O)NHOC₆F₅); and -C(S)SC₆H₅) etc., wherein
   **R^{α}** is not connected to the ring via O or N atoms, such that for example, **R^{α}** is not -OMe, and wherein, whenever H is added at the 4 position of the 1,4 system, **R^{α}** is is not H, and wherein **R^{γ}** is H or carbon-containing group such as substituted or unsubstituted, alkyl, aryl, naphthyl etc.
   **R^{δ}** is H or carbon-containing group such as substituted or unsubstituted, alkyl, aryl, naphthyl etc.
2. A process as defined in any one of processes B1; B1b; B1c; B1d; B2; B2a; B3; B3a; B4; B5; B6; B6a; B7; B8; B9; B10; B11; B12; B13; B14; B15; B16; B16a; B16b; B16c; B16d; B17; B18; B18a; B18b; B18c; B18d; B19; B19a; B19b; B19c; B20; B21a; B21b; B21c; B22; B23; B24; B25; B26; B27a; B27b; B28a; B28b; B29a; B29b; B30a; B30b; B31; B32; B33; B34; B35; B36; B37; B38; B39; B40; B41; B42; B43; B44; B45; B46; B47; B48; B49; B50; and B51 or selected from the group of,
   a) a process **B1b** for preparing a Saturated Target of formula 1 from Unsaturated Precursor of formula **2** 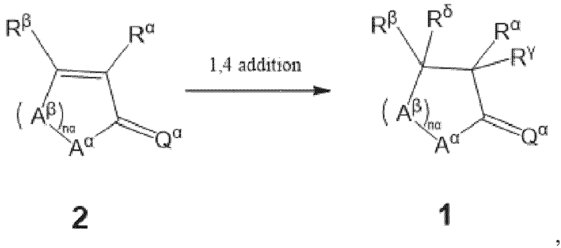 comprising substitution of the 1,4 system exemplified by, 1,4-addition using Grignard reagent, organo-lithium reagent, organo-zinc reagent, hydrogenation , hydride source, electron source, reduction, exemplified by Zn/AcOH or enzyme, wherein each of
      **R^{β}, A^{α}, A^{β}, nu, Q^{α}** and **R^{ε},** have the same meanings as defined in compound **A2,** and wherein
      **R^{α}** is H, halogen or carbon-containing group exemplified by, substituted or unsubstituted, alkyl, aryl, heterocyclic, naphthyl, ester including aliphatic and aromatic esters e.g. C(O)OC₆H₅, or -C(O)OC₆H₄F), amide including aliphatic and aromatic e.g. - C(O)NHC₆H₅), hydrazinamide including aliphatic and aromatic e.g. -C(O)NHNHC₆H₅), thioesters, thionoesters and similar compounds such as -C(O)NHOC₆F₅); -C(S)SC₆H₅) etc.,
      **R^{γ}** is H or carbon-containing group such as substituted or unsubstituted, alkyl, aryl, naphthyl etc.,
      **R^{δ}** is H or carbon containing group such as substituted or unsubstituted, alkyl, aryl, naphthyl etc., with the proviso that
      **R^{α}** is not connected to the ring via O or N atoms, such that for example, **R^{α}** is not -OMe, and that, whenever H is added at the 4 position of the 1,4 system, **R^{α}** is not H with the exception that when **A^{α}** is N(Me) and **R^{β}** is m-(trifluoromethyl)benzene, m-(trifluoromethyl)benzene, or 6-(trifluoromethyl)pyridin-3-yl, then **R^{α}** may be H such as in the following scheme: 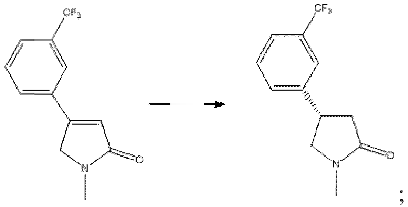
   b) a process **B1c** for preparing a Saturated Target from a corresponding Unsaturated Precursor according to any one of processes **B1** and **B1b** comprising, combining the Unsaturated Precursor, a nucleophile exemplified by Grignard reagent, organo-lithium reagent, organo-zinc reagent, hydrogen source, e.g. H₂/Pd/C or hydride source or electron source exemplified by Zn/AcOH, and at least one solvent to affect a 1,4 system substitution on the Unsaturated Precursor e.g. a 1,4-addition reaction, to obtain the Saturated Target such as defined by the following formula, 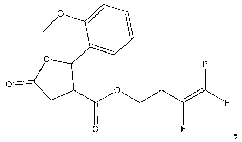 wherein preferably, depending of the reaction condition one or more of the isomers is in excess exemplified by the trans isomers or cis isomers: 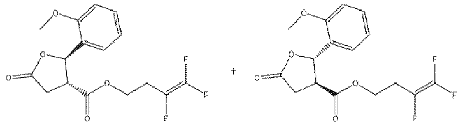 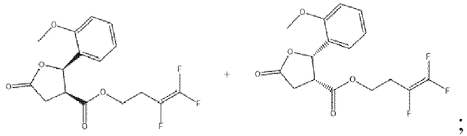
   c) a process **B1d** for preparing a Saturated Target from a corresponding Unsaturated Precursor comprising substitution of the Unsaturated Precursor 1,4 system e.g. by a 1,4-addition using Grignard reagent, organo-lithium reagent, organo-zinc reagent, hydrogenation or hydride source, wherein, the Saturated Target product obtained has excess in one or more of the possible isomers, said process represented by schemes selected from the group of: 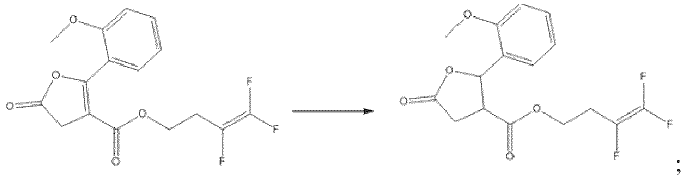 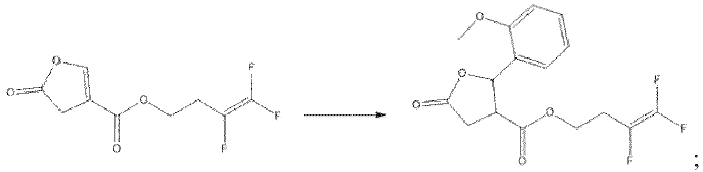 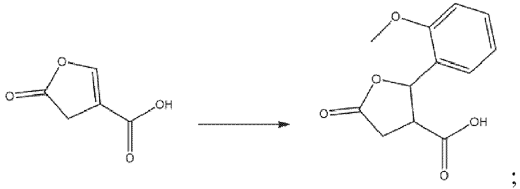 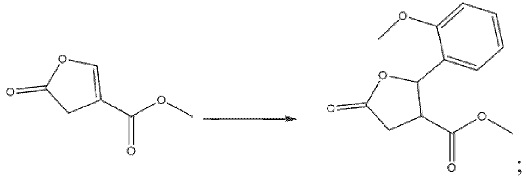 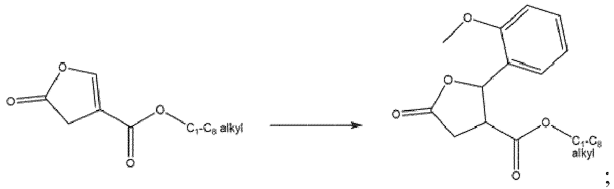 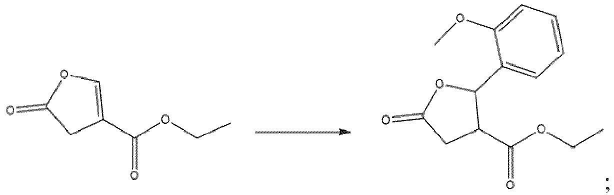 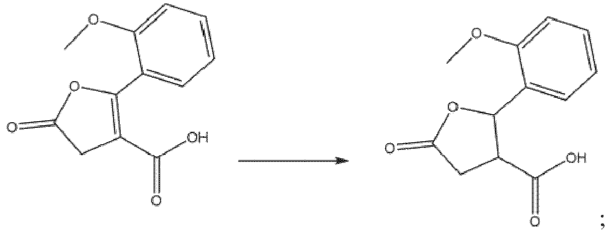 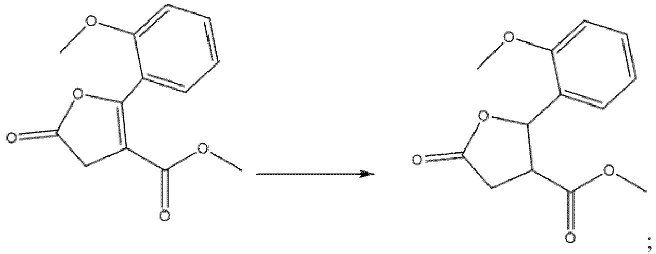 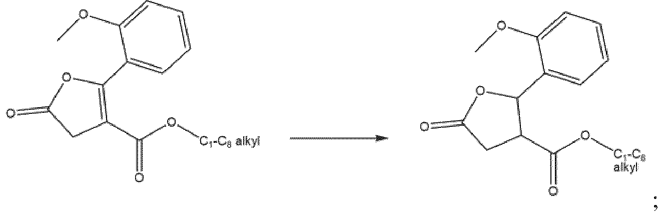 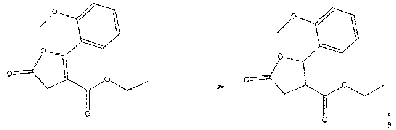 and relevant combinations thereof;
   d) a process **B2** according to any one of processes selected from **B1;** and **B1b,** comprising combining an Unsaturated Precursor, a nucleophile exemplified by Grignard reagent, organo-lithium reagent, organo-zinc reagent, hydrogen source, such as, H₂/Pd/C or hydride source or electron source such as Zn/AcOH, and solvent to affect a 1,4 system substitution of the Unsaturated Precursor such as a 1,4-addition reaction to obtain the Saturated Target.
   e) a process **B2a** for preparing a Saturated Target of formula **1** from an Unsaturated Precursor of formula **2** 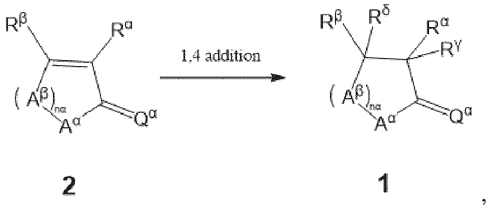 comprising substitution of the 1,4 system such as, 1,4-addition using Grignard reagent, organo-lithium reagent, organo-zinc reagent, hydrogenation or hydride source,
      wherein, the Saturated Target product, has excess in one or more of the possible isomers,
      wherein each of **R^{α}, R^{β}, A^{α}, A^{β}, nα**, **Q^{α}** and **R^{ε},** have the same meanings as defined for compound **A2,**
      **R^{γ}** is H or **R^{α}** and
      **R^{δ}** is H or **R^{α}**
   f) A process **B3** according to any one of process **B2** and process **B2a** comprising, combining an Unsaturated Precursor, a nucleophile such as a Grignard reagent, organo-lithium reagent, organo-zinc reagent, or a hydride source, a solvent and one or more catalytic components each chosen from, catalyst; pre catalyst and co-catalyst, to affect a 1,4 system substitution of the Unsaturated Precursor such as a 1,4-addition reaction to obtain the Saturated Target.
   g) A process **B3a** according to any one of process **B2** and process **B2a** comprising, combining an Unsaturated Precursor, a nucleophile such as a Grignard reagent, organo-lithium reagent, organo-zinc reagent, or a hydride source, a solvent and one or more catalytic components each chosen from, catalyst; pre catalyst and co-catalyst, to affect a 1,4 system substitution of the Unsaturated Precursor such as a 1,4-addition reaction to obtain the Saturated Target, wherein, the, Saturated Target product has one or more of the possible isomers in excess.
   h) A process **B4** according to any one of processes **B2, B2a, B3,** and **B3a,** comprising, including within the combination, an additive which is a source for a reversibly bound adduct, to the Unsaturated Precursor, such as Lewis acid, to provide an improvement in the 1,4-addition reaction on the Unsaturated Precursor to obtain the Saturated Target, in any of the following schemes or any combination thereof, wherein RGA is the reversibly bound adduct; 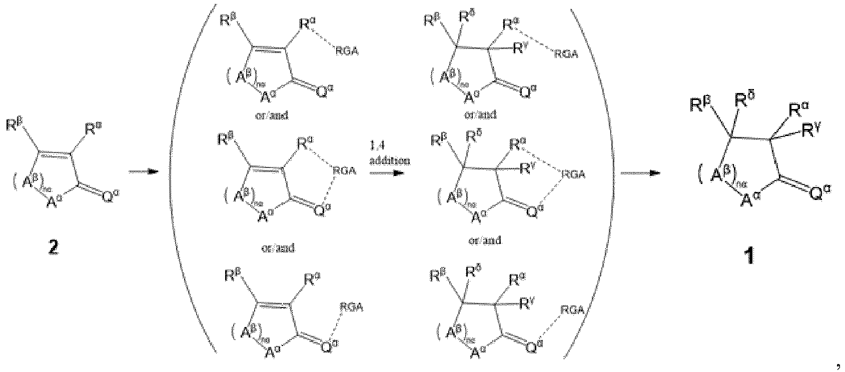
      wherein said improvement provided is any of, protecting part of the Unsaturated Precursor from un-desired reaction; increasing pathway selectivity; increasing the selectivity for specific desired isomer; increasing the reactivity of the Unsaturated Precursor; and enabling reduction in the amount of the **R^{δ}** source such as Grignard reagent, required,
      wherein, **R^{α}**, **R^{β}**, **R^{γ}**, **A^{α}**, **A^{β}**, **nα**, **Q^{α}** and **R^{ε}** and **R^{δ}** have the same meaning as defined in any one of processes **B1; B1a, B1b; B2** and **B2a;**
   i) A process **B5** as defined in any one of processes **B2; B2a; B3; B3a;** and **B4** further comprising a step of purifying the obtained Saturated Target product;
   j) a process **B6** according to any one of processes **B2; B2a; B3; B3a; B4;** and **B5,** further comprising a step of crystalizing the obtained Saturated Target product;
   k) a process **B6a** according to any one of processes **B2; B2a; B3; B3a; B4;** and **B5,** further comprising a step of obtaining a non-crystalline solid Saturated Target product by subjecting the Saturated Target obtained by the process of any one of processes **B2; B2a; B3; B3a; B4** to a process such as spray drying, freeze drying, or by adsorbing a solution of the Saturated Target onto solid carrier material;
   l) a process **B7** according to any one of processes **B2** - **B6,** comprising combining an Unsaturated Precursor, a nucleophile such as a Grignard reagent, organo-lithium reagent, organo-zinc reagent, or hydride source; solvent; one or more catalytic components each selected from, catalyst; pre catalyst; co-catalyst; and an additive which is a source for a reversibly bound adduct, to affect a 1,4-addition reaction to obtain the Saturated Target, further comprising steps of purifying and crystallizing the reaction product to obtain crystalline, purified, Saturated Target product;
   m) a process **B8 B7** wherein the combination step comprises a Grignard reagent; organo-lithium reagent; organo-zinc reagent, or a hydride source, with solvent such as diethyl ether, and two or more catalytic components each selected from catalyst; pre catalyst; co-catalyst and mixtures thereof and Lewis acid;
   n) a process **B9** according to any one of processes **B1** to **B8,** comprising, dissolving the Unsaturated Precursor in a solvent such as, diethyl ether, and preferably adding a catalytic component comprising one or more of, catalyst; pre catalyst; co-catalyst such as a combination of both CuBr.SMe₂ (Copper(I) bromide dimethyl sulfide complex) and R-BINAP or (S)-N-((S)-1-(butylamino)-1-oxo-3-phenylpropan-2-yl)-2-(((E)-2-(diphenylphosphaneyl) benzylidene) amino)-3-methylbutanamide 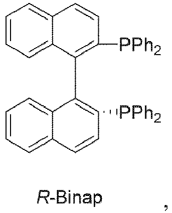 agitating the reaction mass by e.g., stirring, or by mixing in a continuous reactor and the like, and optionally adding a source for a reversibly bound adduct, to the Unsaturated Precursor, such as Lewis acid e.g., Trimethylsilyl chloride, and continuing agitation for a suitable time such as about 20 minutes, adjusting the temperature to optimal levels preferably a cold temperature, such as for example of between -20°C to -25°C, adding nucleophile, such as for example a Grignard reagent or a hydride source, and agitating for a period of time to achieve an optimal product by balancing parameters typified by considerations of, desired quality, and acceptable yield within a reasonable reaction time, quenching the reaction mass, such as for example into aqueous NH₄Cl solution, and optionally extracting with suitable solvent, optionally, washing the combined organic layer with for example, saturated brine solution, and optionally drying e.g., over Na₂SO₄ and optionally concentrating to obtain the Saturated Target product;
   o) a process **B10** according to process **B9** further comprising a step of purifying the obtained Saturated Target product;
   p) a process **B11** according to any one of processes **B9** and **B10** further comprising a step of crystalizing the obtained Saturated Target product;
   q) a process **B12** according to process **B1** comprising the following steps:
      in a first vessel **A,** dissolving an Unsaturated Precursor in a solvent such as diethyl ether, and agitating, optionally cooling the reaction mass to a suitable temperature such as to between 20°C to -25°C, and adding Lewis acid, such as, Trimethylsilyl chloride, and agitating, in a second vessel **B,** introducing a catalytic component comprising one or more of, catalyst; pre-catalyst; co-catalyst such as a combination of both CuBr.SMe₂ (Copper(I) bromide dimethyl sulfide complex) and R-BINAP 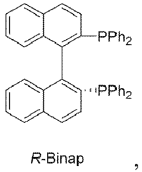 adding a nucleophilic component, for example a Grignard reagent, organo-lithium reagent, organo-zinc reagent, or a hydride source, to vessel **B,** and agitating, adding the contents of vessel A to vessel **B** and agitating the mass for a time such as for example 20 minutes, quenching the reaction mass e.g. into aq. NH₄Cl solution, and optionally extracting with solvent, optionally washing the combined organic layer with e.g., saturated brine solution, and optionally, drying e.g. over Na₂SO₄, and optionally concentrating to obtain the Saturated Target product;
   r) a process **B13** according to process **B12,** further comprising a step of purifying the obtained Saturated Target;
   s) a process **B14** according to any one of processes **B12** and **B13** further comprising a step of crystalizing the obtained Saturated Target product;
   t) a process **B15** according to any one of processes **B1** to **B14,** wherein the Unsaturated Precursor is any one of the compounds **A1** - **A35,** comprising, substitution of the 1,4 system of the Unsaturated Precursor such as 1,4-addition using Grignard reagent, organo-lithium reagent, organo-zinc reagent, or hydride source;
   u) a process **B16** according to any one of processes **B1- B15** wherein the Unsaturated Precursor is a compound **A10,** represented by the following scheme, 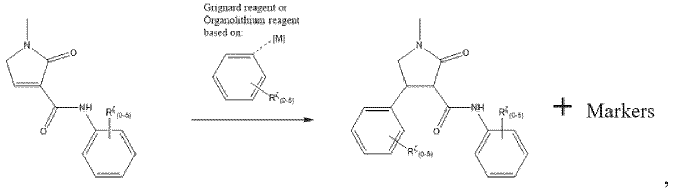 wherein, the markers that can be formed in the above scheme can include one; some, or all of the following structures, 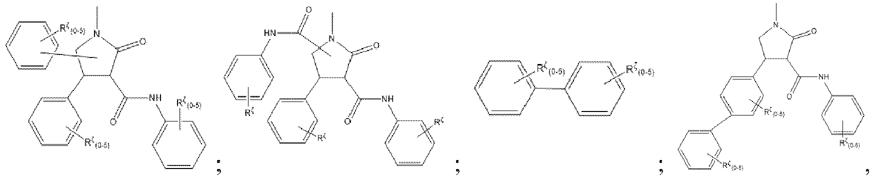 comprising, substitution of the Unsaturated Precursor 1,4 system such as 1,4-addition using Grignard reagent or/and organo-lithium reagent, such as (3-(trifluoromethyl)phenyl)lithium);
   v) a process **B16a** according to any one of processes **B1- B15** wherein the Unsaturated Precursor is a compound **A10** such as represented by the following scheme, 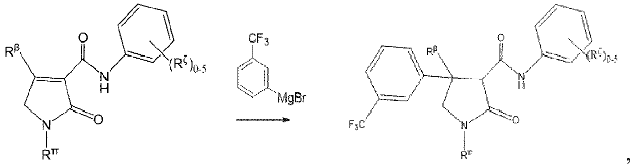 comprising, substitution of the Unsaturated Precursor 1,4 system such as 1,4-addition using Grignard reagent, such as
      3-(trifluoromethyl)phenyl magnesium bromide, or
      3-(trifluoromethyl)phenyl]magnesium chloride etc;
   w) a process **B16b** according to any one of processes **B1- B15** wherein the Unsaturated Precursor is a compound **A10,** such as represented by the following scheme, 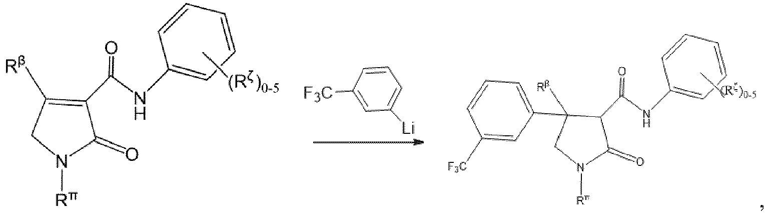 comprising, substitution of the Unsaturated Precursor 1,4 system such as by, 1,4-addition using organo-lithium reagent, e.g., 3-(trifluoromethyl)phenyl lithium;
   x) a process **B16c** according to any one of processes **B1- B15** wherein the Unsaturated Precursor is a compound **A10,** such as represented by the following scheme, 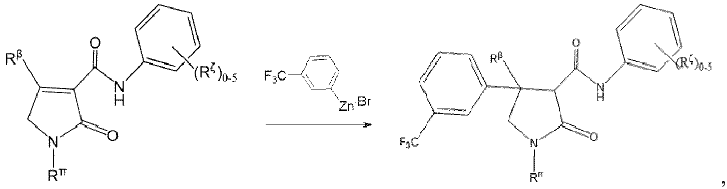 comprising, substitution of the Unsaturated Precursor 1,4 system such as by 1,4-addition using organo-zinc reagent, e.g., 3-(trifluoromethyl)phenyl zinc(II) bromide);
   y) a process **B16d** according to any one of processes **B1- B15** wherein the Unsaturated Precursor is a compound **A10,** such as represented by the following scheme, 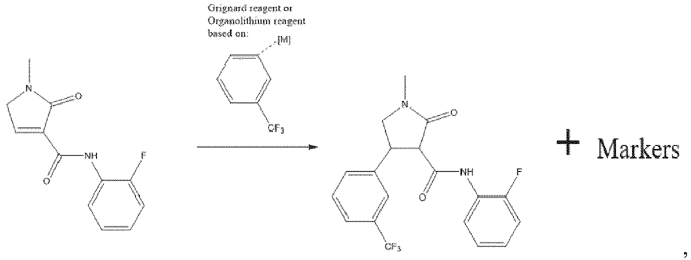 wherein, the markers that can be formed in the above scheme can include one; some; or all of the following structures, 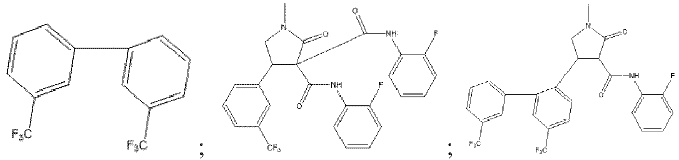 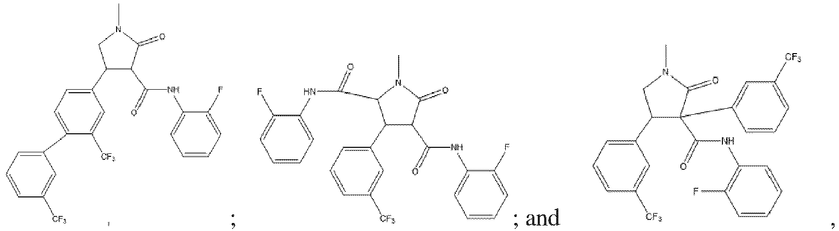 comprising, substitution of the Unsaturated Precursor 1,4 system such as by1,4-addition using Grignard reagent or/and organo-lithium reagent, e.g., 3-(trifluoromethyl)phenyl)lithium);
   z) a process **B17** such as represented by the following scheme, 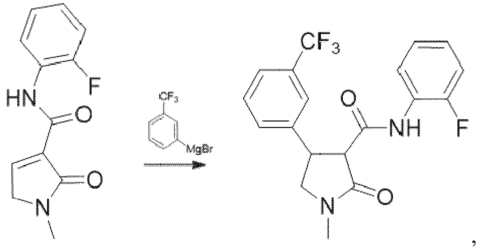 comprising, substitution of the Unsaturated Precursor 1,4 system such as by 1,4-addition using Grignard reagent, e.g, 3-(trifluoromethyl)phenyl magnesium bromide;
   aa) a process **B18** such as represented by the following scheme, 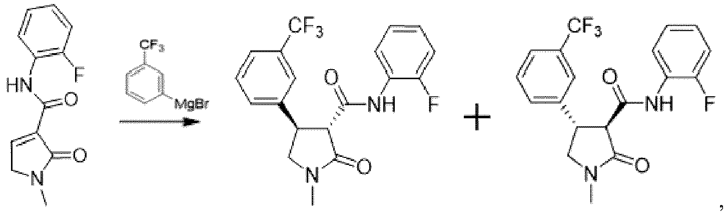 comprising substitution of the 1,4 system such as by a 1,4-addition using Grignard reagent, e.g., 3-(trifluoromethyl)phenyl magnesium bromide;
   bb) a process **B18a** according to process **B18** such as represented by the scheme, 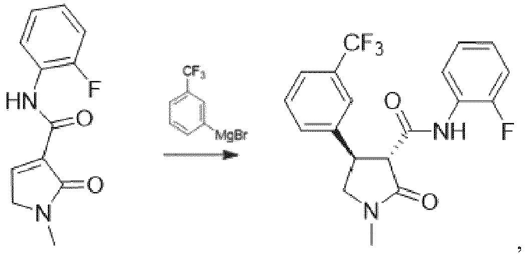 comprising substitution of the 1,4 system such as by a 1,4-addition using Grignard reagent, e.g., 3-(trifluoromethyl)phenyl magnesium bromide;
   cc) a process **B18b** according to process **B18** such as represented by the following scheme, 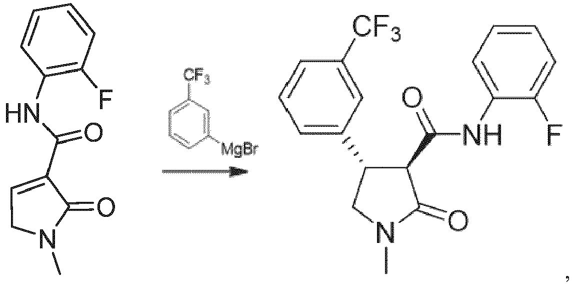 comprising substitution of the 1,4 system such as by a 1,4-addition using Grignard reagent, such as [3-(trifluoromethyl)phenyl] magnesium bromide);
   dd) a process **B18c** such as represented by the following scheme, 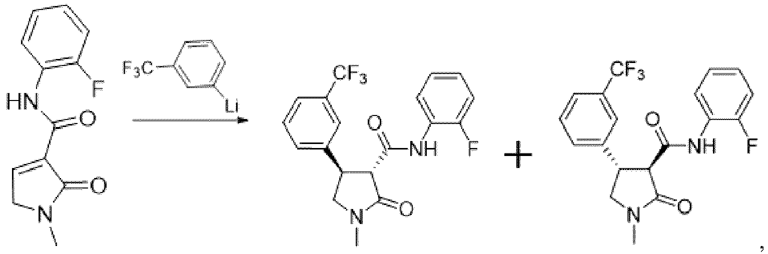 comprising substitution of the 1,4 system such as a 1,4-addition using organo-lithium reagent, such as 3-(trifluoromethyl)phenyl lithium;
   ee) a process **B18d** such as represented by the following scheme, 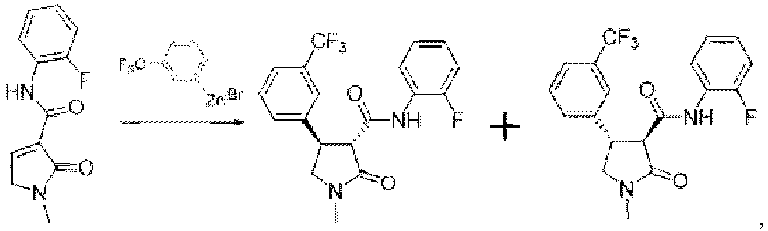 comprising substitution of the 1,4 system such as a 1,4-addition using organo-zinc reagent, such as 3-(trifluoromethyl phenyl zinc(II) bromide;
   ff) a process **B19** as represented by the following scheme, 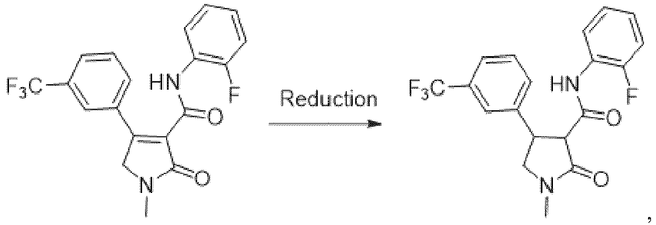 comprising substitution of the 1,4 system by reduction of the 1-Methyl-2H-pyrrol-5-one, double bond, such as by the use of a hydride source;
   gg) a process **B19a** according to process **B19** such as represented by the following scheme, 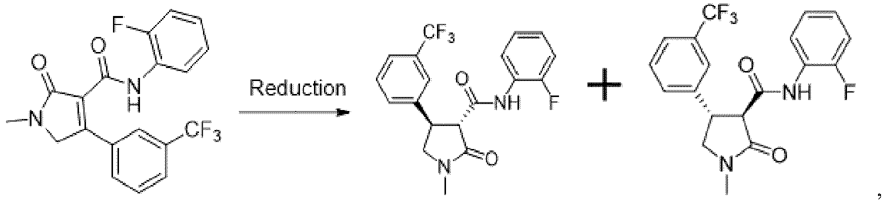 comprising, substitution of the 1,4 system by reduction of the double bond in the 1-Methyl-2H-pyrrol-5-one group, such as by the use of a hydride source;
   hh) a process **B19b** such as represented by the following scheme, 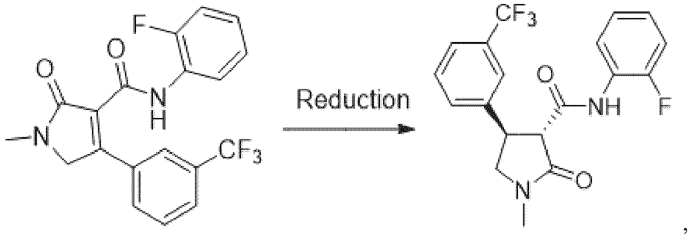 comprising, substitution of the 1,4 system by reduction of the double bond in the 1-Methyl-2H-pyrrol-5-one group, e.g., by the use of a hydride source;
   ii) a process **B19c** such as represented by the following scheme, 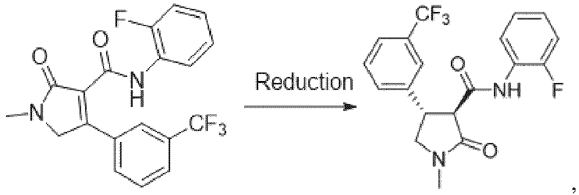 comprising, substitution of the 1,4 system by reduction of the double bond in the 1-Methyl-2H-pyrrol-5-one group, such as by the use of a hydride source;
   jj) a process **B20** as represented by the following scheme, 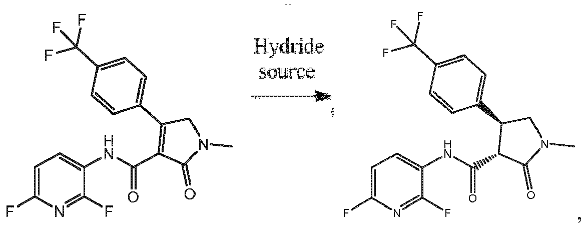 comprising, substitution of the 1,4 system by reduction of the double bond in the 1-Methyl-2H-pyrrol-5-one group, such as by the use of a hydride source;
   kk) a process **B21a** for obtaining a Saturated Target from an Unsaturated Precursor such as represented by the following scheme, 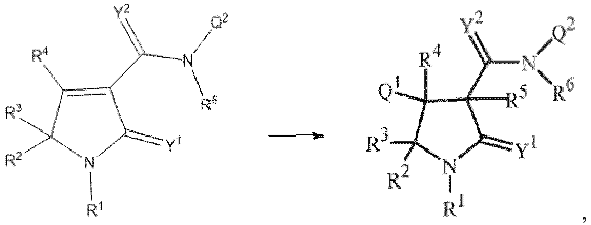 said process comprising substitution of the 1,4 system such as by 1,4-addition using Grignard reagent or hydride source, wherein the Saturated Target is a compound as defined in item 1 of application PCT/US2014/068073 published as WO 2015/084796, wherein Q¹, Q², R¹, R², R³, R⁴, R⁶, Y¹ and Y² etc. have the meanings as defined in item 1 of application PCT/US2014/068073 published as WO 2015/084796, wherein the substituents of the structures of this process are as defined for compound **A35a;**
   ll) a process **B21b** for obtaining a Saturated Target from an Unsaturated Precursor such as represented by the following scheme, 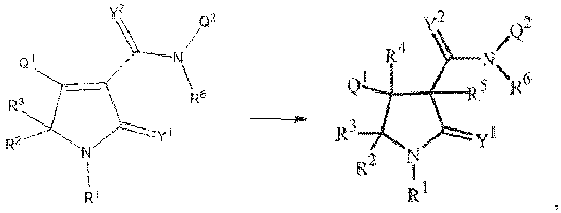 said process comprising substitution of the 1,4 system such as by 1,4-addition using Grignard reagent or hydride source, wherein the Saturated Target is a compound as defined in item 1 of application PCT/US2014/068073 published as WO 2015/084796, wherein Q¹, Q², R¹, R², R³, R⁴, R⁶, Y¹ and Y² etc., have the meanings as defined in item 1 of application PCT/US2014/068073 published as WO 2015/084796, wherein the substituents of the structures of this process are as defined for compound A35a;
   mm) a process **B21c** for obtaining a Saturated Target from Unsaturated Precursor, such as represented by the following scheme, 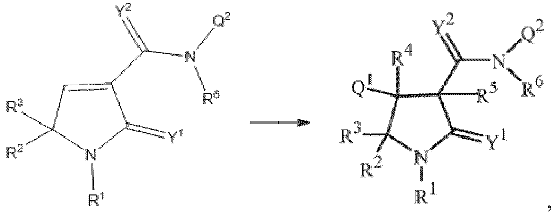 said process comprising substitution of the 1,4 system such as by 1,4-addition using Grignard reagent or hydride source, wherein the Saturated Target is a compound as defined in item 1 of application PCT/US2014/068073 published as WO 2015/084796, wherein Q¹, Q², R¹, R², R³, R⁴, R⁶, Y¹ and Y² etc. have the meanings as defined in item 1 of application PCT/US2014/068073 published as WO 2015/084796 wherein the substituents of the structures of this process are as defined for compound **A35a**
   nn) a process **B22** such as represented by the following scheme, 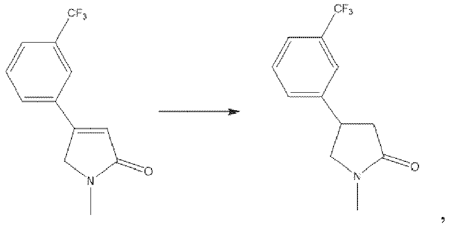 comprising, substitution of the Unsaturated Precursor 1,4 system;
   oo) a process **B23** such as represented by the following scheme, 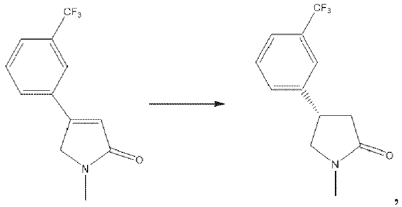 comprising, substitution of the Unsaturated Precursor 1,4 system;
   pp) a process **B24** such as represented by the following scheme, 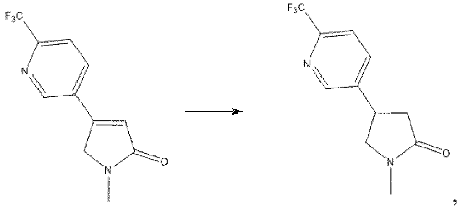 comprising, substitution of the Unsaturated Precursor 1,4 system;
   qq) a process **B25** such as represented by the following scheme, 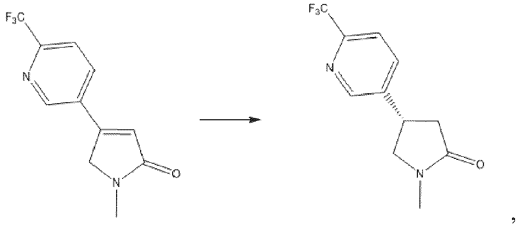 comprising, substitution of the Unsaturated Precursor 1,4 system;
   rr) a process **B26** such as represented by the following scheme, 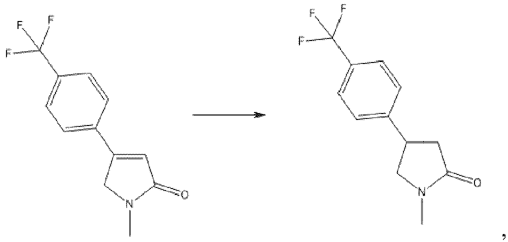 comprising, substitution of the Unsaturated Precursor 1,4 system;
   ss) a process **B27a** such as represented by the following scheme, 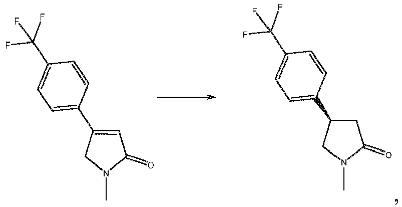 comprising, substitution of the Unsaturated Precursor 1,4 system;
   tt) a process **B27b** such as represented by the following scheme, 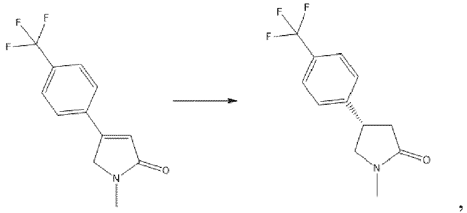 comprising, substitution of the Unsaturated Precursor 1,4 system;
   uu) a process **B28a** such as represented by the following scheme, 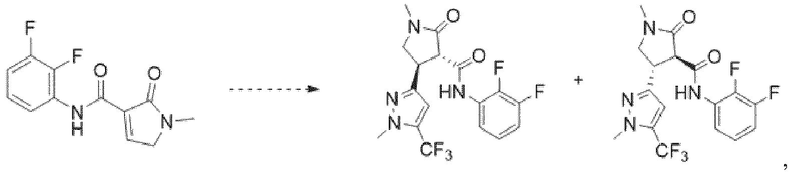 comprising substitution of the 1,4 system such as by a 1,4-addition
   vv) a process **B28b** such as represented by the following scheme, 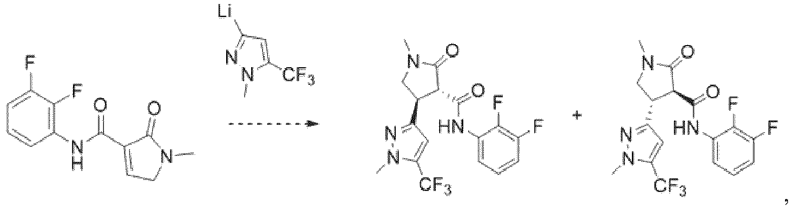 comprising substitution of the 1,4 system such as by a 1,4-addition using organo-lithium reagent, such as by 1-methyl-5-(trifluoromethyl)-1H-pyrazol-3-yl lithium;
   ww) a process **B29a** such as represented by the following scheme, 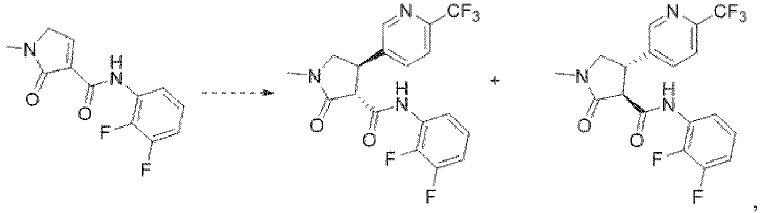 comprising substitution of the 1,4 system such as by a 1,4-addition;
   xx) A process **B29b** such as represented by the following scheme, 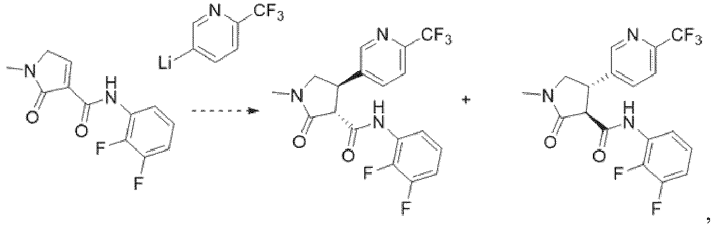 comprising substitution of the 1,4 system such as by a 1,4-addition using organo-lithium reagent, such as (6-(trifluoromethyl)pyridin-3-yl)lithium;
   yy) a process **B30a** such as represented by the following scheme, 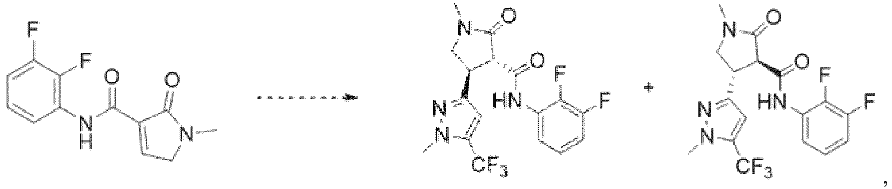 comprising substitution of the 1,4 system such as by a 1,4-addition;
   zz) a process **B30b** such as represented by the following scheme, 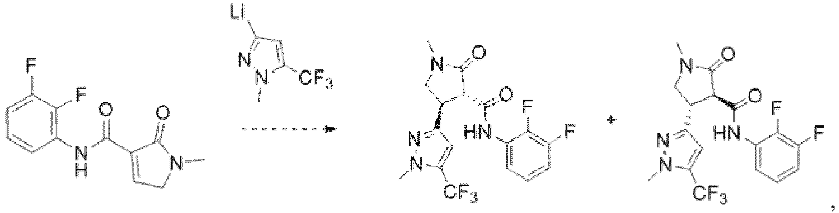 comprising substitution of the 1,4 system such as by a 1,4-addition using organo-lithium reagent e.g., 1-methyl-5-(trifluoromethyl)-1H-pyrazol-3-yl lithium
   aaa) A process **B31** according to any one of processes **B1-B30b** wherein an excess in one or more of the possible isomers is obtained;
   bbb) a process **B32** according to any one of processes **B1-B30b** wherein an excess of the anti-isomer/s is obtained;
   ccc) a process **B33** according to_any one of processes **B1-B30b** wherein an excess of the syn-isomer/s is obtained;
   ddd) a process **B34** according to_any one of processes **B1-B33** wherein an excess of one or more of the possible chiral isomers is obtained relative to the others;
   eee) A process **B35** according to_any one of processes **B1-B34** wherein an excess of chiral anti-isomer is obtained;
   fff) a process **B36** according to any one of processes **B1-B34** wherein an excess of chiral, anti-isomer with S,S configuration, is obtained;
   ggg) a process **B37** according to any one of processes **B1-B36** wherein magnesium, lithium, zinc, iPrMgCl, iPrMgBr, iPrMgl, hexyllithium, butyllithium, tert-butyllithium, 4-methyl-pentyl-lithium, isobutyl lithium or combinations thereof are used to prepare the reagent for the substitution of the 1,4 system, in-situ and/or before its use in the substitution reaction;
   hhh) a process **B38** according to any one of processes **B1-B36** wherein magnesium halogen exchange or lithium halogen exchange is used for the preparation of the reagent for the substitution of the 1,4 system in-situ and/or before its use;
   iii) a process **B39** according to any one of processes **B1-B38** conducted in the presence of metal;
   jjj) a process **B40** according to process **B39** wherein the metal is selected from Cu; Ni; Ti; Co; and Fe;
   kkk) a process **B41** according to process **B39** wherein the metal is selected from Cu; Ni; and Ti;
   lll) a process **B42** according to process **B39** wherein the metal is Cu;
   mmm) a process **B43** according to process **B39** wherein the metal is Ni;
   nnn) a process **B44** according to any one of processes **B39-B43** wherein the metal is present in catalytic amount;
   ooo) a process **B45** according to any one of processes **B1-B44** conducted in the presence of a chiral compound optionally connected to, or part of, a solid material or substrate;
   ppp) a process **B46** according to process **B45** wherein the chiral compound is selected from a group containing, naturally occurring, synthetic, modified, (e.g., substituted etc.), amino acids such as N,N-dimethyl-L-prolinium and L-proline; peptides exemplified by, (S)-N-((S)-1-butylamino-1-oxo-3-phenylpropan-2-yl-2- (E)-2-diphenylphosphaneyl benzylidene amino-3-methylbutanamide; phosphorus ligand such as, R-BINAP; proteins; enzymes; and sugars;
   qqq) a process **B47** according to any one of processes **B45-B46** wherein the chiral compound is selected from (S)-N- (S)-1-(butylamino-1-oxo-3-phenylpropan-2-yl-2-(E)-2-diphenylphosphaneyl benzylidene amino-3-methyl butanamide;
   rrr) a process **B48** according to any one of processes **B45-B47** wherein the chiral compound is present in catalytic amount;
   sss) a process **B49** according to any one of processes **B45-B48** wherein Lewis bases are present in the workup such as, NHa⁺Cl⁻ (ammonium chloride); NHa⁺OH⁻ (ammonium hydroxide); NH₃; CN⁻ ion (e.g. from KCN or NaCN); pyridine; DMA; DMSO; DMF; TEMADA; OH⁻ ion; PH₃; and H₂PO₃⁻ ion; etc.;
   ttt) a process **B50** according to process **B49** wherein the Lewis bases in the workup are selected from, NH4⁺Cl⁻ (ammonium chloride); NH₄⁺OH⁻ (ammonium hydroxide); NH₃; and CN⁻ ion for example, from KCN or NaCN and
   uuu) a process **B51** according to any one of processes **B1-B50** conducted in the presence of strong bases such as, MeMgCl; MeMgBr; iPrMgCl; iPrMgBr; BuLi; tert-BuLi; hexyllithium; NaH; and KH, etc.;
3. A process selected from any one of processes, B16; B16a; B16b; B16c; B16d; B17; B18; B 18a; B18b; B 18c; B18d; B19; B19a; B 19b; and process B 19c;
4. A process as represented by the following scheme, mediated by exposure of the starting compound to sunlight 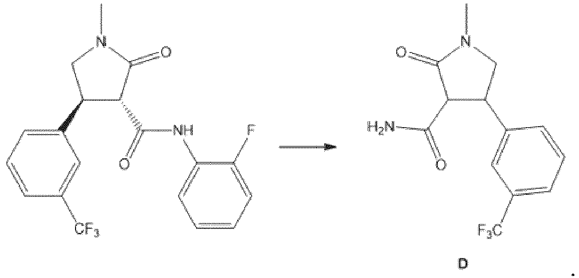
5. A process for producing a Storage Marker compound of formula D as represented by the following scheme, 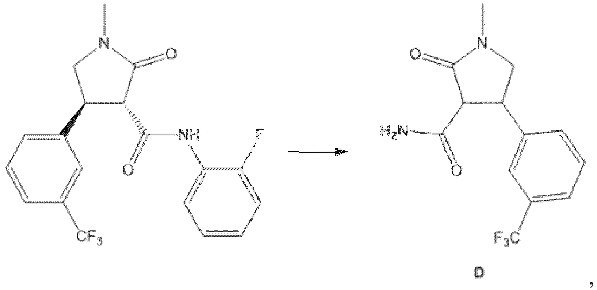 wherein the rate and degree of conversion to, and amount of, Storage Marker compound of formula D produced, and its percentage content in mixtures with compositions of the compound from which it was produced, is mediated by the characteristics of said composition and by the history of its storage at various defined environmental conditions, particularly, exposure to sunlight.
6. An Unsaturated Precursor compound **A1** of Formula 2 and e.g., salts etc. thereof, 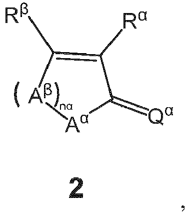 wherein,
   **R^{α}** is halogen; carbon-containing group exemplified by, substituted or unsubstituted, alkyl, aryl, heterocyclic, naphthyl; ester, including aliphatic and aromatic esters exemplified by C(O)OC₆H₅, -C(O)OC₆H₄F; amide, including aliphatic and aromatic amides exemplified by -C(O)NHC₆H₅; hydrazinamide, including aliphatic and aromatic hydrazinamide exemplified by, -C(O)NHNHC₆H₅, thioesters, thionoesters and similar compounds such as, C(O)NHOC₆F₅; -C(S)SC₆H₅ etc., with the proviso that **R^{α}** is not connected to the ring via O or N, such that, **R^{α}** cannot be -OMe,
   **R^{β}** is hydrogen or carbon-containing group such as, substituted or unsubstituted, alkyl, aryl, naphthyl, heterocycles etc. connected to the ring via a C atom, or a Si atom where relevant, with the proviso that
   **R^{β}** is not an amine connected via N to the ring
   **R^{β}** is not an ether connected via O to the ring
   **R^{β}** is not a thioether connected via S to the ring
   **A^{α}** is a C, N, O or S atom with or without substitution, such as by -N(Me)-; or -O-, wherein if present, a substituent of **A^{α}** may also be connected to another part of the molecule such as Q^{α}, A^{β} etc. such as in the following structure . 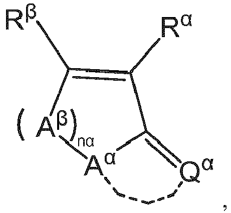
   each **A^{β}** is individually and independently a C, N, O or S atom, with or without substitution such as by -CH₂- or -O-,
   wherein, where present, a substituent of each **A^{β}** may be connected to another part of the molecule such as **A^{α}**, **A^{β}**, **R^{β}** etc., with the proviso, that at least one of **A^{β}** is a saturated atom exemplified by CH₂,
   **nα** is 1, 2, or 3,
   each **Q^{α}** is individually and independently O, S, or **NR^{ε}**,
   **R^{ε}** is a C, N, O or S atom with or without substitution such as by -N(Me)₂,
   the substituent on **R^{ε}** when present, may be hydrogen or a carbon- and/or nitrogen-containing group, such as, substituted or unsubstituted, alkyl, aryl, naphthyl, heterocycles etc. connected to the ring via a C atom, or a Si atom where relevant, and where present, the substituent of **R^{ε}** may also be connected to another part of the molecule such as **Q^{α}**, **A^{β}** etc. in a ring via a C atom, or Si atom if relevant, such as represented in the structure below, 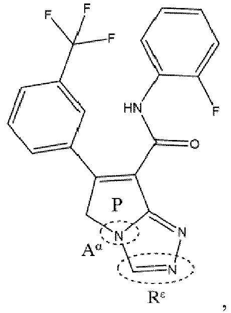 wherein preferably, the ring moiety of the Unsaturated Precursor, labelled **P** in the following structure is not aromatic such that not all atoms are capable of conjugation, 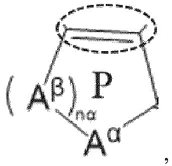 additionally, the double bond in that ring moiety, is not part of an aromatic additional ring external to it.
7. An Unsaturated Precursor compound **A2** of Formula 2 and e.g. salts thereof 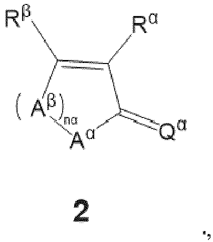 wherein, the Unsaturated Precursor does not include any covalent Nitrogen-Halogen bond, and wherein,
   **R^{α}** is halogen (Hal.), C₁-C₁₀ alkyl, C₁-C₁₀ haloalkyl, **R^{µ}**, or -C(**Q^{α}**)**Q^{β}R^{µ},**
   Optionally **R^{α}** may also be connected to another part of the molecule such as to **Q^{α}**, **A^{β}** etc. in a ring,
   **Q^{β}** is O, S, **NR^{ε}**, N**R^{π}**-N**R^{π}**, N**R^{π}**-O, N**R^{π}**-S, S-N**R^{π}**, or O-N**R^{π}**,
   **R^{β}** is H, **R^{ε}**, or Si(**R^{ε}**)₃
   **A^{α}** is a C, N, O or S atom with 0-2 substituents, wherein the substituents, when present, may be selected from H and/or **R^{θ}**, optionally, **A^{α}** is selected from -N(Me)-, -C(Et)H-, and -O-, and optionally, substituents of **A^{α}** where present are connected to another part of the molecule such as, **Q^{α}**, **A^{β}**, etc. as a ring, as in the following structure: 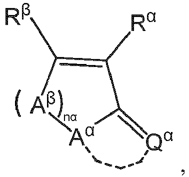 and wherein
   each **A^{β}** is individually and independently a C, N, O or S atom with 0-2 substituents, optionally, **A^{β}** is any of, -N(Me)-, -C(Et)H-, -O- etc. wherein substituents on each **A^{β}** are selected from H and/or **R^{θ}**, with the proviso that at least one of the **A^{β}** atoms on the ring is a saturated atom such as CH₂,
   wherein optionally, each **A^{β}** may be connected to another part of the molecule such as to, **Q^{α}**, **A^{α}** or to another **A^{β}**, etc. to form a ring,
   **nα** is 1-3,
   **Q^{α}** is O, S, **NR^{ε}**, and optionally **Q^{α}** may be connected to another part of the molecule such as an **A^{β}** etc. in a ring,
   each **R^{θ}** is individually and independently halogen (Hal.), hydroxy (OH), cyano (CN), nitro, amino, C₁-C₈ alkyl, C₁-C₈ cyanoalkyl, C₁-C₈ cyanoalkoxy, C₁-C₈ haloalkyl,
   C₁-C₈ hydroxyalkyl, C₁-C₈ nitroalkyl, C₂-C₈ alkenyl, C₂-C₈ haloalkenyl, C₂-C₈ nitroalkenyl, C₂-C₈ alkynyl, C₂-C₈ haloalkynyl, C₂-C₈ alkoxyalkyl, C₃-C₈ alkoxyalkoxyalkyl,
   C₂-C₈ haloalkoxyalkyl, C₂-C₈ haloalkoxyhaloalkoxy, C₃-C₆ cycloalkyl, cyclopropylmethyl, 1-methylcyclopropyl, 2-methylcyclopropyl, C₄-C₁₀ cycloalkylalkyl,
   C₄-C₁₀ halocycloalkylalkyl, C₅-C₁₂ alkylcycloalkylalkyl, C₅-C₁₂ cycloalkylalkenyl,
   C₅-C₁₂ cycloalkylalkynyl, C₃-C₈ cycloalkyl, C₃-C₈ halocycloalkyl, C₄-C₁₀ alkylcycloalkyl,
   C₆-C₁₂ cycloalkylcycloalkyl, C₃-C₈ cycloalkenyl, C₃-C₈ halocycloalkenyl,
   C₂-C₈ haloalkoxyalkoxy, C₂-C₈ alkoxyalkoxy, C₄-C₁₀ cycloalkoxyalkyl,
   C₃-C₁₀ alkoxyalkoxyalkyl, C₂-C₈ alkylthioalkyl, C₂-C₈ alkylsulfinylalkyl,
   C₂-C₈ alkylsulfonylalkyl, C₂-C₈ alkylamino, C₂-C₈ dialkylamino, C₂-C₈ halodialkylamino,
   C₂-C₈ alkylaminoalkyl, C₂-C₈ haloalkylaminoalkyl, C₄-C₁₀ cycloalkylaminoalkyl,
   C₃-C₁₀ dialkylaminoalkyl, -CHO, C₂-C₈ alkylcarbonyl, C₂-C₈ haloalkylcarbonyl, C₄-C₁₀ cycloalkylcarbonyl, -C(isO)OH, C₂-C₈ alkoxycarbonyl, C₂-C₈ haloalkoxycarbonyl,
   C₄-C₁₀ cycloalkoxycarbonyl, C₅-C₁₂ cycloalkylalkoxycarbonyl, -C(isO)NH₂,
   C₂-C₈ alkylaminocarbonyl, C₄-C₁₀ cycloalkylaminocarbonyl, C₃-C₁₀ dialkylaminocarbonyl,
   C₁-C₈ alkoxy, C₁-C₈ haloalkoxy, C₂-C₈ alkoxyalkoxy, C₂-C₈ alkenyloxy,
   C₂-C₈ haloalkenyloxy, C₃-C₈ alkynyloxy, C₃-C₈ haloalkynyloxy, C₃-C₈ cycloalkoxy,
   C₃-C₈ halocycloalkoxy, C₄-C₁₀ cycloalkylalkoxy, C₃-C₁₀ alkylcarbonylalkoxy,
   C₂-C₈ alkylcarbonyloxy, C₂-C₈ haloalkylcarbonyloxy, C₄-C₁₀ cycloalkylcarbonyloxy,
   C₁-C₈ alkylsulfonyloxy, C₁-C₈ haloalkylsulfonyloxy, C₁-C₈ alkylthio, C₁-C₈ haloalkylthio,
   C₃-C₈ cycloalkylthio, C₁-C₈ alkylsulfinyl, C₁-C₈ haloalkylsulfinyl, Ci-Cs alkylsulfonyl,
   C₁-C₈ haloalkylsulfonyl, C₃-C₈ cycloalkylsulfonyl, formylamino, C₂-C₈ alkylcarbonylamino,
   C₂-C₈ haloalkylcarbonylamino, C₃-C₈ cycloalkylamino, C₂-C₈ alkoxycarbonylamino,
   C₁-C₆ alkylsulfonylamino, C₁-C₆ haloalkylsulfonylamino, -SF₅, -SCN, SO₂NH₂,
   C₃-C₁₂ trialkylsilyl, C₄-C₁₂ trialkylsilylalkyl, C₄-C₁₂ trialkylsilylalkoxy, **R^{σ}**, **R^{η}S**(=O)=N-, **R^{η}S**(=O)₂N, **R^{η}**-C(=O)-, **R^{η}**(**R^{η}**N=)**_{q}S**(=O)**ₚ**-cyano, formyl, C₃-C₈ alkylcarbonylalkyl,
   -C(C₁-C₄ alkyl)=N-O(C₁-C₄ alkyl), -C(O)NH₂, C₂-C₆ cyanoalkyl, C₃-C₆ cycloalkyl, C₄-C₈ cycloalkenyl, arylcarbonyl, arylalkenylalkyl, arylcarbonylalkyl or -CPh=N-O(Ci-C₄ alkyl), each of which are optionally substituted on ring members with up to 5 substituents independently selected from **R^{η}**,
   wherein, where **R^{θ}** is connected to another part of the molecule, the connection is through a saturated, partially unsaturated or fully unsaturated, chain containing 2 to 4 atoms selected from,
      up to four C atoms,
      up to one O atom,
      up to one S atom and
      up to two N atoms,
   wherein up to 2 carbon members of the chain are independently selected from C(=O) and C(=S) and a sulfur atom member of the chain is selected from, S(=O)**ᵤ**( N**R^{ζ}**)**ᵥ**; said chain being optionally substituted with up to 5 substituents independently selected from **R^{η}** substituted on carbon and/or nitrogen atoms;
   **R'** is H or **R^{µ}**,
   wherein, **R'** may be connected to another part of the molecule such as **Q^{α}**, **A^{β}** ,
   **R^{µ}** is phenyl ring or a naphthalenyl ring system, each ring or ring system being unsubstituted or substituted with up to 5 substituents independently selected from, **R^{ζ}**; a 4-7 membered heterocyclic ring; and an 8-10 membered bicyclic ring system, each ring or ring system containing ring members selected from carbon atoms and 1 to 4 heteroatoms independently selected from,
      up to two O atoms,
      up to two S atoms, and
      up to five N atoms,
   wherein up to three **C** ring members are independently selected from C(=O) and C(=S), and the sulfur atom ring members are independently selected from S(=O)ᵤ(=N**R^{ζ}**)**ᵥ**, each ring or ring system substituted or unsubstituted with up to 5 substituents independently selected from **R^{ζ}** on carbon atom ring members and selected from **R^{η}** on nitrogen atom ring members,
   **R^{π}** is H or **R^{θ}**,
   **R^{ε}** is C, N, O or S atom with 0-2 substituents, each selected from **R^{π}**, such as, -N(Me)₂,
   wherein substituents are optionally additionally connected to another part of the molecule such as **Q^{α}**, **A^{β}** etc.in a ring,
   each **R^{ζ}** is individually and independently, Hal, OH, CN, nitro, amino, C₁-C₈ alkyl,
   C₁-C₈ cyanoalkyl, C₁-C₈ cyanoalkoxy, C₁-C₈ haloalkyl, C₁-C₈ hydroxyalkyl, C₁-C₈ nitroalkyl,
   C₂-C₈ alkenyl, C₂-C₈ haloalkenyl, C₂-C₈ nitroalkenyl, C₂-C₈ alkynyl, C₂-C₈ haloalkynyl,
   C₂-C₈ alkoxyalkyl, C₃-C₈ alkoxyalkoxyalkyl, C₂-C₈ haloalkoxyalkyl, C₂-C₈ haloalkoxyhaloalkoxy, C₃-C₆ cycloalkyl, cyclopropylmethyl, 1-methylcyclopropyl, 2-methylcyclopropyl,
   C₄-C₁₀ cycloalkylalkyl, C₄-C₁₀ halocycloalkylalkyl, C₅-C₁₂ alkylcycloalkylalkyl,
   C₅-C₁₂ cycloalkylalkenyl, C₅-C₁₂ cycloalkylalkynyl, C₃-C₈ cycloalkyl, C₃-C₈ halocycloalkyl,
   C₄-C₁₀ alkylcycloalkyl, C₆-C₁₂ cycloalkylcycloalkyl, C₃-C₈ cycloalkenyl, C₃-C₈ halocycloalkenyl,
   C₂-C₈ haloalkoxyalkoxy, C₂-C₈ alkoxyalkoxy, C₄-C₁₀ cycloalkoxyalkyl, C₃-C₁₀ alkoxyalkoxyalkyl, C₂-C₈ alkylthioalkyl, C₂-C₈ alkylsulfinylalkyl, C₂-C₈ alkylsulfonylalkyl, C₂-C₈ alkylamino,
   C₂-C₈ dialkylamino, C₂-C₈ halodialkylamino, C₂-C₈ alkylaminoalkyl, C₂-C₈ haloalkylaminoalkyl, C₄-C₁₀ cycloalkylaminoalkyl, C₃-C₁₀ dialkylaminoalkyl, -CHO, C₂-C₈ alkylcarbonyl,
   C₂-C₈ haloalkylcarbonyl, C₄-C₁₀ cycloalkylcarbonyl, -C(isO)OH, C₂-C₈ alkoxycarbonyl,
   C₂-C₈ haloalkoxycarbonyl, C₄-C₁₀ cycloalkoxycarbonyl, C₅-C₁₂ cycloalkylalkoxycarbonyl, - C(=O)NH₂, C₂-C₈ alkylaminocarbonyl, C₄-C₁₀ cycloalkylaminocarbonyl,
   C₃-C₁₀ dialkylaminocarbonyl, C₁-C₈ alkoxy, C₁-C₈ haloalkoxy, C₂-C₈ alkoxyalkoxy,
   C₂-C₈ alkenyloxy, C₂-C₈ haloalkenyloxy, C₃-C₈ alkynyloxy, C₃-C₈ haloalkynyloxy,
   C₃-C₈ cycloalkoxy, C₃-C₈ halocycloalkoxy, C₄-C₁₀ cycloalkylalkoxy,
   C₃-C₁₀ alkylcarbonylalkoxy, C₂-C₈ alkylcarbonyloxy, C₂-C₈ haloalkylcarbonyloxy,
   C₄-C₁₀ cycloalkylcarbonyloxy, C₁-C₈ alkylsulfonyloxy, C₁-C₈ haloalkylsulfonyloxy,
   C₁-C₈ alkylthio, C₁-C₈ haloalkylthio, C₃-C₈ cycloalkylthio, C₁-C₈ alkylsulfinyl,
   C₁-C₈ haloalkylsulfinyl, Ci-Cs alkylsulfonyl, C₁-C₈ haloalkylsulfonyl, C₃-C₈ cycloalkylsulfonyl, formylamino, C₂-C₈ alkylcarbonylamino, C₂-C₈ haloalkylcarbonylamino, C₃-C₈ cycloalkylamino, C₂-C₈ alkoxycarbonylamino, C₁-C₆ alkylsulfonylamino, C₁-C₆ haloalkylsulfonylamino, -SF₅, -SCN, SO₂NH₂, C₃-C₁₂ trialkylsilyl, C₄-C₁₂ trialkylsilylalkyl, C₄-C₁₂ trialkylsilylalkoxy, **R^{η}**, **R^{η}**S(=O)=N-, **R^{η}S**(=O)₂N**R^{η}**-C(=O)- or **R^{η}**(**R^{η}**N=)**_{q}**S(=O)**ₚ**-, wherein the free bond projecting to the right indicates the connecting point such as to **R^{µ}**; optionally **R^{ζ}** may be connected to another part of the molecule such as **Q^{α}**, **A^{β}** etc. to form a ring,
   each **R^{η}** is individually and independently, CN, C₁-C₃ alkyl, C₁-C₈ hydroxyalkyl, C₂-C₃ alkenyl,
   C₂-C₃ alkynyl, C₃-C₆ cycloalkyl, C₂-C₃ alkoxyalkyl, C₁-C₃ alkoxy, C₂-C₃ alkylcarbonyl,
   C₂-C₃ alkoxycarbonyl, C₂-C₃ alkylaminoalkyl or C₃-C₄ dialkylaminoalkyl, wherein
   optionally **R^{η}** may be connected to another part of the molecule such as **Q^{α}**, **A^{β}** etc. forming a ring,
   each **R^{σ}** is individually and independently, phenyl, phenylmethyl (benzyl), pyridinylmethyl, phenylcarbonyl (i.e. benzoyl), phenoxy, phenylethynyl, phenylsulfonyl or a 5- or 6-membered heterocyclic ring, each substituted or unsubstituted on ring members with up to 5 substituents independently selected from **R^{λ}**,
   **q** is 0-4,
   **p** is 0-4,
   **u** is 0-4, and
   **v** is 0-4.
8. An Unsaturated Precursor compound of Formula **2** 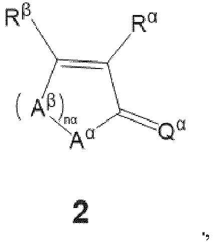 selected from the group as defined, depicted or described in any one of Unsaturated Precursor compounds, as indicated in item 6 (A1); as indicated in item 7(A2), or any of compounds A2; A2a; A2b; A2c; A2d; A3; A4; A5; A6; A7; A8; A9; A10; A11; A12; A13; A14; A15; A16; A16a; A17; A17a; A18; A19; A20; A21; A22; A23; A24; A25; A26; A27; A28; A29; A30; A31; A32; A33; A34; and A35a selected from the group of,
   a. an Unsaturated Precursor compound **A1** of Formula **2** and e.g. salts etc. thereof_as indicated in item 6;
   b. an Unsaturated Precursor compound **A2** of Formula 2 and e.g. salts etc. thereof_as indicated in item 7;
   c. an Unsaturated Precursor compound A2a which is the compound **A2** and e.g. salts thereof wherein,
      **nα** is 1, and wherein,
      **R^{α}**, **R^{β}**, **Q^{α}**, **Q^{β}**, **A^{β}**, **A^{α}**, and **R^{µ}**, have the same meanings as defined in compound **A2;**
   d. an Unsaturated Precursor compound A2b which is the compound **A2** and e.g. salts thereof wherein,
      **nα** is 2, and wherein,
      **R^{α}**, **R^{β}**, **Q^{α}**, **Q^{β}**, **A^{β}**, **A^{α}**, and **R^{µ}**, have the same meanings as defined in compound **A2;**
   e. an Unsaturated Precursor compound **A2c** which is the compound **A2** and e.g. salts thereof wherein,
      **R^{β}** is H and
      **R^{α}**, **Q^{α}, Q^{β}**, **A^{β}**, **A^{α}**, **R^{µ}** and **nα**, have the same meanings as defined in compound **A2;**
   f. an Unsaturated Precursor compound **A2d** which is the compound **A2** and e.g. salts thereof wherein,
      **R^{α}**, **R^{β}**, **Q^{α}**, **Q^{β}**, **A^{β}**, **A^{α}**, **R^{µ}** and **nα**, have the same meanings as defined in compound **A2,** with the proviso that **R^{β}** is not H;
   g. an Unsaturated Precursor compound **A3** which is any one of compounds **A2, A2a, A2b, A2c,** or **A2d** and e.g., salts thereof wherein,
      **R^{α}** is -C(**Q^{α}**)**Q^{β}R^{µ}**, or the following structure 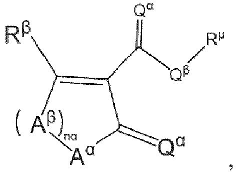 and wherein,
      **R^{β}**, **Q^{α}**, **Q^{β}**, **A^{β}**, **A^{α}**, **R^{µ}** and **nα**, have the same meanings as defined in compound **A2;**
   h. an Unsaturated Precursor compound **A4** which is any one of compounds **A2; A2a;**
      **A2b; A2c;** or **A2d** and e.g. salts thereof, wherein,
      **R^{α}** is -C(**Q^{α}**)**Q^{β}R^{µ}**, and
      **A^{α}** is -N(**R**^{π})-, such as in the following ring structure 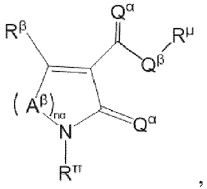 and wherein,
      **R^{β}**, **Q^{α}**, **Q^{β}**, **A^{β}**, **R^{α}**, **R^{µ}** and **nα**, have the same meanings as defined in compound **A2;**
   i. an Unsaturated Precursor compound **A5** which is any one of compounds **A2; A2c;** or **A2d** and e.g. salts thereof wherein,
      **R^{α}** is -C(**Q^{α}**)**Q^{β}R^{µ}**,
      **A^{α}** is -N(**R^{π}**)-, and
      **nα** is 1 such as in the following ring structure 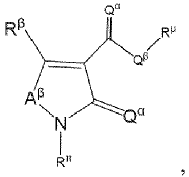 and wherein,
      **R^{β}**, **Q^{α}**, **Q^{β}**, **A^{β}**, **R^{π}** and **R^{µ}**, have the same meanings as defined in compound **A2;**
   j. an Unsaturated Precursor compound **A6** which is any one of compounds **A2; A2c;** and **A2d** and e.g. salts thereof wherein,
      **R^{α}** is -C(**Q^{α}**)**Q^{β}R^{µ}**
         **A^{α}** is -N(**R^{π}**)-
      **nα** is 1
      **A^{β}** is CH₂ , such as in the following ring structure, 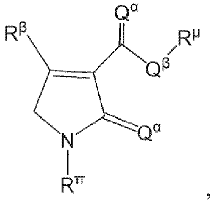 and wherein,
         **R^{β}**, **Q^{α}**, **Q^{β}**, **R^{π}** and **R^{µ}**, have the same meanings as defined in compound **A2;**
   k. an Unsaturated Precursor compound A7 which is any one of compounds **A2; A2c;** and **A2d** and e.g. salts thereof wherein,
      **R^{α}** is -C(**Q^{α}**)**Q^{β}R^{µ}**,
      **A^{α}** is -N(**R^{π}**)-,
         **nα** is 2,
      **A^{β}** is CH₂ such as in the following ring structure, 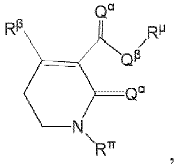 and wherein,
      **R^{β}**, **Q^{α}**, **Q^{β}**, **R^{π}** and **R^{µ}**, have the same meanings as defined in compound **A2;**
   **1.** an Unsaturated Precursor compound **A8** which is any one of compounds **A2; A2c;** and **A2d** and e.g. salts thereof wherein,
      **R^{α}** is -C(O)**Q^{β}R^{µ}**
      **A^{α}** is -N(**R^{π}**)-
      **nα** is 1
      **A^{β}** is CH₂
      **Q^{α}** is O such as in the following ring structure 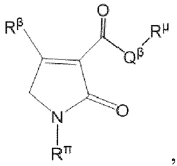 and wherein,
      **R^{β}**, **Q^{β}**, **R^{π}** and **R^{µ}**, have the same meanings as defined in compound **A2;**
   m. an Unsaturated Precursor compound **A9** which is any one of compounds **A2; A2c;** and **A2d** and e.g. salts thereof wherein,
      **R^{α}** is -C(O)NH**R^{µ}**
      **A^{α}** is -N(**R^{π}**)-
      **nα** is 1
      **A^{β}** is CH₂
         **Q^{α}** is O
      **Q^{β}** is NH such as in the following ring structure 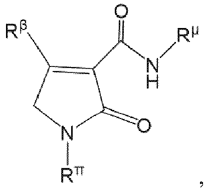 and wherein,
      **R^{β}**, **R^{π}** and **R^{µ}**, have the same meanings as defined in compound **A2;**
   **n.** an Unsaturated Precursor compound **A10** which is any one of compounds **A2; A2c;** and **A2d** and e.g. salts thereof wherein,
      **R^{α}** is -C(O)NH**R^{µ}**
      **A^{α}** is -N(**R^{π}**)-
      **nα** is 1
      **A^{β}** is CH₂
      **Q^{α}** is O,
      **Q^{β}** is NH and
      **R^{µ}** is phenyl ring, unsubstituted or substituted with up to 5 substituents independently selected from **R^{ζ}** such as in the following ring structure 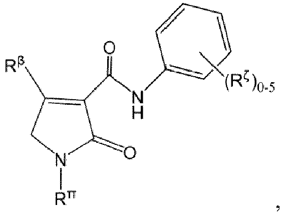 and wherein,
      **R^{β}**, **R^{π}** and **R^{ζ}**, have the same meanings as defined in compound **A2.**
   o. an Unsaturated Precursor compound **A11** which is the compound **A2,** and e.g. salts thereof wherein,
      **R^{α}** is -C(O)NH**R^{µ}**
      **A^{α}** is -N(**R^{π}**)-
      **nα** is 1
      **A^{β}** is CH₂
         **Q^{α}** is O
         **Q^{β}** is NH
      **R^{µ}** is phenyl ring, unsubstituted or substituted with up to 5 substituents independently selected from **R^{ζ}**
      **R^{β}** is **R^{λ}** is phenyl ring, unsubstituted or substituted with up to 5 substituents independently
      selected from **R^{ζ}** such as in the following ring structure 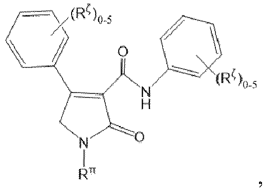 and wherein, **R^{π}** and **R^{ζ}**, have the same meanings as defined in compound **A2;**
   p. an Unsaturated Precursor compound **A12** which is the compound **A2,** and e.g. salts thereof wherein,
      **R^{α}** is -C(O)NH**R^{µ}**
         **A^{α}** is -N(Me)-
         **nα** is 1
         **A^{β}** is CH₂
            **Q^{α}** is O
            **Q^{β}** is NH
      **R^{µ}** is phenyl ring, unsubstituted or substituted with up to 5 substituents independently selected from **R^{ζ}**
         **R^{β}** is **R^{λ}** is phenyl ring, unsubstituted or substituted with up to 5 substituents independently
         selected from **R^{ζ}**, such as in the following ring structure 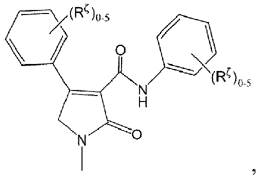 wherein, **R^{ζ}**, has the same meaning as defined in compound **A2;**
   q. an Unsaturated Precursor compound **A13** which is the compound **A2,** and e.g. salts thereof wherein,
      **R^{α}** is -C(O)NH**R^{µ}**
      **A^{α}** is -NH-
         **nα** is 1
      **A^{β}** is CH₂
         **Q^{α}** is O
         **Q^{β}** is NH
      **R^{µ}** is phenyl ring, unsubstituted or substituted with up to 5 substituents independently selected from **R^{ζ}**
      **R^{β}** is **R'** is phenyl ring, unsubstituted or substituted with up to 5 substituents independently
         selected from **R^{ζ}** such as in the following ring structure, 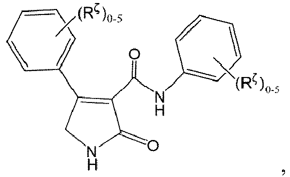 and wherein, **R^{ζ}**, has the same meaning as defined in compound **A2;**
   r. an Unsaturated Precursor compound **A14** which is the compound **A2,** and e.g. salts thereof wherein,
      **R^{α}** is -C(O)NH**R^{µ}**
      **A^{α}** is -NH-
      **nα** is 2
      **A^{β}** is CH₂
         **Q^{α}** is O
         **Q^{β}** is NH
      **R^{µ}** is phenyl ring, unsubstituted or substituted with up to 5 substituents independently selected from **R^{ζ}**
      **R^{β}** is **R'** which is a phenyl ring, unsubstituted or substituted with up to 5 substituents each independently selected from **R^{ζ}** such as in the following ring structure, 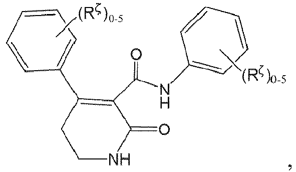 and wherein, **R^{ζ}**, has the same meaning as defined in compound **A2;**
   s. an Unsaturated Precursor compound **A15** which is the compound **A2,** and e.g. salts thereof wherein,
      **R^{α}** is -C(O)NH**R^{µ}**
      **A^{α}** is -N(Me)-
         **nα** is 2
         a. **A^{β}** is CH₂
            **Q^{α}** is O
            **Q^{β}** is NH
      **R^{µ}** is phenyl ring, unsubstituted or substituted with up to 5 substituents independently selected from **R^{ζ}**
      **R^{β}** is **R'** is phenyl ring, unsubstituted or substituted with up to 5 substituents each independently selected from **R^{ζ}** such as in the following ring structure, 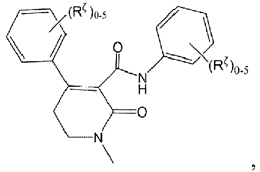 and wherein, **R^{ζ}**, has the same meaning as defined in compound **A2;**
   t. an Unsaturated Precursor compound **A16** which has the following structure, 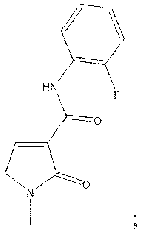
   u. an Unsaturated Precursor compound **A16a** having the following structure, 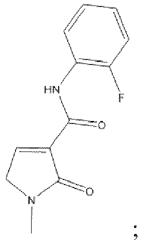
   v. an Unsaturated Precursor compound **A17** which has the following structure, 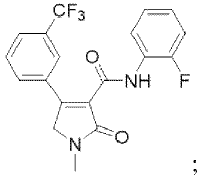
   w. a pesticide synthesis intermediate compound **A17a** having the following structure, 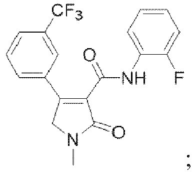
   x. an Unsaturated Precursor compound **A18** which has the following structure, 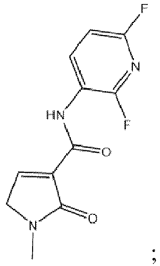
   y. an Unsaturated Precursor compound **A19** which has the following structure, 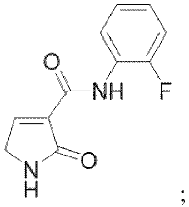
   z. an Unsaturated Precursor compound **A20** which has the following structure, 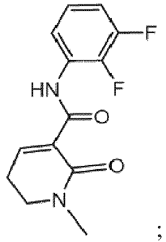
   aa. an Unsaturated Precursor compound **A21** which has the following structure, 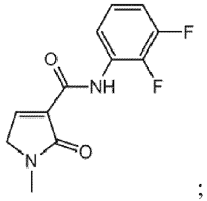
   bb. an Unsaturated Precursor compound **A22** which has the following structure, 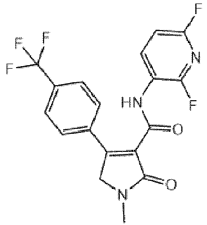
   cc. an Unsaturated Precursor compound **A23** which has the following structure, 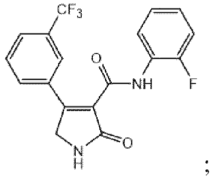
   dd. an Unsaturated Precursor compound **A24** which has the following structure, 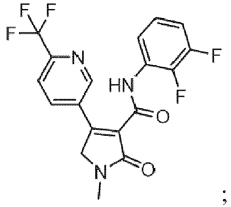
   ee. an Unsaturated Precursor compound **A25** which has the following structure, 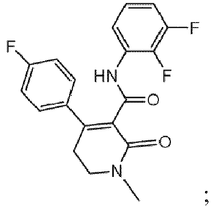
   ff. an Unsaturated Precursor compound **A26** which has the following structure, 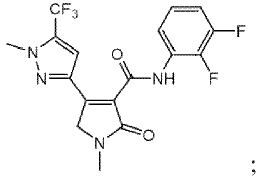
   gg. an Unsaturated Precursor compound **A27** which has the following structure, 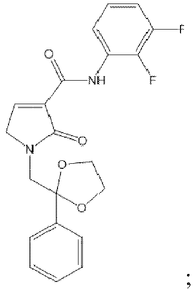
   hh. an Unsaturated Precursor compound **A28** which has the following structure, 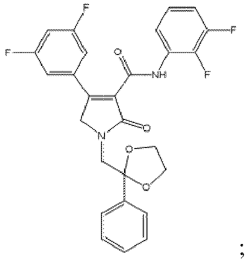
   ii. an Unsaturated Precursor compound **A29** which has the following structure, 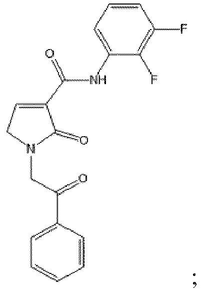
   jj. an Unsaturated Precursor compound **A30** which has the following structure, 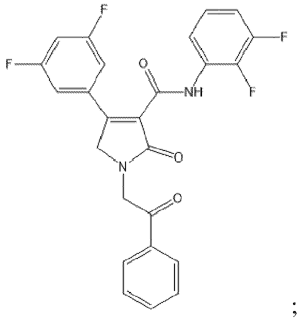
   kk. an Unsaturated Precursor compound **A31** which is the compound A2, and e.g. salts thereof wherein,
      **R^{α}** is -C(O)NH**R^{µ}**,
      **A^{α}** is -N(**R^{π}**)-,
         **nα** is 1,
      **A^{β}** is CH₂ ,
      **Q^{α}** is O,
      **Q^{β}** is NH,
      **R^{µ}** is a phenyl ring, unsubstituted or substituted with up to 5 substituents each independently selected from **R^{ζ}** , and
      **R^{β}** is H such as in the following ring structure,
         i. 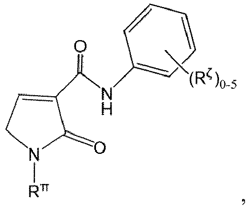 and wherein,
      **R^{π}** and **R^{ζ}**, have the same meanings as defined in compound **A2;**
   ll. an Unsaturated Precursor compound **A32** which is compound **A2** and e.g. salts thereof wherein,
      **R^{α}** is -C(O)NH**R^{µ}** ,
         **A^{α}** is -NH-,
      **nα** is 1,
         **A^{β}** is CH₂ ,
         **Q^{α}** is O,
         **Q^{β}** is NH,
         **R^{µ}** is phenyl ring, unsubstituted or substituted with up to 5 substituents each independently selected from **R^{ζ}**, and
         **R^{β}** is H such as in the following ring structure, 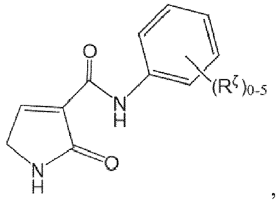 and wherein, **R^{ζ}**, has the same meaning as defined in compound **A2;**
   mm. an Unsaturated Precursor compound **A33** which is compound A2 and e.g. salts thereof wherein,
      **R^{α}** is -C(O)NH**R^{µ}**,
      **A^{α}** is -NH-,
         **nα** is 2,
      **A^{β}** is CH₂ ,
         **Q^{α}** is O,
      **Q^{β}** is NH,
      **R^{µ}** is a phenyl ring, unsubstituted or substituted with up to 5 substituents each independently selected from **R^{ζ}** and
      **R^{β}** is H such as in the following ring structure, 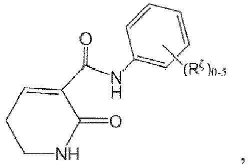 wherein, **R^{ζ}**, has the same meaning as defined in compound **A2;**
   nn. an Unsaturated Precursor compound **A34** which is compound **A2** and e.g. salts thereof wherein,
      **R^{α}** is -C(O)NH**R^{µ}**,
      **A^{α}** is -N(Me)-,
      **not** is 2,
      **A^{β}** is CH₂,
      **Q^{α}** is O,
      **Q^{β}** is NH,
      **R^{µ}** is phenyl ring, unsubstituted or substituted with up to 5 substituents each independently selected from **R^{ζ}**,
      **R^{β}** is H, such as in the following ring structure, 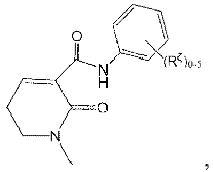 and wherein, **R^{ζ}**, has the same meaning as defined in compound **A2** and
   oo. an Unsaturated Precursor compound **A35a** which is selected from the following structures, 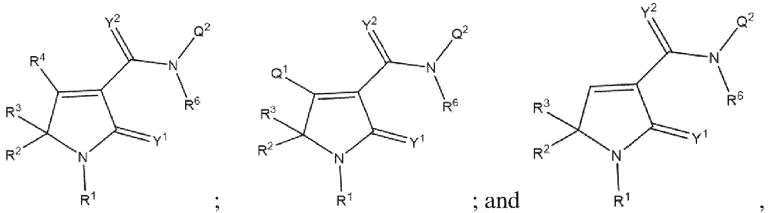 wherein **Q¹**, **Q²**, **R¹**, **R²**, **R³**, **R⁴**, **R⁶**, **Y1** and **Y2** etc. have the meanings as defined in item 1 of application PCT/US2014/068073 published as WO 2015/084796;
9. An Unsaturated Precursor for use as a pesticide, and preferably, as a herbicide synthetic process intermediate and/or marker compound as defined, depicted or described in any one of compounds selected from, A1; A2; A2a; A2b; A2c; A2d; A3; A4; A5; A6; A7; A8; A9; A10; A11; A12; A13; A14; A15; A16; A16a; A17; A17a; A18; A19; A20; A21; A22; A23; A24; A25; A26; A27; A28; A29; A30; A31; A32; A33; A34; and A35a of item 8.
10. A compound as defined, depicted or described in any one of compounds selected from A16; A16a; A17; A17a; A18; and A19 of item 8.
11. A compound as defined, depicted or described in compound A16 of item 8.
12. A compound as defined, depicted or described in compound A17 of item 8.
13. A combination as defined, depicted or described as any one of combinations **A41a; A42a; A43a; B1a; B15b; B15d; C2b; C35b; C36b; C37c; C38a; C41; C42a; C43a; E2; M1; M2; M3; M4,** selected from;
   a. combination **A41a** of a Saturated Target of the following formula, N-oxides, and salts thereof as defined in item 1 of PCT/US2018/035017 published as WO 2018/222647, 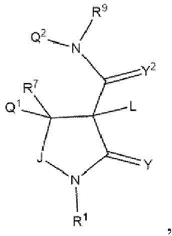 in combination with at least one Unsaturated Precursor, each defined by any one of the following structures, 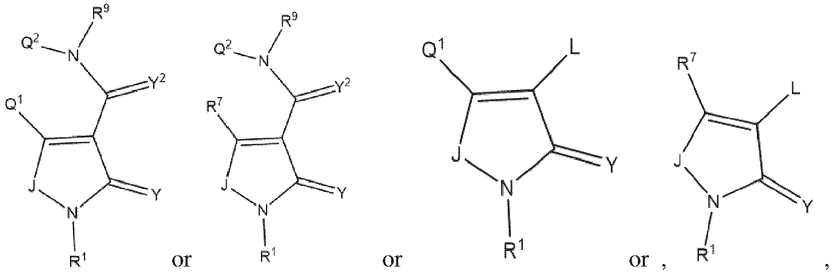 wherein **Q¹**, **Q²**, **R¹**, **R⁷**, **R⁹, J, L, Y** and **Y²** etc. have the meanings as defined in item 1 of application PCT/US2018/035017 published as in WO 2018/222647;
   b. combination **A42a** of a Saturated Target, N-oxides salts and stereoisomers thereof, wherein the Saturated Target is a compound as defined in item 1 of PCT/US2018/035015 published as WO2018/222646 published as WO2018222646, of the following formula, 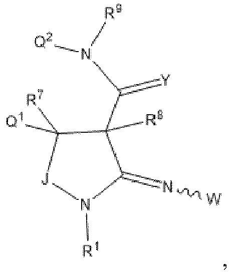 in combination with at least one Saturated Precursor each of which defined by any one of the following structures, 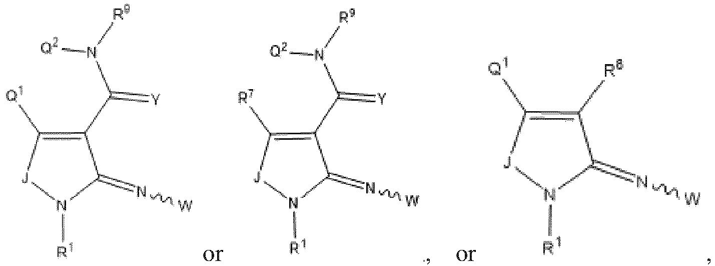 wherein **Q1, Q2, R1**, **R7, R8, R9, J,** Y and W, etc. have the meanings as defined in item 1 of application PCT/US2018/035015 published as WO 2018/222646;
   c. combination **A43a** of a Saturated Target of the following formula and salts thereof, wherein the Saturated Target is a compound as defined in item 1 of PCT/EP2018/057628 published as WO2018/184890 of the following formula, 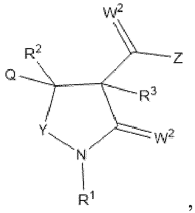 in combination with at least one Unsaturated Precursor, each defined by either one of the structures, 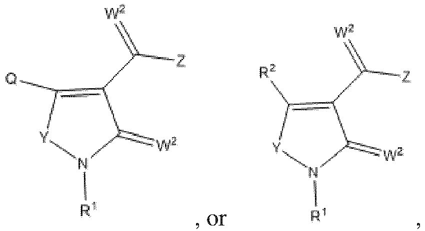 wherein **Q,W², R¹**, **R²**, **Y** and **Z** etc. have the meanings as defined in item 1 of application PCT/EP2018/057628 published as WO2018/184890;
   d. A combination **B1a** of a Saturated Target of formula 1 as defined in process **B1** 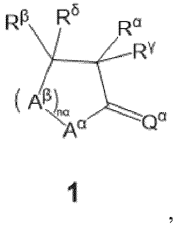 in combination with an amount of the intermediate Unsaturated Precursor compound selected from the compounds **A1** to A52 and from formula 2, of process **B1** 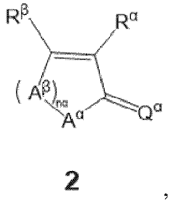 from which it was produced, as an impurity;
   e. a combination **B15b** of a Saturated Target as defined in process **B1** in combination with the intermediate Unsaturated Precursor compound via which it was produced using any one of the processes **B1** to **B14,** as an impurity;
   f. a combination **B15d** of a Saturated Target as defined in process **B1** in combination with the intermediate Unsaturated Precursor compound via which it was produced by the methods of any one of processes **B1** to **B14,** as an impurity;
   g. a combination **C2b** of a Saturated Target compound of **formula 1,** in combination with the intermediate compound of **formula 3,** via which it was produced, as an impurity 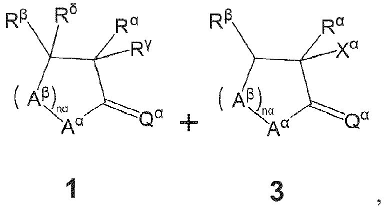 wherein the intermediate is a compound as defined in any one of compounds **C1a**; **C1b**; **C1c**; and **C2,** and wherein, **R^{γ}** has the same meaning as defined in any one of processes **B1; B1a**, **B1b**; **B2** and **B2a;**
   h. a combination **C35b** of a Saturated Target compound having the following structure, 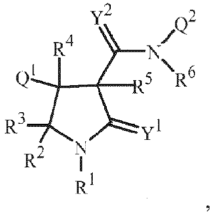 in combination with at least one compound defined by any one of the following formulae: 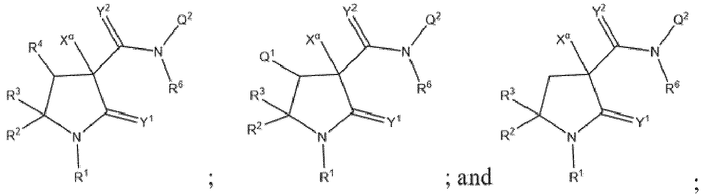
      wherein **Q¹, Q², R¹, R², R³, R⁴, R⁶, Y1** and Y2 etc. have the meanings as defined in item 1 of application PCT/US2014/068073 published as WO 2015/084796, and wherein **X^{α}** = SPh, SePh, SG^{α}, SeG^{α}
      **G^{α}** = alkyl, haloalkyl, phenyl, phenyl substituted with 0-5 groups each selected from halogen; alkyl; and haloalkyl groups;
   i. a combination **C36b** of a Saturated Target compound having the structure, 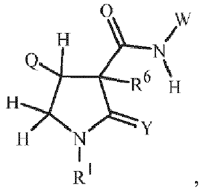 in combination with at least one compound defined by either one of the following formulae, 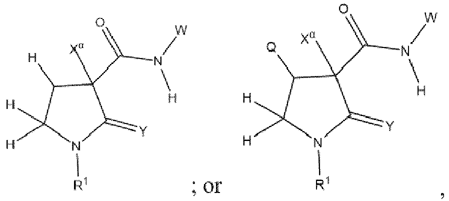 wherein Q, **R¹**, **R⁶**, **Y** and **W** etc. have the meanings as defined in item 1 of application PCT/US2020/034232, published as WO 2020/242946, and wherein,
      **X^{α}** is SPh, SePh, **SG^{α}**, **SeG^{α}**, and
      **G^{α}** is alkyl, haloalkyl, phenyl, phenyl substituted with 0-5 groups each selected from halogen; alkyl; and haloalkyl groups;
   j. a combination **C37c** of a Saturated Target compound having the following structure, 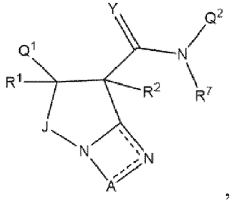 in combination with at least one compound defined by any one of the following structures, 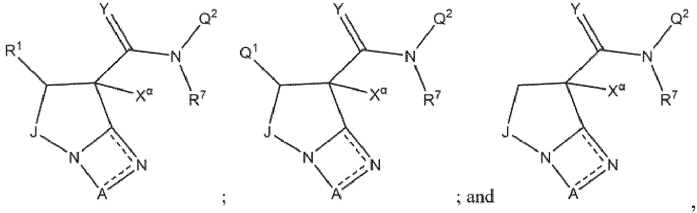 and wherein
      **Q¹, Q², R¹, R²**, **R⁷**, **Y,** A and **J** have the meanings as defined in item 1 of application PCT/US2016/030450, published as WO 2016/182780, and wherein,
      **X^{α}** is SPh, SePh, **SG^{α}**, **SeG^{α}**, and
      **G^{α}** is alkyl, haloalkyl, phenyl, phenyl substituted with 0-5 groups each selected from halogen; alkyl; and haloalkyl groups;
   k. a combination **C38a** of a Saturated Target compound of the following Formula 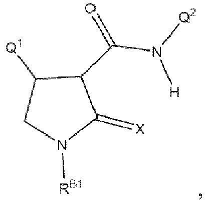 as defined in item 1 (B) of application PCT/EP2020/052780 published as WO 2020/161147, in combination with at least one compound defined by any one of the following structures, 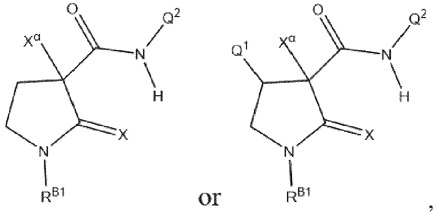 wherein **Q¹, Q²,R^{B1}**, and **X** etc. have the meanings as defined in item 1 of application PCT/EP2020/052780 published as WO 2020/161147, and wherein,
      **X^{α}** is SPh, SePh, **SG^{α}**, **SeG^{α}**,
      **G^{α}** is alkyl, haloalkyl, phenyl, phenyl substituted with 0-5 groups each selected from halogen; alkyl; and haloalkyl groups;
   1. A combination **C41** of a **Saturated Target** of the following formula, 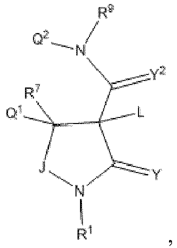 N-oxides, and salts thereof as defined in item 1 of application PCT/US2018/035017 published as WO 2018/222647, in combination with at least one compound each of which is defined by any one of the following structures, 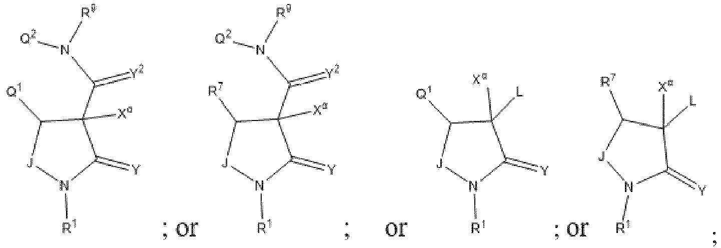 wherein,
      **Q¹, Q², R¹, R⁷**, **R⁹**, **J, L, Y** and **Y²** etc. have the meanings as defined in item 1 of application PCT/US2018/035017 published as WO 2018/222647, and wherein,
      **X^{α}** is SPh, SePh, **SG^{α}**, **SeG^{α}**, and
      **G^{α}** is alkyl, haloalkyl, phenyl, phenyl substituted with 0-5 groups each selected from halogen; alkyl; and haloalkyl groups;
   m. A combination **C42a** of a Saturated Target, N-oxides salts and stereoisomers thereof, wherein the Saturated Target is a compound as defined in item 1 of application PCT/US2018/035015 published as WO 2018/222646, having the following formula, 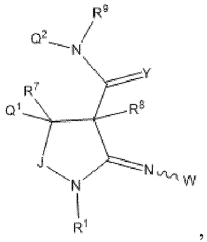 in combination with at least one compound each defined by any one of the following structures, 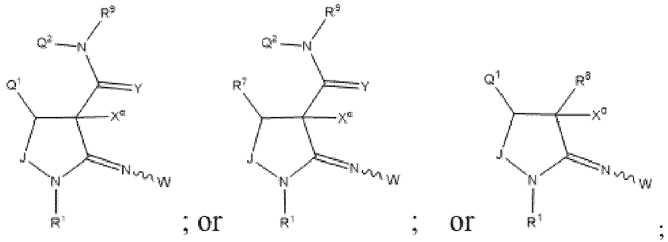 wherein,
      **Q¹, Q², R¹, R⁷**, **R⁸**, **R⁹**, **J, Y** and W, etc. have the meanings as defined in item 1 of application PCT/US2018/035015 published as WO 2018/222646, and wherein,
      **X"** is SPh, SePh, S**G^{α}**, **SeG^{α}**
      **G^{α}** is alkyl, haloalkyl, phenyl, phenyl substituted with 0-5 groups each selected from halogen; alkyl; and haloalkyl groups;
   n. A combination **C43a** of a Saturated Target and salts thereof wherein the Saturated Target is a compound as defined in item 1 of application PCT/EP2018/057628 published as WO2018/184890 having the following formula, 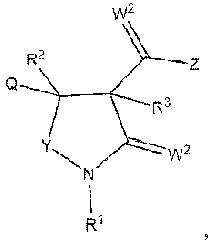 in combination with at least one compound defined by either one of the following structures, 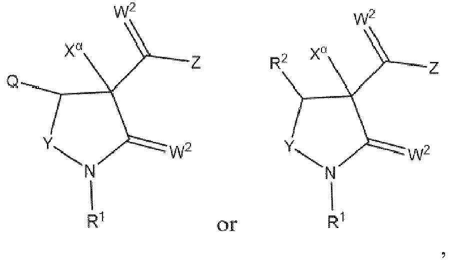 wherein,
      **Q,W², R¹, R², Y** and **Z** etc. have the meanings as defined in item 1 of application PCT/EP2018/057628 published as WO 2018/184890, and wherein
      X" is SPh, SePh, **SG^{α}**, **SeG^{α}**
      **G^{α}** is alkyl, haloalkyl, phenyl, phenyl substituted with 0-5 groups each selected from halogen; alkyl; and haloalkyl groups;
   o. A combination **E2** of a Saturated Target compound of formula **1,** 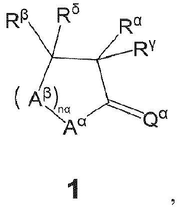 in combination with the intermediate compound of Formula **4** and e.g. salts etc. thereof, 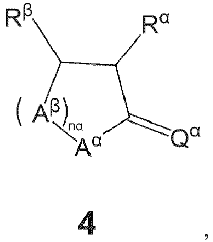 via which it was produced, as an impurity and wherein,
      **R^{α}**, **R^{β}**, **A^{α}**, **A^{β}**, **nα** and **Q^{α}** have the same meanings as defined in compound **A2,** and
      **R^{δ}** and **R^{γ}** have the same meanings as defined in process **B1;**
   p. a combination **M1** of a Saturated Target of formula **1** as defined in process **B1,** 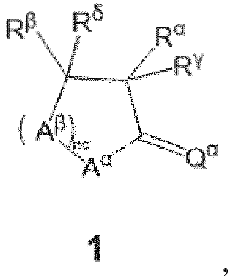 in combination with an amount of any one of the marker, impurity, degradant compounds having the following formulae: 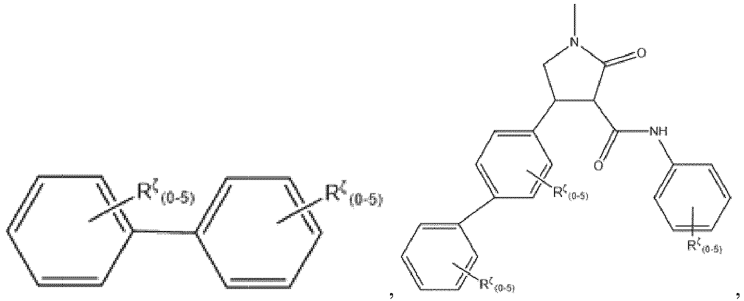 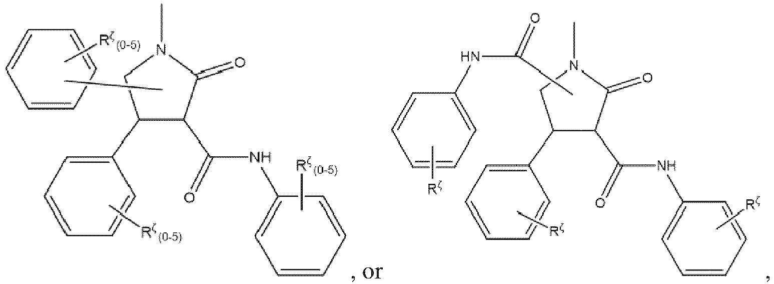 wherein, each **R^{ζ}** is as defined in any one of compounds **A1** to **A52** and process **B1;**
   q. a combination **M2** of_a Saturated Target of formula **1** as defined in process **B1** 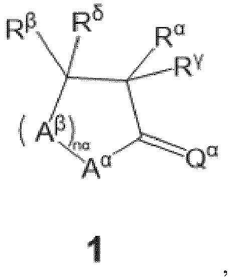 in combination with an amount of any one of the marker, impurity, degradant compounds having the following formulae: 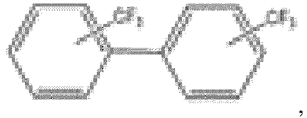 or 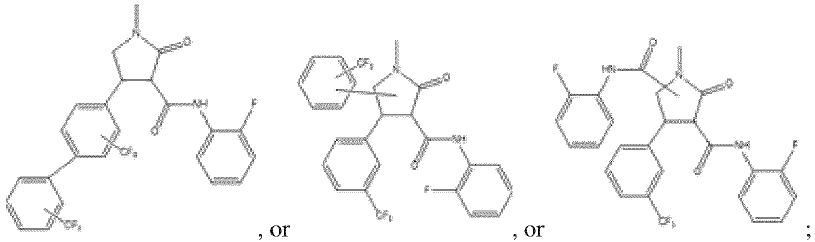
   r. a combination **M3** of a Saturated Target of formula **1** as defined in processes **B1** 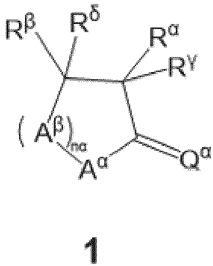 in combination with an amount of any one of the marker, impurity, degradant compounds having the following formulae: 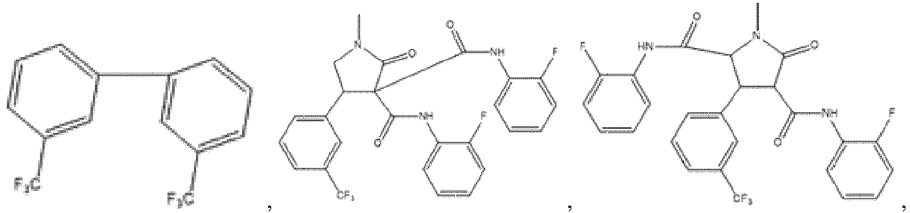 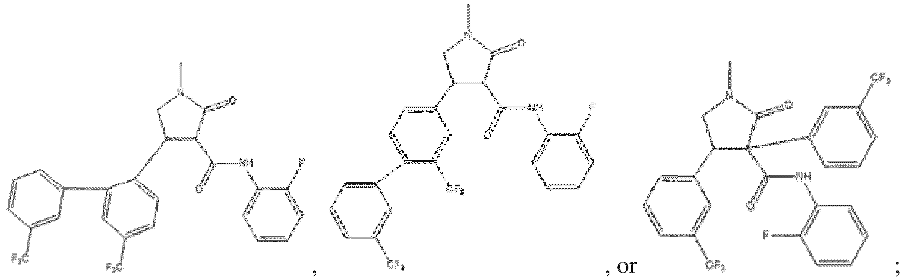
   s. a combination **M4** of a Saturated Target of formula **1** as defined in process **B1** 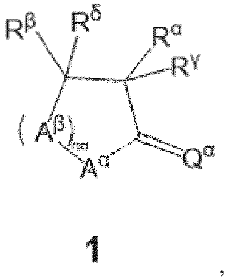 in combination with an amount of any one of the marker, impurity, degradant compounds having the following formulae: 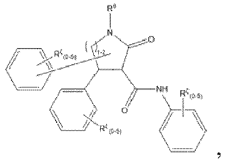 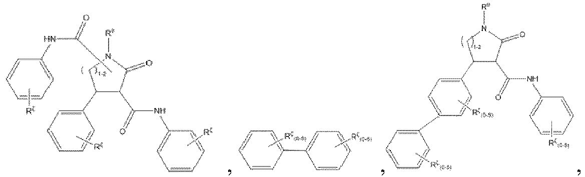 wherein each **R^{θ}** and each **R^{ζ}** is as defined in compound **A2.**
14. A compound as defined, depicted or described in any one of the compounds C1a; C1b; C1c; C2; C3a; C3b; C4a; C4b; C5a; C5b; C5c; C6a; C6b; C7a; C7b; C8a; C8b; C9a; C9b; C9c; C10; C11; C12; C12a; C12b; C13; C14; C15; C16; C16a; C16b; C16c; C16d; C16e; C16f; C16g; C16h; C17a; C17b; C17c; C17d; C17e; C17f; C17g; C17h; C17i; C18a; C18b; C19a; C19b; C20a; C20b; C21a; C21b; C22a; C22b; C23a; C23b; C24a; C24b; C25a; C25b; C26a; C26b; C27a; C27b; C28a; C28b; C29a; C29b; C30a; C30b; C31a; C31b; C32a; C32b; C33; C34; C35a; C36a; C37a; C37b; C38; C39; C40; C42; and C43 or selected from the group of
   (a) compound **C1a** of Formula **3** and e.g. salts etc. thereof 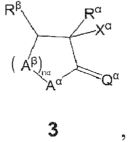 wherein,
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups, and
      **R^{α}, R^{β}, A^{α}, A^{β}, nα** and **Q^{α}** have the same meanings as defined in compound **A2;**
   (b) compound **C1b** of Formulae **3** and e.g. salts etc. thereof 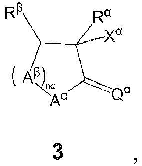 wherein,
      **X^{α}** is halogen, and
      **R^{α}, R^{β}, A^{α}, A^{β}, nα** and **Q^{α}** have the same meanings as defined in compound **A2;**
   (c ) compound **C1c** of Formula **3** and e.g. salts etc. thereof 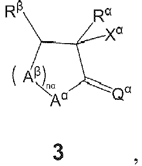 wherein,
      **X^{α}** is N(G^{α})₂, N⁺(O⁻)(G^{α})₂ [i.e. the N-Oxide of N(G^{α})₂];
      **G^{α}** is alkyl, haloalkyl, phenyl, phenyl substituted with 0-5 groups each selected from halogen; alkyl; and haloalkyl groups; and
      **R^{α}, R^{β}, A^{α}, A^{β}, nα** and **Q^{α}** have the same meanings as defined in compound **A2;**
   (d) compound **C2** of Formula **3** and e.g. salts etc. thereof 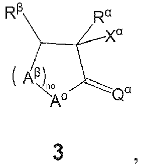 wherein, the compound of Formula **3** does not include any covalent Nitrogen-Halogen bond, and wherein,
      **X^{α}** is halogen, SPh, SePh, SG^{α}, SeG^{α};
      **G^{α}** is alkyl, haloalkyl, phenyl, phenyl substituted with 0-5 groups each selected from halogen; alkyl; and haloalkyl groups; and **R^{α}, R^{β}, A^{α}, A^{β}, nα** and **Q^{α}** have the same meanings as defined in compound **A2;**
   (e) compound **C3a** of compound **C2** and e.g. salts thereof as a process intermediate and/or marker wherein
      **R^{α}** is -C**(Q^{α})Q^{β}R^{µ},**
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl, 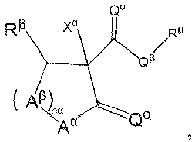 wherein,
      **R^{β}, Q^{α}, Q^{β}, A^{β}, A^{α}, R^{µ}** and **nα,** have the same meanings as defined in compound **A2;**
   (f) compound **C3b** of compound **C2** and e.g. salts thereof as a process intermediate and/or marker wherein,
      **R^{α}** is -C**(Q^{α})Q^{β}R^{µ},**
      **X^{α}** is halogen, N(G^{α})₂, N⁺(O⁻)(G^{α})₂; 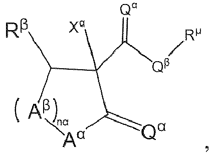 and wherein,
      **R^{β}, Q^{α}, Q^{β}, A^{β}, A^{α}, R^{µ}** and **nα,** have the same meanings as defined in compound **A2;**
   (g) compound **C4a** of compound **C2** and e.g. salts thereof as a process intermediate and/or marker wherein,
      **R^{α}** is -C**(Q^{α})Q^{β}R^{µ}**;
      **A^{α}** is -N(**R^{π}**)-;
      **X^{α}** is SPh, SePh, SG^{α}, SeG^{α};
      **G^{α}** is alkyl, haloalkyl, phenyl, phenyl substituted with 0-5 groups each selected from halogen; alkyl; and haloalkyl, 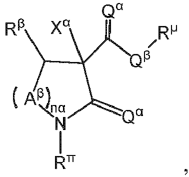 and wherein,
      **R^{β}, Q^{α}, Q^{β}, A^{β}, R^{π}, R^{µ}** and **nα,** have the same meanings as defined in compound **A2;**
   (h) compound **C4b** of compound **C2** and e.g. salts thereof, as a process intermediate and/or marker wherein,
      **R^{α}** is -C**(Q^{α})Q^{β}R^{µ},** and
      **A^{α}** is -N(**R^{π}**)-
      **X^{α}** is halogen, N(**G^{α}**)₂, N⁺(O⁻)(**G^{α}**)₂ 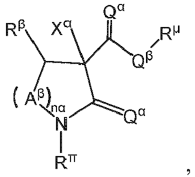 and wherein,
      **R^{β}, Q^{α}, Q^{β}, A^{β}, R^{π}, R^{µ}** and **nα,** have the same meanings as defined in compound **A2;**
   (i) compound **C5a** of compound **C2** and e.g. salts thereof wherein,
      **R^{α}** is **-C(Q^{α})Q^{β}R^{µ},**
      **A^{α}** is -N**(R^{π})-,** and
      **nα** is 1
      **X^{α}** is SPh, SePh, S**G^{α}**, Se**G^{α}**
      **G^{α}** is alkyl, haloalkyl, phenyl, phenyl substituted with 0-5 groups each selected from halogen; alkyl; and haloalkyl groups, 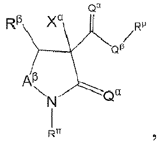 and wherein,
      **R^{β}, Q^{α}, Q^{β}, A^{β}, R^{π}** and **R^{µ},** have the same meanings as defined in compound **A2;**
   (j) compound **CSb** of compound **C2** and e.g. salts thereof, as a process intermediate and/or marker wherein,
      **R^{α}** is **-C(Q^{α})Q^{β}R^{µ};**
      **A^{α}** is **-N(R^{π})-;**
      **nα** is 1;
      **X^{α}** is halogen 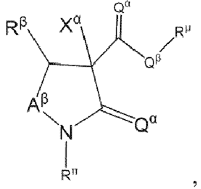 and wherein,
      **R^{β}, Q^{α}, Q^{β}, A^{β}, R^{π}** and **R^{µ},** have the same meanings as defined in compound **A2;**
   (k) compound **C5c** of compound **C2** and e.g. salts thereof wherein,
      **R^{α}** is **-C(Q^{α})Q^{β}R^{µ},**
      **A^{α}** is -N(**R^{π}**)-, and
      **nα** is **1**
      **X^{α}** is SPh, SePh,
      **G^{α}** is alkyl, haloalkyl, phenyl, phenyl substituted with 0-5 groups each selected from halogen; alkyl; and haloalkyl,groups, 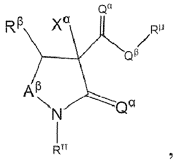 and wherein,
      **R^{β}, Q^{α}, Q^{β}, A^{β}, R^{π}** and **R^{µ},** have the same meanings as defined in compound **A2;**
   (l) compound **C6a** of compound **C2** and e.g. salts thereof, as a process intermediate and/or markerwherein,
      **R^{α}** is -C**(Q^{α})Q^{β}R^{µ}**
      **A^{α}** is -N(**R^{π}**)-
      **nα** is 1
      **A^{β}** is CH₂
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups, 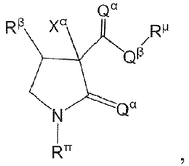 and wherein,
      **R^{β}, Q^{α}, Q^{β}, R^{π}** and **R^{µ},** have the same meanings as defined in compound **A2;**
   (m) compound **C6b** of compound **C2** and e.g. salts thereof, as a process intermediate and/or marker wherein,
      **R^{α}** is -C(Q^{α})Q^{β}R^{µ}
      **A^{α}** is -N(R^{π})-
      **nα** is 1
      **A^{β}** is CH₂
      **X^{α}** is halogen 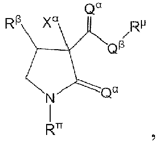 and wherein,
      **R^{β}, Q^{α}, Q^{β}, R^{π}** and **R^{µ},** have the same meanings as defined in compound **A2;**
   (n) compound **C7a** of compound **C2** and e.g. salts thereof, as a process intermediate and/or marker wherein,
      **R^{α}** is -C**(Q^{α})Q^{β}R^{µ},**
      **A^{α}** is -N**(R^{π})-,**
      **nα** is 2,
      **A^{β}** is CH₂
      **X^{α}** is SPh, SePh, SG^{α}, SeG^{α}
      G^{α} is alkyl, haloalkyl, phenyl, phenyl substituted with 0-5 groups each selected from halogen; alkyl; and haloalkyl groups, 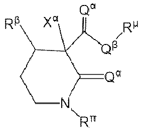
      And wherein,
      R^{β}, Q^{α}, Q^{β}, R^{π} and R^{µ}, have the same meanings as defined in compound A2;
   (o) compound **C7b** of compound **C2** and e.g. salts thereof, as a process intermediate and/or marker wherein,
      **R^{α}** is **-C(Q^{α})Q^{β}R^{µ},**
      **A^{α}** is **-N(R^{π})-,**
      **nα** is 2,
      **A^{β}** is CH₂
      **X^{α}** is halogen 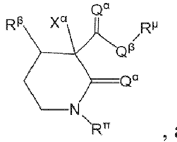 and wherein,
      **R^{β}, Q^{α}, Q^{β}, R^{π}** and **R^{µ},** have the same meanings as defined in compound **A2;**
   (p) compound **C8a** of compound **C2** and e.g. salts thereof wherein,
      **R^{α}** is **-C(O)Q^{β}R^{µ}**
      **A^{α}** is **-N(R^{π})-**
      **nα** is 1
      **A^{β}** is CH₂
      **Q^{α}** is O
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups, 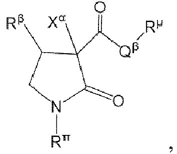 and wherein,
      **R^{β}, Q^{β}, R^{π}** and **R^{µ},** have the same meanings as defined in compound **A2;**
   (q) compound **C8b** of compound **C2** and e.g. salts thereof wherein,
      **R^{α}** is -C(O)Q^{β}R^{µ}
      **A^{α}** is -N(R^{π})-
      **nα** is 1
      **A^{β}** is CH₂
      **Q^{α}** is O
      **X^{α}** is N⁺(O⁻)(G^{α})₂ 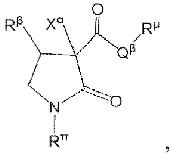 and wherein,
      **R^{β}, Q^{β}, R^{π}** and **R^{µ},** have the same meanings as defined in compound **A2;**
   (r) compound **C9a** of compound **C2** and e.g. salts thereof, as a process intermediate and/or marker wherein,
      **R^{α}** is -C(O)NHR^{w}
      **A"** is -N(R^{π})-
      **nα** is 1
      **A^{β}** is CH₂
      **Q^{α}** is O
      **Q^{β}** is NH
      **X"** is SPh, SePh, SG^{α}, SeG^{α}
      **G^{α}** is alkyl, haloalkyl, phenyl, phenyl substituted with 0-5 groups each selected from halogen; alkyl; and haloalkyl groups, 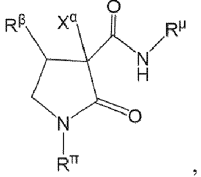 and wherein,
      **R^{β}, R^{π}** and **R^{µ},** have the same meanings as defined in compound **A2;**
   (s) compound **C9b** of compound **C2** and e.g. salts thereof wherein,
      **R^{α}** is -C(O)NHR^{µ}
      **A^{α}** is -N(R^{π})-
      **nα** is 1
      **A^{β}** is CH₂
      **Q^{α}** is O
      **Q^{β}** is NH
      **X^{α}** is SPh, SePh, 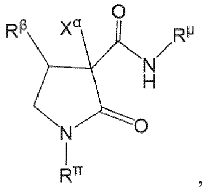 and wherein,
      **R^{β}, R^{π}** and **R^{µ},** have the same meanings as defined in compound **A2;**
   (t) compound **C9c** of compound **C2** and e.g. salts thereof, as a process intermediate and/or marker wherein,
      **R^{α}** is -C(O)NHR^{µ}
      **A^{α}** is -N(R^{π})-
      **nα** is 1
      **A^{β}** is CH₂
      **Q^{α}** is O
      **Q^{β}** is NH
      **X^{a}** is halogen 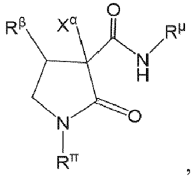 and wherein,
      **R^{β}, R^{π}** and **R^{µ},** have the same meanings as defined in compound **A2;**
   (u) compound **C10** of compound **C2** and e.g. salts thereof wherein,
      **R^{α}** is -C(O)NHR^{µ}
      **A^{α}** is -N(R^{π})-
      **nα** is 1
      **A^{β}** is CH₂
      **Q^{α}** is O,
      **Q^{β}** is NH and
      **R^{µ}** is phenyl ring, unsubstituted or substituted with up to 5 substituents each independently selected from **R^{ζ}**
      **X"** is SPh, SePh, SG^{α}, SeG^{α},
      **G^{α}** is alkyl, haloalkyl, phenyl, phenyl substituted with 0-5 groups each selected from halogen; alkyl; and haloalkyl groups, 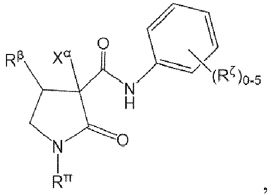 and wherein,
      **R^{β}, R^{π}** and **R^{ζ},** have the same meanings as defined in compound A2;
   (v) compound **C11**of compound **C2** and e.g. salts thereof wherein,
      **R^{α}** is -C(O)NH**R^{µ}**
      **A"** is -N(**R^{π}**)-
      **nα** is 1
      **A^{β}** is CH₂
      **Q^{α}** is O
      **Q^{β}** is NH
      **R^{µ}** is phenyl ring, unsubstituted or substituted with up to 5 substituents independently selected from **R^{ζ}**
      **R^{β}** is **R'** is phenyl ring, unsubstituted or substituted with up to 5 substituents each independently selected from **R^{ζ}**
      **X"** is SPh, SePh, 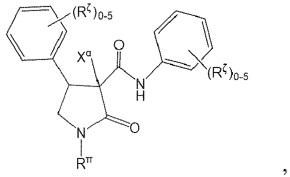 and wherein,
      **R^{π}** and **R^{ζ},** have the same meanings as defined in compound **A2;**
   (w) compound **C12** of compound **C2** and e.g. salts thereof wherein,
      **R^{α}** is -C(O)NHR^{µ}
      **A^{α}** is -N(Me)-
      **nα** is 1
      **A^{β}** is CH₂
      **Q^{α}** is O
      **Q^{β}** is NH
      **R^{µ}** is phenyl ring, unsubstituted or substituted with up to 5 substituents each independently selected from **R^{ζ}**
      **R^{β} = R^{λ}** is phenyl ring, unsubstituted or substituted with up to 5 substituents each independently
         selected from **R^{ζ},**
      **X"** is SPh, SePh, 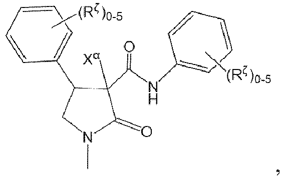 and wherein,
      **R^{ζ},** has the same meaning as defined in compound **A2;**
   (x) compound **C12a** of compound **C2** and e.g. salts thereof wherein,
      **R^{α}** is -C(O)NHR^{µ} ,
      **A^{α}** is -N(Me)-,
      **nα** is 1,
      **A^{β}** is CH₂ ,
      **Q^{α}** is O,
      **Q^{β}** is NH,
      **R^{µ}** is phenyl ring, unsubstituted or substituted with up to 5 substituents each independently selected from **R^{ζ},**
      **R^{β}** = R^{λ} is phenyl ring, unsubstituted or substituted with up to 5 substituents each independently
         selected from **R^{ζ},**
      **X"** is Halogen, 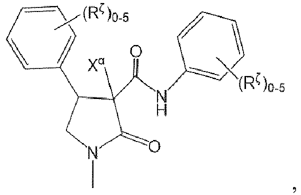 and wherein,
      **R^{ζ},** has the same meaning as defined in compound **A2;**
   (y) compound **C12b** of compound **C2** and e.g. salts thereof wherein,
      **R^{α}** is -C(O)NHR^{µ},
      **A^{α}** is -N(Me)-,
      **nα** is 1,
      **A^{β}** is CH₂ ,
      **Q^{α}** is O,
      **Q^{β}** is NH,
      **R^{µ}** is phenyl ring, unsubstituted or substituted with up to 5 substituents each independently selected from **R^{ζ},**
      **R^{β} = R^{λ}** is phenyl ring, unsubstituted or substituted with up to 5 substituents each independently
         selected from **R^{ζ},**
      **X"** is N⁺(O⁻)(G^{α})₂,
      **G^{α}** is alkyl, haloalkyl, phenyl, phenyl substituted with 0-5 groups each selected from halogen;
         alkyl; and haloalkyl groups, 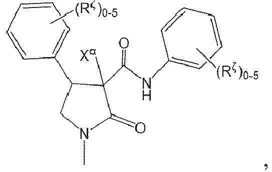 and wherein,
      **R^{ζ},** has the same meaning as defined in compound **A2;**
   (z ) compound **C13** of compound **C2** and e.g. salts thereof wherein,
      **R^{α}** is -C(O)NH**R^{µ}** ,
      **A^{α}** is -NH-,
      **nα** is 1,
      **A^{β}** is CH₂ ,
      **Q^{α}** is O,
      **Q^{β}** is NH,
      **R^{µ}** is phenyl ring, unsubstituted or substituted with up to 5 substituents each independently selected from **R^{ζ},**
      **R^{β}** =R^{λ} is a phenyl ring, unsubstituted or substituted with up to 5 substituents each independently selected from **R^{ζ},**
      **X^{α}** is SPh, SePh, SG^{α}, SeG^{α}, , N⁺(O⁻)(G^{α})₂
      **G^{α}** is alkyl, haloalkyl, phenyl, phenyl substituted with 0-5 groups each selected from halogen;
         alkyl; and haloalkyl groups, 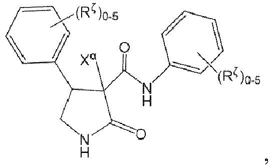 and wherein,
      **R^{ζ},** has the same meaning as defined in compound **A2;**
   (aa) compound C14 of compound **C2** and e.g. salts thereof wherein,
      **R^{α}** is -C(O)NHR^{µ} ,
      **A^{α}** is -NH-,
      **nα** is 2,
      **A^{β}** is CH₂ ,
      **Q^{α}** is O,
      **Q^{β}** is NH,
      **R^{µ}** is phenyl ring, unsubstituted or substituted with up to 5 substituents each independently selected from **R^{ζ},**
      **R^{β}** = R^{λ} is phenyl ring, unsubstituted or substituted with up to 5 substituents independently selected from R^{ζ}
      X^{α} is SPh, SePh, SG^{α}, SeG^{α}, N⁺(O⁻)(G^{α})₂
      **G^{α}** is alkyl, haloalkyl, phenyl, phenyl substituted with 0-5 groups each selected from halogen; alkyl; and haloalkyl groups, 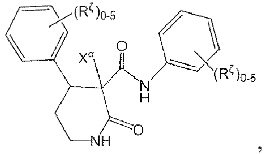 and wherein,
      **R^{ζ},** has the same meaning as defined in compound **A;**
   (bb) compound **C15** of compound **C2** and e.g. salts thereof wherein,
      **R^{α}** is -C(O)NHR^{µ},
      **A^{α}** is -N(Me)-,
      **nα** is 2,
      **A^{β}** is CH₂,
      **Q^{α}** is O,
      **Q^{β}** is NH,
      **R^{µ}** is phenyl ring, unsubstituted or substituted with up to 5 substituents each independently selected from **R^{ζ},**
      **R^{β}** = R^{λ} is phenyl ring, unsubstituted or substituted with up to 5 substituents each independently
         selected from **R^{ζ},**
      **X^{α}** is SPh, SePh, SG^{α}, SeG^{α}, N⁺(O⁻)(G^{α})₂,
      **G^{α}** is alkyl, haloalkyl, phenyl, phenyl substituted with 0-5 groups each selected from halogen; alkyl; and haloalkyl groups, 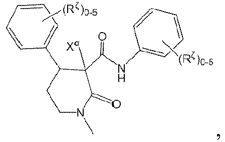
      wherein, **R^{ζ},** has the same meaning as defined in compound **A2;**
   (cc) compound **C16** of any one of compounds **C1-C15** and e.g. salts etc. thereof wherein,
      **X^{α}** is SPh, and wherein,
      **R^{α}, R^{β}, A^{α}, A^{β}, nα,** and **Q^{α}** have the same meanings as in compound **A2;**
   (dd) compound **C16a** of any one of compounds **C1-C15** and e.g. salts etc. thereof, wherein,
      **X^{α}** is N(Me)₂, and wherein,
      **R^{α}, R^{β}, A^{α}, A^{β}, nα,** and **Q^{α}** have the same meanings as in compound **A2;**
   (ee) compound **C16b** which has the following formula, 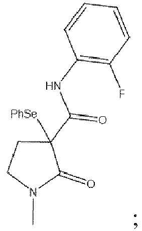
   (ff) compound **C16c** which has the following formula, 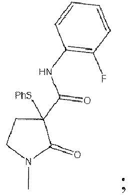
   (gg) compound **C16d** which has the following formula, 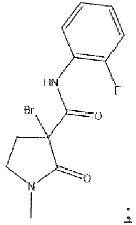
   (hh) compound **C16e** for use as a pesticide, preferably, a herbicide synthetic process intermediate and/or marker compound of the following structure, 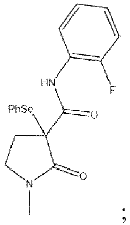
   (ii) compound **C16f** for use as a pesticide, preferably, a herbicide synthetic process intermediate and/or marker compound of the following structure, 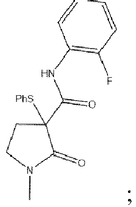
   (jj) compound **C16g** for use as a pesticide, preferably, a herbicide synthetic process intermediate and/or marker compound of the structure, 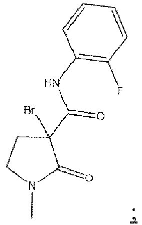
   (kk) compound **C16h** for use as a pesticide, preferably, a herbicide synthetic process intermediate and/or marker compound of the following structure, 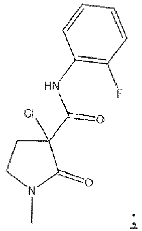
   (ll) compound **C17a** which has the following formula, 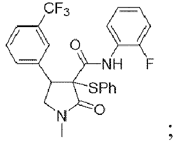
   (mm) compound **C17b** which has the following formula, 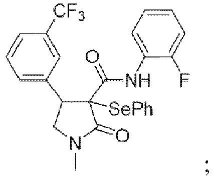
   (nn) compound **C17c** which has the following formula, 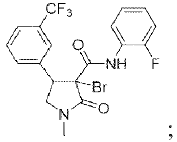
   (oo) compound **C17d** which has the following formula, 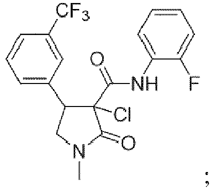
   (pp) compound **C17f** for use as a pesticide, preferably, a herbicide synthetic process intermediate and/or marker compound of the following structure, 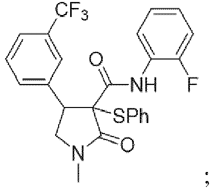
   (qq) compound **C17g** for use as a pesticide, preferably, a herbicide synthetic process intermediate and/or marker compound of the following structure, 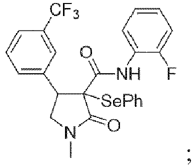
   (rr) compound **C17h** for use as a pesticide, preferably, a herbicide synthetic process intermediate and/or marker compound of the following structure, 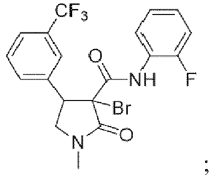
   (ss) compound **C17i** for use as a pesticide, preferably, a herbicide synthetic process intermediate and/or marker compound of the following structure, 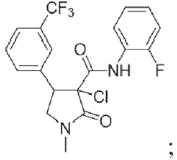
   (tt) compound **C18a** which has the following formula, 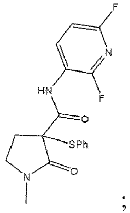
   (uu) compound **C18b** which has the following formula, 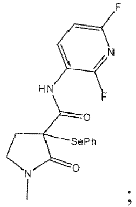
   (vv) compound **C19a** which has the following formula, 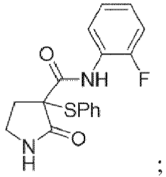
   (ww) compound **C19b** which has the following formula, 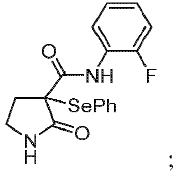
   (xx) compound **C20a** which has the following formula, 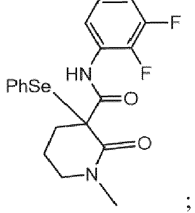
   (yy) compound **C20b** which has the following formula, 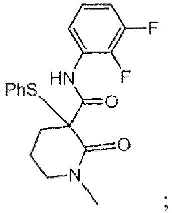
   (zz) compound **C21a** which has the following formula, 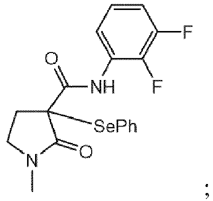
   (aaa) compound **C21b** which has the following formula, 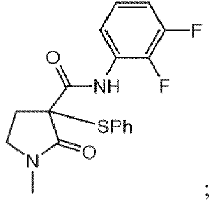
   (bbb) compound **C22a** which has the following formula, 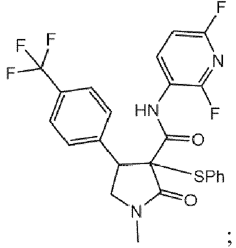
   (ccc) compound **C22b** which has the following formula, 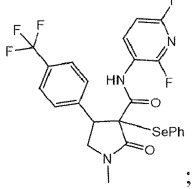
   (ddd) compound C23a which has the following formula, 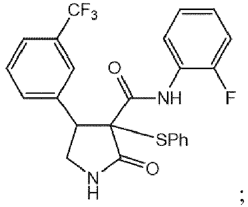
   (eee) compound **C23b** which has the following formula, 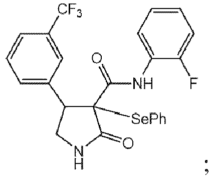
   (fff) compound **C24a** which has the following formula, 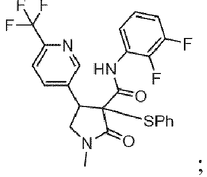
   (ggg) compound **C24b** which has the following formula, 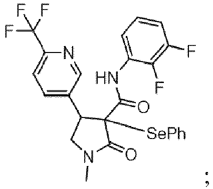
   (hhh) compound **C25a** which has the following formula, 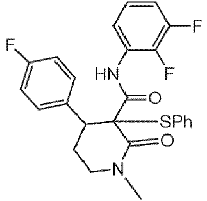
   (iii) compound **C25b** which has the following formula, 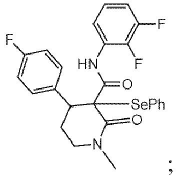
   (jjj) compound **C26a** which has the following formula, 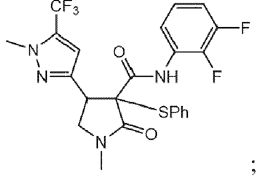
   (kkk) compound **C26b** which has the following formula, 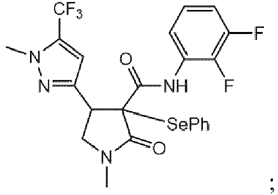
   (lll) compound **C27a** which has the following formula, 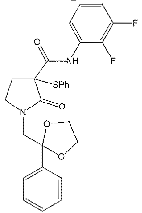
   (mmm) compound **C27b** which has the following formula, 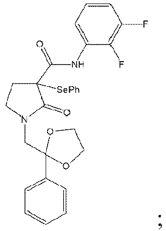
   (nnn) compound **C28a** which has the following formula, 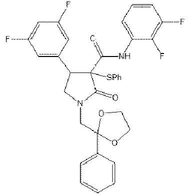
   (ooo) compound **C28b** which has the following formula, 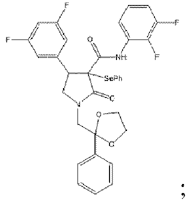
   (ppp) compound **C29a** which has the following formula, 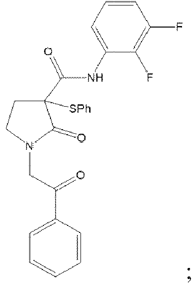
   (qqq) compound **C29b** which has the following formula, 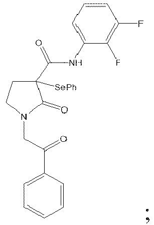
   (rrr) compound **C30a** which has the following formula, 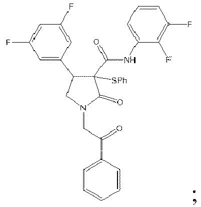
   (sss) compound **C30b** which has the following formula, 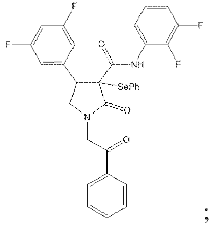
   (ttt) compound **C31a** of compound **C2** and e.g. salts thereof wherein,
      **R^{α}** is -C(O)NHR^{µ}
      **A^{α}** is -N(R^{π})-
      **nα** is 1
      **A^{β}** is CH₂
      **Q^{α}** is O
      **Q^{β}** is NH
      **R^{µ}** is phenyl ring, unsubstituted or substituted with up to 5 substituents each independently selected from **R^{ζ}**
      **R^{β}** is H
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups, 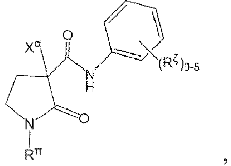 and wherein,
      **R^{π}** and **R^{ζ},** have the same meanings as defined in compound **A2;**
   (uuu) compound **C31b** of compound **C2** and e.g. salts thereof wherein,
      **R^{α}** is -C(O)NHR^{µ}
      **A^{α}** is -N(R^{π})-
      **nα** is 1
      **A^{β}** is CH₂
      **Q^{α}** is O
      **Q^{β}** is NH
      **R^{µ}** is phenyl ring, unsubstituted or substituted with up to 5 substituents each independently selected from **R^{ζ}**
      **R^{β}** is H
      **X^{α}** is Halogen 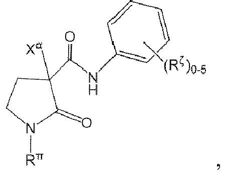 and
      wherein,
      **R^{π}** and **R^{ζ},** have the same meanings as defined in compound **A2;**
   (vvv) compound **C32a** of compound **C2** and e.g. salts thereof wherein,
      **R^{α}** is -C(O)NHR^{µ}
      **A^{α}** is -NH-
      **nα** is 1
      **A^{β}** is CH₂
      **Q^{α}** is O
      **Q^{β}** is NH
      **R^{µ}** is phenyl ring, unsubstituted or substituted with up to 5 substituents each independently selected from **R^{ζ}**
      **R^{β}** is H
      **X^{α}** is SPh, SePh, SG^{α}, SeG^{α}
      G^{α} is alkyl, haloalkyl, phenyl, phenyl substituted with 0-5 groups each selected from halogen; alkyl; and haloalkyl groups, 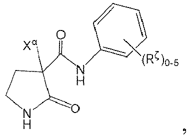 and
      wherein,
      **R^{ζ},** has the same meaning as defined in compound **A2;**
   (www) compound **C32b** of compound **C2** and e.g. salts thereof wherein,
      **R^{α}** is -C(O)NHR^{µ} ,
      **A^{α}** is -NH-,
      **nα** is 1,
      **A^{β}** is CH₂ ,
      **Q^{α}** is O,
      **Q^{β}** is NH,
      **R^{µ}** is phenyl ring, unsubstituted or substituted with up to 5 substituents each independently selected from **R^{ζ},**
      **R^{β}** is H,
      **X^{α}** is Halogen, 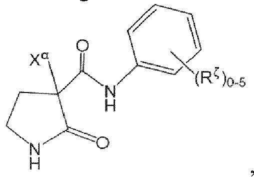 and wherein,
      **R^{ζ},** has the same meaning as defined in compound **A2;**
   (xxx) compound **C33** of compound **C2** and e.g. salts thereof wherein,
      **R^{α}** is -C(O)NHR^{µ} ,
      **A^{α}** is -NH-,
      **nα** is 2,
      **A^{β}** is CH₂ ,
      **Q^{α}** is O,
      **Q^{β}** is NH,
      **R^{µ}** is phenyl ring, unsubstituted or substituted with up to 5 substituents each independently selected from **Pζ**
      **R^{β}** is H
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups, 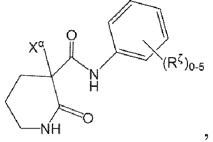 and
      wherein,
      **R^{ζ},** has the same meaning as defined in compound **A2;**
   (yyy) compound **C34** of compound **C2** and e.g. salts thereof wherein,
      **R^{α}** is -C(O)NHR^{µ}
      **A^{α}** is -N(Me)-
      **nα** is 2
      **A^{β}** is CH₂
      **Q^{α}** is O
      **Q^{β}** is NH
      **R^{µ}** is phenyl ring, unsubstituted or substituted with up to 5 substituents each independently selected from **R^{ζ}**
      **R^{β}** is H
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups, 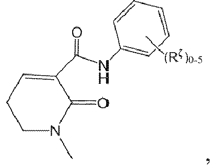 and wherein,
      **R^{ζ},** has the same meaning as defined in compound **A2;**
   (zzz) compound **C35a** defined by any one of the following structures, 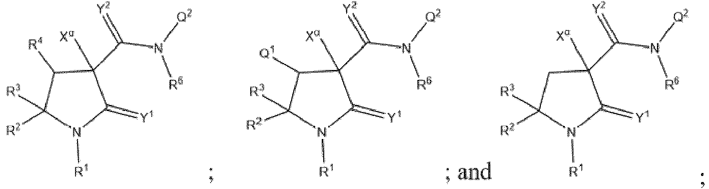
      wherein Q¹, Q², R¹, R², R³, R⁴, R6, Y1 and Y2 etc. have the meanings as defined in item 1 of PCT/US2014/068073 published as WO 2015/084796.
      Wherein
      X^{α} = SPh, SePh, SG^{α}, SeG^{α}
      G^{α} = alkyl, haloalkyl, phenyl, phenyl substituted with 0-5 groups each selected from halogen; alkyl; and haloalkyl groups;
   (aaaa) compound **C36a** defined by any one of the following structures, 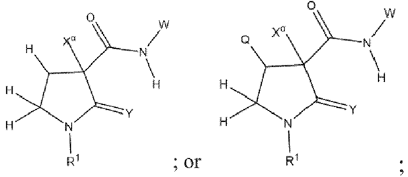
      wherein Q, R¹, Y and W have the meanings as defined in item 1 of PCT/US2020/034232, published as WO2020/242946 and wherein,
      **X^{α}** = SPh, SePh, SG^{α}, SeG^{α}, and
      **G^{α}** = alkyl, haloalkyl, phenyl, phenyl substituted with 0-5 groups each selected from halogen; alkyl; and haloalkyl groups;
   (bbbb) compound **C37a** defined by any one of the following formulae, 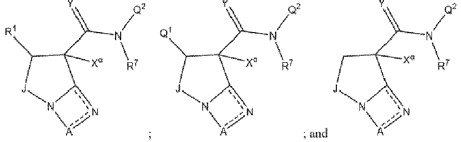
      Wherein Q¹, Q², R¹, R², R⁷, Y, A and J etc. have the meanings as defined in item 1 of PCT/US2016/030450, published as WO 2016/182780 and wherein,
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups;
   (cccc) compound **C37b** for use as a pesticide, preferably, a herbicide synthetic process intermediate and/or marker having any one of the following structures, 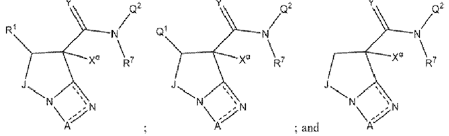
      Wherein Q¹, Q², R¹, R², R⁷, Y, A and J etc. have the meanings as defined in item 1 of PCT/US2016/030450, published as WO 2016/182780 and wherein
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups;
   (dddd) compound **C38** defined by either one of the following formulae, 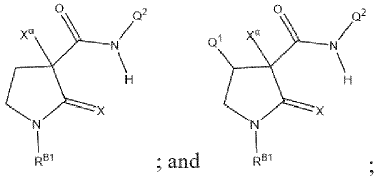
      wherein Q¹, Q², R^{B1}, and X have the meanings as defined in item 1 of PCT/EP2020/052780 published as WO 2020/161147
      and wherein,
      **X^{α}** is SPh, SePh, SG^{α}, SeG^{α}
      G^{α} is alkyl, haloalkyl, phenyl, phenyl substituted with 0-5 groups each selected from halogen; alkyl; and haloalkyl groups;
   (eeee) compound **C39** defined by either one of the following formulae, 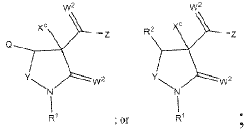
      wherein Q,W², R¹, Y and Z etc. have the meanings as defined in item 1 of PCT/EP2018/069001 published as WO2019/025156 and Wherein
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups;
   (ffff) compound **C40** defined by any one of the following formulae, 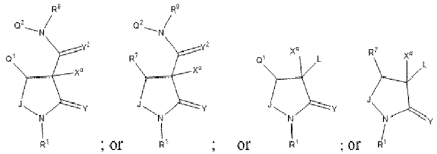
      wherein Q¹, Q², R¹, R⁷, R⁹, J, L, Y and Y² etc. have the meanings as defined in item 1 of PCT/US2018/035017 published as WO 2018/222647 and wherein,
      **X^{α}** is SPh, SePh, SG^{α}, SeG^{α};
      **G^{α}** is alkyl, haloalkyl, phenyl, phenyl substituted with 0-5 groups each selected from halogen; alkyl; and haloalkyl groups;
   (gggg) compound **C42** defined by any one of the following formulae, 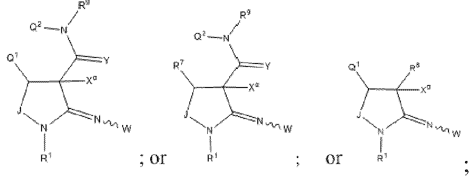
      wherein Q¹, Q², R¹, R⁷, R⁸, R⁹, J, Y and W, etc. have the meanings as defined in item 1 of PCT/US2018/035015 published as WO2018/222646 and Wherein
      X^{α} is SPh, SePh, SG^{α}, SeG^{α}
      G^{α} is alkyl, haloalkyl, phenyl, phenyl substituted with 0-5 groups each selected from halogen; alkyl; and haloalkyl groups and
   (hhhh) compound **C43** defined by either one of the formulae, 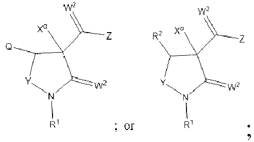 wherein,
      Q,W², R¹, R², Y and Z etc. have the meanings as defined in item 1 of PCT/EP2018/057628 published as WO2018/184890
      and Wherein
      **X^{α}** is SPh, SePh, SG^{α}, SeG^{α}
      **G^{α}** is alkyl, haloalkyl, phenyl, phenyl substituted with 0-5 groups each selected from halogen; alkyl; and haloalkyl groups
15. A compound as defined, depicted or described in compound C16c.
16. A compound as defined, depicted or described in compound C17a.
17. A process as defined, depicted or described in any one of process D1a; D1b; D2a; D2b; D3a; D3b; D3c; D4a; D4b; D5a; D5b; D6a; D6b; D7a; D7b; D8a; D8b; D9a; and D9b or selected from the group of
   (a) process **D1a** as described generally above for preparing an Unsaturated Precursor of formula **2** from a compound of formula 3 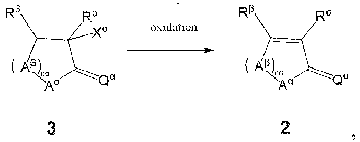 comprising oxidation of the corresponding compound of formula **3** using an Oxidant such as *meta-*Chloroperoxybenzoic acid (mCPBA) or Hydrogen peroxide (H₂O₂), wherein each of **X^{α}, R^{α}, R^{β}, A^{α}, A^{β}, nα,** and **Q^{α},** have the same meanings as in compound **C1a;**
   (b) process **D1b** as described generally above for preparing a Unsaturated Precursor of formula 2 from a compound of formula 3 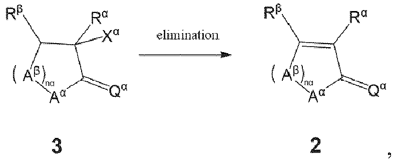 comprising an elimination reaction utilizing an activator such as a Base or a Lewis acid, wherein each of **X^{α}, R^{α}, R^{β}, A^{α}, A^{β}, nα,** and **Q^{α},** have the same meanings as in compound **C1a;**
   (c) process **D2a** of process **D1a** comprising, dissolving a compound of formula 3in suitable solvent e.g. Acetic acid, agitating (e.g. stirring etc.) for a period of time exemplified by about 20mins, adding portion wise, an Oxidant exemplified by *meta*-Chloroperoxy benzoic acid (mCPBA), or Hydrogen peroxide (H₂O₂), at suitable temperature. Then, agitating the reaction mass at substantially the same temperature for some time e.g. about 2 hrs. The reaction mass can be quenched e.g. into aq.NaHCOs solution, and extracted with suitable solvent typically two times. The combined organic layer can be concentrated to obtain the corresponding Unsaturated Precursor of formula **2;**
   (d) process **D2b** of process **D1a** comprising, dissolving a compound of formula **3**in suitable solvent e.g. chlorobenzene, agitating (e.g. by stirring etc.) for a time exemplified by about 20mins, adding portion wise, an Activator exemplified by Base or Lewis acid at suitable temperature. Then, agitating the reaction mass at substantially the same temperature for some time e.g. about 2 hrs. The reaction mass can be quenched e.g. into NH₄Cl solution, and extracted with suitable solvent typically two times. The combined organic layer can be concentrated to obtain the corresponding Unsaturated Precursor of formula **2;**
   (e) process **D3a** exemplified by the following scheme, 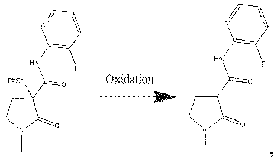 comprising, oxidation of the compound of the following formula, 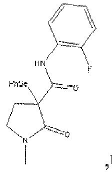 using oxidant, exemplified by *meta*-Chloroperoxy benzoic acid (mCPBA), or Hydrogen peroxide (H₂O₂). to obtain the corresponding Unsaturated Precursor compound;
   (f) process **D3b** exemplified by the following scheme, 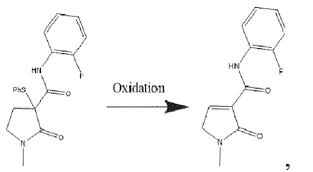 comprising, oxidation of the compound of the following formula, 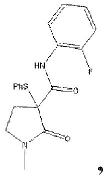 using oxidant, exemplified by *meta*-Chloroperoxy benzoic acid (mCPBA), or Hydrogen peroxide (H₂O₂). to obtain the corresponding Unsaturated Precursor;
   (g) process **D3c** exemplified by the following scheme, 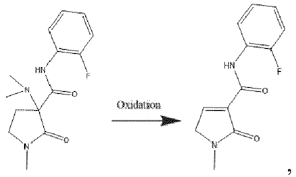 comprising, oxidation of the compound of the following formula, 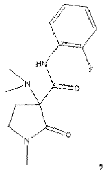 using oxidant exemplified by *meta*-Chloroperoxy benzoic acid (mCPBA), or Hydrogen peroxide (H₂O₂). to obtain the corresponding Unsaturated Precursor compound;
   (h) process **D4a** exemplified by the following scheme, 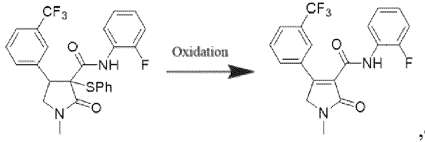 comprising, oxidation of the compound of the following formula, 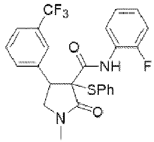 using oxidant exemplified by *meta*-Chloroperoxy benzoic acid (mCPBA), or Hydrogen peroxide (H₂O₂). to obtain the corresponding Unsaturated Precursor compound;
   (i) process **D4b** exemplified by the following scheme, 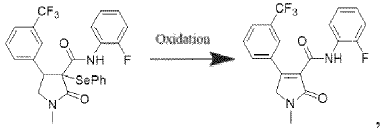 comprising, oxidation of the compound of the following formula, 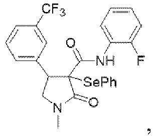 using oxidant exemplified by *meta*-Chloroperoxy benzoic acid (mCPBA), or Hydrogen peroxide (H₂O₂). to obtain the corresponding Unsaturated Precursor compound;
   (j) process **D5a** exemplified by the following scheme, 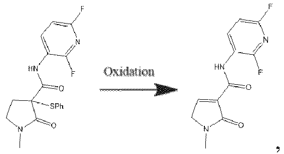 comprising, oxidation of the compound of the following formula, 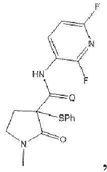 using oxidant exemplified by *meta*-Chloroperoxy benzoic acid (mCPBA), or Hydrogen peroxide (H₂O₂). to obtain the corresponding Unsaturated Precursor compound;
   (k) process **D5b** exemplified by the following scheme, 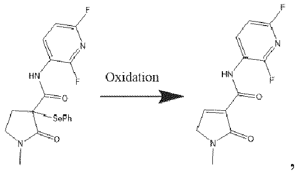 comprising, oxidation of the compound of the following formula, 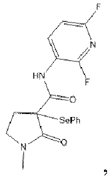 using oxidant exemplified by *meta*-Chloroperoxy benzoic acid (mCPBA), or Hydrogen peroxide (H₂O₂). to obtain the corresponding Unsaturated Precursor compound;
   (l) process **D6a** exemplified by the scheme, 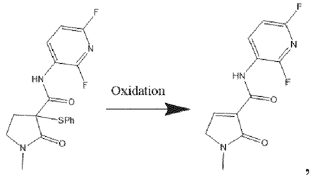 comprising, oxidation of the compound of the following formula, 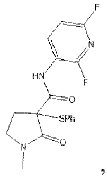 using oxidant exemplified by *meta*-Chloroperoxy benzoic acid (mCPBA), or Hydrogen peroxide (H₂O₂) to obtain the corresponding Unsaturated Precursor compound;
   (m) process **D6b** exemplified by the following scheme, 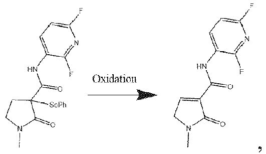 comprising, Oxidation of the compound of the following formula, 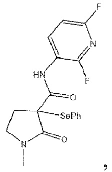 using oxidant exemplified by *meta*-Chloroperoxy benzoic acid (mCPBA), or Hydrogen peroxide (H₂O₂) to obtain the corresponding Unsaturated Precursor compound;
   (n) process **D7a** exemplified by the scheme, 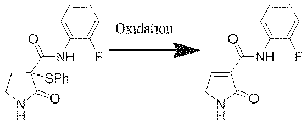 comprising, Oxidation of the compound of the following formula, 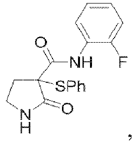 using oxidant exemplified by *meta*-Chloroperoxy benzoic acid (mCPBA), or Hydrogen peroxide (H₂O₂) to obtain the corresponding Unsaturated Precursor compound;
   (o) process **D7b** exemplified by the following scheme, 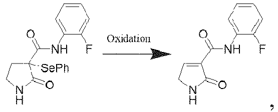 comprising, oxidation of the compound of the following formula, 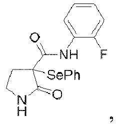 using oxidant exemplified by *meta*-Chloroperoxy benzoic acid (mCPBA), or Hydrogen peroxide (H₂O₂) to obtain the corresponding Unsaturated Precursor compound;
   (p) process **D8a** exemplified by the following scheme, 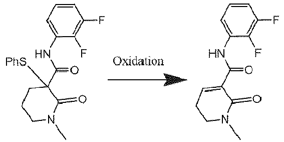 comprising, Oxidation of the compound of the following formula, 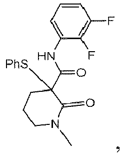 using oxidant exemplified by *meta*-Chloroperoxy benzoic acid (mCPBA), or Hydrogen peroxide (H₂O₂) to obtain the corresponding Unsaturated Precursor compound;
   (q) process **D8b** exemplified by the following scheme, 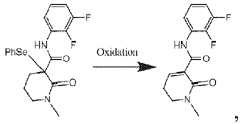 comprising, Oxidation of the compound of the following formula, 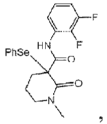 using Oxidant exemplified by *meta*-Chloroperoxy benzoic acid (mCPBA), or Hydrogen peroxide (H₂O₂) to obtain the corresponding Unsaturated Precursor compound;
   (r) process **D9a** exemplified by the following scheme, 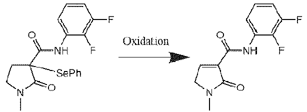 comprising, Oxidation of the compound of the following formula, 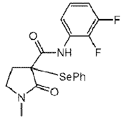 using Oxidant exemplified by *meta*-Chloroperoxy benzoic acid (mCPBA), or Hydrogen peroxide (H₂O₂) to obtain the corresponding Unsaturated Precursor compound and
   (s) process **D9b** exemplified by the scheme, 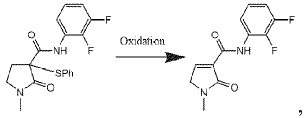 comprising, oxidation of the compound of the following formula, 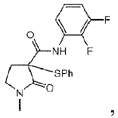 using Oxidant exemplified by *meta*-Chloroperoxy benzoic acid (mCPBA), or Hydrogen peroxide (H₂O₂) to obtain the corresponding Unsaturated Precursor compound.
18. A compound as defined, depicted or described in compound E1 or a compound **E1** of Formula 4 and e.g. salts etc. thereof, 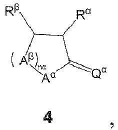 for use as an intermediate in the method of preparation of a corresponding Saturated Target of formula 1, 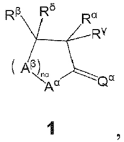 wherein, **R^{α}, R^{β}, A^{α}, A^{β}, nα** and **Q^{α}** have the same meanings as defined in compound **A2,** and R^{δ} and R^{γ} have the same meanings as defined in process **B.**
19. The process as defined, depicted or described in any one of process F1a; F1b; F1c; F2a; F2b; F3; and F4 or selected from group of
   (a) process **F1a,** for preparing a compound of formula **3** from a compound of formula **4,** 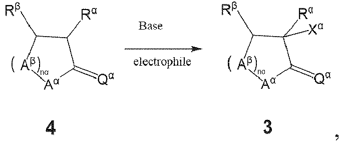
      comprising addition **of a X^{α}** "leaving" group by utilizing Base and Electrophile,
      wherein each of **X^{α}, R^{α}, R^{β}, A^{α}, A^{β}, nα,** and **Q^{α},** have the same meanings as in compound **C1a;**
   (b) process **F1b,** for preparing a compound of formula **3** from a compound of formula **4,** 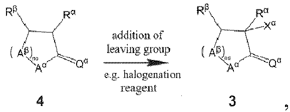 comprising addition **of a X^{α}** leaving group by utilizing a leaving group source such as halogenation reagent, wherein each of **X^{α}, R^{α}, R^{β}, A^{α}, A^{β}, nα,** and **Q^{α},** have the same meanings as in compound **C1a;**
   (c ) process **F1c** for preparing a compound of formula **3** from a compound of formula **4** 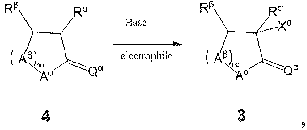 comprising addition of a **X^{α}** group, by utilizing Base and Electrophile, wherein each of **X^{α}, R^{α}, R^{β}, A^{α}, A^{β}, nα,** and **Q^{α},** have the same meanings as in compound **C1b;**
   (d) process **F2a** of process **F1a** comprising, dissolving a compound of formula 4 in suitable solvent with Base such as Sodium Hydride at a specified temperature, typically by cooling, agitating (e.g. stirring etc.), for a time exemplified by about 20mins, adding Electrophile exemplified by any of diphenyl disulfide; phenyl hypochlorothioite; phenyl hypobromoselenoite, dissolved in suitable solvent at substantially the same temperature, agitating the reaction mass at substantially the same temperature for some time. The reaction mass can be quenched e.g. into NH₄Cl solution and extracted with suitable solvent typically two or more times, the combined organic layer can be washed and dried over Na₂SO₄ and concentrated to obtain the corresponding compound of formula 3;
   (e) process **F2b** of process **F1b** comprising, dissolving a compound of formula 4 in suitable solvent together with a leaving group source exemplified by a halogenation reagent (e.g. Bromine source), at a specific temperature, optionally adding a Base and/or catalyst, (catalytic component comprising one or more of, catalyst; pre-catalyst; co-catalyst). Agitating the reaction mass (e.g. stirring etc.), at substantially the same temperature for some time exemplified by about 2 hours, quenching the reaction mass and extracting with a suitable solvent. Preferably the mass is washed again and/or the combined organic layers are washed, optionally dried e.g. over Na₂SO₄ and concentrated to obtain the corresponding compound of formula 3;
   (f) process **F3** of any one of processes **F2a** or **F2b,** further comprising a step of purifying the compound of formula 3 obtained from the process and
   (g) process **F4** of any one of processes **F1-F3** employing any of diphenyl disulfide, and phenyl hypochlorothioite. as Electrophile.
20. A compound as defined, depicted or described in any one of compounds G1; G2; DD1; DD2; DD3; DD4; DD5; DD6; DD7; DD8; DD9; DD10; and DD11 or selected from group of
   (a) storage Marker **G1,** having the following structure, and e.g. salts thereof, 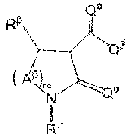 wherein,
      **R^{β}, Q^{α}, Q^{β}** and **R^{π},** have the same meanings as defined in compound **A2;**
   (b) storage Marker compound **G2** of formula **D,** obtainable by exposure of Tetflupyrolimet to sunlight 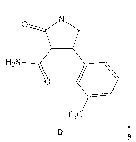
   (c ) storage Marker compound **DD1**of the following formula, 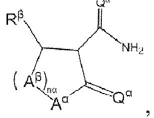 wherein,
      **R^{β}, Q^{α}, A^{β}, A^{α}** and nα, have the same meanings as defined in compound **A2;**
   (d) storage Marker compound **DD2**of the following formula, 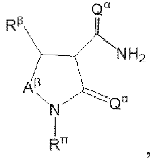 wherein,
      **R^{β}, Q^{α}, A^{β}** and **R^{π},** have the same meanings as defined in compound **A2;**
   (e) storage Marker compound **DD3** of the following formula, 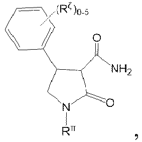 wherein,
      **R^{π}** and **R^{ζ},** have the same meanings as defined in compound **A2;**
   (f) storage Marker compound **DD4** of the following formula, 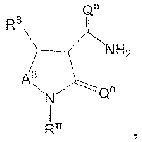 wherein,
      **R^{β}, Q^{α}, A^{β}** and **R^{π},** have the same meanings as defined in compound **A2;**
   (g) storage Marker compound **DD5** of the following formula, 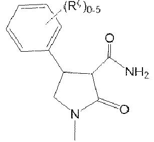 wherein, each **R^{ζ},** has the same meanings as defined in compound **A2;**
   (h) storage Marker compound **DD6** of the following formula, 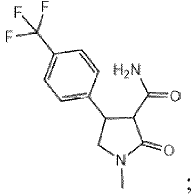
   (i) storage Marker compound **DD7** of the following formula, 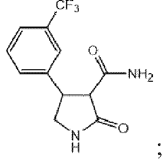
   (j) storage Marker compound **DD8** of the following formula, 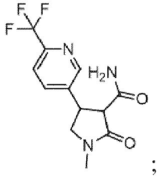
   (k) storage Marker compound **DD9** of the following formula, 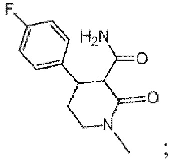
   (l) storage Marker compound **DD10** of the following formula, 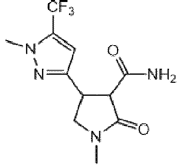 and
   (m) storage Marker compound **DD11** of the following formula, 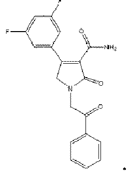
21. A Storage Marker compound as defined, depicted or described in any one of compounds G1; G2; DD1; DD2; DD3; DD4; DD5; DD6; DD7; DD8; DD9; DD10; and DD11 or selected from group of
   (a) storage Marker G1, having the following structure, and e.g. salts thereof, 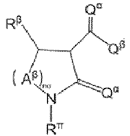 wherein,
      **R^{β}, Q^{α}, Q^{β}** and **R^{π},** have the same meanings as defined in compound **A2;**
   (b) storage Marker compound **G2** of formula **D**, obtainable by exposure of Tetflupyrolimet to sunlight 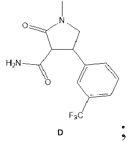
   (c ) storage Marker compound DD1of the following formula, 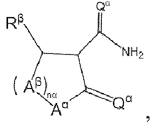 wherein,
      **R^{β}, Q^{α}, A^{β}, A^{α}** and nα, have the same meanings as defined in compound **A2;**
   (d) storage Marker compound DD2of the following formula, 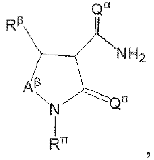 wherein,
      **R^{β}, Q^{α}, A^{β}** and **R^{π},** have the same meanings as defined in compound **A2;**
   (e) storage Marker compound **DD3** of the following formula, 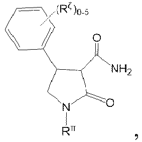 wherein,
      **R^{π}** and **R^{ζ},** have the same meanings as defined in compound A2;
   (f ) storage Marker compound **DD4** of the following formula, 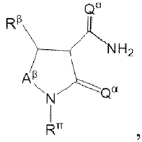 wherein,
      **R^{β}, Q^{α}, A^{β}** and **R^{π},** have the same meanings as defined in compound **A2;**
   (g) storage Marker compound **DD5** of the following formula, 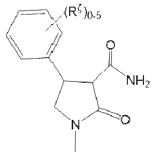 wherein, each **R^{ζ},** has the same meanings as defined in compound **A2;**
   (h) storage Marker compound **DD6** of the following formula, 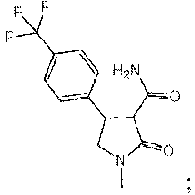
   (i) storage Marker compound **DD7** of the following formula, 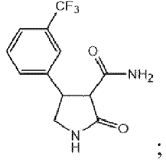
   (j) storage Marker compound **DD8of** the following formula, 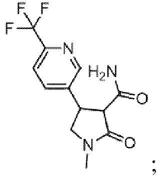
   (k) storage Marker compound **DD9of** the following formula, 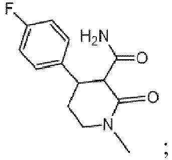
   (l) storage Marker compound **DD10** of the following formula, 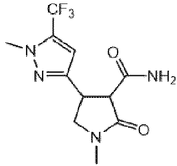 and
   (m) storage Marker compound **DD11** of the following formula, 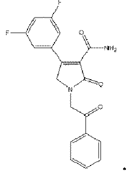
22. A Storage Marker compound as defined, depicted or described as compound G2.
23. A process as defined, depicted or described in any one of processes H1; H2; H2a; H3; H4; H5; and H6 or selected from group of
   (a) process **H1** for preparing a Storage Marker compound from a corresponding Saturated Target compound, in the following general scheme, wherein the Saturated Target is exposed to sunlight for sufficient time for detectable quantities of the corresponding Storage Marker to be formed when measured by an analytical method exemplified by HPLC and particularly by HPLC-MS. 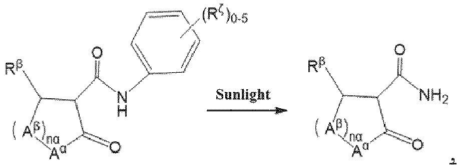 wherein,
      **R^{β}, A^{β}, A^{α}, R^{ζ}** and **nα,** have the same meanings as defined in compound **A2;**
   (b) process **H2** for preparing a Storage Marker compound from a corresponding Saturated Target compound, in the following general scheme, 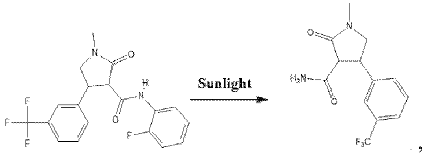 comprising exposing a composition, such as a solution, of the Saturated Target to sunlight for sufficient time for detectable quantities of the corresponding Storage Marker to be formed in the composition, and particularly, quantities above the limit of quantitation, using analytical techniques exemplified by HPLC-MS;
   (c ) process **H2a** for preparing the corresponding Storage Marker compound from a Saturated Target compound which is, (3S,4S)-2'-fluoro-1-methyl-2-oxo-4-[3-(trifluoromethyl)phenyl]pyrrolidine-3-carboxanilide, in the following scheme, 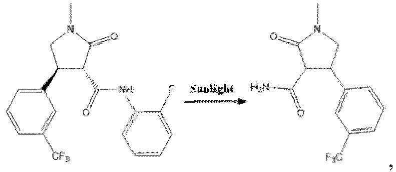 comprising exposing a composition, such as a solution, of the (3S,4S)-2'-fluoro-1-methyl-2-oxo-4-[3-(trifluoromethyl)phenyl]pyrrolidine-3-carboxanilide to sunlight for sufficient time for detectable quantities of the corresponding Storage Marker to be formed in the composition, and particularly, quantities above the limit of quantitation, using analytical techniques exemplified by HPLC-MS;
   (d) process **H3** of either one of processes H2, or **H2a,** wherein the composition comprises a solution, especially an aqueous solution, that is maintained at a predefined level of acidity/basicity, preferably, of pH, by a method exemplified by inclusion of a suitable buffer in the preferably, aqueous, solution;
   (e ) process **H4** of process **H3,** wherein the solution of the Saturated Target is maintained at a level of Acidity/Basicity exemplified by an aqueous solution of Saturated Target having a pH selected from about 4±1; 7±1; and 10±1, by inclusion of effective concentrations of suitable buffers in the preferably aqueous solution;
   (f) process **H5** of any one of processes **H2; H2a; H3;** and **H4,** wherein the rate at which the corresponding Storage Marker is formed from a Saturated Target compound by exposure to sunlight, is further mediated by the selection of the acidity/basicity of the composition of comprising the Saturated Target and
   (g) process **H6** of any one of processes **H3, H4** and **H5,** wherein the composition is an aqueous solution, and wherein the rate at which the corresponding Storage Marker is formed from the Saturated Target by exposure to sunlight, is mediated by the selection of the pH of the aqueous solution of the Saturated Target.
24. A process as defined, depicted or described in any one processes H7; H8; H9; H10; H11; and H12 or selected from group of
   (a) process **H7** for minimizing the rate of conversion of 2'-fluoro-1-methyl-2-oxo-4-[3-(trifluoromethyl)phenyl]pyrrolidine-3-carboxanilide in a composition thereof, to the corresponding Storage Marker, comprising minimizing and/or avoiding the exposure to sunlight of said composition and/or mitigating the converting effect of said exposure to sunlight;
   (b) process **H8** for minimizing the rate of conversion of Tetflupyrolimet in a composition thereof to the corresponding Storage Marker, comprising minimizing the time and extent to which said composition is exposed to sunlight and/or mitigating the converting effect of said exposure to sunlight;
   (c ) process **H9** for minimizing the rate of conversion of Tetflupyrolimet in a composition thereof, to the corresponding Storage Marker, , comprising including in said composition, at least one of, ingredient; excipient; adjuvant; carrier; or any additive that can mitigate the converting effect of said exposure to sunlight, or reduce the rate at which said conversion would occur on exposure to sunlight absent said ingredient, excipient, adjuvant, carrier or additive;
   (d) process **H10** for minimizing the rate of conversion of Tetflupyrolimet in a product comprising a Tetflupyrolimet composition, including solutions thereof, to the corresponding Storage Marker, during manufacturing, packaging, labeling, transportation, and storage of said product, comprising mitigating or overcoming the effects of exposure to sunlight of said product, by including said composition within light-resistant, preferably substantially opaque, packaging and/or containers (e.g. a container-closure system), that protects the contents from the converting effects of sunlight by virtue of the specific properties of the material of which it is composed, including any coating applied to it, including clear, colorless, or translucent containers made light-resistant by means of a light-resistant or opaque covering or by use of secondary packaging, or within amber colored containers, wherein light-resistance and opacity relates to the wavelengths of light mediating conversion of Tetflupyrolimet to the corresponding Storage Marker;
   (e ) process **H11** and/or composition of any one of processes **H1** to **H10** wherein the Saturated Target compound, preferably, Tetflupyrolimet, is in a liquid, solution, emulsion or suspension composition and
   (f) process **H12** for minimizing the rate at which an aqueous solution of Tetflupyrolimet is converted to the corresponding Storage Marker compound when said solution is exposed to sunlight, comprising maintaining the pH of said aqueous solution to a range of between about 6 to about 8 and preferably about 7.
25. A process as defined, depicted or described in any one of processes H13; H14; H15; and H16 or selected from group of
   (a) process **H13** to determine the time period that a stored composition comprising a Saturated Target compound, especially, a Tetflupyrolimet composition, was exposed to sunlight while in storage, said process comprising measuring and quantifying the relative content of the corresponding Storage Marker compound as compared to the content of the Saturated Target in the composition, and evaluating the period of exposure to sunlight by reference to a standard calibrating rate of conversion in one or more reference compositions comprising the Saturated Target compound;
   (b) process **H14** of process **H13** wherein said measurement and quantifying of said relative content is by a method exemplified by calculating the ratio of the respective areas under the resolved characterizing peaks of each compound in a test sample by appropriate HPLC analysis;
   (c) process **H15** of any one of processes **H13** and **H14,** wherein said standard calibrating rate is determined for a reference composition with the same acidity/basicity as the stored test composition and
   (d) process **H16** of any one of processes **H13**, **H14** and **H15**, wherein said standard calibrating rate is determined for a reference composition packaged in the same container system as the stored test composition.
26. A stored packaged composition as defined, depicted or described in any one of compositions 11; 12; 13; 14; 15 and 113 or selected from group of
   (a) stored packaged composition **I1** comprising a combination of detectable quantities of a Storage Marker compound and a corresponding Saturated Target compound from which it was converted, by a process exemplified by the process of process **H2;**
   (b) stored packaged composition **I2** of composition **I1** comprising detectable, preferably measurable, quantities of a Storage Marker compound of the following formula, 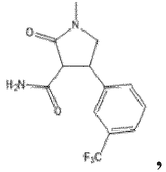 including any isomer or enantiomer thereof, in combination with a Saturated Target compound of the following formula, 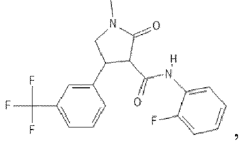 including any isomer or enantiomer thereof, especially exemplified by
      (3S,4S)-2'-fluoro-1-methyl-2-oxo-4-[3-(trifluoromethyl)phenyl]pyrrolidine-3-carboxanilide, of the following formula, 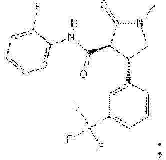
   (c ) stored packaged composition **I3** of any one of compositions **I1** and **I2,** wherein the composition comprises a solution, especially an aqueous solution, of the Saturated Target that is maintained at a predefined level of acidity/basicity, preferably pH, by a method exemplified by inclusion of a suitable buffer system in the preferably aqueous, solution;
   (d) stored packaged composition **I4** of composition **I3,** wherein the solution of the Saturated Target is maintained at a level of Acidity/Basicity exemplified by an aqueous solution of the Saturated Target having a pH selected from about 4±1; about 7±1; and about 10±1 maintained, by inclusion of suitable buffers in the preferably aqueous solution at concentrations effective to maintain said level of Acidity/Basicity and
   (d) stored packaged composition **I5** of any one of compositions **I1 - I4**, wherein the rate at which the Storage Marker compound, especially the compound of the following formula, 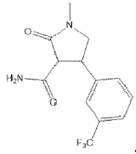 was formed from the corresponding Saturated Target, especially from the compound of the following formula, 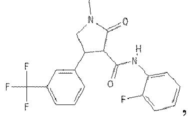 by exposure to sunlight on storage, was further mediated by the selection of the acidity/basicity of the composition of said Saturated Target over the storage period.
   (e) stored packaged composition **I13** comprising a Saturated Target compound, comprising less than a measurable, preferably less than a detectable, amount of, the corresponding Storage Marker compound after storage for a period greater than 2 months in a sunlit environment.
27. A packaged aqueous-based composition of Tetflupyrolimet as defined and described in composition 16 or
   a packaged aqueous-based composition **I6** of Tetflupyrolimet characterized in that the rate at which the Tetflupyrolimet is converted to a corresponding Storage Marker of the following formula, 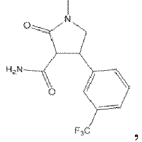 including any isomer, enantiomer or mixture thereof, when the composition is exposed to sunlight during storage is minimized, wherein said composition is adapted to maintain pH during storage, at a range of between about 6 to about 8 and preferably about 7, by inclusion of an ingredient, excipient or other component, exemplified by a buffer or buffering system in the composition.
28. An agricultural product as defined, depicted or described in any one of product 17 or 18 or selected from group of
   (a) agricultural product **I7** comprising a composition of an 2'-fluoro-1-methyl-2-oxo-4-[3-(trifluoromethyl)phenyl]pyrrolidine-3-carboxanilide especially, Tetflupyrolimet, characterized in that the rate at which conversion to the corresponding Storage Marker of the following formula, 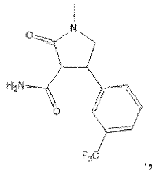 including any isomer, enantiomer or mixture thereof, occurs during storage, is minimized, wherein said product is adapted to limit, reduce or minimize the time and extent or to substantially avoid exposure to sunlight of said composition within said product during storage, wherein said minimization is as compared to an agricultural Tetflupyrolimet product without said adaptation and
   (b) agricultural product **I8** comprising a Tetflupyrolimet composition protected to a detectable degree against sunlight-mediated conversion to the corresponding Storage Marker compound of the following formula, 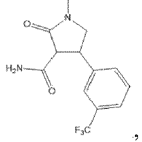 during manufacturing, packaging, labeling, transportation, or storage, of said composition, characterized in that the product comprises said composition contained within and/or shielded by substantially light-resistant, preferably, opaque packaging and/or a container-closure system that protects the contents from the effects of sunlight by virtue of the specific properties of the material of which it is composed, including any coating applied to it, including clear and colorless or translucent container made light-resistant by means of an opaque covering or by use of secondary packaging, or within amber colored containers.
29. A composition as defined, depicted or described in any one of products 17 or 18 or selected from group of
   (a) agricultural product **I7** comprising a composition of an 2'-fluoro-1-methyl-2-oxo-4-[3-(trifluoromethyl)phenyl]pyrrolidine-3-carboxanilide especially, Tetflupyrolimet, characterized in that the rate at which conversion to the corresponding Storage Marker of the following formula, 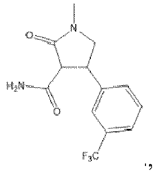 including any isomer, enantiomer or mixture thereof, occurs during storage, is minimized, wherein said product is adapted to limit, reduce or minimize the time and extent or to substantially avoid exposure to sunlight of said composition within said product during storage, wherein said minimization is as compared to an agricultural Tetflupyrolimet product without said adaptation and
   (b) agricultural product **I8,** comprising a Tetflupyrolimet composition protected to a detectable degree against sunlight-mediated conversion to the corresponding Storage Marker compound of the following formula, 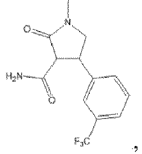 during manufacturing, packaging, labeling, transportation, or storage, of said composition, characterized in that the product comprises said composition contained within and/or shielded by substantially light-resistant, preferably, opaque packaging and/or a container-closure system that protects the contents from the effects of sunlight by virtue of the specific properties of the material of which it is composed, including any coating applied to it, including clear and colorless or translucent container made light-resistant by means of an opaque covering or by use of secondary packaging, or within amber colored containers.
30. A Tetflupyrolimet composition as defined, depicted or described in any one of compositions 19; 110; and 112 or selected from group of
   (a) Tetflupyrolimet composition **I9** characterized by a minimized rate or extent of sunlight-mediated conversion of Tetflupyrolimet to the corresponding Storage Marker of the following formula, 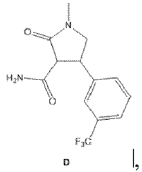 comprising at least one of, ingredient, excipient, adjuvant, carrier or additive that can mitigate the converting effect of exposure to sunlight or reduce the rate at which said conversion would occur on exposure to sunlight absent said ingredient, excipient, adjuvant, carrier or additive;
   (b) Tetflupyrolimet composition **I10** stabilized to at least a measurable degree against sunlight-mediated conversion to the corresponding Storage Marker, during manufacturing, packaging, labeling, transportation, and storage, characterized in that said composition comprises at least one of, ingredient, excipient, adjuvant, carrier or additive that can mitigate the converting effect of exposure to sunlight and/or reduce the rate at which said conversion occurs absent said ingredient, excipient, adjuvant, carrier or additive and
   (c ) Tetflupyrolimet composition **I12** stabilized to at least a measurable degree against conversion to the corresponding Storage Marker or displaying a measurably reduced rate of conversion to the corresponding Storage Marker during manufacturing, packaging, labeling, transportation, and storage, as compared to the standard calibrating rate of conversion in one or more reference compositions comprising the Tetflupyrolimet, characterized in that said composition comprises at least one component which is any, some or all, of,

   i) a buffer which maintains the pH of the composition to 7± 1;
   ii) a UV absorber;
   iii) an ingredient etc. that exhibits the absorption spectra substantially similar to that of the Tetflupyrolimet exemplified by a suitable dye, colorant or pigment;
   iv) an ingredient etc. capable of reducing the amount of sunlight impinging on the material;
   v) an opacifier;
   vi) a reflecting pigment such as titanium dioxide;
   vii) a light protecting coating over a least a portion of said Tetflupyrolimet in said composition;
   viii) a complex of Tetflupyrolimet with a complexing agent exemplified by an inclusion complex with e.g. a cyclodextrin;
      wherein said reference compositions comprise Tetflupyrolimet absent said component.
31. A product as defined, depicted or described in any one of products 111; and 115 or selected from group of
   (a) product **I11** comprising a Tetflupyrolimet composition shielded against sunlight-mediated conversion to the corresponding Storage Marker during any of, manufacturing; packaging; labeling; transportation; or storage; of said composition, characterized in that the product comprises said Tetflupyrolimet composition contained within substantially light-resistant, preferably, opaque packaging substantially as detailed in process **H10.** and
   (b) product and /or composition **I15** of any one of compositions **I1** to **I12** wherein the Saturated Target preferably, Tetflupyrolimet is in a liquid, solution, emulsion, or suspension composition.
32. A buffered Tetflupyrolimet composition as defined, depicted or described in composition 114 or
   a buffered Tetflupyrolimet composition comprising less than a measurable, preferably less than a detectable, amount of, the corresponding Storage Marker compound after storage for
   a period equal to, or greater than 2 months in a sunlit environment.
33. A crystalline polymorph Form I of N-(2-fluorophenyl)-1-methyl-2-oxo-4-[3-(trifluoromethyl)phenyl]-3-pyrrolidinecarboxamide or Tetflupyrolimet, as defined in any one of crystalline polymorph J1 or J2 or selected from group of
   (a) crystalline polymorph **J1, Form I** of *N*-(2-fluorophenyl)-1-methyl-2-oxo-4-[3-(trifluoromethyl)phenyl]-3-pyrrolidinecarboxamide which exhibits at least one of the following properties:
      an infrared spectrum substantially as shown in **Figure 9****.**
      an X-ray powder diffraction pattern substantially as shown in **Figure 5****.**
      an X-ray powder diffraction pattern having a differentiating peak expressed in 2θ (± 0.20) at 9.6,
      an X-ray powder diffraction pattern having at least 4 of the characteristic peaks expressed in 2θ (± 0.20) at 9.6, 15.0, 17.3, 17.9, 21.6 and 24.6 and
   (b) crystalline polymorph **J2, Form I** of Tetflupyrolimet which exhibits at least one of the following properties:
      an infrared spectrum substantially as shown in **Figure 9****.**
      an X-ray powder diffraction pattern substantially as shown in **Figure 5****.**
      an X-ray powder diffraction pattern having a differentiating peak expressed in 2θ (± 0.20) at 9.6,
      an X-ray powder diffraction pattern having at least 4 of the characteristic peaks expressed in 2θ (± 0.20) at 9.6, 15.0, 17.3, 17.9, 21.6 and 24.6.
34. A composition as defined in any one of compositions 13; J4 and J5 or selected from group of
   (a) composition **J3** comprising the crystalline polymorph **Form I** Tetflupyrolimet as defined in any one of crystalline polymorph **J1** or **J2;**
   (b) pesticidal, preferably, herbicidal composition **J4** comprising the crystalline polymorph **Form I** of any one of crystalline polymorph **J1** and **J2;** and an herbicidally acceptable diluent or carrier and
   (c ) pesticidal, preferably, herbicidal composition **J5** prepared from a crystalline polymorph **Form I** of any one of crystalline polymorph **J1** and **J2.**
35. A crystalline polymorph Form II of N-(2-fluorophenyl)-1-methyl-2-oxo-4-[3-(trifluoromethyl)phenyl]-3-pyrrolidinecarboxamide or Tetflupyrolimet, as defined in any one of crystalline polymorphsK1 and K2 or selected from group of
   (a) crystalline polymorph **K1, Form II** of *N*-(2-fluorophenyl)-1-methyl-2-oxo-4-[3-(trifluoromethyl)phenyl]-3-pyrrolidinecarboxamide which exhibits at least one of the following properties:
      An infrared (IR) absorption spectrum substantially as shown in **Figure 10****.**
      An infrared (IR) absorption spectrum having at least one characteristic peak selected from the following values expressed as cm-1 (± 1 cm-1) at 1678, 1546, 1395, 946, 924, 885, 825 and 662.
      An X-ray powder diffraction pattern substantially as shown in **Figure 6****.**
      An X-ray powder diffraction pattern having a differentiating peak expressed in 2θ (± 0.20) at 6.9,
      an X-ray powder diffraction pattern having at least 4 of the characteristic peaks expressed in 2θ (± 0.20) selected from the following values at 6.9, 11.2, 17.7, 23.5 and 26.3 and
   (b) crystalline polymorph **K2,** Form **II** of Tetflupyrolimet which exhibits at least one of the following properties:
      An infrared (IR) absorption spectrum substantially as shown in **Figure 10****.**
      An infrared (IR) absorption spectrum having at least one characteristic peak selected from the following values expressed as cm-1 (± 1 cm-1) at 1678, 1546, 1395, 946, 924, 885, 825 and 662.
      An X-ray powder diffraction pattern substantially as shown in **Figure 6****.**
      An X-ray powder diffraction pattern having a differentiating peak expressed in 2θ (± 0.20) at 6.9,
      an X-ray powder diffraction pattern having at least 4 of the characteristic peaks expressed in 2θ (± 0.20) selected from the following values at 6.9, 11.2, 17.7, 23.5 and 26.3.
36. A non-crystalline solid form of N-(2-fluorophenyl)-1-methyl-2-oxo-4-[3-(trifluoromethyl)phenyl]-3-pyrrolidinecarboxamide or Tetflupyrolimet prepared by a process exemplified by any one of, precipitation; spray drying; lyophilization; adsorption in or on a solid carrier; solid solution in a polymer, wax, saccharide, or salt and complexation with polyelectrolytes or cyclodextrins.
37. A combination of solid forms of N-(2-fluorophenyl)-1-methyl-2-oxo-4-[3-(trifluoromethyl)phenyl]-3-pyrrolidinecarboxamide or Tetflupyrolimet comprising at least two selected from the group of, crystalline polymorph Form I; crystalline polymorph Form II and crystalline polymorph Form II; of N-(2-fluorophenyl)-1-methyl-2-oxo-4-[3-(trifluoromethyl)phenyl]-3-pyrrolidinecarboxamide or Tetflupyrolimet.
38. A composition as defined in any one of composition K3; K4 and K5 or selected from group of.
   (a) composition **K3** comprising the crystalline polymorph Form **II** according to any one of crystalline polymorph **K1** or **K2.**
   (b) pesticidal, preferably, herbicidal composition **K4** comprising the crystalline polymorph Form **II** according to any one of crystalline polymorph **K1** and **K2;** and a pesticidally, preferably, herbicidally acceptable diluent or carrier and
   (c) pesticidal, preferably, herbicidal composition **K5** prepared from the crystalline polymorph Form **II** according to any one of crystalline polymorph **K1** and **K2.**
39. A method for preparation as defined and or described in any one of methods K6; and K6a or selected from group of
   (a) crystallization method, K6, for preparing the crystalline polymorph **Form I** according any one of crystalline polymorph **J1 and J2** comprising recrystallization from *tert*-butyl methyl ether and
   (b) method, **K6a** , for preparing the crystalline polymorph **Form I** according any one of crystalline polymorph **J1** and **J2** comprising:
      i) dissolving (3R,4S)-1-methyl-2-oxo-4-(3-(trifluoromethyl)phenyl)pyrrolidine-3-carboxylic acid in acetonitrile (200 mL),
      ii) adding 2-fluoroaniline and N-methylimidazole,
      iii) mixing the resulting contents for a period exemplified by 10 min at ambient temperature,
      iv) adding Chloro-N,N,N',N'-tetramethylformamidinium hexafluorophosphate,
      v) stirring the resulting mixture at ambient temperature for a period exemplified by for 10h,
      vi) removing the solvent e.g. by evaporation,
      vii) dissolving the residue in e.g. dichloromethane ,
      viii) washing with e.g. sodium hydrosulfate aqueous solution and brine,
      ix) drying and concentrating under reduced pressure to obtain crude product,
      x) recrystallizing crude product from tert-butyl methyl ether.
40. A method for preparation as defined and or described in any one of methods K7; and K7a or selected from group of
   (a) method of preparing a crystalline polymorph **Form II,** **K7** according to any one of crystalline polymorph **K1** and **K2** comprising: trituration with heptane and
   (b) method of preparing a crystalline polymorph **Form II** **K7a** according to any one of crystalline polymorph **K1** and **K2** comprising,
      i) dissolving N-(2-fluorophenyl)-1-methyl-2-oxo-2,5-dihydro-1H-pyrrole-3-carboxamide in Diethyl ether,
      ii) adding e.g. CuBr.SMe₂,
      iii) adding e.g. 2,2'-bis(diphenylphosphaneyl)-1,1'-binaphthalene,
      iv) stirring the reaction mass for a period exemplified by 20 minutes.
      v) cooling the reaction mass,
      vi) adding e.g. Trimethylsilyl trifluoromethanesulfonate
      vii) stirring for a period exemplified by 20 minutes,
      viii) adding 3-(trifluoromethyl) phenyl) magnesium bromide
      ix) stirring for a period exemplified by 2 hours,
      x) quench the reaction mass into e.g. aq. NH₄Cl solution
      xi) extract with ethyl acetate
      xii) wash combined organic layers with brine solution,
      xiii) dry over Na₂SO₄ and concentrate to obtain crude
      xiv) purifyby e.g. column chromatography using Ethyl acetate /Hexane (20:80) as eluent, and
      xv) triturate the solid obtained with heptane (5 mL) .
41. A procedure as defined, depicted or described in any one of procedures N1; and N2 or selected from group of
   (a) general procedure **N1** for the Preparation of a Storage Marker from a Saturated Target: 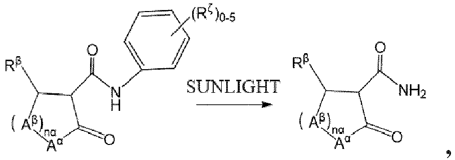 wherein,
      **R^{β}, A^{β}, A^{α}** and **R^{ζ},** have the same meanings as defined in compound **A2** and
   (b) general Procedure **N2** for Preparation of a Storage Marker useful for calibration of pH dependency, based on three aqueous compositions of a Saturated Target each buffered at a different pH.

   Three lots of about 50 mg of Saturated Target are each suspended in about 2ml of a 2% solution of acetonitrile in water, and sonicated for about 20 minutes.
   About 0.1 ml of one of three different buffers is added to each, of pH=4, pH=7 and pH=10 respectively.
   The three buffered solutions are each filtered and introduced to quartz tubes and exposed to natural sunlight for approximately 2 months.
   Analysis by HPLC-MS shows the formation of the corresponding Storage Marker in each solution in pH related concentrations.
42. A compound as defined, depicted or described in any one of compounds P1; P1a; P2; P2a; P3; P3a; Q1; Q2; and Q3 or selected from group of
   (a) a compound **P1** of the following formula, 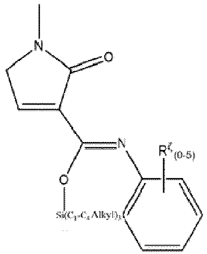 or salts or N-oxides thereof wherein,
      Each **R^{ζ},** has the same meaning as defined in compound **A2;**
   (b) a synthetic intermediate compound **P1a** for the preparation of a Saturated Target of the following formula, 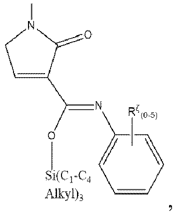 or salts or N-oxides thereof wherein,
      **R^{ζ},** has the same meaning as defined in compound **A2;**
   (c ) a compound **P2** of the following formula, 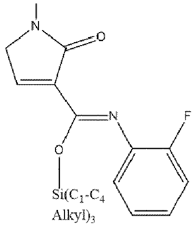 or salts or N-oxides thereof;
   (d) synthetic intermediate compound **P2a** for the preparation of a Saturated Target of the following formula, 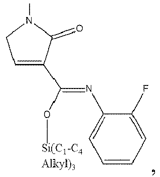 or salts or N-oxides thereof;
   (e ) a compound **P3** of the following formula, 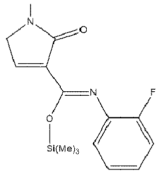 or salts or N-oxides thereof;
   (f) a synthetic intermediate compound **P3a** for the preparation of a Saturated Target of the following formula, 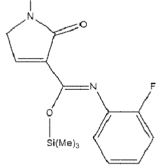 or salts or N-oxides thereof;
   (g) a compound **Q1** of the following formula, 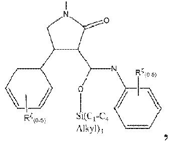 or salts or N-oxides thereof wherein,
      each R^{ζ}, has the same meaning as defined in compound A2;
   (h) a compound **Q2** of the following formula, 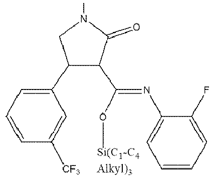 or salts or N-oxides thereof and
   (i) a compound **Q3** of the following formula, 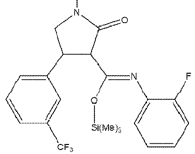 or salts or N-oxides thereof.
43. A process, method, or synthesis defined, depicted, or described in any one of Preparations A; B; C; D1; D2; D3; D4; E; F; G; H; I; J; K; L; M; N; O; P; Q; R; S; T; U; V; W; X; Y; Z; AA; AB; AC; AD; AE; AF; AG; AH; AI; AJ; AK; AL; and AM .
44. A process **B15a** wherein the Unsaturated Precursor of any one of the compounds **A1 - A35,** is used as an intermediate in the method of preparation of a Saturated Target as defined in any one of processes **B1** to **B14.**
45. A process **B15c** comprising the use of an Unsaturated Precursor of any one of the processes **B1,** as an intermediate in the method of preparation of a Saturated Target in any one of processes **B1** to **B14.**

## Claims

1. A solid form of N-(2-fluorophenyl)-1-methyl-2-oxo-4-[3-(trifluoromethyl)phenyl]-3-pyrrolidinecarboxamide selected from the group of:
a. crystalline polymorph **J1, Form I** of N-(2-fluorophenyl)-1-methyl-2-oxo-4-[3-(trifluoromethyl)phenyl]-3-pyrrolidinecarboxamide (Embodiment **J1**);
b. crystalline polymorph **J2, Form I** of (3S,4S)-N-(2-fluorophenyl)-1-methyl-2-oxo-4-[3-(trifluoromethyl)phenyl]-3-pyrrolidinecarboxamide (Tetflupyrolimet) (Embodiment **J2**);
c. crystalline polymorph **K1, Form II** of N-(2-fluorophenyl)-1-methyl-2-oxo-4-[3-(trifluoromethyl)phenyl] -3 -pyrrolidinecarboxamide (Embodiment **K1**);
d. crystalline polymorph **K2, Form II** of Tetflupyrolimet (Embodiment **K2**); and
e. non-crystalline amorphous solid form of N-(2-fluorophenyl)-1-methyl-2-oxo-4-[3-(trifluoromethyl)phenyl]-3-pyrrolidinecarboxamide or Tetflupyrolimet.

2. The crystalline polymorph **J1, Form I** of claim 1a., which exhibits at least one of the following properties:
a. an infrared spectrum substantially as shown in **Figure 9,**
b. an X-ray powder diffraction pattern substantially as shown in **Figure 5,**
c. an X-ray powder diffraction pattern having a differentiating peak expressed in 2θ (± 0.20) at 9.6,
d. an X-ray powder diffraction pattern having at least 4 of the characteristic peaks expressed in 2θ (± 0.20) at 9.6, 15.0, 17.3, 17.9, 21.6 and 24.6.

3. The crystalline polymorph **J2, Form I** of claim 1b. which exhibits at least one of the following properties:
a) an infrared spectrum substantially as shown in **Figure 9.**
b) an X-ray powder diffraction pattern substantially as shown in **Figure 5.**
c) an X-ray powder diffraction pattern having a differentiating peak expressed in 2θ (± 0.20) at 9.6,
d) an X-ray powder diffraction pattern having at least 4 of the characteristic peaks expressed in 2θ (± 0.20) at 9.6, 15.0, 17.3, 17.9, 21.6 and 24.6.

4. The crystalline polymorph **K1, Form II** of claim 1c., which exhibits at least one of the following properties:
a. an infrared (IR) absorption spectrum substantially as shown in **Figure 10,**
b. an infrared (IR) absorption spectrum having at least one characteristic peak selected from the following values expressed as cm⁻¹ (± 1 cm⁻¹) at 1678, 1546, 1395, 946, 924, 885, 825 and 662,
c. an X-ray powder diffraction pattern substantially as shown in **Figure 6,**
d. an X-ray powder diffraction pattern having a differentiating peak expressed in 2θ (± 0.20) at 6.9,
e. an X-ray powder diffraction pattern having at least 4 of the characteristic peaks expressed in 2θ (± 0.20) selected from the following values at 6.9, 11.2, 17.7, 23.5 and 26.3.

5. The crystalline polymorph **K2, Form II,** of claim 1d. which exhibits at least one of the following properties:
a. an infrared (IR) absorption spectrum substantially as shown in **Figure 10,**
b. an infrared (IR) absorption spectrum having at least one characteristic peak selected from the following values expressed as cm⁻¹ (± 1 cm⁻¹) at 1678, 1546, 1395, 946, 924, 885, 825 and 662,
c. an X-ray powder diffraction pattern substantially as shown in **Figure 6,**
d. an X-ray powder diffraction pattern having a differentiating peak expressed in 2θ (± 0.20) at 6.9,
e. an X-ray powder diffraction pattern having at least 4 of the characteristic peaks expressed in 2θ (± 0.20) selected from the following values at 6.9, 11.2, 17.7, 23.5 and 26.3.

6. A composition of solid forms of N-(2-fluorophenyl)-1-methyl-2-oxo-4-[3-(trifluoromethyl)phenyl]-3-pyrrolidinecarboxamide of claim 1 comprising a combination of 2 or more of crystalline solid forms, selected from **J1 Form I, J2, Form I, K1 Form II, and K2 Form II.**

7. A composition of solid forms of N-(2-fluorophenyl)-1-methyl-2-oxo-4-[3-(trifluoromethyl)phenyl]-3-pyrrolidinecarboxamide of claim 1 comprising a combination of one or more non-crystalline amorphous solid forms of N-(2-fluorophenyl)-1-methyl-2-oxo-4-[3-(trifluoromethyl)phenyl]-3-pyrrolidinecarboxamide or Tetflupyrolimet with one or more of crystalline solid forms, selected from **J1 Form I; J2; Form I; K1 Form II;** and **K2Form II.**

8. A solid composition comprising, the non-crystalline amorphous solid form of, N-(2-fluorophenyl)-1 -methyl-2-oxo-4- [3 -(trifluoromethyl)phenyl] -3 - pyrrolidinecarboxamide or Tetflupyrolimet of claim 1e., and excipient, adjuvant; solid solvent; carrier; diluent; formulation additive; solid solution solvent, such as polymer, wax, saccharide, or salt; complexation polyelectrolyte; complexation cyclodextrin and any and all combinations thereof, wherein the solid composition is in a form selected from the group of:
a. solid precipitate;
b. spray dried solid;
c. freeze dried solid (lyophilizate);
d. adsorbed in or on a solid carrier;
e. solid solution in a polymer, wax, saccharide, salt; and
f. solid complex with polyelectrolyte or cyclodextrin.

9. A composition selected from the group of:
(a) a composition **J3** comprising, the crystalline polymorph **J1, Form I** of N-(2-fluorophenyl)-1-methyl-2-oxo-4-[3-(trifluoromethyl)phenyl]-3-pyrrolidinecarboxamide, or the crystalline polymorph **J2, Form I** of (3S,4S)-N-(2-fluorophenyl)-1 -methyl-2-oxo-4- [3 -(trifluoromethyl)phenyl] -3 - pyrrolidinecarboxamide (Tetflupyrolimet), and a diluent or carrier;
(b) a pesticidal, preferably, herbicidal composition **J4** comprising the crystalline polymorph **J1, Form I** of N-(2-fluorophenyl)-1-methyl-2-oxo-4-[3-(trifluoromethyl)phenyl]-3-pyrrolidinecarboxamide, or the crystalline polymorph **J2, Form I** of (3S,4S)-N-(2-fluorophenyl)-1-methyl-2-oxo-4-[3-(trifluoromethyl)phenyl]-3-pyrrolidinecarboxamide (Tetflupyrolimet)and an herbicidally acceptable diluent or carrier; and
(c) a pesticidal, preferably, herbicidal composition **J5** prepared from (by using), the crystalline polymorph **J1, Form I** of N-(2-fluorophenyl)-1-methyl-2-oxo-4-[3-(trifluoromethyl)phenyl]-3-pyrrolidinecarboxamide, or the crystalline polymorph **J2, Form I** of (3S,4S)-N-(2-fluorophenyl)-1-methyl-2-oxo-4-[3-(trifluoromethyl)phenyl]-3-pyrrolidinecarboxamide (Tetflupyrolimet) and an herbicidally acceptable diluent or carrier.

10. A composition selected from the group of:
(a) a composition **K3** comprising the crystalline polymorph **K1, Form II** of N-(2-fluorophenyl)-1-methyl-2-oxo-4-[3-(trifluoromethyl)phenyl]-3-pyrrolidinecarboxamide or the crystalline polymorph **K2, Form II** of Tetflupyrolimet, and a diluent or carrier;
(b) a pesticidal, preferably herbicidal, composition **K4** comprising the crystalline polymorph **K1, Form II** of N-(2-fluorophenyl)-1-methyl-2-oxo-4-[3-(trifluoromethyl)phenyl]-3-pyrrolidinecarboxamide or the crystalline polymorph **K2, Form II** of Tetflupyrolimet, and a pesticidally, preferably herbicidally, acceptable diluent or carrier; and
(c) a pesticidal, preferably herbicidal, composition **K5** prepared from (by using), the crystalline polymorph **K1, Form II** of N-(2-fluorophenyl)-1-methyl-2-oxo-4-[3-(trifluoromethyl)phenyl]-3-pyrrolidinecarboxamide or the crystalline polymorph **K2, Form II** of Tetflupyrolimet and a pesticidally, preferably herbicidally acceptable diluent or carrier.

11. A method for preparation selected the from group of:
(a) a crystallization method, **K6,** for preparing the crystalline polymorph **J1, Form I** of N-(2-fluorophenyl)-1-methyl-2-oxo-4-[3-(trifluoromethyl)phenyl]-3-pyrrolidinecarboxamide, or the crystalline polymorph **J2, Form I** of (3S,4S)-N-(2-fluorophenyl)-1-methyl-2-oxo-4-[3-(trifluoromethyl)phenyl]-3-pyrrolidinecarboxamide (Tetflupyrolimet), comprising recrystallization of N-(2-fluorophenyl)-1-methyl-2-oxo-4-[3-(trifluoromethyl)phenyl]-3-pyrrolidinecarboxamide, or (3S,4S)-N-(2-fluorophenyl)-1-methyl-2-oxo-4-[3-(trifluoromethyl)phenyl]-3-pyrrolidinecarboxamide (Tetflupyrolimet), respectively from *tert*-butyl methyl ether; and
(b) method **K6a** for preparing the crystalline polymorph **J1, Form I** of N-(2-fluorophenyl)-1-methyl-2-oxo-4-[3-(trifluoromethyl)phenyl]-3-pyrrolidinecarboxamide, or the crystalline polymorph **J2, Form I** of (3S,4S)-N-(2-fluorophenyl)-1-methyl-2-oxo-4-[3-(trifluoromethyl)phenyl]-3-pyrrolidinecarboxamide (Tetflupyrolimet),comprising:
i) dissolving N-(2-fluorophenyl)-1-methyl-2-oxo-4-[3-(trifluoromethyl)phenyl]-3-pyrrolidinecarboxamide or (3R,4S)-1-methyl-2-oxo-4-(3-(trifluoromethyl)phenyl)pyrrolidine-3-carboxylic acid in acetonitrile (200 mL),
ii) adding 2-fluoroaniline and N-methylimidazole,
iii) mixing the resulting contents for a period exemplified by 10 min at ambient temperature,
iv) adding Chloro-N,N,N',N'-tetramethylformamidinium hexafluorophosphate,
v) stirring the resulting mixture at ambient temperature for a period exemplified by for 10h,
vi) removing the solvent e.g. by evaporation,
vii) dissolving the residue in e.g. dichloromethane,
viii) washing with e.g. sodium hydrosulfate aqueous solution and brine,
ix) drying and concentrating under reduced pressure to obtain crude product,
x) recrystallizing crude product from tert-butyl methyl ether.

12. A method for preparation selected from the group of
(a) a method **K7** of preparing the crystalline polymorph **K1, Form II,** of N-(2-fluorophenyl)-1-methyl-2-oxo-4-[3-(trifluoromethyl)phenyl]-3-pyrrolidinecarboxamide, or the crystalline polymorph **K2, Form II** of Tetflupyrolimet comprising trituration of N-(2-fluorophenyl)-1-methyl-2-oxo-4-[3-(trifluoromethyl)phenyl]-3-pyrrolidinecarboxamide, or Tetflupyrolimet with heptane respectively; and
(b) method **K7a** of preparing the crystalline polymorph **K1, Form II** of N-(2-fluorophenyl)-1-methyl-2-oxo-4-[3-(trifluoromethyl)phenyl]-3-pyrrolidinecarboxamide, or the crystalline polymorph **K2, Form II** of Tetflupyrolimet comprising:
i) dissolving N-(2-fluorophenyl)-1-methyl-2-oxo-2,5-dihydro-1H-pyrrole-3-carboxamide or Tetflupyrolimet in Diethyl ether,
ii) adding e.g. CuBr.SMe₂,
iii) adding e.g. 2,2'-bis(diphenylphosphaneyl)-1,1'-binaphthalene,
iv) stirring the reaction mass for a period exemplified by 20 minutes,
v) cooling the reaction mass,
vi) adding e.g. Trimethylsilyl trifluoromethanesulfonate
vii) stirring for a period exemplified by 20 minutes,
viii) adding 3-(trifluoromethyl) phenyl) magnesium bromide,
ix) stirring for a period exemplified by 2 hours,
x) quenching the reaction mass into e.g. aq. NH₄Cl solution
xi) extracting with ethyl acetate,
xii) washing the combined organic layers with brine solution,
xiii) drying over Na₂SO₄ and concentrate to obtain crude material,
xiv) purifying the crude material such as by column chromatography using Ethyl acetate /Hexane (20:80) as eluent, and
xv) triturating the solid obtained with heptane (5 mL).

13. A method for preparation of a non-crystalline amorphous solid form of, N-(2-fluorophenyl)-1 -methyl-2-oxo-4- [3 -(trifluoromethyl)phenyl] -3 - pyrrolidinecarboxamide or Tetflupyrolimet comprising a process selected from the group of:
a. precipitation optionally in combination with one or more of, excipient, adjuvant; carrier; diluent; formulation additive; solid solution solvent, polymer, wax, saccharide, salt; complexation polyelectrolyte; complexation cyclodextrin and any and all combinations thereof,
b. spray drying, optionally in combination with one or more of, excipient, adjuvant; carrier; diluent; formulation additive; solid solution solvent, such as polymer, wax, saccharide, salt, polyelectrolyte; cyclodextrin and any and all combinations thereof;
c. freeze drying (lyophilizing) optionally in combination with one or more of, excipient, adjuvant; carrier; diluent; formulation additive; solid solution solvent, such as polymer, wax, saccharide, salt, polyelectrolyte; cyclodextrin and any and all combinations thereof;
d. adsorbing in or on a solid carrier;
e. forming a solid solution in a polymer, wax, saccharide, or salt; and
f. forming a solid complex with polyelectrolyte and/or cyclodextrin.
